(19) Europäisches Patentamt
European Patent Office
Office européen des brevets

(11) EP 1 058 679 B1

(12) EUROPEAN PATENT SPECIFICATION

(45) Date of publication and mention of the grant of the patent:
**19.10.2005 Bulletin 2005/42**

(21) Application number: **99908420.5**

(22) Date of filing: **24.02.1999**

(51) Int Cl.[7]: **C07D 277/30**, C07D 263/32, C07D 493/04, C07D 313/00, C07D 417/06, C07C 59/90, C07C 69/738, C07F 7/18

(86) International application number:
**PCT/US1999/004008**

(87) International publication number:
**WO 1999/043653 (02.09.1999 Gazette 1999/35)**

(54) **SYNTHESIS OF EPOTHILONES, INTERMEDIATES THERETO AND ANALOGUES THEROF**

SYNTHESE VON EPOTHILONEN, IHREN ZWISCHENPRODUKTEN UND ANALOGEN VERBINDUNGEN

SYNTHESE D'EPOTHILONES, DE LEURS INTERMEDIAIRES ET DE LEURS ANALOGUES

(84) Designated Contracting States:
**AT BE CH CY DE DK ES FI FR GB GR IE IT LI LU MC NL PT SE**
Designated Extension States:
**AL LT LV MK RO SI**

(30) Priority: **25.02.1998 US 75947 P**
**09.07.1998 US 92319 P**
**24.08.1998 US 97733 P**

(43) Date of publication of application:
**13.12.2000 Bulletin 2000/50**

(73) Proprietor: **Sloan-Kettering Institute For Cancer Research**
**New York, New York 10021 (US)**

(72) Inventors:
- **DANISHEFSKY, Samuel, J.**
  **Englewood, NJ 07631 (US)**
- **BALOG, Aaron**
  **New York, NY 10028 (US)**
- **BERTINATO, Peter**
  **Old Lyme, Connecticut 06371 (US)**
- **SU, Dai-Shi**
  **Pennsylvania 19025 (US)**
- **CHOU, Ting-Chau Memorial Sloan-Kettering Cancer C.**
  **1275 York Avenue NY 10021 (US)**
- **MENG, DongFang**
  **Westfield New Jersey 07090 (US)**
- **KAMENECKA, Ted**
  **New York, NY 10021 (US)**
- **SORENSEN, Erik, J.**
  **San Diego, CA 92130 (US)**
- **KUDUK, Scott**
  **Harleysville, PA 14398 (US)**
- **HARRIS, Christina**
  **New York, NY 10021 (US)**
- **ZHANG, Xiu-Guo**
  **New York, NY 10021 (US)**
- **BERTINO, Joseph R.**
  **Branford, CT 06405 (US)**

(74) Representative:
**Cornish, Kristina Victoria Joy et al**
**Kilburn & Strode,**
**20 Red Lion Street**
**London WC1R 4PJ (GB)**

(56) References cited:
**WO-A-99/28324**

- **MENG et al., "Total Syntheses of Epothilones A and B", J. AM. CHEM. SOC., June 1997, Vol. 119, No. 42, pages 10073-10092.**
- **BALOG et al., "Stereoselective Syntheses and Evaluation of Compounds in the 8-Desmethylepothilone A Series: Some Surprising Observations Regarding Their Chemical and Biological Properties", TETRAHEDRON LETTERS, May 1997, Vol. 38, No. 26, pages 4529-4532.**
- **NICOLAOU et al., "Synthesis of Epothilones A and B in Solid and Solution Phase", NATURE, 15 May 1997, Vol. 387, pages 268-272.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• MARCH, Advanced Organic Chemistry, 2nd Ed., McGRAW-HILL, (1977), page 940, Section 7-21.

## Description

This application is based on U.S. Provisional Applications Serial Nos. 60/075,947, 60/092,319, and 60/097,733, filed February 25, 1998, July 9, 1998, and August 24, 1998, respectively, and is a continuation-in-part of U.S. Serial No. 08/986,025, filed December 3, 1997, which was based on U.S. Provisional Applications Serial Nos. 60/032,282,60/033,767,60/047,566,60/047,941, and 60/055,533, filed December 3, 1996, January 14, 1997, May 22, 1997, May 29, 1997, and August 13, 1997, respectively. This invention was made with government support under grants CA-28824, CA-39821, CA-GM 72231, GM-18248, CA-62948, and AIO-9355 from the National Institutes of Health, and grant CHE-9504805 from the National Science Foundation.

## Field of the Invention

The present invention is in the field of epothilone macrolides. In particular, the present invention relates to processes for the preparation of desoxyepothilones A and B, and analogues thereof which are useful as highly specific, non-toxic anticancer therapeutics. In addition, the invention provides methods of inhibiting multidrug resistant cells. The present invention also provides novel compositions of matter which serve as intermediates for preparing the epothilones.

## Background of the Invention

Epothilones A and B are highly active anticancer compounds isolated from the Myxobacteria of the genus *Sorangium.* The full structures of these compounds, arising from an x-ray crystallographic analysis were determined by Höfle. G. Höfle *et al., Angew. Chem. Int. Ed. Engl*., **1996**, *35,* 1567. The total synthesis of the epothilones is an important goal for several reasons. Taxol® is already a useful resource in chemotherapy against ovarian and breast cancer and its range of clinical applicability is expanding. G.I. Georg *et al., Taxane Anticancer Agents;* American Cancer Society: San Diego, **1995.** The mechanism of the cytotoxic action of Taxol®, at least at the *in vitro* level, involves stabilization of microtubule assemblies. P.B. Schiff *et al., Nature* (*London*), **1979,** *277,* 665. A series of complementary *in vitro* investigations with the epothilones indicated that they share the mechanistic theme of the taxoids, possibly down to the binding sites to their protein target. D.M. Bollag *et al., Cancer Res*., **1995**, *55,* 2325. Moreover, the epothilones surpass Taxol® in terms of cytotoxicity and far surpass it in terms of *in vitro* efficacy against drug resistant cells. Since multiple drug resistance (MDR) is one of the serious limitations of Taxol® (L.M. Landino and T.L. MacDonald in *The Chemistry and Pharmacology of Taxol® and its Derivatives,* V. Farin, Ed., Elsevier: New York, **1995,** ch. 7, p. 301), any agent which promises relief from this problem merits serious attention. Furthermore, formulating the epothilones for clinical use is more straightforward than Taxol®.
WO 99/28324 discloses the reduction of oxiranyl epothilones to olefinic epothilones using a metallocene.
Accordingly, the present inventors undertook the total synthesis of the epothilones, and as a result, have developed efficient processes for synthesizing epothilones A and B, the corresponding desoxyepothilones, as well as analogues thereof. The present invention also provides novel intermediates useful in the synthesis of epothilones A and B and analogues thereof, compositions derived from such epothilones and analogues, purified compounds of epothilones A and B, and desoxyepothilones A and B, in addition to methods of use of the epothilone analogues in the treatment of cancer. Unexpectedly, certain epothilones have been found to be effective not only in reversing multi-drug resistance in cancer cells, both *in vitro* and *in vivo*, but have been determined to be active as collateral sensitive agents, which are more cytotoxic towards MDR cells than normal cells, and as synergistic agents, which are more active in combination with other cytotoxic agents, such as vinblastin, than the individual drugs would be alone at the same concentrations. Remarkably, the desoxyepothilones of the invention have exceptionally high specificity as tumor cytotoxic agents *in vivo,* more effective and less toxic to normal cells than the principal chemotherapeutics currently in use, including Taxol®, vinblastin, adriamycin and camptothecin.

## Summary of the Invention

One object of the present invention is to provide processes for the preparation of desoxyepothilones A and B, and related compounds useful as anticancer therapeutics.

## Brief Description of the Drawings

**Comparative Figure 1(A)** shows a retrosynthetic analysis for epothilone A and B.

**Comparative Figure 1(B)** provides synthesis of compound **11.** (a) t-$BuMe_2OTf$, 2,6-lutidine, $CH_2Cl_2$, 98%; (b) (1) DDQ, $CH_2Cl_2/H_2O$, 89%; (2) $(COCl)_2$, DMSO, $CH_2Cl_2$, -78°C; then $Et_3N$, -78°C → rt, 90%; (c) $MeOCH_2PPh_3Cl$, t-BuOK, THF, 0°C → rt, 86%; (d) (1) p-TsOH, dioxane/$H_2O$, 50 °C, 99%; (2) $CH_3PPh_3Br$, NaHMDS, $PhCH_3$, 0°C → rt, 76%; (e) $PhI(OCOCF_3)_2$, MeOH/THF, rt, 0.25 h, 92%.

**Comparative Figure 2** provides key intermediates in the preparation of 12, 13-*E*- and -Z-deoxyepothilones.

**Comparative Figure 3(A)** provides syntheses of key iodinated intermediates used to prepare hydroxymethylene- and hydroxypropylene-substituted epothilone derivatives.

**Comparative Figure 3(B)** provides methods of preparing hydroxymethylene- and hydroxypropylene-substituted epothilone derivatives, said methods being useful generally to prepare 12,13-*E* epothilones wherein R is methyl, ethyl, n-propyl, and n-hexyl from the corresponding *E*-vinyl iodides.

**Comparative Figure 3(C)** shows reactions leading to benzoylated hydroxymethyl-substituted desoxyepothilone and hydroxymethylene-substituted epothilone (epoxide).

**Comparative Figure 4(A)** provides synthesis of compound **19.** (a) DHP, PPTS, $CH_2Cl_2$, rt: (b) (1) $Me_3SiCCLi$, $BF_3 \cdot OEt_2$, THF, -78°C; (2) MOMCl, I-$Pr_2NEt$, $Cl(CH_2)_2Cl$, 55°C; (3) PPTS, MeOH, rt; (c) (1)$(COCl)_2$, DMSO, $CH_2Cl_2$, -78°C; then $Et_3N$, -78°C → rt; (2) MeMgBr, $Et_2O$, 0°C → rt, (3) TPAP, NMO, 4Å mol. sieves, $CH_2Cl_2$, 0°C → rt; (d) 16, n-BuLi, THF, -78°C; then 15, THF, -78°C → rt; (e) (1) N-iodosuccinimide, $AgNO_3$, $(CH_3)_2CO$; (2) $Cy_2BH$, $Et_2O$, AcOH; (f) (1) PhSH, $BF_3$-$OEt_2$, $CH_2Cl_2$, rt; (2) $Ac_2O$, pyridine, 4-DMAP, $CH_2Cl_2$, rt.

**Comparative Figure 4(B)** presents synthesis of compound **1**. (a) **11**, 9-BBN, THF, rt; then $PdCl_2(dppf)_2$, $Cs_2CO_3$, $Ph_3As$, $H_2O$, DMF, **19**, rt, 71%; (b) p-TsOH, dioxane/$H_2O$, 50°C; (c) KHMDS, THF, -78°C, 51%; (d) (1) HF-pyridine, pyridine, THF, rt, 97%; (2) t-$BuMe_2$ SiOTf, 2,6-lutidine, $CH_2Cl_2$, -25°C, 93%; (3) Dess-Martin periodinane, $CH_2Cl_2$, 87%; (4) HF·pyridine, THF, rt, 99%; (e) dimethyldioxirane, $CH_2Cl_2$, 0.5 h, -50°C, 45% (≥ 20: 1).

**Comparative Figure 5** shows a scheme of the synthesis of the "left wing" of epothilone A.

**Comparative Figure 6** provides a scheme of an olefin metathesis route to epothilone A and other analogues.

**Comparative Figure 7** illustrates a convergent strategy for a total synthesis of epothilone A (1) and the glycal cyclopropane solvolysis strategy for the introduction of geminal methyl groups.

**Comparative Figure 8** provides an enantioselective synthesis of compound **15B.**

**Comparative Figure 9** shows the construction of epothilone model systems **20B, 21B,** and **22B** by ring-closing olefin metathesis.

**Comparative Figure 10** illustrates a sedimentation test for natural, synthetic and desoxyepothilone A.

**Comparative Figure 11** illustrates a sedimentation test for natural, synthetic and desoxyepothilone A after cold treatment at 4°C.

**Comparative Figure 12** illustrates (A) structures of epothilones A (1) and B (2) and (B) of Taxol® **(1A)**.

**Comparative Figure 13** shows a method of elaborating acyclic stereochemical relationships based on dihydropyrone matrices.

**Comparative Figure 14** shows the preparation of intermediates **4A.**

**Comparative Figure 15** shows an alternative enantioselective synthesis of compound **17A.**

**Comparative Figure 16** provides a synthetic pathway to intermediate **13C. (a) 1**. tributyl allyltin, (*S*)-(-)- BINOL, $Ti(Oi\text{-}Pr)_4$, $CH_2Cl_2$, -20 °C, 60%, >95% e.e.; 2. $Ac_2O$, $Et_3N$, DMAP, $CH_2Cl_2$, 95%; (b) 1. $OsO_4$, NMO, acetone/$H_2O$, 0°C; 2. $NaIO_4$, THF/$H_2O$; (c) 12, THF, - 20 °C, Z isomer only, 25% from **10**; (d) $Pd(dppf)_2$, $Cs_2CO_3$, $Ph_3As$ $H_2O$, DMF, rt. 77%.

**Comparative Figure 17** provides a synthetic pathway to intermediate epothilone **B (2)**. (a) *p*-TsOH, dioxane/$H_2O$, 55 °C, 71%; (b) KHMDS, THF, -78 °C, 67%, α/β: 1.5:1; (c) Dess-Marti n periodinane, $CH_2Cl_2$; (d) $NaBH_4$, MeOH, 67% for two steps; (e) 1. HF-pyridine, pyridine, THF, rt, 93%; 2. TBSOTf, 2,6-lutidine, $CH_2Cl_2$, -30 °C, 89%; 3. Dess-Martin periodinane, $CH_2Cl_2$, 67%; (f) HF-pyridine, THF, rt, 80%; (g) dimethyldioxirane, $CH_2Cl_2$, -50 °C, 70%.

**Comparative Figure 18** provides a synthetic pathway to a protected intermediate for 8-desmethyl deoxyepothilone A.

**Comparative Figure 19** provides a synthetic pathway to 8-desmethyl deoxyepothilone A, and structures of *trans*-8-desmethyl-desoxyepothiolone A and a trans-iodoolefin intermediate thereto.

**Comparative Figure 20** shows (top) structures of epothilones A and B and 8-desmethylepothilone and (bottom) a synthetic pathway to intermediate TBS ester **10** used in the preparation of desmethylepothilone A. (a) (Z)-Crotyl-B[(-)-lpc]$_2$, -78°C, $Et_2O$, then 3N NaOH, 30% $H_2O_2$; (b) TBSOTf, 2,6-lutidine, $CH_2Cl_2$ (74% for two steps, 87% ee); (c) $O_3$, $CH_2Cl_2$/MeOH, -78°C, then DMS, (82%); (d) *t*-butyl isobutyrylacetate, NaH, BuLi, 0°C, then 6 (60%, 10:1); (e) $Me_4NBH(OAc)_3$, -10°C (50%, 10:1 α/β) or $NaBH_4$, MeOH, THF, 0°C, (88%, 1:1 α/β); (f) TBSOTf, 2,6-lutidine, -40°C, (88%); (g) Dess-Martin periodinane, (90%); (h) $Pd(OH)_2$, $H_2$, EtOH (96%); (I) DMSO, oxalyl chloride, $CH_2Cl_2$, -78°C (78%); (j) Methyl triphenylphosphonium bromide, NaHMOS, THF, 0°C (85%); (k) TBSOTf, 2,6-lutidine, $CH_2Cl_2$, rt (87%).

**Comparative Figure 21** shows a synthetic pathway to 8-desmethylepothilone A. (a) $Pd(dppf)_2Cl_2$, $Ph_3As$, $Cs_2CO_3$, $H_2O$, DMF, rt (62%); (b) $K_2CO_3$, MeOH, $H_2O$ (78%); (c) DCC, 4-DMAP, 4-DMAP-HCl, $CHCl_3$ (78%); (d) HF-pyr, THF, rt (82%), (e) 3,3-dimethyl dioxirane, $CH_2Cl_2$, -35°C (72%, 1.5:1).

**Comparative Figure 22** shows a synthetic pathway to prepare epothilone analogue **27D.**

**Comparative Figure 23** shows a synthetic pathway to prepare epothilone analogue **24D.**

**Comparative Figure 24** shows a synthetic pathway to prepare epothilone analogue **19D.**

**Comparative Figure 25** shows a synthetic pathway to prepare epothilone analogue **20D**.

**Comparative Figure 26** shows a synthetic pathway to prepare epothilone analogue **22D.**

**Comparative Figure 27** shows a synthetic pathway to prepare epothilone analogue 12-hydroxy ethyl-epothilone.

**Comparative Figure 28** shows the activity of epothilone analogues in a sedimentation test in comparison with DMSO, epothilone A and/or B. Structures 17-20, 22, and 24-27 are shown in Figures 29- 37, respectively. Compounds were added to tubulin (1mg/ml) to a concentration of 10 μM. The quantity of microtubules formed with epothilone A was defined as 100%.

**Comparative Figure 29** shows a high resolution $^1H$ NMR spectrum of epothilone analogue **#17.**

**Comparative Figure 30** shows a high resolution $^1H$ NMR spectrum of epothilone analogue **#18.**

**Comparative Figure 31** shows a high resolution $^1H$ NMR spectrum of epothilone analogue **#19.**

**Comparative Figure 32** shows a high resolution $^1H$ NMR spectrum of epothilone analogue **#20.**

**Comparative Figure 33** shows a high resolution $^1H$ NMR spectrum of epothilone analogue **#22.**

**Comparative Figure 34** shows a high resolution $^1H$ NMR spectrum of epothilone analogue **#24.**

**Comparative Figure 35** shows a high resolution $^1H$ NMR spectrum of epothilone analogue **#25.**

**Comparative Figure 36** shows a high resolution $^1H$ NMR spectrum of epothilone analogue **#26.**

**Comparative Figure 37** shows a high resolution $^1H$ NMR spectrum of epothilone analogue **#27.**

**Comparative Figure 38** provides a graphical representation of the effect of fractional combinations of cytotoxic agents.

**Comparative Figure 39** shows epothilone A and epothilone analogues #1-7. Potencies against human leukemia CCRF-CEM (sensitive) and CCRF-CEM/VBL MDR (resistant) sublines are shown in round and square brackets, respectively.

**Comparative Figure 40** shows epothilone B and epothilone analogues #8-16. Potencies against human leukemia CCRF-CEM (sensitive) and CCRF-CEM/VBL MDR (resistant) sublines are shown in round and square brackets, respectively.

**Comparative Figure 41** shows epothilone analogues #17-25. Potencies against human leukemia CCRF-CEM (sensitive) and CCRF-CEM/VBL MDR (resistant) sublines are shown in round and square brackets, respectively.

**Comparative Figure 42(A)** shows epothilone analogues #26-34. Potencies against human leukemia CCRF-CEM (sensitive) and CCRF-CEM/VBL MDR (resistant) sublines are shown in round and square brackets, respectively. **(B)** shows epothilone analogues #35-46.Potencies against human leukemia CCRF-CEM (sensitive) and CCRF-CEMNBL MDR (resistant) sublines are shown in round and square brackets, respectively. **(C)** shows epothilone analogues #47-49.

**Comparative Figure 43 (A)** shows antitumor activity of desoxyepothilone B against MDR MCF-7/Adr xenograft in comparison with Taxol®. Control (♦); desoxyepothilone B (■; 35mg/kg); Taxol® (▲; 6mg/kg); adriamycin (×; 1.8mg/kg); i.p. Q2Dx5; start on day 8. **(B)** shows antitumor activity of epothilone B against MDR MCF-7/Adr xenograft in comparison with Taxol®. Control (♦); epothilone B (■; 25mg/kg; non-toxic dose); Taxol® (▲; 6mg/kg; half $LD_{50}$); adriamycin (×;1.8mg/kg); i.p. Q2Dx5; start on day 8.

**Comparative Figure 44(A)** shows toxicity of desoxyepothilone B in B6D2F, mice bearing B16 melanoma. Body weight was determined at 0, 2, 4, 6, 8, 10 and 12 days. Control (▲); desoxyepothilone B (○; 10mg/kg QDx8; 0 of 8 died); desoxyepothilone B (●; 20mg/kg QDx6; 0 of 8 died). Injections were started on day 1. **(B)** shows toxicity of epothilone B in E6D2F, mice bearing B16 melanoma. Body weight was determined at 0, 2, 4, 6, 8, 10 and 12 days. Control (▲); epothilone B (○; 0.4mg/kg QDx6; 1 of 8 died of toxicity); epothilone B (●; 0.8mg/kg QDx5; 5| of 8 died). Injections were started on day 1.

**Comparative Figure 45(A)** shows comparative therapeutic effect of desoxyepothilone B and Taxol® on nude mice bearing MX-1 xenoplant. Tumor, s.c.; drug administered i.p., Q2Dx5, start on day 7. control (♦); Taxol® (□; 5mg/kg, one half of $LD_{50}$); desoxyepothilone B (Δ; 25mg/kg; nontoxic dose). **(B)** shows comparative therapeutic effect of desoxyepothilone B and Taxol® on nude mice bearing MX-1 xenoplant. Tumor, s.c.; drug administered i.p., Q2Dx5, start on day 7. control (♦); Taxol® (□; 5mg/kg, one half of $LD_{50}$, given on days 7, 9, 11, 13, 15; then 6 mg/kg, given on days 17. 19, 23, 24, 25); desoxyepothilone B (n = 3; Δ, x, *; 25mg/kg, nontoxic dose, given to three mice on days 7, 9, 11, 13, 15; then 35 mg/kg, given on days 17. 19, 23, 24, 25).

**Comparative Figure 46** shows the effect of treatment with desoxyepothilone B (35 mg/kg), Taxol® (5 mg/kg) and adriamycin (2 mg/kg) of nude mice bearing human MX-1 xenograft on tumor size between 8 and 18 days after implantation. Desoxyepothilone B (□), Taxol® (Δ), adriamycin (X), control (♦); i.p. treatments were given on day 8, 10, 12, 14 and 16.

**Comparative Figure 47** show the relative toxicity of epothilone B (0; 0.6 mg/kg QDx4; i.p.) and desoxyepothilone **B** (Δ; 25 mg/kg QDx4; i.p.) versus control (♦) in normal nude mice. Body weight of mice was determined daily after injection. For epothilone B, 8 of 8 mice died of toxicity on days 5, 6, 6, 7, 7, 7, 7, and 7; for desoxyepothilone B, all six mice survived.

**Comparative Figure 48** shows a high resolution $^1$H NMR spectrum of epothilone analogue **#43.**

**Comparative Figure 49** shows a high resolution $^1$H NMR spectrum of epothilone analogue **#45.**

**Comparative Figure 50** shows a high resolution $^1$H NMR spectrum of epothilone analogue **#46.**

**Comparative Figure 51** shows a high resolution $^1$H NMR spectrum of epothilone analogue **#47.**

**Comparative Figure 52** shows a high resolution $^1$H NMR spectrum of epothilone analogue **#48.**

**Comparative Figure 53(A)** shows an approach to the preparation of desoxyepothilones. **(B)** represents a key step involving dianion addition to an aldehyde reactant.

**Comparative Figure 54** illustrates the acylation of t-butyl 4-methylpentan-3-on-1-ate to provide t-butyl 4,4-dimethylheptan-3,5-dion-1-ate.

**Comparative Figure 55** shows the preparation of a key intermediate hydroxyacid in the preparation of desoxyepothilones. The final synthetic steps leading to various desoxyepothilones from the hydroxyacid are found in, e. g., Figs. 21-26. R is selected from the group consisting of H, Me, Et, Pr, Hx, $CH_2OH$ and $(CH_2)_3OH$. Ar is selected from the group consisting of phenyl, tolyl, xylyl, thiazolyl, 2-methylthiazolyl, pyrryl and pyridyl, and is either unsubstituted or substituted with an $C_{1-6}$ alkyl, phenyl or benzyl group. Conditions for the Noyori reduction are disclosed in Taber *et al*., *Tetrahedron Lett*., **1991,** *32*, 4227, and Noyori *et al*., *J.Amer.Chem.Soc*., **1987,** *109*, 5856, the contents of which are incorporated herein by reference. Conditions for the DDQ deprotection are disclosed in Horita *et al*., *Tetrahedron,* **1986**, *42*, 3021, the contents of which is incorporated herein by reference.

**Comparative Figure 56** illustrates an application of the Noyori reduction of a substrate with C-15 hydroxyl useful in the preparation of epothilone analogues.

**Comparative Figure 57** exemplifies a dianion addition to a coupled aldehyde intermediate useful in the preparation of epothilone analogues.

**Comparative Figure 58** provides an application of the Noyori reduction of a coupled substrate useful in the preparation of epothilone analogues.

**Figure 59** shows the preparation of desoxyepothilone analogues by deoxygenation of the epoxide using Zn/Cu couple, exemplified by the conversion of desoxyepothilone B from epothilone B. In a sample procedure, Zn/Cu couple is added to a solution of epothilone B (6mg, 0.012 mmol) in i-PrOH (0.3 mL) and water (3 drops). The suspension was heated to 90°C for 13 hours, cooled to room temperature, filtered through a pad of Celite™ and concentrated. Flash chromatography afforded 1.5 mg of epothilone B (75% conversion) and 3.2 mg of desoxyepothilone B (73% yield, as a mixture of cis and trans isomers in a 0.7:1 ratio).

**Comparative Figure 60** illustrates the therapeutic effect of dEpoB, Taxol® and adriamycin in nude mice bearing the human mammary carcinoma MX-1 xenograft. MX-1 tissue preparation 100μl/mouse was implanted s.c. on day 0. Every other day i.p. treatments were given on day 8, 10, 12, 14 and 16 with dEpoB 35 mg/kg (■), Taxol® 5 mg/kg (▲), adriamycin (2 mg/kg (x), and vehicle (DMSO, 30μl) treated control (♦). For Taxol®, 2/10 mice died of toxicity on day 18. For adriamycin, 1/10 mice died of toxicity on day 22. For dEpoB, 10/10 mice survived and were subjected to the second cycle of treatment at 40 mg/kg on day 18, 20, 22, 24 and 26. This led to 3/10 mice tumor-free up to day 80, whereas 7/10 mice were with markedly suppressed tumors and were sacrificed on day 50.

**Comparative Figure 61(A)** shows a procedure for preparing intermediate **49**. [a]NaH, THF, 25°C, then 0°C, then propionyl chloride, -50°C, 71%; [b]NaH, 0°C then TESOTf, -50°C, 78%; LDA, THF, -33°C. 5 min.

**Comparative Figure 61(B)** shows a procedure for preparing desoxyepothilone B. [a]TrocCl, pyridine, $CH_2Cl_2$, 0°C - 25°C; then0.5N HCl in MeOH, 0°C, 87%; [b]9-BBN, THF, **50** then **51**, $(Pd(dppf)_2)Cl_2$, $Ph_3As$, $Cs_2CO_3$, $H_2O$, DMF; [c]0.4N HCl in MeOH (50% for two steps); [d](R)-(BINAP)$RuCl_2$, $H_2$-(1200 psi), MeOH, HCl, 25°C, 7 h, 88%, >95:5; [e]TESOTf, 2,6-lutidine, $CH_2Cl_2$, -78-25°C, then HCl/MeOH, 77%; [f]2,4,6-trichlorobenzoyl chloride, TEA, 4-DMAP, toluene, 78%; [g]$SmI_2$, cat. $NiI_2$, THF, -78°C, 95%; [h]HF•pyridine, THF, 98%; [i]2,2-dimethyldioxirane, $CH_2Cl_2$, -50°C, 98%, >20:1.

**Comparative Figure 62** shows the therapeutic effect of dEpoB and Taxol® in nude mice bearing MX-1 with Q2Dx5 i.v. 6 h infusion, using dEpoB 30 mg/kg (x), Taxol® 15 mg/kg (■), Taxol® 24 mg/kg (Δ) and control (●).

**Comparative Figure 63** shows the therapeutic effect of dEpoB and Taxol® in nude mice bearing MCF-7/Adr with Q2Dx5 i.v. 6 h infusion, using dEpoB 30 mg/kg (x), Taxol® 15 mg/kg (■), Taxol® 24 mg/kg (Δ) and control (●).

**Comparative Figure 64** shows the therapeutic effect of dEpoB and Taxol® in nude mice bearing CCRF/Taxol®

with Q2Dx5 i.v. 6 h infusion, using dEpoB 30 mg/kg (x), Taxol® 20 mg/kg (■), Taxol® 24 mg/kg (A) and control (●). Treatment on days 6, 8, 10, 12 and 14.

**Comparative Figure 65 (A) and (B)** show a procedure for preparing desoxyepothilone B **(14E)**.

**Comparative Figure 66 (A) and (B)** shows a procedure for preparing intermediate **8E**.

**Comparative Figure 67** shows the therapeutic effect of dEpoB and Taxol® in nude mice bearing CCRF/CEM tumor with Q2Dx4 i.v. 6 h infusion, using dEpoB 30 mg/kg (□), Taxol® 20 mg/kg (Δ), and control (○). Treatment on days 21, 23, 25, and 27.

**Comparative Figure 68** shows the therapeutic effect of dEpoB and Taxol® in nude mice bearing CCRF/Taxol® with Q2Dx5 i.v. 6 h infusion, using using dEpoB 30 mg/kg (□), Taxol® 20 mg/kg (Δ) and control (◊). Treatment on days 19, 21, 23, 25, 27, 39, 41, 43, 45 and 47.

**Comparative Figure 69** shows the therapeutic effect of dEpoB and Taxol® in nude mice bearing SK-OV-3 Tumor with Q2Dx6 i.v. 6 h infusion, using dEpoB 30 mg/kg (Δ), Taxol® 15 mg/kg (□), and control (○). Treatment on days 10, 12, 14, 16, 18 and 20.

**Comparative Figure 70** shows changes in body weight following treatment with desoxyepothilone B and Taxol® in nude mice bearing SK-OV-3 Tumor by i.v. infusion, using dEpoB 30 mg/kg (□), Taxol® 15 mg/kg (Δ) and control (◊).

**Comparative Figure 71** shows the therapeutic effect of dEpoB and Taxol® in nude mice bearing PC-3 Human Prostate Carcinoma with Q2Dx3 i.v. 6 or 18 h infusion, using dEpoB 30 mg/kg, 18 h (x), dEpoB 40 mg/kg, 6 h (*), dEpoB 50 mg/kg, 6 h (○) Taxol® 15 mg/kg, 6 h (□), Taxol® 24 mg/kg, 6 h (Δ) and control (♦). Treatment on days 5, 7 and 9.

**Comparative Figure 72** shows the therapeutic effect of dEpoB and Taxol® in nude mice bearing CCRF-CEM/VBL with Q2Dx5 i.v. 6 h infusion, using using dEpoB 30 mg/kg (□), Taxol® 20 mg/kg (Δ) and control (◊). Treatment on days 19, 21, 23, 25, 27, 39, 41, 43, 45, 47 and 53.

**Comparative Figure 73** shows the therapeutic effect of dEpoB and Taxol® in nude mice bearing HT-29 Colon Adenocarcinoma with Q2Dx6 i.v. 6 h infusion, using dEpoB 30 mg/kg (Δ), Taxol® 15 mg/kg (□) and control (◊).

**Comparative Figure 74** shows the therapeutic effect of dEpoB and Taxol® in nude mice bearing A549 Human Lung Carcinoma with Q2Dx3 i.v. 6 or 18 h infusion, using dEpoB 30 mg/kg 18h (x), dEpoB 40 mg/kg, 6 h (*), dEpoB 50 mg/kg, 6 h (o), Taxol® 15 mg/kg, 6 h (□), Taxol® 24 mg/kg, 6 h (Δ) and control (♦). Treatment on days 7, 9 and 11.

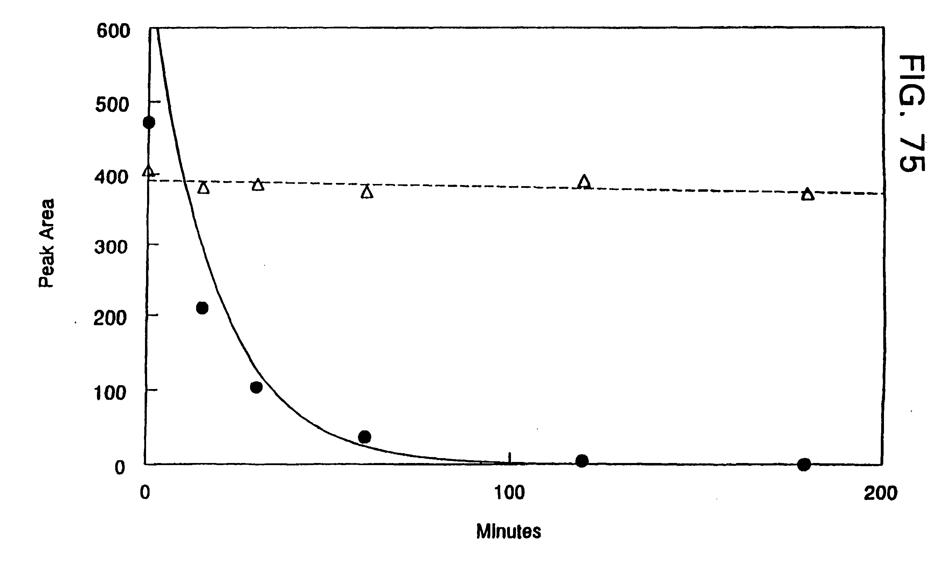

**Comparative Figure 75** shows the Epi stability in plasma of dEpoB : human (Δ) and mice (♦).

## Detailed Description of the Invention

As used herein, the term "linear or branched chain alkyl" encompasses, but is not limited to, methyl, ethyl, propyl, isopropyl, t-butyl, see-butyl, cyclopentyl or cyclohexyl. The alkyl group may contain one carbon atom or as many as fourteen carbon atoms, but preferably contains one carbon atom or as many as nine carbon atoms, and may be substituted by various groups, which include, but are not limited to, acyl, aryl, alkoxy, aryloxy, carboxy, hydroxy, carboxamido and/or N-acylamino moieties.

As used herein, the terms "alkoxycarbonyl", "acyl" and "alkoxy" encompass, but are not limited to, methoxycarbonyl, ethoxycarbonyl, propoxycarbonyl, n-butoxycarbonyl, benzyloxycarbonyl, hydroxypropylcarbonyl, aminoethoxycarbonyl, sec-butoxycarbonyl and cyclopentyloxycarbonyl. Examples of acyl groups include, but are not limited to, formyl, acetyl, propionyl, butyryl and penanoyl. Examples of alkoxy groups include, but are not limited to, methoxy, ethoxy, propoxy, n-butoxy, sec-butoxy and cyclopentyloxy.

As used herein, an "aryl" encompasses, but is not limited to, a phenyl, pyridyl, pyrryl, indolyl, naphthyl, thiophenyl or furyl group, each of which may be substituted by various groups, which include, but are not limited, acyl, aryl alkoxy, aryloxy, carboxy, hydroxy, carboxamido or N-acylamino moieties. Examples of aryloxy groups include, but are not limited to, a phenoxy, 2-methylphenoxy, 3-methylphenoxy and 2-naphthoxy. Examples of acyloxy groups include, but are not limited to, acetoxy, propanoyloxy, butyryloxy, pentanoyloxy and hexanoyloxy.

The present invention provides a method of preparing a desoxyepothilone having the structure:

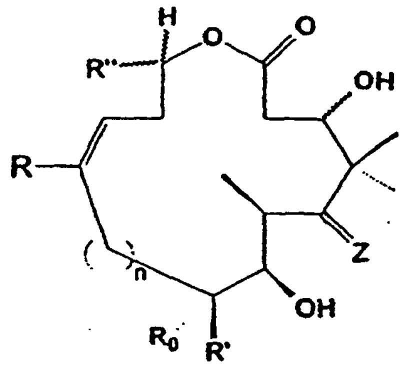

wherein R, $R_0$, and R' are independently H, linear or branched chain alkyl, optionally substituted by hydroxy, alkoxy, carboxy, carboxaldehyde, linear or branched alkyl or cyclic acetal, fluorine, $NR_1R_2$, N-hydroximino, or N-alkoxyimino, wherein $R_1$ and $R_2$ are independently H, phenyl, benzyl, linear or branched chain alkyl; wherein R" is -CY=CX-, or H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-roethyl-1,3-oxazolinyl, 3-indolyl or 6-indolyl; wherein X is H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein Y is H or linear or branched chain alkyl; wherein Z is O, $N(OR_3)$ or $N\text{-}NR_4R_5$, wherein $R_3$, $R_4$ and $R_5$ are independently H or a linear or branched alkyl; and wherein n is 0, 1, 2, or 3; which comprises treating an epothilone having a structure:

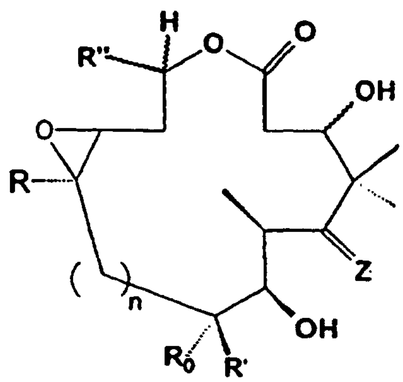

wherein R, $R_0$, $R_1$, $R_2$, $R_3$, $R_4$, $R_5$, R', R", X, Y, Z and n are defined as for the desoxyepothilone, under suitable conditions so as to deoxygenate the epothilone, and thereby to provide the desoxyepothilone. In one embodiment, the method is effected wherein the desoxyepothilone has the structure:

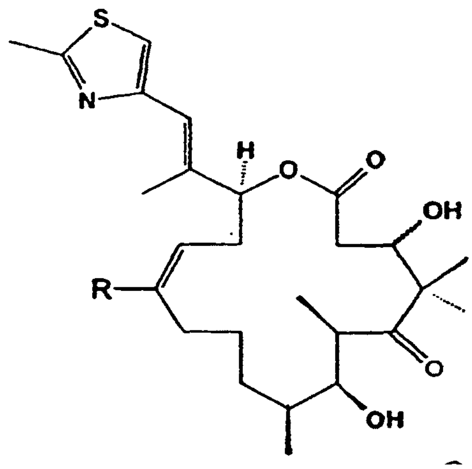

wherein R is H, methyl, ethyl, n-propyl, n-butyl, n-hexyl,

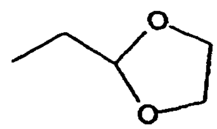

or $(CH_2)_3$-OH.

The method is effected wherein the epothilone is deoxygenated using a zinc/copper couple. Preferably, the method is carried out wherein the epothilone is deoxygenated in the presence of a polar solvent comprising isopropanol and water.

The present invention further provides a method of preparing a desoxyepothilone having the structure:

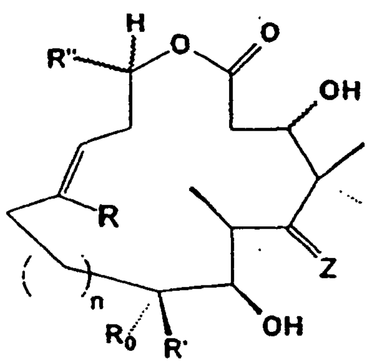

wherein R, $R_0$, and R' are independently H, linear or branched chain alkyl, optionally substituted by hydroxy, alkoxy, carboxy, carboxaldedyde, linear or branched alkyl or cyclic acetal, fluorine, $NR_1R_2$, N-hydroximino, or N-alkoxyimino, wherein $R_1$ and $R_2$ are independently H, phenyl, benzyl, linear or branched chain alkyl; wherein R" is -CY=CHX, or H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein X is H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein Y is H or linear or branched chain alkyl; wherein Z is O, $N(OR_3)$ or $N-NR_4R_5$, wherein $R_3$, $R_4$ and $R_5$ are independently H or a linear or branched chain alkyl; and wherein n is 0, 1, 2, or 3; which comprises treating an epothilone having a structure:

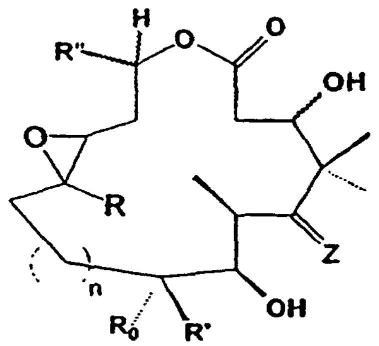

wherein R, $R_0$, $R_1$, $R_2$, $R_3$, $R_4$, $R_5$, R', R", X, Y, Z and n are defined as for the desoxyepothilone, under suitable conditions so as to deoxygenate the epothilone, and thereby to provide the desoxyepothilone. In one embodiment, the method is performed wherein the desoxyepothilone has the structure:

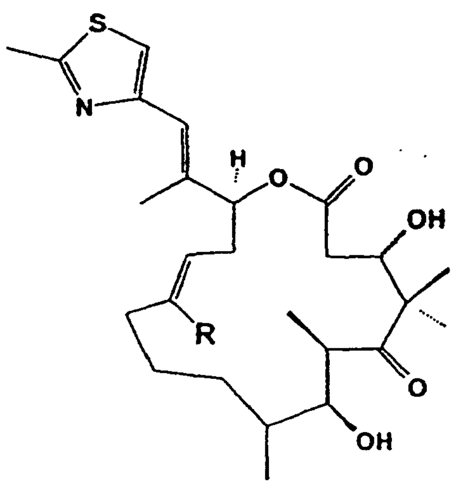

wherein R is H, methyl, ethyl, n-propyl, n-butyl, n-hexyl or hydroxypropyl. The method is effected wherein the epothilone is deoxygenated using a zinc/copper couple. Favorably, the epothilone is deoxygenated in the presence of a polar solvent comprising isopropanol and water.

Methods of preparation of intermediates are disclosed in U.S. Patent Applications Serial Nos. 60/032,282,60/033,767,60/047,566,60/047,941,and 60/055,533, filed December 3, 1996, January 14, 1997, May 22, 1997, May 29, 1997, and August 13, 1997, respectively, the contents of which are hereby incorporated by reference into this application.

The present invention will be better understood from the Experimental Details which follow. However, one skilled in the art will readily appreciate that the specific methods and results discussed are merely illustrative of the invention as described in the claims which follow thereafter. It will be understood that the processes of the present invention for preparing epothilones A and B, analogues thereof and intermediates thereto encompass the use of various alternate protecting groups known in the art. Those protecting groups used in the disclosure including the Examples below are merely illustrative.

## Comparative Example 1

THP glycidol: **13:** A solution of (*R*)-(+)-glycidol 12 (20 g; 270 mmol) and freshly distilled 3,4-dihydro-2H-pyran (68.1 g; 810 mmol) in $CH_2Cl_2$ (900 ml) was treated with pyridinium *p*-toluenesulfonate (2.1 g; 8.36 mmol) at rt and the resulting solution was stirred for 16 h. Approximately 50% of the solvent was then removed in vacuo and the remaining solution was diluted with ether (1 L). The organic layer was then washed with two portions of saturated aqueous sodium bicarbonate (500 ml), dried ($Na_2SO_4$), filtered, and concentrated. Purification of the residue by flash chromatography (silica, 25 $\rightarrow$ 50% ether:hexanes) afforded THP glycidol 13 (31.2 g; 73%) as a colorless liquid: IR(film): 2941, 1122, 1034 $cm^{-1}$; $^1H$ NMR($CDCl_3$, 500MHz)$\delta$4.66(t, $J$ = 3.5Hz, 1H), 4.64 (t, $J$ = 3.5 Hz, 1H), 3.93 (dd, $J$ = 11.7, 3.1 Hz, 1H),

3.86 (m, 2H), 3.73 (dd, $J$ = 11.8, 5.03 Hz, 1H), 3.67 (dd, $J$ = 11.8, 3.4Hz, 1 H), 3.51 (m, 2H), 3.40 (dd, $J$ = 11.7, 6.4, 1 H), 3.18 (m, 2H), 2.80 (m, 2H), 2.67 (dd, $J$ = 5.2, 2.7 Hz, 1H), 2.58 (dd, $J$ = 5.0, 2.7Hz, 1H), 1.82 (m, 2H), 1.73 (m, 2H), 1.52 (m, 4H); $^{13}$C NMR ($CDCl_3$,125MHz)δ98.9, 98.8, 68.5, 67.3, 62.4, 62.2, 50.9, 50.6, 44.6, 44.5, 30.5, 30.4, 25.4, 19.3, 19.2; $[a]_D$ = +4.98 (c=2.15, $CHCl_3$).

## Comparative Example 2

Alcohol **13a:** Trimethylsilylacetylene (32.3 g; 329 mmol) was added via syringe to THF (290 ml), and the resulting solution was cooled to -78 °C and treated with *n*-butyllithium (154 ml of a 1.6 M solution in hexanes; 246.4 mmol). After 15 min, boron trifluoride diethyl etherate (34.9 g; 246 mmol) was added, and the resulting mixture was stirred for 10 min. A solution of epoxide 13 (26 g; 164.3 mmol) in THF (130 ml) was then added via a cannula and the resulting solution was stirred for 5.5 h at -78 °C. The reaction was quenched by the addition of saturated aqueous sodium bicarbonate solution (250 ml) and the solution was allowed to warm to rt. The mixture was then diluted with ether (600 ml) and washed successively with saturated aqueous sodium bicarbonate solution (250 ml), water (250 ml), and brine (250 ml). The organic layer was then dried ($Na_2SO_4$), filtered, and concentrated in vacuo. Purification of the residue by flash chromatography (silica, 20% etherhexanes) provided alcohol **13a** (34 g; 76%).

## Comparative Example 3

MOM ether **13b**: A solution of alcohol **13a** (24 g; 88.9 mmol) and *N,N*-diisopropylethylamine (108 ml; 622 mmol) in anhydrous 1,2-dichlwoethane (600 ml) was treated with chloromethyl methyl ether (17 ml; 196 mmol), and the resulting mixture was heated to 55 °C for 28 h. The dark mixture was then cooled to rt and treated with saturated aqueous sodium bicarbonate solution (300 ml). The layers were separated, and the organic layer was washed successively with saturated aqueous sodium bicarbonate solution (200 ml) and brine (200 ml). The organic layer was then dried ($MgSO_4$) and filtered through a pad of silica gel (ether rinse). Purification of the residue by flash chromatography (silica, 20 - 30% ether:hexanes) afforded MOM ether **13b** (23.7 g; 85%) as a pale yellow oil.

## Comparative Example 4

Alcohol **14**: A solution of THP ether **13b** (20 g; 63.7 mmol) in methanol (90 ml) was treated with pyridinium p-toluenesulfonate (4.0 g; 15.9 mmol) and the resulting mixture was stirred at rt for 16 h. The reaction was then quenched by the addition of saturated aqueous sodium bicarbonate solution (100 ml), and the excess methanol was removed in vacuo. The residue was diluted with ether (300 ml), and the organic layer was washed successively with saturated aqueous sodium bicarbonate solution (200 ml) and brine (200 ml). The organic layer was dried ($MgSO_4$), filtered, and concentrated. Purification of the residue by flash chromatography (silica, 40 - 50% ether:hexanes) provided alcohol **14** (13.1 g; 95%) as a colorless oil.

## Comparative Example 5

Alcohol **14a**: To a cooled (-78 °C) solution of oxalyl chloride (24.04 ml of a 2.0 M solution in $CH_2Cl_2$; 48.08 mmol) in $CH_2Cl_2$ (165 ml) was added anhydrous DMSO (4.6 ml; 64.1 mmol) in dropwise fashion. After 30 min, a solution of alcohol **14** (6.93 g; 32.05 mmol) in $CH_2Cl_2$ (65 ml + 10 ml rinse) was added and the resulting solution was stirred at -78 °C for 40 min. Freshly distilled triethylamine (13.4 ml; 96.15 mmol) was then added, the cooling bath was removed, and the mixture was allowed to warm to 0 °C. The reaction mixture was then diluted with ether (500 ml), and washed successively with two portions of water (250 ml) and one portion of brine (250 ml). The organic layer was then dried ($MgSO_4$), filtered, and concentrated.

The crude aldehyde (6.9 g) prepared in the above reaction was dissolved in ether (160 ml) and cooled to 0 °C. Methylmagnesium bromide (32.1 ml of a 3.0 M solution in butyl ether; 96.15 mmol) was then added, and the solution was allowed to warm slowly to rt. After 10 h, the reaction mixture was cooled to 0 °C and the reaction was quenched by the addition of saturated aqueous ammonium chloride solution. The mixture was diluted with ether (200 ml) and washed successively with water (150 ml) and brine (150 ml). The organic layer was dried ($MgSO_4$), filtered, and concentrated. Purification of the residue by flash chromatography (silica, 40 → 50% ether:hexanes) provided alcohol **14a** (6.3 g; 85% from **14).**

## Comparative Example 6

Ketone **15**: A solution of alcohol **14** (1.0 g; 4.35 mmol), 4 Å mol. sieves, and N-methytmorphotine-*N*-oxide (1.0 g; 8.7 mmol) in $CH_2Cl_2$ (20 ml) at rt was treated with a catalytic amount of tetra-*n*-propylammonium perruthenate,

and the resulting black suspension was stirred for 3 h. The reaction mixture was then filtered through a pad of silica gel (ether rinse), and the filtrate was concentrated in vacuo. Purification of the residue by flash chromatography (silica, 10% ether:hexanes) afforded ketone 15 (924 mg; 93%) as a light yellow oil.

**Comparative Example 7**

Alkene **17**: A cooled (-78 °C) solution of phosphine oxide **16** (1.53 g; 4.88 mmol) in THF (15.2 ml) was treated with *n*-butyllithium (1.79 ml of a 2.45 M solution in hexanes). After 15 min, the orange solution was treated with a solution of ketone 15 (557 mg; 2.44 mmol) in THF (4.6 ml). After 10 min, the cooling bath was removed, and the solution was allowed to warm to rt. The formation of a precipitate was observed as the solution warmed. The reaction was quenched by the addition of saturated aqueous ammonium chloride solution (20 ml). The mixture was then poured into ether (150 ml) and washed successively with water (50 ml) and brine (50 ml). The organic layer was dried ($Na_2SO_4$), filtered, and concentrated. Purification of the residue by flash chromatography (silica, 10% ether:hexanes) afforded alkene 17 (767 mg; 97%) as a colorless oil: IR(film): 2956, 2177, 1506, 1249, 1149, 1032, 842, $cm^{-1}$; $^1H$ NMR($COCl_3$, 500MHz)δ6.95(s, 1H), 6.53(s, 1H), 4.67(d, *J* = 6.7Hz, 1H), 4.57 (d, *J* = 6.8Hz, 1H), 4.29 (dd, *J* = 8.1, 5.4 Hz, 1H), 3.43 (s, 3H), 2.70 (s, 3H), 2.62 (dd, *J* = 16.9, 8.2Hz, 1H), 2.51(dd, *J* = 17.0,5.4Hz, 1H), 2.02(s, 3H); $^{13}C$ NMR ($CDCl_3$, 125 MHz)δ164.4, 152.5, 137.1, 121.8, 116.2, 103.7, 93.6, 86.1, 79.6, 55.4, 25.9, 19.1, 13.5; $[\alpha]_D$ = -27.3 (c = 2.2, $CHCl_3$).

**Comparative Example 8**

Alkynyl iodide formation: To a solution of the alkyne **17** (3.00 g, 9.29 mmol) in acetone (100 mL) at 0°C was added NIS (2.51 g; 11.2 mmol) and $AgNO_3$ (0.160 g; 0.929 mmol). The mixture was then slowly warmed to rt. After 1.5 h, the reaction was poured into $Et_2O$ (250 mL) and washed once with sat bisulfite (40 mL), once with sat $NaHCO_3$ (40 mL), once with brine (40 mL) and dried over anhydrous $MgSO_4$. Purification by flash chromatography on silica gel using gradient elution with hexanes/ethyl acetate (10:1 - 7:1) gave 2.22 g (64%) of the iodide **17a** as an amber oil.

**Comparative Example 9**

Reduction of the alkynyl iodide: $BH_3$-DMS (0.846 mL, 8.92 mmol) was added to a solution of cyclohexene (1.47 mL, 17.9 mmol) in $Et_2O$ (60 mL) at 0°C. The reaction was then warmed to rt. After 1 h, the iodide x (2.22 g, 5.95 mmol) was added to $Et_2O$. After 3 h, AcOH (1.0 mL) was added. After 30 additional min, the solution was poured into sat $NaHCO_3$ and extracted with $Et_2O$ (3 x 100 mL). The combined organics were then washed once with brine (50 mL) and dried over anhydrous $MgSO_4$. Purification by flash chromatography on silica gel eluting with hexanes/ethyl acetate (6:1) gave 1.45 g (65%) of the vinyl iodide 18 as a yellow oil.

**Comparative Example 10**

MOM removal: To a solution of iodide **18** (1.45 g, 3.86 mmol) in $CH_2Cl_2$ (40 mL) at rt was added thiophenol (1.98 mL, 19.3 mmol) and $BF_3oEt_2O$ (1.90 mL, 15.43 mmol). After 22h, the reaction was poured into EtOAc (150 mL) and washed with 1N NaOH (2 x 50 mL) and dried over anhydrous $MgSO_4$. Purification by flash chromatography on silica gel using gradient elution with hexanes/ethyl acetate (4:1 - 2:1 - 1:1) gave 1.075 g (86%) of the alcohol **18a** as a pale yellow oil.

**Comparative Example 11**

Acetate formation: To a solution of alcohol **18a** (1.04 g, 3.15 mmol) in $CH_2Cl_2$ (30 mL) was added pyridine (2.52 mL, 25.4 mmol), acetic anhydride (1.19 mL, 12.61 mmol) and DMAP (0.005 g). After 1 h, the volatiles were removed in vacuo. Purification of the resulting residue by flash chromatography on silica gel eluting with hexanes/ethyl acetate (7:1) gave 1.16 g (99%) of the acetate **19** as a paleyellow oil. IR(film): 1737, 1368, 1232, 1018 $cm^{-1}$; $^1H$ NMR ($CDCl_3$, 500MHz)δ6.97 (s, 1H), 6.53 (s, 1H), 6.34 (dd, *J* = 17.5, 1.0Hz, 1H), 6.18 (dd, *J* = 13.7, 6.9Hz, 1h), 5.40 (t, *J* = 6.4Hz, 1H), 2.70 (s, 3h), 2.61 (m, 2H), 2.08 (2s, 6H). $^{13}C$ NMR ($CDCl_3$, 125 MHz)δ169.8, 164.4, 152.2, 136.4, 136.1, 120.6, 116.4, 85.1, 38.3, 21.0, 19.1, 14.7; $[\alpha]_D$ = -28.8 (c = 1.47, $CHCl_3$).

**Comparative Example 12**

To a solution of alcohol **4** (2.34 g, 3.62 mmol) and 2,6-lutidine (1.26 mL, 10.86 mmol) in $CH_2Cl_2$ (23 mL) at 0 °C was treated with TBSOTf (1.0 mL, 4.34 mmol). After stirrring for 1.5 h at 0 °C the reaction mixture was quenched with MeOH (200 uL) and the mixture stirred an additional 5 min. The reaction mixture was diluted with $Et_2O$ (100 mL)

and washed successively with 1 N HCl (25 mL), water (25 mL), and brine (25 mL). The solution was dried over $MgSO_4$, filtered, and concentrated. The residue was purified by flash chromatography on silica gel eluting with 5% $Et_2O$ in hexanes to provide compund 7 (2.70 g, 98%) as a colorless foam.

## Comparative Example 13

A solution of compound 7 (2.93 g, 3.85 mmol) in $CH_2Cl_2/H_2O$ (20:1, 80 mL) was treated with DDQ (5.23 g, 23.07 mmol) and the resulting suspension was stirred at room temperature for 24 h. The reaction mixture was diluted with $Et_2O$ (200 mL) and washed with aqueous $NaHCO_3$ (2 x 40 mL). The aqueous layer was extracted with $Et_2O$ (3 x 40 mL) and the combined organic fractions were washed with brine (50 mL), dried over $MgSO_4$, filtered, and concentrated. Purification of the crude oil by flash chromatography on silica gel eluting with 30% ether in hexanes afforded alcohol 7A (2.30 g, 89%) as a colorless oil: tR (film) 3488, 1471, 1428, 1115, 1054 $cm^{-1}$; $^1H$ NMR ($CDCl_3$, 500 MHz) δ7.70 (6 H, dd, *J* = 8.0, 1.5 Hz), 7.44 (9 H, s), 4.57 (1 H, d, *J* = 3.5 Hz), 4.19 (1 H, s), 3.67 (1 H, d, *J* = 8.5 Hz), 3.06 (1 H, dd, *J* = 11.5, 5.0 Hz), 2.89 (1 H, dd, *J* = 11.5, 5.0 Hz), 2.68 (1 H, d, *J* = 13.5 Hz), 2.59 (1 H, d, *J* = 13.5 Hz), 2.34 (1 H, dt, *J* = 12.0, 2.5 Hz), 2.11 (1 H, m), 1.84 (1 H, dt, *J* = 12.0, 2.5 Hz), 1.76 (2 H, m), 1.59 (2 H, m), 1.34 (3 H, s), 1.13 (3 H, d, *J* = 7.5 Hz), 1.10 (3 H, s), 0.87 (9 H, s), 0.84 (3 H, d, *J* = 12.0 Hz), 0.02 (3 H, s), 0.01 (3 H, s); $^{13}C$ NMR ($CDCl_3$, 125 MHz)δ136.18, 134.66, 130.16, 127.84, 78.41, 75.91; 63.65, 59.69, 45.43, 45.09, 37.72, 30.84, 30.50, 26.23, 25.89, 22.42, 21.05, 18.40, 15.60, 14.41, -3.23, -3.51; $[\alpha]_D$ = -0.95 (c = 0.173, $CHCl_3$).

## Comparative Example 14

To a solution of oxalyl chloride (414 μL, 4.74 mmol) in $CH_2Cl_2$ (40 mL) at -78 °C was added dropwise DMSO (448 uL, 6.32 mmol) and the resulting solution was stirred at -78 °C for 30 min. Alcohol 7a (2.12 g, 3.16 mmol) in $CH_2Cl_2$ (20 mL) was added and the resulting white suspension was stirred at -78°C for 45 min. The reaction mixture was quenched with $Et_3N$ (2.2 mL, 15.8 mmol) and the solution was allowed to warm to 0 °C and stirred at this temperature for 30 min. The reaction mixture was diluted with $Et_2O$ (100 mL) and washed successively with aqueous $NH_4Cl$ (20 mL), water (20 mL), and brine (20 mL). The crude aldehyde was purified by flash chromatography on silica gel eluting with 5% $Et_2O$ in hexanes to provide aldehyde 8 (1.90 g, 90%) as a colorless oil.

## Comparative Example 15

A solution of (methoxymethyl)triphenylphosphonium chloride (2.97 g, 8.55 mmol) in THF (25 mL) at 0 °C was treated with KO'Bu (8.21 mL, 1M in THF, 8.1 mmol). The mixture was stirred at 0 °C for 30 min. Aldehyde **8** (3.1 g, 4.07 mmol) in THF (10 ml) was added and the resulting solution was allowed to warm to room temperature and stirred at this temperature for 2 h. The reaction mixture was quenched with aqueous $NH_4Cl$ (40 mL) and the resulting solution extracted with $Et_2O$ (3 x 30 mL). The combined $Et_2O$ fractions were washed with brine (20 ml), dried over $MgSO_4$, filtered, and concentrated. The residue was purified by flash chromatography on silica gel eluting with 5% $Et_2O$ in hexanes to yield compound 9 (2.83 g, 86%) as a colorless foam.

## Comparative Example 16

To a solution of compound **9** (2.83g, 3.50 mmol) in dioxane/$H_2O$ (9:1, 28 mL) was added pTSA-$H_2O$ (1.0 g, 5.30 mmol) and the resulting mixture was heated to 50°C for 2 h. After cooling to room temperature the mixture was diluted with $Et_2O$ (50 mL) and washed with aqueous $NaHCO_3$ (15 mL), brine (20 ml), dried over $MgSO_4$, filtered, and concentrated to provide aldehyde 9a (2.75 g, 99%) as a colorless foam.

## Comparative Example 17

Methyltriphenylphosphonium bromide (1.98 g, 5-54 mmol) in THF (50 ml) at 0 °C was treated with lithium bis (trimethylsilyl)amide (5.04 ml, 1M in THF, 5.04 mmol) and the resulting solution was stirred at 0 °C for 30 min. Aldehyde **9a** (2.0 g, 2.52 mmol) in THF (5.0 mL) was added and the mixture was allowed to warm to room temperature and stirred at this temperature for 1 h. The reaction mixture was quenched with aqueous $NH_4Cl$ (15 mL) and extracted with $Et_2O$ (3 x 20 mL). The combined $Et_2O$ fractions were washed with brine (15 mL), dried over $MgSO_4$, filtered, and concentrated. The residue was purified by flash chromatography on silica gel eluting with 5% $Et_2O$ in hexanes to afford compound **10** (1.42 g, 76%) as a colorless foam.

**Comparative Example 18**

A solution of compound **10** (1.0g, 1.34 mmol) in MeOH/THF (2:1, 13 mL) was treated with [bis(trifluoroacetoxy) iodobenzene] (865 mg, 2.01 mmol) at room temperature. After 15 min the reaction mixture was quenched with aqueous $NaHCO_3$ (25 mL). The mixture was extracted with $Et_2O$ (3 x 25 mL) and the combined $Et_2O$ fractions were washed with brine, dried over $MgSO_4$, filtered, and concentrated. Purification of the residue by flash chromatography on silica gel eluting with 5% $Et_2O$ in hexanes provided compound 11 (865 mg, 92%) as a colorless foam: IR (film) 1428, 1252, 1114, 1075, 1046 $cm^{-1}$; $^1H$ NMR ($CDCl_3$, 500 MHz)$\delta$7.61 (6 H, dd, *J* = 7.9, 1.4 Hz), 7.38 (9 H, s), 5.47 (1 H, m), 4.87 (1 H, d, *J* = 10.0 Hz), 4.76 (1 H, d, *J* = 15.9 Hz), 4.30 (1 H, d, *J* = 3.7 Hz), 3.95 (1 H, s), 3.56 (1 H, dd, *J* = 7.5, 1.4 Hz), 3.39 (3 H, s), 2.84 (3 H, s), 2.02 (1 H, m), 1.64 (2 H, m), 1.34 (1 H, m), 1.11 (3 H, s), 1.02 (3 H, d, *J* = 7.4 Hz), 0.90 (3 H, s), 0.85 (9 H, s), 0.62 (3 H, d, *J* = 6.8 Hz), -0.04 (3 H, s), -0.05 (3 H, s); $^{13}C$ NMR ($CDCl_3$, 125 MHz)$\delta$138.29, 135.79, 135.04, 129.86, 127.78, 114.98, 110.49, 60.11, 55.57, 46.47, 43.91, 36.82, 34.21, 26.26, 19.60, 18.60, 17.08, 16.16, 13.92, -2.96, -3.84; $[\alpha]_D$ = +1.74 (c = 0.77, $CHCl_3$).

**Comparative Example 19**

Suzuki Coupling: To a solution of olefin **11** (0.680 g, 1.07 mmol) in THF (8.0 mL) was added 9-BBN (0.5 M soln in THF, 2.99 mL, 1.50 mmol). In a separate flask, the iodide **19** (0.478 g, 1.284 mmol) was dissolved in DMF (10.0 mL). $CsCO_3$ (0.696 g, 2.14 mmol) was then added with vigorous stirring followed by sequential addition of $Ph_3As$ (0.034 g, 0.111 mmol), $PdCl_2(dppf)_2$ (0.091 g, 0.111 mmol) and $H_2O$ (0.693 mL, 38.5 mmol). After 4 h, then borane solution was added to the iodide mixture in DMF. The reaction quickly turned dark brown in color and slowly became pale yellow after 2 h. The reaction was then poured into $H_2O$ (100 mL) and extracted with $Et_2O$ (3 x 50 mL). The combined organics were washed with $H_2O$ (2 x 50 mL), once with brine (50 mL) and dried over anhydrous $MgSO_4$. Purification by flash chromatography on silica gel eluting with hexanes/ethyl acetate (7:1) gave 0.630 g (75%) of the coupled product 20 as a pale yellow oil.

**Comparative Example 20**

Hydrolysis of dimethyl acetal **21**: The acetate 20 (0.610 g, 0.770 mmol) was dissolved in dioxane/$H_2O$ (9:1,15 mL) and *p*-TSA·H $_2$O (0.442 g, 2.32 mmol) was added. The mixture was then heated to 55°C. After 3 h, the mixture was cooled to rt and poured into $Et_2O$. This solution was washed once with sat $NaHCO_3$ (30 mL), once with brine (30 mL) and dried over anhydrous $MgSO_4$. Purification by flash chromatography on silica gel eluting with hexanes/ethyl acetate (7:1) gave 0.486 g (85%) of the aldehyde **21** as a pale yellow oil. IR (film) 1737, 1429, 1237, 1115, 1053 $cm^{-1}$; $^1H$ NMR ($CDCl_3$, 500 MHz)$\delta$9.74 (1 H, s), 7.61 (6 H, dd, *J* = 7.8, 1.2 Hz), 7.38 (9 H, m), 6.94 (1 H, s), 6.53 (1 H, s), 5.39 (1 H, m), 5.31 (1 H, m), 5.29 (1 H, t, *J* = 6.9 Hz), 4.61 (1 H, d, *J* = 4.3 Hz), 3.50 (1 H, dd, *J* = 5.2, 2.6 Hz), 2.70 (3 H, s), 2.48 (2 H, m), 2.14 (1 H, m), 2.09 (3 H, s), 2.07 (3 H, s), 1.83 (2 H, m), 1.41 (1 H, m), 1.18 (1 H, m), 1.01 (3 H, s), 0.99 (3 H, s), 0.91 (3 H, d, *J* = 7.4 Hz), 0.85 (9 H, s), 0.69 (1 H, m), 0.58 (3 H, d, *J* = 6.8 Hz), -0.05 (3 H, s), -0.06 (3 H, s); $^{13}C$ NMR ($CDCl_3$, 125 MHz)$\delta$205.46, 170.01, 164.49, 152.46, 137.10, 135.60, 134.22, 132.55, 130.65, 127.84, 123.82, 120.66, 116.19, 81.09, 78.47, 76.73, 51.66, 43.14, 38.98, 30.99, 30.42, 27.63, 26.10, 21.15, 20.92, 20.05, 19.15, 18.49, 15.12, 14.70, 12.75, -3.25, -4.08; $[\alpha]_D$ = -18.7 (c = 0.53, $CHCl_3$).

**Comparative Example 21**

Aldol: To a solution of the acetate-aldehyde 21 (84 mg, 0.099 mmol) in THF at -78°C was added KHMDS (0.5M in toluene, 1.0 ml, 0.5 mmol)) dropwise.The resulting solution was stirred at -78°C for 30 min. Then the reaction mixure was cannulated to a short pad of silica gel and washed with ether. The residue was purified by flash chromatography (silica, 12% EtOAc in hexane) to give the lactone 22 (37 mg of 3-S and 6 mg of 3-R, 51 %) as white foam.

**Comparative Example 22**

Monodeprotection: Lactone **22** (32 mg, 0.0376 mmol) was treated with 1ml of pyridine buffered HF pyridine - THF solution at room temperture for 2 h. The reaction mixure was poured into saturated aqueous $NaHCO_3$ and extracted with ether . The organic layer was washed in sequence with saturated $CuSO_4$ (10 ml x 3) and saturated $NaHCO_3$ (10 ml), then dried over $Na_2SO_4$ and concentrated under vacuum. The residue was purified by flash chromatography (silica, 25% EtOAc in hexane) and to give diol **22a** (22 mg, 99%) as white foam.

**Comparative Example 23**

TBS-protection: To a cooled (-30°C) solution of diol **22a** (29 mg, 0.0489 mmol) and 2,6-lutidine (0.017 ml, 0.147 mmol) in anhydrous $CH_2Cl_2$ (1ml) was added TBSOTf (0.015 ml, 0.0646 mmol). The resulting solution was then stirred at -30°C for 30 min. The reaction was quenched with 0.5M HCl (10 ml) and extracted with ether (15 ml). Ether layer was washed with saturated $NaHCO_3$, dried ($Na_2SO_4$) and concentrated in vacuo. Purifiction of the residue by flash chromatogrphy (silica, 8% EtOAc in hexane) afforded TBS ether **22B** (32 mg, 93%) as white foam.

**Comparative Example 24**

Ketone Formation: To a solution of alcohol **22B** (30 mg, 0.0424 mmol) in $CH_2Cl_2$ (2.0 mL) at 25°C was added Dess-Martin periodinane (36 mg, 0.0848 mmol) in one portion. The resulting solution was then allowed to stir at 25°C for 1.5 h. The reaction was quenched by the addition of 1:1 saturated aqueous sodium bicarbonate: sodium thiosulfate (10 ml) and stirred for 5 min. The mixture was then extracted with ether (3 x 15 ml). The organic layer was dried ($Na_2SO_4$), filtered, and concentrated in vacuo . Purification of the residue by flash chromatography (silica, 8% EtOAc in hexane) provided ketone **22C** (25 mg, 84%) as white foam. IR(film): 2928, 1745, 1692, 1254, 1175, 836 $cm^{-1}$; $^1H$ NMR($CDCl_3$, 500 MHz)δ6.97 (s, 1H), 6.57 (s, 1H), 5.53 (dt, *J* = 3.4, 11.1 Hz, 1 H), 5.37 (dd, *J* = 16.4, 9.9Hz, 1H), 5.00 (d, *J* = 10.3Hz, 1 H), 4.02 (d, *J* = 9.7Hz, 1 H), 3.89 (d, *J* = 8.7Hz, 1H), 3.00 (m, 1 H), 2.82 (d, *J* = 6.5Hz, 1H), 2.71 (m, 5H), 2.36 (q, *J* =10.7Hz, 1 H), 2.12 (, 3H), 2.07 (dd, *J* - 8.2, 1H), 1.87 (bs, 1 H), 1.49 (m, 3H), 1.19 (m, 5H), 1.14 (s, 3H), 1.08 (d, *J* = 6.8Hz, 3H), 0.94 (m, 12H), 0.84 (s, 9H), 0.12 (s, 3H), 0.10 (s, 3H), 0.07 (s, 3H), -0.098 (s, 3H); $^{13}C$ NMR ($CDCl_3$, 125 MHz)δ 218.7, 170.1, 164.5, 152.6, 137.9, 133.9, 124.8, 119.6, 115.9, 72.7, 53.2, 43.9, 41.0, 40.3, 32.9, 32.3, 28.4, 27.1, 26.3, 26.1, 26.0, 19.2, 19.1, 18.3, 18.2, 17.1, 16.0, 15.2, 14.3, -4.2, -4.4, -4.6, -4.8 ; $[\alpha]_D$ = -21.93 (c = 1.4, $CHCl_3$).

**Comparative Example 25**

Desoxy compound: To a solution of TBS ether **22C** (27 mg, 0.038 mmol) in THF(1 ml) at 25°C in a plastic vial was added dropwise HF-pyridine (0.5 ml). The resulting solution was allowed to stir at 25°C for 2 h. The reaction mixture was diluted with chloroform (2 ml) and very slowly added to satured sodium bicarbonate (20 ml). The mixture was extracted with $CHCl_3$ (20ml x 3). The organic layer was dried ($Na_2SO_4$), filtered, and concentrated in vacuo. Purification of the residue by flash chromatography (silica , 30% EtOAc in hexane) provided diol **23** (18 mg, 99%) as white foam: IR(film): 3493, 2925, 1728, 1689, 1249 $cm^{-1}$; $^1H$ NMR ($CDCl_3$, 500 MHz)δ6.96 (s, 1 H), 6.59 (s, 1H), 5.44 (dt, *J* = 4.3, 10.4 Hz, 1H), 5.36 (dt, *J* = 5.1, 10.2 Hz, 1H), 5.28 (dd, *J* = 1.7, 9.8 Hz, 1H), 4.11 (d, *J* = 7.2 Hz, 1H), 3.74 (s, 1H), 3.20 (d, *J* = 4.5 Hz, 1H), 3.14 (dd, *J* = 2.2, 6.8 Hz, 1H), 3.00 (s, 1H), 2.69 (m, 4H), 2.49 (dd, *J* = 11.3, 15.1 Hz, 1H), 2.35 (dd, *J* -2.5, 15.1 Hz, 1H), 2.27 (m, 1H), 2.05 (m, 1H), 2.04 (s, 3H), 2.01 (m, 1H) 1.75 (m, 1 H), 1.67 (m, 1H), 1.33 (m, 4H), 1.21 (s, 1H), 1.19 (m, 2H), 1.08 (d, *J* = 7.0 Hz, 3H), 1.00 (s, 3H), 0.93 (d, *J* = 7.1 Hz, 3H); $^{13}C$ NMR ($CDCl_3$, 125 MHz)δ226.5, 176.5, 171.1, 158.2, 144.7, 139.6, 131.1, 125.7, 122.0, 84.6, 80.2, 78.6, 59.4, 47.9, 45.4, 44.6, 38.5, 37.9, 33.7, 33.6, 28.7, 25.1, 25.0, 21.9, 21.7, 19.6 ; $[\alpha]_D$ = -84.7 (c = 0.85, $CHCl_3$).

**Comparative Example 26**

Epothilone: To a cooled (-50°C) solution of desoxyepothilone (9 mg, 0.0189 mmol) in dry $CH_2Cl_2$ (1 ml) was added freshly prepared dimethyldioxirane (0.95 ml, 0.1 M in acetone). The resulting solution was allowed to warm up to -30°C for 2 h. A stream of nitrogen was then bubbled through the solution to remove excess DMDO. The residue was purified by flash chromatography (silica, 40% EtOAc in hexane) and afforded epothilone A (4.6 mg, 49%) as colorless solid and 0.1 mg of cis-epoxide diastereomer. This material was identical with the natural epothilone A in all respects.

**Comparative Example 27**

Procedure for Rine-closine Olefin Metathesis:

To a stirred solution of diene **24** (5 mg, 0.0068 mmol) in dry benzene (1.5 mL) was added Grubbs's catalyst (2.8 mg, 0.0034 mmol). After 12 h, an additional portion of catalyst was added (2.8 mg). After an additional 5 h, the reaction was concentrated. Purification by silica gel chromatography eluting with hexanes/ethyl acetate (11:1) gave the lactone 23 (3.5 mg, 94%, 2:1 E/Z).

**Comparative Example 28**

Preparation of Compound **19**:

Alcohol **2A:** A mixture of (S)-(-)-1,1'-bi-2-naphthol (259 mg. 0.91 mmoL), $Ti(O\text{-}i\text{-}Pr)_4$ (261 µL;0.90 mmol), and 4 Å sieves (3.23g) in $CH_2Cl_2$ (16 ml) was heated at reflux for 1 h. The mixture was cooled to rt and aldehyde 1 was added. After 10 min. the suspension was cooled to -78°C, and allyl tributyltin (3.6 mL; 11.60 mmol) was added. The reaction mixture was stirred for 10 min at -78 °C and then placed in a -20 °C freezer for 70 h. Saturated $NaHCO_3$ (2mL) was added, and the mixture was stirred for 1 h, poured over $Na_2SO_4$, and then filtered through a pad of $MgSO_4$ and celite. The crude material was purified by flash chromatography (hexanes/ethyl acetate, 1:1) to give alcohol **2A** as a yellow oil (1.11 g; 60%).

**Comparative Example 29**

Acetate **3A**: To a solution of alcohol **2A** (264 mg; 1.26 mmol) in $CH_2Cl_2$ (12 mL) was added DMAP (15mg: 0.098 mmol), $Et_3N$ (0.45 mL; 3.22 mmol), and $Ac_2O$ (0.18 mL; 1.90 mmol). After 2h, the reaction mixture was quenched by 20 mL of $H_2O$, and extracted with EtOAC (4 x 20 mL). The combined organic layer was dried with $MgSO_4$, filtered, and concentrated. Flash chromatrography (EtOAC/hexanes, 1:3) afforded acetate **3A** as a yellow oil (302 mg; 96%).

**Comparative Example 30**

Vinyl Iodide **19:** To a solution of acetate **3A** (99 mg; 0.39 mmol) in acetone at 0 °C was added $H_2O$ (4 drops), $OsO_4$ (2.5% wt. in butyl alcohol; 175 µL; 0.018 mmol), and N-methyl-morpholine-N-oxide (69 mg; 0.59 mmol). The mixture was stirred at 0 °C for 2h and 45 min and then quenched with $Na_2SO_3$. The solution was poured to 10 mL of $H_2O$ and extracted with EtOAc (5 x 10mL). The combined organic layer was dried over MgSO4, filtered, and concentrated.

To a solution of this crude product in $THF/H_2O$ (4 mL, 3:1) was added $NaIO_4$ (260 mg; 1.22 mmol). After 1.25 h, the reaction mixture was then quenched with 10 mL of $H_2O$ and concentrated. The residue was extracted with EtOAc (5 x 10mL). The organic layer was dried over $MgSO_4$, filtered, and concentrated. Flash chromatography (EtOAc/hexanes, 1:1) gave a yellow oil (80 mg) which contained unidentified by-product(s). This mixture was used without further purification.

To a solution of $(Ph_3P\ {}^+CH_2I)I^-$ (100 mg; 0.19 mmol) in 0.25 mL of THF at rt was added 0.15 mL (0.15 mmol) of NaHMDS (1 M in THF). To the resulting solution at -78°C was added HMPA (22 µL; 0.13 mmol) and the product from previous step (16 mg) in THF (0.25 mL). The reaction mixture was then stirred at rt for 30 min. After the addition of hexanes (10mL), the solution was extracted with EtOAc (4 x 10ml). The combined EtOAC layer was dried ($MgSO_4$), filtered, and concentrated. Preparative TLC (EtOAc/hexanes, 2.3) afforded vinyl iodide **19** as a yellow oil (14 mg; 50% for three steps).

**Comparative Example 31**

Iodoolefin acetate **8C:** To a suspension of ethyltriphenylphosphonium iodide (1.125 g, 2.69 mmol) in THF (10 mL) was added *n*BuLi (2.5 M soln in hexanes, 1.05 mL, 2.62 mmol) at rt. After disappearance of the solid material, the solution was added to a mixture of iodine (0.613 g, 2.41 mmol) in THF (20 mL) at -78 °C. The resulting suspension was vigorously stirred for 5 min at -78 °C, then warmed up -20 °C, and treated with sodium hexamethyldisilazane (1 M soln in THF, 2.4 mL, 2.4 mmol). The resulting red solution was stirred for 5 min followed by the slow addition of aldehyde **9C** (0.339 g, 1.34 mmol). The mixture was stirred at -20 °C for 40 min, diluted with pentane (50 mL), filtered through a pad of celite, and concentrated. Purification of the residue by flash column chromatography (hexanes/ethyl acetate, 85:15) gave 0.202 g (25% overall from vinyl acetate **10C**) of the vinyl iodide **8C** as a yellow oil. IR (film): 2920, 1738, 1234 $cm^{-1}$; $^1H$ NMR ($CDCl_3$): δ 6.98 (s, 1H), 6.56 (s, 1H), 5.42 (dd, *J* = 5.43, 6.57 Hz, 1H), 5.35 (t, *J* = 6.6 Hz, 1 H), 2.71 (s, 3H), 2.54 (q, *J* = 6.33, 2H), 2.50 (s, 3H), 2.09 (s, 6H); $^{13}C$ NMR ($CDCl_3$): δ 170.1, 164.6, 152.4, 136.9, 130.2, 120.6, 116.4, 103.6, 40.3, 33.7, 21.2, 19.2, 14.9; $[\alpha]_D$ = -20.7 ° (c = 2.45, $CHCl_3$).

**Comparative Example 32**

Acetal **13C:** To a solution of olefin "**7C**" (0.082 g, 0.13 mmol) in THF (0.5 mL) was added 9-BBN (0.5 M soln in THF, 0.4 mL, 0.2 mmol). After stirring at rt. for 3.5 h, an additional portion of 9-BBN (0.5 M soln in THF, 0.26 mL, 0.13 mmol) was added. In a separate flask, iodide **8C** (0.063 g, 0.16 mmol) was dissolved in DMF (0.5 mL). $Cs_2CO_3$ (0.097 g, 0.30 mmol) was then added with vigorous stirring followed by sequential addition of $PdCl_2(dppf)_2$ (0.018 g,

0.022 mmol), $Ph_3As$ (0.0059 g, 0.019 mmol), and $H_2O$ (0.035 mL, 1.94 mmol). After 6 h, then borane solution was added to the iodide mixture in DMF. The reaction quickly turned dark brown in color and slowly became pale yellow after 3 h. The reaction was then poured into $H_2O$ (10 mL) and extracted with $Et_2O$ (3 x 15 mL). The combined organic layers were washed with $H_2O$ (3 x 15 mL), brine (1 x 20 mL), dried over $MgSO_4$, filtered, and concentrated. Flash column chromatography (hexanes/ethyl acetate, 9:1) gave 0.089 g (77%) of the coupled product **13C** as a yellow oil.

## Comparative Example 33

Aldehyde **14C:** Acetal **13C** (0.069 g, 0.077 mmol) was dissolved in dioxane/$H_2O$ (9:1, 1 mL) and *p*TSA·$H_2O$ (0.045 g, 0.237 mmol) was added. The mixture was then heated to 55°C. After 3 h, the mixture was cooled to rt, poured into $Et_2O$, and extracted with $Et_2O$ (4 x 15 mL). The combined ether solutions were washed with sat $NaHCO_3$ (1 x 30 mL), brine (1 x 30 mL), dried over $MgSO_4$, filtered, and concentrated. Flash column chromatography (hexanes/ethyl acetate, 3:1) gave 0.046 g (71 %) of the aldehyde **14C** as a pale yellow oil.

## Comparative Example 34

Macrocycle **15C-*(SR)*:** To a solution of aldehyde **14C** (0.021 g, 0.024 mmol) in THF (5 mL) at -78 °C was added KHMDS (0.5 M soln in toluene, 0.145 mL, 0.073 mmol). The solution was stirred at -78 °C for 1 h, then quenched with sat'd $NH_4Cl$, and extracted with ether (3 x 15 mL). The combined organic layers were dried with $MgSO_4$, filtered, and concentrated. Flash column chromatography (hexaneslethyl acetate, 7:1) gave 0.008 g of the desired α-alcohol **15C-(*S*)** and 0.006 g of β-alcohol **15C-(*R*)** (67% total) as pale yellow oils.

## Comparative Example 35

Macrocycle **15C-(*S*):** To a solution of β-alcohol **15C-(*R*)** (0.006 g, 0.0070 mmol) in 0.5 mL of $CH_2Cl_2$ at rt. was added Dess-Martin periodinane (0.028g, 0.066 mmol). After 0.5 h, an additional portion of Dess-Martin periodinane (0.025 mg, 0.059 mmol) was added. The resulting solution was stirred at rt for additional 1 h, then treated with ether (2 mL) and sat'd $Na_2S_2O_3$/sat'd $NaHCO_3$ (3 mL, 1:1), poured into $H_2O$ (20 mL), and extracted with ether (4 x 10 mL). The combined ether solutions were washed with $H_2O$ (1 x 30 mL), brine (1 x 30 mL), dried with $MgSO_4$, filtered, and concentrated. To a solution of crude ketone **15C'** in MeOH/THF (2 mL, 1:1) at -78 °C was added $NaBH_4$ (0.015 g, 0.395 mmol). The resulting solution was stirred at rt for 1 h, quenched with sat $NH_4Cl$, and extracted with ether (3 x 15 mL). The organic layers were dried with $MgSO_4$, filtered, and concentrated. Flash column chromatography (hexanes/ethyl acetate, 9:1) gave 0.0040 g (67%) of the α-alcohol **15C-(*S*)** as a pale yellow oil and 0.0006 g of β-alcohol **15C-(*R*).**

## Comparative Example 36

Diol **15C":** The silyl ether **15C-(*S*)** (0.010 g, 0.012 mmol) was dissolved in HF-pyridinelpyridine/THF (1 mL). The solution was stirred at rt. for 2 h, then diluted with $Et_2O$ (1 mL), poured into a mixture of $Et_2O$/sat. $NaHCO_3$ (20 mL, 1:1), and extracted with $Et_2O$ (4x10 mL). The $Et_2O$ solutions were washed with sat $CuSO_4$ (3 x 30 mL), sat $NaHCO_3$ (1 x 30 mL), brine (1 x 30 mL), dried with $MgSO_4$, filtered, and concentrated. Flash column chromatography (hexanes/ethyl acetate, 9:1) gave 0.0066 g (93%) of the diol **15C"** as a pale yellow oil.

## Comparative Example 37

Alcohol **15C'''**: To a solution of diol **15C"** (0.0066 g, 0.011 mmol) in 0.5 mL of $CH_2Cl_2$ at -78 °C was added 2,6-lutidine (7 µL, 0.060 mmol) and TBSOTf (5 µL, 0.022 mmol). The resulting solution was stirred at -30 °C for 0.5 h, then quenched with $H_2O$ (5 mL), and extracted with $Et_2O$ (4 x 10 mL). The ether solutions were washed with 0.5 M HCl (1 x 10 mL), sat'd $NaHCO_3$ (1 x 10 mL), dried over $MgSO_4$, filtered, and concentrated. Flash column chromatography (hexanes/ethyl acetate, 93:7) gave 0.0070 g (89%) of the alcohol **15C'''** as a pale yellow oil.

## Comparative Example 38

Ketone **16C:** To a solution of alcohol **15C'''** (0.006 g, 0.0083 mmol) in 0.5 mL of $CH_2Cl_2$ at rt. was added Dess-Martin periodinane (0.030g, 0.071 mmol). After 1.25 h, another portion of Dess-Martin periodinane (0.025 mg, 0.059 mmol) was added. The resulting solution was stirred at rt for additional 0.75 h, treated with ether (1 mL) and sat'd $Na_2S_2O_3$/sat'd $NaHCO_3$ (2 mL, 1:1), poured into $H_2O$ (20 mL), and extracted with ether (4 x 10 mL). The ether solution was washed with sat $NaHCO_3$ (1 x 20 mL), dried with $MgSO_4$, filtered, and concentrated. Flash column chromatography (hexanes/ethyl acetate, 9:1) gave 0.0040 g (67%) of the ketone **16C** as a pale yellow oil.

**Comparative Example 39**

Desoxyepothiolone B **2C**:To a solution of ketone **16C** (0.004 g, 0.0056 mmol) in THF (0.35 mL) was added HF·pyridine (0.25 mL) dropwise over 20 min. The solution was stirred at rt for 1.5 h, diluted with $CHCl_3$ (2 mL), poured into sat'd $NaHCO_3$/$CHCl_3$ (20 mL, 1:1) slowly, and extracted with $CHCl_3$ (4 x 10 mL). The combined $CHCl_3$ layers were dried with $MgSO_4$, filtered, and concentrated. Flash column chromatography (hexanes/ethyl acetate, 3:1) gave 0.0022 g (80%) of the desoxyepothilone B **2C** as a pale yellow oil.

**Comparative Example 40**

Epothilone B **2:** To a solution of desoxyepothilone B (0.0022 g, 0.0041 mmol) in $CH_2Cl_2$ (0.25 mL) at -50 °C was added dimethyldioxirane (0.1 mL, 0.0095 mmol) dropwise. The resulting solution was stirred at -50 °C for 1 h. The dimethyldioxirane and solvent were removed by a stream of $N_2$. The residue was purified by flash column chromatography (hexanes/ethyl acetate, 1:1) gave 0.0015 g (70%) of epothiolone B (2) as a pale yellow oil which was identical with an authentic sample in $^1$H NMR, IR, mass spectrum, and $[\alpha]_D$.

**Comparative Example 41**

**8-Desmethylepothilone A**

**Crotylation product:** To a stirred mixture of potassium *tert*-butoxide (1.0 M soln in THF, 50.4 mL, 50.4 mmol), THF (14 mL), and *cis*-2-butene (9.0 mL, 101 mmol) at -78°C was added *n*-BuLi (1.6 M, in hexanes, 31.5 mL, 50.4 mmol). After complete addition of *n*-BuLi, the mixture was stirred at -45°C for 10 min and then cooled to -78°C. (+)-*B*-Methoxydiisopinocampheylborane (19.21 g, 60.74 mmol) was then added dropwise in $Et_2O$ (10 mL). After 30 min, $BF_3 \cdot Et_2O$ (7.47 mL, 60.74 mmol) was added followed by aldehyde **4D** (9.84 g, 60.74 mmol) in THF (15 mL) generating a viscous solution which could not be stirred. The mixture was shaken vigorously every 10 min to ensure homogeneity. After 3 h at -78°C, the reaction was treated with 3N NaOH (36.6 mL, 110 mmol) and 30% $H_2O_2$ (15 mL) and the solution brought to reflux for 1 h. The reaction was poured into $Et_2O$ (300 mL) and washed with $H_2O$ (100 mL), brine (30 mL) and dried over anhydrous $MgSO_4$. The crude material was placed in a bulb-to-bulb distillation apparatus to remove the ligand from the desired product. Heating at 80°C at 2 mm Hg removed 90% of the lower boiling ligand. Further purification of the alcohol **4D** was achieved by flash chromatography on silica gel eluting with $Et_2O$ in $CH_2Cl_2$ (2% - 4%) to give pure alcohol **4D** as a clear oil. The erythro selectivty was >50:1 as judged by $^1$H NMR spectroscopy. The product was determined to be 87% ee by formation of the Mosher ester: IR (film): 3435, 2861, 1454, 1363, 1099 cm$^{-1}$; $^1$H NMR ($COCl_3$, 400 MHz) δ 7.34 (5 H, m), 5.80 (1 H, m), 5.09 (1 H, dd, *J* = 1.6, 8.3 Hz), 5.04 (1 H, d, *J* = 1.6 Hz), 4.52 (2 H, s), 3.51 (2 H, t, *J* = 5.8 Hz), 3.47 (1 H, m), 2.27 (2 H, m), 1.73 (3 H, m), 1.42 (1 H, m), 1.04 (3 H, d, *J* = 6.9 Hz); $^{13}$C NMR ($CDCl_3$, 100 MHz) δ 141.1, 138.2, 128.3, 127.6, 127.5, 115.0, 74.5, 72.9, 70.4, 43.7, 31.3, 26.5, 14.6.

**Comparative Example 42**

TBS ether **5D**: Alcohol **4D** (5.00 g, 21.4 mmol) was dissolved in $CH_2Cl_2$ (150 mL) and 2,6-lutidine (9.97 mL, 85.6 mmol) was added. The mixture was cooled to 0°C and TBSOTf (9.83 mL, 42.8 mmol) was slowly added. The reaction was then warmed to rt. After 1 h, the reaction was poured into $Et_2O$ (300 mL) and washed once with 1 N HCl (50 mL), once with sat $NaHCO_3$ (50 mL), once with brine (30 mL) and dried over anhydrous $MgSO_4$. Purification by flash chromatography on silica gel eluting with hexanes/diethyl ether (97:3) gave 6.33 g (85%) of pure olefin **5D** as a clearoil: IR (film): 1472, 1361, 1255, 1097, 1068 cm$^{-1}$; $^1$H NMR ($CDCl_3$, 400 MHz) δ 7.30 (5 H, m), 5.81 (1 H, m), 4.97 (1 H, dd, *J* = 1.4, 4.8 Hz), 4.94 (1 H, d, *J* = 1.1 Hz), 3.51 (1 H, q, *J* = 5.1 Hz), 3.41 (2 H, dt, *J* = 2.1, 6.6 Hz), 2.27 (1 H, q, *J* = 5.5 Hz), 1.68 (1 h, m), 1.55 (1 H, m), 1.41 (2 H, m), 0.93 (3 H, d, *J* = 6.9 Hz), 0.85 (9 H, s), -0.01 (6 H, s); $^{13}$C NMR ($COCl_3$, 100 MHz) δ 141.2, 138.6, 128.3, 127.6, 127.4, 113.9, 75.6, 72.7, 70.6, 42.7, 30.1, 25.9, 25.4, 18.1, 15.1, -4.3, -4.4.

**Comparative Example 43**

Aldehyde **6D**: The olefin 5 (4.00 g, 11.49 mmol) was dissolved in 1:1 MeOH/$CH_2Cl_2$ (100 mL). Pyridine (4.0 mL) was then added and the mixture cooled to -78°C. Ozone was then bubbled through the reaction for 10 minutes before the color turned light blue in color. Oxygen was then bubbled through the reaction for 10 min. Dimethyl sulfide (4.0 mL) was then added and the reaction slowly warmed to rt. The reaction was stirred overnight and then the volatiles were removed in vacuo. Purification by flash chromatography on silica gel eluting with hexanes/ethyl acetate (9:1)

gave 3.31 g (82%) of the aldehyde **6D** as a clear oil: IR (film): 2856, 1727, 1475, 1361, 1253, 1102 $cm^{-1}$; $^1H$ NMR ($COCl_3$, 400 MHz) 6 9.76 (1 H, s), 7.33 (5 H, m), 4.50 (2 H, s), 4.11 (1 H, m), 3.47 (2 H, m), 2.46 (1 H, m), 1.50-1.70 (4 H, band), 1.05 (3 H, d, *J* = 7.0 Hz), 0.86 (9 H, s), 0.06 (3 H, s), 0.03 (3 H, s); $^{13}C$ NMR ($CDCl_3$, 100 MHz) δ 204.8, 138.3, 128.2, 127.4, 127.3, 72.7, 71.7, 69.9, 51.1, 31.1, 25.9, 25.6, 17.8, 7.5, -4.4, -4.8.

## Comparative Example 44

Dianion addition product **7D**: The *tert*-butyl isobutyrylacetate (0.653 g, 3.51 mmol) was added to a suspension of NaH (60% in mineral oil, 0.188 g, 4.69 mmol) in THF (50 mL) at rt. After 10 min, the mixture was cooled to 0°C. After an additional 10 min, *n*-BuLi (1.6 M in hexanes, 2.20 mL, 3.52 mmol) was slowly added. After 30 min, the aldehyde **6D** (1.03 g, 2.93 mmol) was added neat. After 10 min, the reaction was quenched with $H_2O$ (10 mL) and extracted with $Et_2O$ (2 x 75 mL). The combined organics were washed once with brine (30 mL) and dried over anhydrous $MgSO_4$. The crude reaction mixture contained a 15:1 ratio of diastereomers at C5. Purification by flash chromatography on silica gel eluting with hexaneslethyl acetate (9:1 - 7:1) gave 0.723 g (47%) of the desired alcohol **7D** as a clear oil: IR (film): 3531, 2953, 1739, 1702, 1367, 1255, 1153 $cm^{-1}$; $^1H$ NMR ($CDCl_3$, 400 MHz) δ 7.33 (5 H, m), 4.49 (2 H, s), 3.75 (1 H, d, *J* = 2.6 Hz), 3.71 (1 H, m), 3.62 (1 H, d, *J* = 16.0 Hz), 3.53 (1 H, d, *J* = 16.0 Hz), 3.44 (2 H, t, *J* = 5.1 Hz), 2.70 (1 H, d, *J* = 2.6 Hz), 1.83 (1 H, m), 1.55 (4 H, m), 1.46 (9 H, s), 1.17 (3 H, s), 1.11 (3 H, s), 0.89 (9 H, s), 0.82 (3 H, d, *J* = 7.0 Hz), 0.09 (6 H, s); $^{13}C$ NMK ($CDCl_3$, 100 MHz) 6 208.9, 167.3, 138.4, 128.3, 127.6, 127.5,81.3, 79.5, 78.7, 72.8, 70.1, 52.4, 47.6, 35.8, 30.6, 28.2, 25.9, 25.8, 22.6, 20.5, 17.9, 7.05, -4.0, -4.5.

## Comparative Example 45

Directed reduction: To a solution of tetramethylammonium triacetoxyborohydride (1.54 g, 5.88 mmol) in acetonitrile (4.0 mL) was added anhydrous AcOH (4.0 mL). The mixture was stirred at rt for 30 min before cooling to -10°C. A solution of the ester **7D** (0.200 g, 0.39 mmol) in acetonitrile (1.0 mL) was added to the reaction and it was stirred at -10°C for 20 h. The reaction was quenched with 1 N sodium-potassium tartrate (10 mL) and stirred at rt for 10 min. The solution was then poured into sat $NaHCO_3$ (25 mL) and neutralized by the addition of solid $Na_2CO_3$. The mixture was then extracted with EtOAc (3 x 30 mL) and the organics were washed with brine (20 mL) and dried over anydrous $MgSO_4$. Purification by flash chromatography on silica gel eluting with hexanes/ethyt acetate (4:1) gave 0.100 g (50%) of the diol as 10:1 ratio of diastereomeric alcohols.

## Comparative Example 46

Monoprotection of the diol: The diol (1.76 g, 3.31 mmol) was dissolved in $CH_2Cl_2$ (100 mL) and cooled to 0°C. 2,6-lutidine (12.2 mL, 9.92 mmol) was added followed by TBSOTf (1.14 mL, 4.96 mmol) and the reaction slowly warmed to rt. After 1 h, the reaction was poured into $Et_2O$ (300 mL) and washed once with 1 N HCl (50 mL), once with sat $NaHCO_3$ (50 mL), once with brine (30 mL) and dried over anhydrous $MgSO_4$. Purification by flash chromatography on silica gel eluting with hexanes/ethyl acetate (20:1 - 15:1) gave 2.03 g (95%) of the alcohol 80 as a clear oil, which was used as a mixture of diastereomers.

## Comparative Example 47

C5 Ketone formation: The alcohol **8D** (2.03 g, 3.14 mmol) was dissolved in $CH_2Cl_2$ (50 mL) and Dess-Martin periodinane (2.66 g, 6.28 mmol) was added. After 2 h, a 1:1 mixture of sat'd $NaHCO_3$/sat $Na_2S_2O_3$ (20 mL) was added. After 10 min, the mixture was poured into $Et_2O$ (300 mL) and the organic layer was washed with brine (30 mL) and dried over anhydrous $MgSO_4$. Purification by flash chromatography on silica gel eluting with hexanes/ethyl acetate (15:1) gave 1.85 g (91%) of the ketone (benzyl ether) as a clear oil, which was used as a mixture of diastereomers.

## Comparative Example 48

Debenzylation: The ketone (benzyl ether) (1.85 g, 2.87 mmol) was dissolved in EtOH (50 mL), and $Pd(OH)_2$ (0.5 g) was added. The mixture was then stirred under an atmosphere of $H_2$. After 3 h, the reaction was purged with $N_2$ and then filtered through a pad of celite rinsing with $CHCl_3$ (100 mL). Purification by flash chromatography on silica gel eluting with ethyl acetate in hexanes (12% - 15%) gave 1.43 g (90%) of the diastereomeric alcohols as a clear oil. The C3 diastereomers were separated by flash chromatography on TLC-grade $SiO_2$ eluting with ethyl acetate in hexanes (15%):
Alpha isomer: IR (film): 3447, 1732, 1695, 1254, 1156 $cm^{-1}$; $^1H$ NMR ($CDCl_3$, 400 MHz) δ 4.24 (1 H, dd, *J* = 3.6, 5.8 Hz), 3.83 (1 H, m), 3.53 (1 H, m), 3.06 (1 H, t, *J* = 7.1 Hz), 2.36 (1 H, dd, *J* = 3.6, 17.2 Hz), 2.12 (1 H, dd, *J* = 3.9, 17.2

Hz), 1.68 (1 H, t, *J* = 5.4 Hz), 1.54 (2 H, m), 1.41 (1 H , m), 1.37 (9 H, s), 1.31 (1 H, m), 1.16 (3 H, s), 1.02 (3 H, s), 0.99 (3 H, d, *J* = 6.8 Hz), 0.84 (9 H, s), 0.81 (9 H, s), 0.05 (3 H, s), 0.01 (6 H, s), -0.01 (3 H, s); $^{13}C$ NMR ($CDCl_3$, 100 MHz) δ 217.7, 171.3, 80.57, 73.5, 73.1, 63.0, 53.4, 26.8, 41.2, 32.1, 28.1, 28.0, 26.0, 25.9, 23.1, 19.8, 18.1 (overlapping), 15.3, -4.0, -4.3 (overlapping), -4.8.

Beta isomer: IR (film): 3442, 2857, 1732, 1700, 1472, 1368, 1255 $cm^{-1}$; $^1H$ NMR ($CDCl_3$, 400 MHz) δ 4.45 (1 H, t, *J* = 5.3 Hz), 3.86 (1 H, m), 3.52 (2 H, q, *J* = 5.9 Hz), 3.01 (1 H, m), 2.28 (1 H, dd, *J* = 4.3, 17.1 Hz), 2.16 (1 H, dd, *J* = 5.5, 17.1 Hz), 1.67 (1 H, t, *J* = 5.6 Hz), 1.56 (2 H, m), 1.44 (1 H, m), 1.37 (9 H, s), 1.34 (1 H, m), 1.13 (3 H, s), 0.97 (3 H, d, *J* = 7.4 Hz), 0.96 (3 H, s), 0.83 (9 H, s), 0.79 (9 H, s), 0.01 (3 H, s), 0.00 (6 H, s), -0.07 (3 H, s); $^{13}C$ NMR ($CDCl_3$, 100 MHz) δ 217.1, 171.2, 80.6, 73.5, 72.1, 62.9, 63.9, 46.4, 41.2, 32.0, 28.1, 28.0, 26.0, 25.9, 21.5, 19.5, 18.2, 18.1, 15.8, -4.0, -4.3, -4.4, -4.7.

## Comparative Example 49

Aldehyde formation: DMSO (0.177 mL, 2.50 mmol) was added to a mixture of oxalyl chloride (0.11 mL, 1.25 mmol) in $CH_2Cl_2$ (15 mL) at -78°C. After 10 min, the alcohol (0.531 g, 0.96 mmol) was added in $CH_2Cl_2$ (4 mL). After 20 min, TEA (0.697 mL, 5.00 mmol) was added to the reaction followed by warming to rt. The reaction was then poured into $H_2O$ (50 mL) and extracted with $Et_2O$ (3 x 50 mL). The organics were washed once with $H_2O$ (30 mL), once with brine (30 mL) and dried over anhydrous $MgSO_4$. The aldehyde was used in crude form.

## Comparative Example 50

Wittig olefination to give **9D:** NaHMDS (1.0 M soln in THF, 1.54 mL, 1.54 mmol) was added to a suspension of methyl triphenylphosphonium bromide (0.690 g, 1.92 mmol) in THF (20 mL) at 0°C. After 1 h, the crude aldhyde (0.96 mmol) was added in THF (5 mL). After 15 min at 0°C, $H_2O$ (0.1 mL) was added and the reaction poured into hexanes (50 mL). This was filtered through a plug of silica gel eluting with hexanes/$Et_2O$ (9:1, 150 mL). The crude olefin **9D** was further purified by flash chromatography on silica gel eluting with ethyl acetate in hexanes (5%) to give 0.437 g (83% for two steps) of the olefin **9D** as a clear oil: IR (film): 2857, 1732, 1695, 1472, 1368, 1255, 1156 $cm^{-1}$; $^1H$ NMR ($CDCl_3$, 400 MHz) δ 5.72 (1 H, m), 4.91 (2 H, m), 4.25 (1 H, dd, *J* = 3.9, 5.4 Hz), 3.81 (1H, m), 3.05 (1 H, m), 2.38 (1H, dd, *J* = 7.9, 17.2 Hz), 2.12 (1 H, dd, *J* = 6.6, 17.2 Hz), 2.04 (2 H, q, *J* = 7.5 Hz), 1.47 (1 H, m), 1.39 (9 H, s), 1.34 (1 H, m), 1.20 (3 H, s), 1.00 (3 H, s), 3.00 (3 H, d, *J* = 6.7 Hz), 0.85 (9 H, s), 0.83 (9 H, s), 0.07 (3 H, s), 0.00 (6 H, s), -0.05 (3 H, s); $^{13}C$ NMR ($CDCl_3$, 100 MHz) δ 217.5, 172.1, 137.9, 114.0, 80.4, 74.0, 73.0, 53.0,46.9, 41.3, 35.1, 29.0, 28.1, 26.0, 25.9, 22.8, 20.2, 18.2 (overlapping), 14.9, -4.1, -4.2, -4.3, -4.8.

## Comparative Example 51

TBS ester 10D: The olefin **9D** (0.420 g, 0.76 mmol) was dissolved in $CH_2Cl_2$ (15 mL) and treated successively with 2,6-lutidine (1.33 mL, 11.4 mmol) and TBSOTf (1.32 mL, 5.73 mmol). After 7 h, the reaction was poured into $Et_2O$ (100 mL) and washed successively with 0.2N HCl (25 mL), brine (20 mL) and dried over anhydrous $MgSO_4$. The residue was purified by flash chromatography on a short pad of silica gel with fast elution with hexanes/ethyl acetate (20:1) to give the TBS ester **10D** as a clear oil. The purification must be done quickly to avoid hydrolysis of the silyl ester: IR (film): 2930, 1721, 1695, 1472, 1254, 1091 $cm^{-1}$; $^1H$ NMR ($CDCl_3$, 400 MHz) δ 5.73 (1 H, m), 4.91 (2 H, m), 4.25 (1 H, dd, *J* = 3.8, 5.4 Hz) 3.80 (1 H, q, *J* = 6.8 Hz), 3.06 (1 H, m), 2.50 (1 H, dd, *J* = 3.7, 17.3 Hz), 2.19 (1 H, dd, *J* = 5.7, 17.3 Hz), 2.04 (2 H, dd, *J* = 7.6, 15.3 Hz), 1.49 (1 H, m), 1.36 (1H, m), 1.21 (3 H, s), 1.00 (3 H, d, *J* = 6.8 Hz), 0.88 (9 H, s), 0.85 (9 H, s), 0.83 (9 H, s), 0.22 (3 H, s), 0.22 (3 H, s), 0.21 (3 H, s), 0.06 (3 H, s), 0.01 (6 H, s), -0.05 (3 H, s); $^{13}C$ NMR ($CDCl_3$, 100 MHz) δ 217.3, 172.3, 138.5, 114.4, 74.5, 73.0, 53.2, 46.9, 41.8, 35.1, 29.0, 26.0, 25.7, 25.5, 22.8, 20.4, 18.2, 18.1, 17.5, 14.9, -2.9, -4.0, -4.2, -4.3, -4.8, -4.9.

## Comparative Example 52

Suzuki coupling: The acetate acid **13D** was purified by flash chromatography on silica gel eluting with hexanes/ethyl acetate (7:1 - 4:1). This was further purified by preparative-TLC eluting with hexanes/ethyl acetate (2:1) to remove unreacted vinyl iodide **12D** from the acetate acid **13D**. Isolated yield of the acid was 0.297 g (62% based on 90% purity with borane residues).

## Comparative Example 53

Hydrolysis of acetate acid **13D:** The acetate **13D** (0.220 g, 0.297 mmol) was dissolved in MeOH/$H_2O$ (2:1, 15 mL) and $K_2CO_3$ (0.300 g) was added. After 3 h, the reaction was diluted with sat $NH_4Cl$ (20 mL) and extracted with

$CHCl_3$ (5 x 20 mL). The hydroxy-acid **14D** was purified by flash chromatography on silica gel eluting with hexanes/ethyl acetate (4:1 - 2: 1) to give 0.146 g (70%) of the pure hydroxy acid **14D**. IR (film): 3510-2400, 1712, 1694, 1471, 1254, 1093 $cm^{-1}$; $^1H$ NMR ($CDCl_3$, 400 MHz) δ 6.96 (1 H, s), 6.66 (1 H, s), 5.55 (1 H, m), 5.38 (1H, m), 4.38 (1 H, dd, *J* = 3.4, 6.1 Hz), 4.19 (1 H, t, *J* = 7.5 Hz), 3.84 (1 H, m), 3.05 (1 H, t, *J* = 7.0 Hz), 2.72 (3 H, s), 2.49 (1 H, dd, *J* = 3.2, 16.4 Hz), 2.42 (2 H, m), 2.33 (1 H, dd, *J* = 6.2, 16.4 Hz), 2.07 (2 H, m), 2.02 (3 H, s), 1.33 (4 H, m), 1.19 (3 H, s), 1.14 (3 H, s), 1.06 (3 H, d, *J* = 6.7 Hz), 0.89 (9 H, s), 0.88 (9 H, s), 0.11 (3 H, s), 0.07 (3 H, s), 0.04 (6 H, s); $^{13}C$ NMR ($CDCl_3$, 100 MHz) δ 217.8, 176.6, 164.9, 152.5, 141.7, 132.9, 125.0, 119.0, 115.3, 73.5, 73.3, 53.4,47.0, 40.1,35.8, 33.2, 29.8, 27.4, 26.0, 25.9, 24.5, 19.0, 18.1, 15.2, 14.3, -4.0, -4.2, -4.2, -4.7.

## Comparative Example 54

Macrolactonization: DCC (0.150 g, 0.725 mmol), 4-DMAP (0.078 g, 0.64 mmol) and 4-DMAP·HCl (0.110 g, 0.696 mmol) were dissolved in $CHCl_3$ (80 mL) at 80°C. To this refluxing solution was added by syringe pump the hydroxy acid **14D** (0.020 g, 0.029 mmol) and DMAP (0.010 g) in $CHCl_3$ (10 mL) over 20 h. The syringe needle was placed at the base of the condensor to ensure proper addition. After 20 h, the reaction was cooled to 50°C and AcOH (0.046 mL, 0.812 mmol) was added. After 2 h, the reaction was cooled to rt and washed with sat $NaHCO_3$ (30 mL), brine (30 mL) and dried over anhydrous $Na_2SO_4$. The lactone **15D** was purified by flash chromatography on silica gel eluting with hexanes/ethyl acetate (20:1-15:1) to give 0.014 g (75%): IR (film): 2929, 1741, 1696, 1254, 1097 $cm^{-1}$; $^1H$ NMR ($CDCl_3$, 400 MHz) δ 6.95 (1 H, s), 6.55 (1 H, s), 5.48 (1 H, m), 5.37 (1 H, m), 5.16 (1 H, d, *J* = 9.8 Hz), 4.17 (1 H, d, *J* = 8.3 Hz), 4.07 (1 H, t, *J* = 7.2 Hz), 3.02 (1 H, t, *J* = 7.2 Hz), 2.77 (1 H, m), 2.70 (3 H, s), 2.64 (2 H, m), 2.29 (1 H, m), 2.15 (1 H, m), 2.12 (3 H, s), 1.92 (1 H, m), 1.71 (1 H, m), 1.44 (2 H, m), 1.26 (1 H, m), 1.17 (3H, s), 1.12 (3 H, s), 1.11 (3 H, d, *J* = 7.0 Hz), 0.91 (9 H, s), 0.85 (9 H, s), 0.09 (3 H, s), 0.06 (6 H, s), -0.04 (3 H, s); $^{13}C$ NMR ($CDCl_3$, 100 MHz) δ 215.2, 171.9, 164.5, 152.5, 138.0, 133.5, 123.8, 120.0, 116.7, 79.4, 76.2, 72.5, 53.5, 47.4, 39.9, 34.5, 31.9, 31.5, 30.2, 27.7, 26.1, 25.9, 24.1, 23.8, 23.1, 22.6, 19.2, 18.5, 18.2, 16.3, 14.9, 14.1, -3.7, -4.2, -4.7, -5.2.

## Comparative Example 55

Desmethyldesoxyepothilone A **(16D)**: To the lactone **15D** (0.038 g, 0.056 mmol) in THF (2.0 mL) was added HF-pyridine (1.0 mL). After 2 h, the reaction was poured into sat $NaHCO_3$ (30 mL) and extracted with $CHCl_3$ (5 x 20 mL). The organics were dried over $Na_2SO_4$. The crude diol **16D** was purified by flash chromatography on silica gel eluting with hexanes/ethyl acetate (3:1 - 2:1) to give 0.023 g (89%): IR (film): 3501, 2933, 1734, 1684, 1290, 1248, 1045 $cm^{-1}$; $^1H$ NMR ($CDCl_3$, 400 MHz) δ 6.95 (1 H, s), 6.59 (1 H, s), 5.40 (2 H, m), 5.23 (1 H, dd, *J* = 1.4, 9.5 Hz), 4.38 (1 H, bd, *J* = 11.1 Hz), 3.78 (1 H, t, *J* = 6.9 Hz), 3.59 (1 H, bs), 3.47 (1 H, s), 2.99 (1 H, q, *J* = 7.0 Hz), 2.68 (3 H, s), 2.66 (1 H, m), 2.46 (1 H, dd, *J* = 11.4, 14.4 Hz), 2.26 (1 H, dd, *J* = 2.2, 14.4 Hz), 2.22 (1 H, m), 2.06 (3 H, s), 1.96 (1 H, m), 1.49 (3 H, m), 1.35 (3 H, m), 1.30 (3 H, s), 1.15 (3 H, d, *J* = 6.9 Hz), 1.06 (3 H, s); $^{13}C$ NMR ($CDCl_3$, 100 MHz) δ 221.5, 170.3, 165.1, 151.8, 139.1, 132.8, 125.2, 119.1, 115.5, 78.4, 72.5, 70.8, 53.8, 42.7, 39.6, 32.3, 31.8, 28.3, 26.8, 24.8, 23.1, 19.0, 17.2, 16.0, 11.1.

## Comparative Example 56

Epoxide formation: Diol **16D** (0.008 g, 0.017 mmol) was dissolved in $CH_2Cl_2$ (1.0 mL) and cooled to -60°C. Dimethyldioxirane (0.06 M, 0.570 mL, 0.0034 mmol) was then slowly added. The reaction temperature was slowly warmed to -25°C. After 2 h at -25°C, the volatiles were removed from the reaction at -25°C under vacuum. The resulting residue was purified by flash chromatography on silica gel eluting with MeOH in $CH_2Cl_2$ (1% - 2%) to give a 1.6:1 mixture of cis -epoxides **3D** and the diastereomeric cis-epoxide (0.0058 g, 74%). The diastereomeric epoxides were separated by preparative-TLC eluting with hexanes/ethyl acetate (1:1) after 3 elutions to give pure diastereomers:

Beta epoxide **3D**: IR (film): 3458, 2928, 1737, 1685, 1456, 1261, 1150, 1043, 1014 $cm^{-1}$; $^1H$ NMR ($CD_2Cl_2$, 500 MHz) δ 7.01 (1 H, s), 6.56 (1 H, s), 5.35 (1 H, dd, *J* = 2.3, 9.6 Hz), 4.30 (1 H, dd, *J* = 3.0, 5.7 Hz), 3.85 (1 H, m), 3.81 (1 H, d, *J* = 5.7 Hz), 3.42 (1 H, d, *J* = 2.0 Hz), 3.03 (1 H, q, *J* = 6.8 Hz), 2.97 (1 H, m), 2.88 (1 H, m), 2.67 (3 H, s), 2.46 (1 H, dd, *J* = 9.0, 14.5 Hz), 2.33 (1 H, dd, *J* = 2.6, 14.5 Hz), 2.13 (1 H, dt, *J* = 3.0, 15.0 Hz), 2.08 (3 H, s), 1.82 (1 H, m), 1.52 (6 H, m), 1.41 (1 H, m), 1.33 (3 H, s), 1.21 (4 H, m), 1.12 (3 H, d, *J* = 7.0 Hz), 1.06 (3 H, s); $^{13}C$ NMR ($CD_2Cl_2$, 125 MHz) δ 221.9, 170.6, 165.6, 152.2, 138.3, 120.2, 116.6, 77.3, 73.4, 69.9, 57.7, 55.3, 43.7, 39.7, 32.6, 32.0, 29.8, 27.2, 25.7, 24.7, 22.5, 19.2, 19.0, 15.6, 15.6, 11.5;

Alpha epoxide: IR (film): 3439, 2918, 1735, 1684, 1455, 1262, 1048, 1014 $cm^{-1}$; $^1H$ NMR ($CD_2Cl_2$, 500 MHz) δ 7.02 (1 H, s), 6.56 (1 H, s), 5.62 (1 H, d, *J* = 8.1 Hz), 4.33 (1 H, dd, *J* = 2.7, 11.0 Hz), 3.85 (1 H, t, *J* = 5.9 Hz), 3.27 (1 H, d, *J* = 5.3 Hz), 3.11 (1 H, m), 3.07 (1 H, d, *J* = 7.0 Hz), 3.04 (1 H, s), 2.87 (1 H, m), 2.68 (3 H, s), 2.46 (1 H, dd, *J* = 11.1, 14.1 Hz), 2.35 (1 H, dd, *J* = 2.3, 14.1 Hz), 2.11 (3 H, s), 2.06 (1 H, ddd, *J* = 1.9, 4.5, 15.1 Hz), 1.87 (1 H, m), 1.52 (6 H, m), 1.38 (2 H, m), 1.29 (3 H, s), 1.08 (3 H, d, *J* = 6.9 Hz), 1.03 (3 H, s); $^{13}C$ NMR ($CD_2Cl_2$, 125 MHz) δ 222.1, 170.2,

165.3, 152.5, 137.6, 119.7, 116.7, 76.7, 72.9, 70.6, 57.1, 55.1, 44.7, 40.0, 32.1, 31.4, 30.0, 26.6, 25.5, 24.7, 21.3, 19.3, 18.7, 15.7, 11.5.

**Comparative Example 57**

Experimental Data for C-12 Hydroxy Epothilone Analogs

Propyl hydroxy compound **43:** $^1$H NMR ($CDCl_3$, 400 MHz) δ 6.96 (1 H, s), 6.59 (1 H, s), 5.16-5.23 (2 H, band), 4.28 (1 H, m), 3.72 (1 H, m), 3.63 (2 H, t, J = 6.3 Hz), 3.17 (1 H, dq, J = 2.2, 0.5 Hz), 3.02 (1 H, s), 2.70 (3 H, s), 2.65 (2 H, m), 2.46 (1 H, dd, J = 10.9, 14.6 Hz), 2.29 (2 H, m), 1.98-2.09 (6 H, band), 1.60-1.91 (6 H, band), 1.35 (3 H, s), 1.33 (3 H, s), 1.18 (3 H, d, J = 6.8 Hz), 1.07 (3 H, s), 1.01 (3 H, d, J = 7.1 Hz); $^{13}$C NMR ($CDCl_3$, 100 MHz) δ 220.69, 170.29, 165.00, 151.81, 141.63, 138.93, 120.64, 118.81, 115.52, 78.53, 77.23, 73.93, 71.85, 62.26, 53.63, 41.57, 39.54, 37.98, 32.33, 32.14, 31.54, 30.75, 29.67, 25.27, 22.89, 18.92, 17.67, 15.98, 15.74, 13.28; MS e/m 536.2, calc 535.29.

Hydroxy methyl compound **46:** $^1$H NMR ($CDCl_3$, 400 MHz)δ 6.97 (1 H, s), 6.63 (1 H, s), 5.43 (1 H, dd, J = 5.7, 9.1 Hz), 5.24 (1 H, d, J = 7.4 Hz), 4.31 (1 H, d, J = 9.7 Hz), 4.05 (2 H, dd, J = 7.3, 31.0 Hz). 3.87 (1 H, bs), 3.69 (1 H, bs), 3.17 (1 H, dd, J = 2.0, 6.9 Hz), 3.03 (1 H, s), 2.69 (3 H, s), 2.63 (1 H, m), 2.45 (1 H, dd, J = 11.2, 14.6 Hz), 2.37 (1 H, m), 2.25 (2 H, m), 2.11 (1 H, m), 2.05 (3 H, s), 1.78 (1 H, m), 1.70 (1 H, m), 1.35 (3 H, s), 1.34 (2 H, m), 1.29 (1 H, m), 1.18 (3 H, d, J = 6.8 Hz), 1.06 (3 H, s), 1.00 (3 H, d, J = 7.0 Hz); $^{13}$C NMR ($CDCl_3$, 100MHz) δ 220.70, 170.16, 165.02, 151.63, 141.56, 138.41, 121.33, 118.65, 115.33, 77.74, 77.25, 74.11, 71.37, 65.75, 53.86, 41.52, 39.52, 37.98, 31.46, 27.70, 25.10, 22.86, 18.74, 17.20, 16.17, 15.63, 13.41.

**Comparative Example 58**

4,4-Dimethyl-3,5-dioxoheptanoate, tert-butyl ester **47**. t-Butyl 4-methyl-3-oxo- 4-methyl pentanoate (22.5 g, 121 mmol) was added dropwise in 20 mL of dry THF to a slurry of NaH (6.29 g, 60% dispersion in mineral oil, 157.2 mmol) in 500 mL of dry THF. The reaction mixture was stirred at 0 °C for 30 min and then the cold bath was cooled to -50 °C. Freshly distilled propionyl chloride (12.3 g, 133.0 mmol) was added rapidly (neat) via syringe to the cold solution. The reaction was monitored by TLC and the cold bath was maintained below -30 °C until the reaction was complete- After 1 hr, the reaction was quenched by pouring into a solution of saturated aqueous $NH_4Cl$ and subjected to an aqueous workup. Flash column chromatography with 2% EtOAc/hexanes afforded the desired tricarbonyl **42** (16.4 g, 67.8 mmol) in 56% yield; $^1$H NMR (400 MHz, $CDCl_3$): δ 12.43 (s, 0.20H), 5.07 (s, 0.20H), 3.36 (s, 1.6H), 2.47 (q, J = 7.13 Hz, 2H), 1.44 (s, 9H), 1.35 (s, 6H), 1.03 (t, J = 7.18 Hz, 3H); $^{13}$C NMR (100 MHz, $CDCl_3$): δ 209.7, 202.9, 166.1, 81.91, 62.53, 43.34, 31.67, 27.82 (3), 21.03 (2), 7.82; IR (neat) 3411.8, 1742.6, 1718.5, 1702.0, 1644.2, 1460.6, 1156.1 $cm^{-1}$.

4,4-Dimethyl-5-oxo-3-triethylsilyloxy-2-hentenoate, tert-butyl ester **48**. The ester **47** (5.79 g, 23.9 mmol) in THF was added to a suspension of NaH (60% in mineral oil, 1.24 g, 31.1 mmol) in THF (200 mL) at 0 °C. After 20 min, the reaction was cooled to -50 °C and TESOTf (5.95 mL, 26.32 mmol) was added. After an additional 20 min, the reaction was poured into saturated aqueous $NaHCO_3$ (300 mL). This mixture was extracted with $Et_2O$ (2 x 200 mL) and the combined organic layers were dried over anhydrous $MgSO_4O$. The resulting oil was purified by flash column chromatography on SiO2 eluting with $Et_2O$/hexanes (1:20 to 1:15) to give 6.65 g (78%) of the desired enol ether 48 as a clear oil; $^1$H NMR (400 MHz, $CDCl_3$): δ 5.16 (s, 1H), 2.48 (q, J = 7.2 Hz, 2H), 1.45 (s, 9H), 1.24 (s, 6H), 1.02 (t, J = 7.2 Hz, 3H), 0.95 (t, J = 8.1 Hz, 9H), 0.74 (q, J = 8.1 Hz, 6H); $^{13}$C NMR (100 MHz, $CDCl_3$): 6 211.2, 169.4, 165.1, 97.77, 78.93, 55.54, 30.33, 28.21, 22.94, 8.15, 6.78, 6.02; IR (neat) 1712, 1619, 1384, 1243, 1150 $cm^{-1}$.

**Comparative Example 59**

(6R,7R,8S)-7-Hydroxy-5-oxo-4,4,6,8-tetramethyl-3-triethylsilyloxy-2,10-undecadienoate, *tert*-butyl ester **49**. The keto enol ether **48** (7.80 g, 22.0 mmol) in 175 ml of dry THF was cooled to -30 °C in a cold bath ($CO_2$(s)/$CH_3CN$) and then, a cooled solution of LDA (27.2 mmol, 0.90 M in THF) was added dropwise via syringe over 5 min. Immediately after the addition of the keto enol ether, the reaction vessel was placed in a -120 °C cold bath (N2(liq)/pentane) and the reaction mixture was stirred for 10 min. Then, the aldehyde (2.0 g, 20.0 mmol) was added via syringe in 1 ml of dry THF. The reaction was complete after 30 min and was quenched by pouring into a solution of saturated aqueous $NaHCO_3$. The desired aldol product **49** (6.0 g, 13.2 mmol) was isolated in 60% yield (yield of the major product of a 5.5:1 mixture of diastereomers, epimeric at C-8) after flash column chromatography with 6-8% $Et_2O$/hexanes; (major diastereomer, higher Rf); $^1$H NMR (400 MHz, $CDCl_3$): δ 5.78 (m, 1H), 5.22 (m, 1H), 5.05 (m, 2H), 3.37 (m, 2H), 3.18 (q, J = 7.08 Hz, 1H), 2.52 (m, 1H), 1.85 (dt, J = 14.0,8.37 Hz, 1 H), 1.62 (m, 1H), 1.55 (s, 3H), 1.46 (s, 9H), 1.22 (s, 3H), 1.25 (s, 3H), 1.04 (d, J = 6.90 Hz, 3H), 0.95 (t, J = 7.94 Hz, 6H), 0.76 (q, J = 8.26 Hz, 9H); $^{13}$C NMR (100 MHz,

$CDCl_3$): δ 217.8, 168.1, 164.6, 137.1, 116.2, 99.03, 79.21, 74.82, 56.74, 40.65, 37.39, 35.06, 28.24, 22.53, 22.29, 14.77, 10.54, 6.95, 6.04; (minor diastereomer, lower Rf) $^1H$ NMR (400 MHz, $CDCl_3$): δ 5.73 (m, 1 H), 5.23 (s, 1 H), 5.02 (m, 2H), 3.43 (d, J = 8.74 Hz, 1H), 3.21 (m, 2H), 2.06 (m, 1H), 1.84 (m, 1H), 1.62 (m, 1H), 1.55 (s, 3H), 1.46 (s, 9H), 1.27 (s, 3H), 1.24 (s, 3H), 1.06 (d, J = 6.91 Hz, 3H), 0.96 (t, J = 8.06 Hz, 9H), 0.77 (q, J = 7.53 Hz, 6H); $^{13}C$ NMR (100 MHz, $CDCl_3$): δ 217.8, 168.5, 165.0, 136.8, 116.8, 99.46, 79.62, 75.47, 57.17, 41.43, 37.86, 35.58, 30.70, 28.66, 28.31, 22.90, 22.74, 16.53, 11.77, 7.37, 6.44.

## Comparative Example 60

(6R,7R,8S)-7-Trichloroethoxyethylcarbonate-3,5-dioxo-4,4,6,8-tetramethyl-10-und ecenoate, tert-butyl ester **50**. The alcohol **49** (1.61 g, 3.55 mmol) was dissolved in 20 ml of dry $CH_2Cl_2$ and cooled to 0 °C in an ice bath. Then, pyridine (1.12 g, 14.2 mmol) and trichloroethoxyethylcarbonoyl chloride (TrocCl) (1.50 g, 7.10 mmol) were added via syringe in that order. The reaction was stirred at 0 °C for 5 min and then the ice bath was removed and the reaction was allowed to come to rt and stir for 30 minutes. After this period of time, TLC analysis showed the complete consumption of the starting material. The reaction mixture was cooled to 0 °C and the TES enol ether was hydrolyzed by the addition of 20 mL of 0.5 M methanolic HCl. The reaction mixture was stirred for 5 min at 0 °C and then quenched by pouring into a solution of saturated aqueous $NaHCO_3$. The desired tricarbonyl **50** (1.54 g, 3.01 mmol) was isolated after an aqueous workup and flash column chromatography with 7-9% $Et_2O$/hexanes; $^1H$ NMR (400 MHz, $CDCl_3$): δ 12.63 (s, 0.25H), 5.70 (m, 1H), 5.15 (s, 0.25H), 5.08-4.88 (m, 2H), 4.91 (dd, J = 6.60, 5.01 Hz, 0.30H), 4.78 (m, 1H), 4.77 (dd, J = 7.86, 3.58 Hz, 0.70H), 4.72 (dd, J = 11.8, 9.66 Hz, 1H), 3.48 (d, J = 16.2 Hz, 0.75H), 3.42 (d, J = 16.2 Hz, 0.75H), 3.36 (m, 0.30H), 3.30 (m, 0.70H), 1.88 (m, 2H), 1.50 (s, 3H), 1.46 (s, 9H), 1.39 (s, 3H), 1.12 (d, J = 6.88 Hz, 0.70H), 1.10 (d, J = 6.88 Hz, 1.3H), 0.93 (d, J = 6.63 Hz, 1.3H), 0.88 (d, J = 6.86 Hz, 0.70H); $^{13}C$ NMR (100 MHz, $CDCl_3$): δ 210.5, 209.5, 203.16, 178.3, 172.6, 166.2, 154.1, 135.9, 135.6, 117.2, 116.9, 94.69, 94.56, 90.69, 82.68, 81.98, 81.65, 81.53, 63.58; 54.34, 46.56, 41.99, 41.62, 36.41, 35.84, 34.49, 34.44, 31.56, 28.23 (3), 27.94 (3), 22.62, 22.08, 21.56, 20.80, 15.95, 15.58, 14.09, 13.02, 12.98, 11.35; IR (neat) 1757.9, 1718.9, 1700.2, 1642.2, 1620.7, 1250.6, 1156.3 $cm^{-1}$.

## Comparative Example 61

TBS vinyl iodide **51**. n-BuLi (1.6 M in hexanes, 7.69 mL, 12.3 mmol) was added to a suspension of ethyl triphenylphosphonium iodide (5.15 g, 12.3 mmol) in THF (50 mL) at 25 °C. After 20 min, the clear red solution was transferred dropwise via syringe to a vigorously stirred solution of $I_2$ (3.12 g, 12.3 mmol) in THF (100 mL) at -78 °C. The-resulting pale yellow suspension was stirred rapidly and warmed to 20 °C. Then, NaHMDS (1.0 M soln in THF, 12.3 mL, 12.3 mmol) was added dropwise via syringe. During the addition of the NaHMDS, the reaction mixture changed from a yellow-orange slurry and to bright red solution. The TBS aldehyde (D.-S. Su *et al*., *Angew.Chem.Int.Ed.Engl*., **1997,** *36, 757*; 2.00 g, 6.15 mmol) was then added in THF. After 30 min, the reaction mixture was poured into hexanes (100 mL) and $H_2O$ (0.5 mL) was added. The solution was then passed through a plug of SiO2 eluting with 2:1 hexanes/ $Et_2O$. The iodide was purified by flash chromatography on SiO2 eluting with hexanes/ethyl acetate (20:1 to 15:1) to give the vinyl iodide 51 (1.46 g, 55%) as a yellow oil: $^1H$ NMR (400 MHz, $COCl_3$): δ 6.95 (s, 1 H), 6.50 (s, 1 H), 5.45 (dt, J = 1.5, 6.8 Hz, 1H), 4.22 (t, J = 6.4 Hz, 1H), 2.73 (s, 3H), 2.48 (s, 3H), 2.39 (m, 2H), 2.02 (s, 3H), 0.90 (s, 9H), 0.06 (3, s), 0.02 (3, s); $^{13}C$ NMR (100 MHz, $COCl_3$): δ 164.86, 153.46, 142.17, 132.54, 119.23, 115.68, 102.79, 77.70, 44.40, 39.09, 26.35, 19.65, 18.63, 14.54, -4.59, -4.84; IR (neat) 2928, 1470, 1252, 1068 $cm^{-1}$.

Post-Suzuki, C-15 Hydroxy Tricarbonyl **52**. 9-BBN (0.5 M soln in THF, 6.68 mL, 3.34 mmol) was added over a 45 min period to a solution of the olefin **50** (1.43 g, 2.78 mmol) in THF (15 mL) at 25°C. After 2 h, TLC analysis revealed the complete consumption of the starting olefin. In a separate flask, containing the vinyl iodide **51** (1.20 g, 2.80 mmol) and DMF (20 mL), were added successively and with vigorous stirring: $Cs_2CO_3$ (1.82 g. 5.60 mmol), Pd $(dppf)_2Cl_2$ (0.454 g, 0.56 mmol), $AsPh_3$ (0.171 g, 0.56 mmol) and $H_2O$ (1.82 mL, 0.1 mmol). Then the borane solution, prepared above, was added rapidly to the vigorously stirred solution containing the vinyl iodide. After 2 h, the reaction was complete and the reaction mixture was poured into $Et_2O$ (300 ml) and washed with $H_2O$ (3 x 200 mL), brine (1 x 50 mL) and dried over anhydrous $MgSO_4$. This crude product was purified by flash column chromatography on $SiO_2$ eluting with hexanes/ethyl acetate (18:1 to 13:1 to 10: 1) to afford the TBS protected coupled product as an impure mixture which was taken on to the next step.

The crude TBS protected coupled product (- 2.78 mmol) was dissolved in 0.36 N HCl in MeOH (30 mL) at 25 °C. After 3.5 h, the mixture was poured into a solution of saturated aqueous $NaHCO_3$ and extracted with $CHCl_3$ (4 x 60 mL). The combined organic layers were washed once with brine (50 mL) and dried over anhydrous $Na_2SO_4$. The diol was purified by flash column chromatography on $SiO_2$ eluting with hexanes/ethyl acetate (4:1 to 3:1 to 2:1) to give the pure product **52** as a clear oil (0.910 g, 46% for two steps): $^1H$ NMR (400 MHz, $CDCl_3$): δ 6.96 (s, 1H), 6.56 (s, 1H), 5.16 (t, J = 6.9 Hz, 1H), 4.83 (d, J = 11.9 Hz, 1H), 4.75 (dd, J = 3.4, 8.0 Hz, 1H), 4.70 (d, J = 11.9 Hz, 1H), 4.14

(t, J = 6.4 Hz, 1H), 3.45 (q, J = 13.2 Hz, 2H), 3.32 (m, 1H), 2.72 (s, 3H), 2.32 (t, J = 6.5 Hz, 2H), 2.04 (s, 3H), 2.01 (m, 2H), 1.74 (m, 2H), 1.69 (s, 3H), 1.45 (s, 9H), 1.38 (s, 6H), 1.09 (d, J = 6.9 Hz, 3H), 0.93 (d, J = 6.9 Hz, 3H); $^{13}C$ NMR (100MHz, $CDCl_3$): δ 209.51, 203.04, 166.15, 164.39, 154.14, 152.72, 141.71, 138.24, 120.70, 118.76, 115.28, 94.54, 81.85, 77.31, 76.57, 63.41, 54.16, 46.47, 41.48, 34.56, 33.95, 31.98, 31.53, 27.85, 24.85, 23.45, 21.47, 20.75, 19.04, 15.60, 14.33, 11.35; IR (neat) 3546, 3395, 1756, 1717, 1699, 1644, 1621, 1506, 1456, 1251 $cm^{-1}$.

**Comparative Example 62**

Noyori C-3/C-15 Diol Product **53**. The diketone 52 (0.900 g, 1.27 mmol) was dissolved in 0.12 N HCl in MeOH (10 mL) at 25 °C. The RuBINAP catalyst (0.018 M in THF, 1.0 ml, 0.018 mmol) was then added and the mixture transferred to a Parr apparatus. The vessel was purged with $H_2$ for 5 min and then pressurized to 1200 psi. After 12 h at 25 °C, the reaction was returned to atmospheric pressure and poured into a saturated solution of $NaHCO_3$. This mixture was extracted with $CHCl_3$ (4 x 50 mL) and the combined organic layers were dried over anhydrous $Na_2SO_4$. The product was purified by flash column chromatography on silica gel eluting with hexanes/ethyl acetate (4:1 to 2:1) to give 0.75 g (81 %) of the hydroxy ester 53 as a white foam; $^1H$ NMR (400 MHz, $CDCl_3$): δ 6.94 (s, 1H), 6.55 (s, 1H), 5.15 (t, J = 6.9 Hz, 1H), 4.85 (t, J = 5.3 Hz, 1H), 4.81 (d, J = 12.0 Hz, 1H), 4.71 (d, J = 12.0 Hz, 1H), 4.12 (m, 2H), 3.43 (m, 2H), 2.70 (s, 3H), 2.37 (dd, J = 2.2, 6.2 Hz, 1H), 2.30 (t, J = 6.7 Hz, 2H), 2.24 (dd, J = 10.6, 16.2 Hz, 1H), 2.03 (s, 3H), 1.99 (m, 2H), 1.68 (s, 3H), 1.44 (s, 9H), 1.18 (s, 3H), 1.16 (s, 3H), 1.09 (d, J = 6.8 Hz, 3H), 0.94 (d, J = 6.8 Hz, 3H); $^{13}C$ NMR (100 MHz, $CDCl_3$): δ 215.95, 172.39, 164.39, 154.21, 152.74, 141.70, 138.33, 120.59, 118.77, 115.27, 94.64, 82.98, 81.26, 76.51, 72.78, 51.82, 41.40, 37.36, 34.66, 33.96, 32.08, 31.10, 30.20, 27.96, 25.06, 23.45, 21.73, 21.07, 19.17, 19.01, 16.12, 15.16, 14.33, 12.17; IR (neat) 3434.0, 1757.5, 1704.5, 1249.9, 1152.8 $cm^{-1}$.

**Comparative Example 63**

C-3/C-15 Bis(TES) Carboxylic Acid **54**. 2,6-Lutidine (0.48 g, 4.5 mmol) and TESOTf (0.59 g, 2.25 mmol) were added successively to a cooled solution of the diol 53 (164 mg, 0.225 mmol) in $CH_2Cl_2$ (2.5 mL) at -78 °C. The reaction mixture was stirred at -78 °C for 5 min and then warmed to rt. The reaction was stirred at rt for 6 hr and then quenched with saturated aqueous $NH_4Cl$ and subjected to an aqueous workup. The crude product was concentrated in vacuo and subjected directly to the next set of reaction conditions; $^1H$ NMR (400 MHz, $CDCl_3$): δ 6.96 (s, 1H), 6.66 (s, 1H), 5.04 (t, J = 6.93 Hz, 1H), 4.90 (d, J = 12.0 Hz, 1H), 4.77 (dd, J = 7.99, 3.21 Hz, 1H), 4.66 (d, J = 12.0 Hz, 1H), 4.46 (m, 1H), 4.10 (dq, J = 12.3, 7.11 Hz, 2H), 3.42 (m, 1H), 2.70 (s, 3H), 2.60 (dd, J = 16.7, 2.34 Hz, 1H), 2.34 (dd, J = 16.7, 7.94 Hz, 1H), 2.27 (dd, J = 14.0, 6.97 Hz, 1H), 2.18 (m, 1H), 2.09 (m, 1H), 2.04 (s, 1H), 1.95 (s, 3H), 1.82 (m, 2H), 1.61 (s, 3H), 1.44 (m, 2H), 1.27-1.22 (m, 4H), 1.14 (d, J = 8.45 Hz, 3H), 1.11 (d, J = 6.81 Hz, 2H), 1.04 (d, J = 6.88 Hz, 2H), 1.15-1.01 (m, 2H), 0.94 (t, J = 7.92 Hz, 18H), 0.65-0.57 (m, 12H); $^{13}C$ NMR (100 MHz, $COCl_3$): δ 215.11, 175.34, 165.00, 154.14, 152.80, 142.60, 136.84, 121.31, 118.79, 114.60, 94.77, 81.60, 79.06, 76.64, 73.87, 54.19, 41.18, 39.56, 35.09, 34.52, 32.29, 31.95, 24.76, 23.62, 22.55, 18.95, 18.64, 15.87, 13.69, 11.33, 6.94, 6.83, 5.07, 4.76; IR (neat) 3100-2390, 1756.8, 1708.8, 1459.3, 1250.6, 816.1 $cm^{-1}$.

**Comparative Example 64**

C-15 Hydroxy Acid for Macrolactonization **55**. The crude bis(triethylsilyl)ether 54, prepared above, was dissolved in 5 mL of dry THF and then cooled to 0 °C. Then, 1 mL of 0.12 M HCl/MeOH was added. The reaction mixture was stirred at 0 °C for 20 min and then checked for completion. TLC analysis at this time revealed the complete consumption of starting material. The reaction was quenched by pouring into a solution of saturated aqueous $NaHCO_3$ and subjected to an aqueous workup. Flash column chromatography with 25 to 30:1 $CHCl_3$/MeOH afforded the desired carboxylic acid **55** in 77% yield; $^1H$ NMR (400 MHz, $CDCl_3$): δ 6.96 (s, 1H), 6.69 (1, s), 5.11 (t, J = 6.9 Hz, 1 H), 4.91 (d, J = 12.0 Hz, 1H), 4.71 (dd, J = 3.1, 8.2 Hz, 1 H), 4.64 (d, J = 12.0 Hz, 1H), 4.42 (d, J = 5.9 Hz, 1 H), 4.10 (m, 1H), 3.43 (m, 1H), 2.71 (s, 3H), 2.57 (dd, J = 2.1, 10.5 Hz, 1H), 2.25 (m, 3H), 2.11 (m, 1H), 1.98 (s, 3H), 1.95 (m, 2H), 1.72 (m 1), 1.67 (s, 3H), 1.45 (m, 2H), 1.16 (s, 3H), 1.13 (s, 3H), 1.09 (d, J = 6.7 Hz, 3H), 0.99 (d, J = 6.7 Hz, 3H), 0.95 (t, J = 7.9 Hz, 9H), 0.64 (dq, J = 2.3, 7.9 Hz, 6H); $^{13}C$ NMR (100 MHz, $CDCl_3$): δ 215.11, 176.00 (165.10, 154.18, 152.35, 142.24, 138.55, 120.74, 118.21, 115.02, 94.76, 81.91, 76.86, 76.63, 73.95, 54.08, 41.28, 39.64, 34.73, 34.16, 32.02, 31.67, 24.71, 23.41, 22.49, 19.17, 18.62, 15.71, 14.86, 11.20, 6.93, 5.05); IR (neat) 3400-2390, 1755.9, 1703.8, 1250.4, 735.4 $cm^{-1}$.

**Comparative Example 65**

C-3 Triethylsilyl/C-7 Trichloroethoxyethylcarbonate Macrolactonization Product **56**. Triethylamine (155 mg, 1.53 mmol) and 2,4,6-trichlorobenzoyl chloride (312 mg, 1.28 mmol) were added to a solution of the hydroxy acid 55

(198 mg, 0.256 mmol) in 3.6 mL of dry THF. The reaction mixture was stirred for 0.25 h at rt and then diluted with 45 mL of dry toluene. The resultant solution was added slowly dropwise, via syringe pump, over 3 hr to a stirred solution of DMAP (328 mg, 2.68 mmol) in 145 mL of dry toluene. After the addition of the substrate was complete, the reaction was stirred for an additional 0.5 h and then taken up in an equal volume of $Et_2O$ and washed with 1N HCl (1x), saturated aqueous $NaHCO_3$ (1x), and brine (1x). The organic layer was dried over $MgSO_4$ and concentrated in vacuo. Flash column chromatography of the crude product with 10% EtOAc/hexanes afforded the desired macrolactone (153 mg, 0.20 mmol) in 78% yield; $^1H$ NMR (400 MHz, $CDCl_3$): δ 6.96 (s, 1 H), 6.53 (s, 1H), 5.20 (m, 2H), 5.04 (d, J = 10.2 Hz, 1H), 4.84 (d, J = 12.0 Hz, 1H), 4.78 (d, J = 12.0 Hz, 1H), 4.07 (m, 1H), 3.32 (m, 1H), 2.86-2.63 (m, 3H), 2.70 (s, 3H), 2.48 (m, 1H), 2.11 (s, 3H), 2.04 (dd, J = 6.17, 14.7 Hz, 1H), 1.73 (m, 4H), 1.66 (s, 3H), 1.25 (m, 2H), 1.19 (s, 3H), 1.15 (s, 3H), 1.12 (d, J = 6.68 Hz, 3H), 1.01 (d, J = 6.83 Hz, 3H), 0.89 (t, J = 8.00 Hz, 9H), 0.58 (q, J = 7.83 Hz, 6H); $^{13}C$ NMR (100 MHz, $CDCl_3$): δ 212.75, 170.66, 164.62, 154.60, 152.52, 140.29, 138.44, 119.81, 119.38, 116.28, 94.84, 86.44, 80.14, 76.59, 76.10, 53.55, 45.89, 39.23, 35.47, 32.39, 31.69, 31.57, 31.16, 29.68, 27.41, 25.00, 23.44, 22.94, 19.23, 18.66, 16.28, 14.83, 6.89, 5.22; IR (neat) 1760.5, 1742.6, 1698.0, 1378.8, 1246.2, 1106.0, 729.8 $cm^{-1}$.

## Comparative Example 66

Sml, Mediated Deprotection of Troc Group **57**. Samarium metal (0.334 g, 2.22 mmol) and iodine (0.51 g, 2.0 mmol) in 25 mL of dry, deoxygenated THF were stirred together vigorously for 2.5 hr at ambient temperature. During this period of time, the reaction mixture progressed from a dark orange to an olive green to deep blue color. The resultant deep blue solution of Sml2 was used directly in the following reaction. Sml2 (25 mL of a 0.08 M stock solution, 2.0 mmol) was added rapidly via syringe to a stirred solution of the macrolactone 57 (200 mg, 0.26 mmol) and a catalytic amount of $NiI_2$ (10 mg) in 10 mL of dry THF at-78 °C. The resultant deep blue solution was maintained at -78 °C with continued vigorous stirring for 2.5 hr. TLC analysis at this time revealed the complete consumption of the starting material and formation of a single, lower Rf product. The reaction mixture was quenched with saturated aqueous $NaHCO_3$ and subjected to an aqueous woricup. Flash column chromatography with 25% EtOAc/hexanes afforded the desired alcohol 57 (143 mg, 0.24 mmol) in 91 % yield; $^1H$ NMR (400 MHz, $CDCl_3$): δ 6.95 (s, 1 H), 6.54 (s, 1H), 5.15 (m, 1H), 5.05 (d, J = 10.15 Hz, 1H), 4.08 (dd, J = 10.1, 2.66 Hz, 1H), 3.87 (m, 1 H), 3.01 (s, 1H), 3.06 (m, 1H), 2.83-2.65 (m, 3H), 2.70 (s, 3H), 2.44 (m, 1H), 2.10 (s, 3H), 2.07 (m, 1H), 1.83 (m, 1H), 1.77 (m, 1H), 1.71 (m, 1H), 1.64 (s, 3H), 1.60 (s, 1H), 1.37 (m, 1H), 1.31 (m, 1H), 1.20 (m, 1H), 1.15 (s, 3H), 1.14 (m, 5H), 1.02 (d, J = 7.02 Hz, 3H), 0.89 (t, J = 7.97 Hz, 9H), 0.64-0.52 (m, 6H); $^{13}C$ NMR (100 MHz, $CDCl_3$): δ 218.34, 170.73, 164.59, 152.46, 139.07, 138.49, 120.48, 119.54, 116.00, 79.31, 75.81, 73.48, 53.62, 42.98, 39.48, 39.01, 32.85, 32.41, 31.20, 26.12, 24.26, 22.01, 22.46, 19.18, 16.44, 15.30, 13.99, 6.98 (3), 5.27 (3); IR (neat) 3524.0, 1740.3, 1693.4, 1457.2, 1378.4, 733.2 $cm^{-1}$.

## Comparative Example 67

Desoxyepothilone B **2C**. The TES protected alcohol 57 (143 mg, 0.24 mmol) was dissolved in 2 mL of dry THF in a plastic reaction vessel and cooled to 0 °C in an ice bath. The resultant solution was treated with 1 mL of HF-pyridine. The reaction mixture was stirred for 80 min at 0 °C and then quenched by pouring into a saturated aqueous solution of $NaHCO_3$. An aqueous workup followed by flash column chromatography with 10% EtOAc/hexanes afforded desoxyepothilone B (112 mg, 0.23 mmol) in 95% yield. The resultant product exhibited a $^1H$ NMR spectrum identical to that of authentic desoxyepothilone B.

## Total Synthesis of Desoxyepothilone B

## Comparative Example 68

***tert*-Butyl 4-methyl-3-oxopentanoate (1E).** Meldrum's acid (80 g, 555 mmol) was dissolved in 600 mL of $CH_2Cl_2$ and cooled to 0 °C. Freshly distilled pyridine (87.8 g, 1.11 mol) was added to the $CH_2Cl_2$ solution and then *iso*-butyryl chloride (65.0 g, 610.5 mmol) was added to the mixture via a pressure equalizing addition funnel. The reaction was stirred at 0 °C for 1 hr and then warmed to rt and stirred for 2 hr. Then, the reaction was quenched with water (200 mL) and washed with 0.5 M HCl (x2), water (x1), and brine (x1). The organize layer was dried over $MgSO_4$ and concentrated *in vacuo*. The crude product was azeotropically dried with benzene (250 mL), and then dissolved in 200 mL of benzene and 200 mL of *tert*-butanol was added. The resultant reaction was heated at reflux for 4 hr. After this period, the volatiles were removed *in vacuo* and the product then distilled on the high vacuum pump (bp 62-63 °C, 0.1 mm Hg). The desired β-keto ester 1E was obtained (58.6 g, 315.5 mmol) in 57% yield as a clear, colorless, light oil.

**Comparative Example 69**

***tert*-Butyl 4,4-dimethy-3,5-dioxoheptanoate (2E).** β-Keto ester 1E (55.0 g, 295.3 mmol) was added dropwise in 50 mL of dry THF to a slurry of NaH (9.7 g, 60% dispersion in mineral oil, 383.9 mmol) in 1.15 L of dry THF. The reaction mixture was stirred at 0 °C for 30 min and then the cold bath was cooled to -50 °C. Propionyl chloride (27.3 g, 295.3 mmol) was added rapidly (neat) by syringe to the cold solution. The reaction was monitored by TLC and the cold bath was maintained below -30 °C until the reaction was complete. After 1 hr, the reaction was quenched by pouring into a solution of saturated aqueous $NH_4Cl$ and subjected to an aqueous workup. The aqueous layer was extracted with $Et_2O$ (x2, 200 mL). Flash column chromatography with 2% EtOAc/hexanes afforded the desired tricarbonyl 2E (50.7 g, 209.6 mmol) in 71% yield; $^1H$ NMR (400 MHz, $CDCl_3$); δ 12.43 (s, 0.20H), 5.07 (s, 0.20H), 3.36 (s, 1.6H), 2.47 (q, *J* = 7.13 Hz, 2H), 1.44 (s, 9H), 1.35 (s, 6H), 1.03 (t, *J* = 7.18 Hz, 3H); $^{13}C$ NMR (100 MHz, $CDCl_3$): δ 209.7, 242.9, 166.1, 81.91, 62.53, 43.34, 31.67, 27.82 (3), 21.03 (2), 7.82; IR (neat) 3411.8, 1742.6, 1716.5, 1702.0, 1644.2, 1460.6, 1156.1 $cm^{-1}$.

**Comparative Example 70**

***tert*-Butyl-4,4-dimethyl-3-methoxy-5-oxo-2-heptenoate (3E).** Trimethylsilyldiazomethane ($TMSCHN_2$, 46.2 mL of a 2.0 M solution in THF, 92.4 mmol) was added by syringe to a stirred solution of the tricarbonyl (16.0 g, 66.0 mmol) and diisopropylethylamine (Hunig's base, 16.1 mL, 92.4 mmol) in 330 mL of a 9:1 solution of acetonitrile: methanol at rt. The resultant reaction mixture was stirred at rt for 18-20 hr. The reaction mixture was then quenched with saturated aqueous $NaHCO_3$ and the enol ether extracted with $Et_2O$ (x3, 50 mL). The combined organic layers were washed with brine and then dried of $MgSO_4$, filtered and concentrated *in vacuo*. Flash column chromatography of the crude product (2% EtOAc/hexanes) afforded the desired enol ether 3E (12.5 g, 48.4 mmol) in 74% yield; $^1H$ NMR (400 MHz, $CDCl_3$): δ 5.18 (s, 1H), 3.88 (s, 3H), 2.45 (q, *J* = 7.33 Hz, 2H), 1.48 (s, 9H), 1.25 (s, 6H), 1.02 (t, *J* = 7.21 Hz, 3H).

**Comparative Example 71**

**(67*R*,7*R*,8*S*)-7-Hydroxy-5-oxo-4,4,6,8-tetramethy-3-triethysilyloxy-2,10-undecadienoate, *tert*-butyl ester (6E).** The keto enol ether 3E (8.0 g, 31.3 mmol) in 750 mL of dry THF was cooled to -30 °C in a cold bath ($CO_2$(s) /$CH_3CN$) and then a solution of LDA (37.5 mmol, 0.90 M in THF) was added dropwise via syringe over 10 min. The reaction mixture was stirred at -30 to -33 °C for 20 min. Then the reaction vessel was placed in a -120 °C cold bath ($N_2$(liq)/pentane) and the reaction mixture was stirred for 10 min. Finally the aldehyde 5 (3.6 g, 36.7 mmol; aldehyde 5E was readily prepared according to the procedure outlined in: Lin, N.-H., *et al*., *J. Am. Chem. Soc.* **1996,** *118,* 9062.) was added via syringe in 5 mL of $CH_2Cl_2$. The reaction was complete after 10 min and was quenched by pouring into a solution of saturated aqueous $NH_4Cl$. The desired aldol product **6E** (5.2 g, 14.7 mmol) was isolated in 47% yield (yield of the major product of a 5.5:1 mixture of diastereomers, epimeric at C-8) after flash column chromatography with 6-5% EtOAc/hexanes; (major diasteromer, high $R_f$); $^1H$ NMR (400 MHz, $CDCl_3$): δ 5.78 (m, 1H), 5.18 (s, 1H), 4.98 (m, 2H), 3.90 (s, 3H), 3.37 (m, 1H), 3.35 (s, 1H), 3.35 (s, 1H), 3.12 (q, *J* = 7.74 Hz, 1H), 2.53 (m, 1H), 1.87 (dt, *J* = 13.8, 8.47 Hz, 1H), 1.61 (m, 1H), 1.55 (s, 1 H), 1.48 (s, 9H), 1.28 (s, 3H), 1.27 (s, 3H), 1.05 (d, *J* = 6.91 Hz, 3H), .079 (d, *J* = 6.76 Hz, 3H); $^{13}C$ NMR (100 MHz, $CDCl_3$): δ 217.7, 171.2, 164.8, 137.0, 116.3, 97.29, 80.22, 74.67, 62.17, 56.05, 41.05, 37.31, 34.99, 28.13, 22.69, 22.67, 15.00, 10.44; (minor diastereomer, low $R_f$): $^1H$ NMR (400 MHz, $CDCl_3$): δ 5.76 (m, 1H), 5.19 (s, 1H), 5.06 (m, 2H), 1.48 (s, 3H), 3.41 (m, 1H), 3.17 (m, 1H), 3.12 (m, 1H), 2.11 (m, 1H), 1.86 (m, 1H), 1.63 (m, 1H), 1.48 (s, 9H), 1.28 (s, 3H), 1.27 (s, 3H), 1.07 (s, *J* = 6.91 Hz, 3H), 0.99 (d, *J* = 6.64 Hz, 3H).

**Comparative Example 72**

**(6*R*,7*R*,8*S*)-7-(2,2,2-Trichloroethoxycarbonate)-5-oxo-4,4,6,8-tetramethyl-3-triethylsilyloxy-2,10-undecadienoate, *tert*-butyl ester.** Alcohol **6E** (5.2 g, 14.7 mmol) was dissolved in 70 mL of dry $CH_2Cl_2$ and cooled to 0°C in an ice bath. Then, pyridine (4.65 g, 58.8 mmol) and trichloroethoxyethylcarbonoyl chloride (TrocCl) (6.23 g, 29.4 mmol) were added by syringe in that order. The reaction was stirred at 0 °C for 5 min and then the ice bath was removed and the reaction was allowed to come to rt and stir for 30 minutes. After this period of time, TLC analysis showed the complete consumption of the starting material. The reaction mixture was quenched by pouring it into a solution of saturated aqueous $NaHCO_3$. Flash column chromatography with 3% EtOAc/hexanes through a short plug of silica gel afforded the desired enol ether which was subjected immediately to hydrolysis; $^1H$ NMR (400 MHz, $COCl_3$): δ 5.71 (m, 1H), 5.21 (s, 1 H), 5.02 (m, 2H), 4.86 (d, *J* = 11.9 Hz, 1H), 4.85 (m, 1 H), 4.72 (d, *J* = 11.9 Hz, 1H), 3.91 (s, 3H), 3.25 (m, 1H), 2.26 (m, 1H), 1.87 (m, 1H), 1.81 (m, 1 H), 1.48 (s, 9H), 1.31 (s, 3H), 1.26 (2, 3H), 1.11 (d, *J* = 6.85 Hz, 3H), 0.91 (d, *J* = 6.64 Hz, 3H).

**Comparative Example 73**

**(6*R*,7*R*,8*S*)-7-Trichloroethoxyethylcarbonate-3,5-dioxo-4,4,6,8-tetramethyl-10-undecenoate, *tert*-butyl ester (7E).** The Troc-protected enol ether (as above) was dissolved in acetone and treated with 300 mg (catalytic) of ρ-TsOH at rt for 5-6 hrs. The reaction was monitored by TLC and after complete consumption of the starting enol ether was apparent, the reaction mixture was quenched with saturated aqueous $NaHCO_3$. The desired tricarbonyl 7E (6.8 g, 12.8 mmol), 87% (2 steps) was isolated after an aqueous workup and flash column chromatography with 7-9% EtOAc/hexanes; $^1H$ NMR (400 MHz, $CDCl_3$): δ 12.63 (s, 0.25H), 5.70 (m, 1 H), 5.15 (s, 0.25H), 5.08-4.88 (m, 2H), 4.91 (dd, *J* = 6.60, 5.01 Hz, 0.30H), 4.78 (m, 1H), 4.77 (dd, *J* = 7.86, 3.58 Hz, 0.70H), 4.72 (dd, *J* = 11.8, 9.66 Hz, 1H), 3.48 (d, *J* = 16.2 Hz, 0.75H), 3.42 (d, *J* = 16.2 Hz, 0.75H), 3.36 (m, 0.30H), 3.30 (m, 0.70H), 1.88 (m, 2H), 1.50 (s, 3H), 1.46 (s, 9H), 1.39 (s, 3H), 1.12 (d, *J* = 6.88 Hz, 0.70H), 1.10 (d, *J* = 6.88 Hz, 1.3H), 0.93 (d, *J* = 6.63 Hz, 1.3H), 0.88 (d, *J* = 6.86 Hz, 0.70H); $^{13}C$ NMR (100 MHz, $CDCl_3$): δ 210.5, 209.5, 203.16, 178.3, 172.6, 166.2, 154.1, 135.9, 135.6, 117.2, 116.9, 94.69, 94.56, 90.69, 82.68, 81.98, 81.65, 81.53, 63.58, 54.34, 46.56, 41.99, 41.62, 36.41, 35.84, 34.49, 34.44, 31.56, 28.23 (3), 27.94 (3), 22.62, 22.08, 21.56, 20.80, 15.95, 15.58, 14.09, 13.02, 12.98, 11.35; IR (neat) 1757.98, 1718.9, 1700.2, 1642.2, 1620.7, 1250.6, 1156.3 $cm^{-1}$.

**Comparative Example 74**

**Allylic Alcohol (16E).** A mixture of (*S*)-(-)-1,1'-bi-2-naphthol (1.37 g, 4.8 mmol), Ti(O-*i*-Pr)$_4$ (1.36 g, 4.8 mmol), and 4 Å sieves (11 g) in $CH_2Cl_2$ (300 mL) was heated at reflux for 1 h. The mixture was cooled to rt and aldehyde **15**E (8.0 g, 47.9 mmol; prepared according to the procedure outlined in an earlier Danishefsky synthesis of the epothilones: Meng, D.; Sorensen., E. J.; Bertinato, P.; Danishefsky, S. J. *J. Org. Chem.* **1996**, 61, 7998) was added. After 10 min, the suspension was cooled to -78 °C, and allyl tri-n-butyltin (20.9 g, 67.1 mmol) was added. The reaction mixture was stirred for 10 min at -78 °C and then placed in a 20 °C freezer for 70 h. Saturated aqueous $NaHCO_c$ solution (2 mL) was added, and the mixture was stirred for 1 h, poured over $Na_2SO_4$, and then filtered through a pad of $MgSO_4$ and celite. The crude material was purified by flash chromatography with EtOAc/hexanes (5%→10% → 15% → 20% → 25% → 30%, two column volumes each) to give alcohol 16E as a yellow oil (6.0 g, 88.0 mmol) in 60% yield; $[\alpha]_D$ = -15.9 (c. 4.9, $CHCl_3$); IR (film) 3360, 1641, 1509, 1434, 1188, 1017, 914 $cm^{-1}$; $^1H$ NMR (500 MHz, $CDCl_3$, 25 °C) δ 6.92 (s, 1 H), 6.55 (s, 1 H), 5.82 (m, 1 H), 5.13 (dd, *J* = 17.1, 1.3 Hz, 1 H), 5.09 (d, *J* = 10.2 Hz, 1 H), 4.21 (t, *J* = 6.0 Hz, 1 H), 2.76 (br s, 1 H), 2.69 (s, 3 H), 2.40 (m, 2 H), 2.02 (s, 3 H); $^{13}C$ NMR (125 MHz, $CDCl_3$, 25 °C) δ 164.5, 152.6, 141.5, 134.6, 119.2, 117.6, 115.3, 76.4, 39.9, 19.0, 14.2; HRMS calcd. for $C_{11}H_{15}NOS$: 209.0874 found: 209.0872 (M+H).

**Comparative Example 75**

**TBS allylic ether (17E).** Alcohol **16E** (5.70 g, 27.3 mmol) was dissolved in 50 mL of dry $CH_2Cl_2$ and cooled to -78 °C. Then, 2,6-lutidine (7.6 g, 70.9 mmol) and TBSOTf (9.36 g, 35.4 mmol) were added via syringe successively and in that order. The reaction mixture was stirred at this temperature for 30 minutes and then quenched by pouring the reaction mixture into saturated aqueous $NaHCO_3$. An aqueous workup followed by flash column chromatography with 2% EtOAc/hexanes afforded the desired TBS ether (7.37 g, 22.8 mmol) in 84% yield. The allylic alcohol, **17E**, could also be prepared according to the general procedure outlined in the following references: (a) Racherla, U. S.; Brown, H. C. *J. Org. Chem*. **1991**, 56, 401. (b) Yang, Z.; He, Y.; Vourloumis, D.; Vallberg, H.; Nicolaou, K. C. *Angew. Chem., Int. Ed. Engl.*, **1997**, 36, 166.

**Comparative Example 76**

**Aldehyde (18E).** To a solution of TBS ether **17E** (7.37 g, 22.8 mmol) in acetone (150 mL) at 0 °C was added 6.7 g of a 60% solution of *N*-methyl-morpholine-*N*-oxide (NMO) in water (34.2 mmol), $OsO_4$ (0.039 M in THF, 6 mL, 0.23 mmol). The resultant mixture was stirred at 0 °C for 2 h and then quenched with saturated aqueous $Na_2SO_3$ solution (100 mL). The solution was poured into $H_2O$ (100 mL) and extracted with EtOAc (8 x 50 mL). The combined organic layer was dried over $MgSO_4$, filtered, concentrated, and flashed through a short plug of silica gel to afford (7.8 g, 21.8 mmol) the crude diol in 96% yield.

To a solution of the crude diol (7.8 g, 21.8 mmol; the oxidation procedure described here may also be acomplished with $NalO_4$ as outlined in a previous Danishefsky synthesis of the epothilones: Meng, D., e*t al., J. Ann. Chem. Soc.* **1997**, 119, 10073.) in 400 mL benzene at 0 °C was added $Pb(OAc)_4$ (19.4 g, 43.7 mmol) and $Na_2CO_3$ (9.24 g, 87.2 mmol). The reaction mixture was stirred at 0 °C for 10 min and then rt for 1.5 hr. After this period of time, the reaction mixture was quenched by pouring into brine. The reaction was filtered through Celite™ and then the resultant aqueous layer was extracted with EtOAc (5 x 50 mL) dried over $MgSO_4$. Flash column chromatography on silica gel

with 20% EtOAc/hexanes on a short pad of silica gave the aldehyde **18E** as a yellow oil (5.02 g, 15.5 mmol) in 71 % yield.

## Comparative Example 77

**TBS vinyl iodide (19E).** *n*-BuLi (2.5 M in hexanes, 22.6 mL, 55.4 mmol) was added to a suspension of ethyl triphenylphosonium iodide (23.2 g, 55.4 mmol) in THF (100 mL) at 25°C. After 30 min, the clear red solution was transferred dropwise by syringe to a vigorously stirred solution of $I_2$ (14.1 g, 55.4 mmol) in THF (1100 mL) at -78°C. After addition of the Wittig reagent was completed, the resulting pale yellow suspension was stirred rapidly and warmed to 20° C. Then, NaHMDS (1.0 M soln in THF, 55.4 mL, 55.4 mmol) was added dropwise by syringe. During the addition of the NaHMDS, the reaction mixture changed from a yellow-orange slurry and to bright red solution. Aldehyde **18E** (6.0 g, 18.5 mmol) was then added in THF. After 30 min, the reaction mixture was poured into hexanes (400 mL) and then 0.5 mL brine was added. The solution was then passed through a plug of $SiO_2$ eluting with 2:1 hexanes/$Et_2O$. The iodide was purified by flash chromatography on $SiO_2$ eluting with hexanes/ethyl acetate (20:1 to 15:1) to give the vinyl iodide **19E** (5.0 g, 10.2 mmol, 50%) as a yellow oil: $^1H$ NMR (400 MHz, $CDCl_3$): δ 6.95 (s, 1H), 6.50 (s, 1H), 5.45 (dt, *J* = 1.5, 6.8 Hz, 1H), 4.22 (t, *J* = 6.4 Hz, 1H), 2.73 (s, 3H), 2.48 (s, 3H), 2.39 (m, 2H), 2.02 (s, 3H), 0.90 (s, 9H), 0.06 (3, s), 0.02 (3, s); $^{13}C$ NMR (100 MHz, $CDCl_3$): δ 164.86, 153.46, 142.17, 132.54, 119.23; 115.68, 102.79, 77.70, 44.40, 39.09, 26.35, 19.65, 18.63, 14.54, -4.59, -4.84; IR (neat) 2928, 1470, 1252, 1068 $cm^{-1}$.

## Comparative Example 78

**Post-Suzuki, C-15 Hydroxy Tricarbonyl (10E).** 9-BBN (0.5 M soln in THF, 14.1 mL, 7.03 mmol) was added over a 45 min period to a solution of the olefin 7**E** (2.78 g, 5.41 mmol) in THF (25 mL) at 25°C. After 2 h, TLC analysis revealed the complete consumption of the starting olefin.

In a separate flask, containing the vinyl iodide **18E** (2.65 g, 5.41 mmol) and DMF (45 mL), were added successively and with vigorous stirring: $Cs_2CO_3$ (3.52 g, 10.82 mmol); $Pd(dppf)_2Cl_2$ (1.10 g, 1.35 mmol), $AsPh_3$ (0.41 g, 1.35 mmol) and $H_2O$ (3.5 mL, 0.19 mol). Then the borane solution, prepared above, was added rapidly by syringe to the vigorously stirred solution containing the vinyl ioidide. After 2 h, the reaction TLC analysis revealed that the reaction was complete. The reaction mixture was poured into $Et_2O$ (3 x 200 mL), brine (1 x 50 mL) dried over anhydrous $MgSO_4$. This crude product was purified by flash column chromatography on $SiO_2$ eluting with hexanes/ethyl acetate (18:1 to 13:1 to 10:1) to afford the TBS protected coupled product **9E** as an impure mixture which was taken on to the next step without further purification.

The crude TBS protected coupled product **9E** was dissolved in 0.5 M HCl in MeOH (30 mL) at 25°C. The reaction was monitored by TLC for corruption and after 3.5 h (disappearance of starting TBS ether), the mixture was poured into a solution of saturated aqueous $NaHCO_3$ and extracted with $CHCl_3$ (4 x 60 mL). The combined organic layers were washed once with brine (50 mL) and dried over with anhydrous $MgSO_4$. The diol was purified by flash column chromatography on $SiO_2$ eluting with hexaneslethyl acetate (4:1 to 3:1 to 2:1) to give the pure product 10E as a clear oil (2.44 g, 3.35 mmol, 62% for two steps): $^1H$ NMR (400 MHz, $CDCl_3$: δ 6.96 (s, 1 H), 6.56 (s, 1H), 5.16 (t, *J* = 6.9 Hz, 1 H), 4.83 (d, *J* = 11.9 Hz, 1 H), 4.75 (dd, *J* = 3.4, 8.0 Hz, 1H), 4.70 (d, *J* = 11.9 Hz, 1H), (t, *J* = 6.4 Hz, 1H), 3.45 (q, *J* = 13.2 Hz, 2H), 3.32 (m, 1H), 2.72 (s, 3H), 2.32 (t, *J* = 6.5 Hz, 2H), 2.04 (s, 3H), 2.01 (m, 2H), 1.74 (m, 2H), 1.69 (s, 3H), 1.45 (s, 9H), 1.38 (s, 6H), 1.09 (d, *J* = 6.9 Hz, 3H), 0.93 (d, *J* = 6.9 Hz, 3H); $^{13}C$ NMR (100 MHz $CDCl_3$: δ 209.51, 203.04, 166.15, 164.39, 154.14, 152.72, 141.71, 138.24, 120.70, 118.76, 115.28, 94.54, 81.85, 77.31, 76.57, 63.41, 54.16, 46.47, 41.48, 34.56, 33.95, 31.98, 31.53, 27.85, 24.85, 23.45, 21.47, 20.75, 19.04, 15.60, 14.33, 11.35; IR (neat) 3546, 3395, 1756, 1717, 1699, 1644, 1621, 1506, 1456, 1251, $cm^{-1}$.

## Comparative Example 79

**Noyori C-3/C-15 Diol Product (11E).** The diketone **10E** (1.77 g, 2.43 mmol) was dissolved in 0.12 N HCl in MeOH (21 mL, 1.3 eq) at 25°C. The (*R*)-RuBINAP catalyst (0.045) M in THF, 8.0 mL, 0.36 mmol) was then added and the mixture transferred to a Parr apparatus. The vessel was purged with $H_2$ for 5 min and then pressurized to 1200 psi. After 12-14 h at 25°C, the reaction was returned to atmospheric pressure and poured into a saturated solution of $NaHCO_3$. This mixture was extracted with $CHCl_3$ (4 x 50mL) and the combined organic layers were dried over anhydrous $MgSO_4$. The product was purified by flash column chromatography on silica gel eluting with hexaneslethyl acetate (4: 1 to 2:1) to give 1.42 g (81%) of the hydroxy ester 11E as a green foam; $^1H$ NMR (400 MHz, $CDCl_3$: δ 6.96 (s, 1H), 6.55 (s, 1H), 5.15 (t, *J* = 6.9 Hz, 1 H), 4.85 (t, *J* = 5.3 Hz, 1 H), 4.81 (d, *J* = 12.0 Hz, 1 H), 4.71 (d, *J* = 12.0 Hz, 1H), 4.12 (m, 2H), 3.43 (m, 2H), 2.70 (s, 3H), 2.37 (dd, *J* = 2.2, 6.2 Hz, 1H), 2.30 (t, *J* = 6.7 Hz, 2H), 2.24 (dd, *J* = 10.6, 16.2 Hz, 1H), 2.03 (s, 3H), 1.99 (m, 2H), 1.68 (S, 3h), 1.44 (s, 9h), 1.18 (s, 3h), 1.16 (s, 3h), 1.09 (d, *J* = 6.8 Hz, 3H), 0.94 (d, *J* = 6.8 Hz, 3H); $^{13}C$ NMR (100 MHz $CDCl_3$): δ 215.95, 172.39, 164.39, 154.21, 152.74, 141.70, 138.33, 120.59, 118.77, 115.27, 94.64, 82.98, 81.26, 76.51, 72.78, 51.82, 41.40, 37.36, 34.66, 33.96, 32.08, 31.10, 30.20, 27.96, 25.06,

23.45, 21.73, 21.07, 19.17, 19.01, 16.12, 15.16, 14.33, 12.17; IR (neat) 3434.0, 1757.5, 1704.5, 1249.9, 1152.8 $cm^{-1}$.

**C-3/C-15 Bis(TES) Carboxylic Acid.** 2,6-Lutidine (2.1 g, 19.6 mmol) and TESOTf (2.6 g, 9.8 mmol) were added successively to a cooled solution of the diol **11E** (2.38, 3.26 mmol) in $CH_2Cl_2$ (30 mL) at -78°C. The reaction mixture was stirred at -78°C for 5 min and then warmed to rt and stirred for 1 hr. Then 2,6-lutidine (4.9 g, 45.6 mmol) and TESOTF (6.0 g, 22.8 mmol) were added successively to a -78°C cooled solution. The reaction was stirred at rt for 6 hr and then quenched with saturated aqueous $NH_4Cl$ and subjected to an aqueous workup. The crude product was concentrated *in vacuo* and the 2,6-lutidine removed on high vacuum pump and then subjected directly to the next set of reaction conditions; $^1H$ NMR (400 MHz, $CDCl_3$): δ 6.96 (s, 1 H), 6.66 (s, 1H), 5.04 (t, *J* = 6.93 Hz, 1H), 4.90 (d, *J* = 12.0 Hz, 1 H), 4.77 (dd, *J* = 7.99, 3.21 Hz, 1H), 4.66 (d, *J* = 12.0 Hz, 1H), 4.46 (m, 1H), 4.10 (dq, *J* = 12.3, 7.11 Hz, 2H), 3.42 (m, 1H), 2.70 (s, 3H), 2.60 (dd, *J* = 16.7, 2.34 Hz, 1H), 2.34 (dd, *J* = 16.7, 7.94 Hz, 1H), 2.27 (dd, *J* = 14.0, 6.97 Hz, 1H), 2.18 (m, 1H), 2.09 (m, 1H), 2.04 (s, 1H), 1.95 (s, 3H), 1.82 (m, 2H), 1.61 (s, 3H), 1.44 (m, 2H), 1.27-1.22 (m, 4H), 1.14 (d, *J* = 8.45 Hz, 3H), 1.11 (d, *J* = 6.81 Hz, 2H), 1.04 (d, *J* = 6.88 Hz, 2H), 1.15-1.01 (m, 2H), 0.94 (t, *J* = 7.92 Hz, 18H), 0.65-0.57 (m, 12H); $^{13}C$ NMR (100 MHz, $CDCl_3$): δ 215.11, 175.34, 165.00, 154.14, 152.80, 142.60, 136.84, 121.31, 118.79, 114.60, 94.77, 81.60, 79.06, 76.64, 73.87, 54.19, 41.18, 39.56, 35.09, 34.52, 32.29, 31.95, 24.76, 23.62, 22.55, 18.95, 18.64, 15.87, 13.69, 11.33, 6.94, 6.83, 5.07, 4.76; IR (neat) 3100-2390, 1756.8, 1708.8, 1459.3, 1250.6,816.1 $cm^{-1}$.

## Comparative Example 80

**C-15 Hydroxy Acid for Macrolactonization (12E).** The crude bis(triethylsilyl)ether, prepared above, was dissolved in 20 mL of dry THF and then cooled to 0 °C. Then, 6 ml of 0.12 M HCl/MeOH was added. The reaction mixture was stirred at 0 °C for 3 min and maintained at 0°C for the duration. The reaction was monitored closely by TLC analysis. Methanolic HCl (0.12 M) was added in small portions, and roughly 1.3 equivalents of 0.12 M HCl was required for the hydrolysis of the C-15 TBS ether (approximately 30-40 mL). The reaction was complete in appoximately 30 min. The reaction was quenched by pouring into a solution of saturated aqueous $NaHCO_3$ and subjected to an aqueous workup. Flash column chromatography with 40% EtOAc/hexanes afforded the desired carboxylic acid **12E** (1.71 g, 2.20 mmol) in 67% yield; $^1H$ NMR (400 MHz, $CDCl_3$): δ 6.96 (s, 1H), 6.69 (1, s), 5.11 (t, *J* = 6.9 Hz, 1 H), 4.91 (d, *J* = 12.0 Hz, 1H), 4.71 (dd, *J* = 3.1, 8.2 Hz, 1H), 4.64 (d, *J* = 12.0 Hz, 1H), 4.42 (d, *J* = 5.9 Hz, 1H), 4.10 (m, 1H), 3.43 (m, 1H), 2.71 (s, 3H), 2.57 (dd, *J* = 2.1, 10.5 Hz, 1 H), 2.25 (m, 3H), 2.11 (m, 1 H), 1.98 (s, 3H), 1.95 (m, 2H), 1.72 (m, 1), 1.67 (s, 3H), 1.45 (m, 2H), 1.16 (s, 3H), 1.13 (s, 3H), 1.09 (d, *J* = 6.7 Hz, 3H), 0.99 (d, *J* = 6.7 Hz, 3H), 0.95 (t, *J* = 7.9 Hz, 9H), 0.64 (dq, *J* = 2.3, 7.9 Hz, 6H); $^{13}C$ NMR (100 MHz, $CDCl_3$): δ 215.11, 176.00 (165.10, 154.18, 152.35, 142.24, 138.55, 120.74, 118.21, 115.02, 94.76, 81.91, 76.86, 76.63, 73.95, 54.08, 41.28, 39.64, 34.73, 34.16, 32.02, 31.67, 24.71, 23.41, 22.49, 19.17, 18.62, 15.71, 14.86, 11.20, 6.93, 5.05); IR (neat) 3400-2390, 1755.9, 1703.8, 1250.4, 735.4 $cm^{-1}$.

## Comparative Example 81

**C-3 Triethylsilyl/C-7 Trichloroethoxyethylcarbonate Macrolactonization Product (13E).** Triethylamine (155 mg, 1.53 mmol) and 2,4,6-trichlorobenzoyl chloride (312 mg, 1.28 mmol) were added to a solution of the hydroxy acid **12E** (198 mg, 0.256 mmol) in 3.6 mL of dry THF. The readion mixture was stirred for 15 min (and NO LONGER) at rt and then diluted with 20 mL of dry toluene. The resultant solution was added slowly dropwise, via syringe pump, over 3 hr to a previously prepared; stirred solution of DMAP (328 mg, 2.68 mmol) in 300 mL of dry toluene. After the addition of the substrate was complete, the reaction was stirred for an additional 0.5 h and then concentrated *in vacuo.* Flash column chromatography of the crude product with 10% EtOAc/hexanes afforded the macrolactone **13E** (153 mg, 0.20 mmol) in 78% yield; $^1H$ NMR (400 MHz, $CDCl_3$): δ 6.96 (s, 1H), 6.53 (s, 1H), 5.20 (m, 2H), 5.04 (d, *J* = 10.2 Hz, 1 H), 4.84 (d, *J* = 12.0 Hz, 1 H), 4.78 (d, *J* = 12.0 Hz, 1H), 4.07 (m, 1H), 3.32 (m, 1H), 2.86-2.63 (m, 3H), 2.70 (s, 3H), 2.48 (m, 1H), 2.1 (s, 3H), 2.04 (dd, *J* = 6.17, 14.7 Hz, 1H), 1.73 (m, 4H), 1.66 (s, 3H), 1.25 (m, 2H), 1.19 (s, 3H), 1.15 (s, 3H), 1.12 (d, *J* = 6.68 Hz, 3H), 1.01 (d, *J* = 6.83 Hz, 3H), 0.89 (t, *J* = 8.00 Hz, 9H), 0.58 (q, *J* = 7.83 Hz, 6H); $^{13}C$ NMR (100 MHz, $CDCl_3$): δ 212.75, 170.66, 164.62, 154.60, 152.52, 140.29, 138.44, 119.81, 119.38, 116.28, 94.84, 86.44, 80.14, 76.59, 76.10, 53.55, 45.89, 39.23, 35.47, 32.39, 31.69, 31.57, 31.16, 29.68, 27.41, 25.00, 23.44, 22.94, 19.23, 18.66, 16.28, 14.83, 6.89, 5.22; IR (neat) 1760.5, 1742.6, 1698.0, 1378.8, 1246.2, 1106.0, 729.8 $cm^{-1}$.

## Comparative Example 82

**$SmI_2$ Mediated Deprotection of Troc Group.** Samarium metal (0.52 g, 3.43 mmol) and iodine (0.78 g, 3.09 mmol) in 40 mL of dry, deoxygenated THF were stirred together vigorously at reflux for 2.5 hr. During this period of time, the reaction mixture progressed from a dark orange to an olive green to deep blue color. The resultant deep blue solution of $SmI_2$ was used directly in the following reaction. A catalytic amount of $NiI_2$ (10 mg) was added in one portion

to the vigorously stirted solution of $SmI_2$. The reaction mixture was stirred 5 min at rt and then cooled to -78°C in a a dry ice/acetone bath. Then, the macrolactone **13E** (297 mg, 0.386 mmol), in 10 mL of dry THF, was added over 1 min to the rapidly stirred, cold solution of $SmI_2/NiI_2$. The resultant deep blue solution was maintained at -78°C with continued vigorous stirring for 1 hr. TLC analysis at this time revealed the complete consumption of the starting material and formation of a single, lower $R_f$ product. The reaction mixture was quenched with saturated aqueous $NaHCO_3$ and subjected to an aqueous workup. Flash column chromatography with 25% EtOAc/hexanes afforded the C-7 alcohol (204 mg, 0.343 mmol) in 89% yield: $^1H$ NMR (400 MHz, $CDCl_3$): δ 6.95 (s, 1 H), 6.54 (s, 1H), 5.15 (m, 1H), 5.05 (d, *J* = 10.15 Hz, 1H), 4.08 (dd, *J* = 10.1, 2.66 Hz, 1H), 3.87 (m, 1H), 3.01 (s, 1H), 3.06 (m, 1H), 2.83-2.65 (m, 3H), 2.70 (s, 3H), 2.44 (m, 1H), 2.10 (s, 3H), 2.07 (m, 1H), 1.83 (m, 1H), 1.77 (m, 1H), 1.71 (m, 1H), 1.64 (s, 3H), 1.60 (s, 1H), 1.37 (m, 1H), 1.31 (m, 1H), 1.20 (m, 1H), 1.15 (s, 3H), 1.14 (m, 5H), 1.02 (d, *J* = 7.02 Hz, 3H), 0.89 (t, *J* = 7.97 Hz, 9H), 0.64-0.52 (m, 6H); $^{13}C$ NMR (100 MHz, $CDCl_3$): δ 218.34, 170.73, 164.59, 152.46, 139.07, 138.49, 120.48, 119.54, 116.00, 79.31, 75.81, 73.48, 53.62, 42.98, 39.48, 39.01, 32.85, 32.41, 31.20, 26.12, 24.26, 22.01, 22.46, 19.18, 16.44, 15.30, 13.99, 6.98 (3),5.27 (3); IR (neat) 3524.0, 1740.3, 1693.4, 1457.2, 1378.4, 733.2 $cm^{-1}$.

**Compatative Example 83**

**Desoxyepothilone B (12E).** The C-3 TES protected alcohol (204 mg, 0.343 mmol) was dissolved in 6 ml of dry THF in a plastic reaction vessel and cooled to 0 °C in an ice bath. The resultant solution was treated with 3 mL of HF-pyridine. The reaction mixture was stirred for 80 min at 0°C and then quenched by pouring into a saturated aqueous solution of $NaHCO_3$. An aqueous workup followed by flash column chromatography with 10% EtOAc/hexanes afforded desoxyepothilone **B 12E** (160 mg, 0.32 mmol) in 95% yield. The resultant product exhibited a $^1H$ NMR spectrum identical to desoxyepothilone B prepared as described hereinabove.

Comparative Discussion - not part of the invention

Desmethylepothilone A

Total syntheses of epothilones A and B have not been previously disclosed. Balog, A., *et al., Angew. Chem., Int. Ed. Engl.* **1996**, 35, 2801; Nicolaou, K.C., *et al., Angew. Chem., Int. Ed. Engl.* **1997**, *36,* 166. Nicolaou, K.C., *et al., Angew. Chem., Int: Ed. Engl.* **1997**, 36, 525; Schinzer, D., *et al., Angew. Chem., Int. Ed. Engl.* **1997**, *36*, 523. Su, D. -S., *et al., Angew. Chem. Int. Ed. Engl.* **1997**, *36*, *757*. The mode of antitumor action of the epothilones closely mimics that of Taxol® (paclitaxel). Höfle, G., *et al*., H. *Angew. Chem., Int. Ed. Engl.* **1996**, *35*, 1567. Although Taxol° is a clinically proven drug, its formulation continues to be difficult. In addition, Taxol® induces the multidrug resistance (MDR) phenotype. Hence, any novel agent that has the same mechanism of action as Taxol® and has the prospect of having superior therapeutic activity warrants serious study. Bollag, D. M., *et al*., *Cancer Res.* **1995,** 55, 2325.

The present invention provides epothilone analogs that are more effective and more readily synthesized than epothilone A or B. The syntheses of the natural products provide ample material for preliminary biological evaluation, but not for producing adequate amounts for full development. One particular area where a structural change could bring significant relief from the complexities of the synthesis would be in the deletion of the C8 methyl group from the polypropionate domain (see target system 3D). The need to deal with this C8 chiral center complicates all of the syntheses of epothilone disclosed thus far. Deletion of the C8 methyl group prompts a major change in synthetic strategy related to an earlier diene-aldehyde cyclocondensation route. Danishefsky, S. J. *Chemtracts* **1989,** 2, 273; Meng, D., *et al*., *J. Org. Chem.* **1996,** *61*, 7998; Bertinato, P., *et al*., *J. Org. Chem.* **1996,** *61*, 8000.

As shown in Fig. 20, asymmetric crotylation (87% ee) of **4D** (Brown, H. C.; Bhat, K. S. *J. Am. Chem. Soc.* **1986**, *108*, 5919), followed by protection led to TBS ether **5D.** The double bond was readily cleaved to give aldehyde **6D**. The aldehyde was coupled to the dianion derived from t-butyl isobutyrylacetate to provide **7D**. The ratio of the $C_{5S}$ (**7D**): $C_{5R}$ compound (not shown) is ca 10:1. That the Weiler-type β-ketoester dianion chemistry (Weiler, L *J. Am. Chem. Soc.* **1970,** *92,* 6702.; Weiler, L; Huckin, S. N. *J. Am. Chem. Soc.* **1974,** 96, 1082) can be conducted in the context of the isobutyryl group suggested several alternate approaches for still more concise syntheses. Directed reduction of the $C_3$ ketone of **7D** following literature precedents (Evans, D. A., *et al*., *J. Org. Chem.* **1991,** *56*, 741), followed by selective silylation of the $C_3$ hydroxyl gave a 50% yield of a 10:1 ratio of the required $C_{3S}$ (see compound **8D**) to $C_{3R}$ isomer (not shown). Reduction with sodium borohydride afforded a ca. 1:1 mixture of $C_3$ epimers. The carbinol, produced upon debenzylation, was oxidized to an aldehyde which, following methylenation through a simple Wittig reaction, afforded olefin **9D.** Treatment of this compound with TBSOTf provided ester **10D** which was used directly in the Suzuki coupling with the vinyl iodide **12D.**

The hydroboration of **10D** with 9-BBN produced intermediate **11D** which, on coupling with the vinyl iodide **12D** and *in situ* cleavage of the TBS ester led to **13D** (Fig. 21). After deacetylation, the hydroxy acid **14D** was in hand. Macrolactonization of this compound (Boden, E. P.; Keck, G. E. *J. Org. Chem.* **1985,** *50*, 2394) produced **15D** which,

after desilylation, afforded $C_8$-desmethyldesoxyepothilone **(16D).** Finally, epoxidation of this compound with dimethyldioxirane produced the goal structure **3D.** The stereoselectivity of epoxidation was surprisingly poor (1.5:1) given that epoxidation of desoxyepothilone A occurred with >20:1 stereoselectivity. Deletion of the $C_8$ methyl group appears to shift the conformational distribution of **16D** to forms in which the epoxidation by dimethyl dioxirane is less β-selective. It is undetermined whether the effect of the $C_8$ methyl on the stereoselectivity of epoxidation by dimethydioxirane and the dramatic reduction of biological activity are related.

Compounds **3D** and **16D** were tested for cytotoxicity in cell cultures and assembly of tubulin in the absence of GTP. Microtubule protein (MTP) was purified from calf brains by two cycles of temperature dependent assembly and disassembly. Weisenberg, R.C. *Science* **1972**, 177, 1104. In control assembly experiments, MTP (1 mg/mL) was diluted in assembly buffer containing 0.1 M MES (2-(N-morpholino) ethanesulfonic acid), 1 mM EGTA, 0.5 mM $MgCl_2$, 1mM GTP and 3M glycerol, pH 6.6. The concentration of tubulin in MTP was estimated to be about 85%. Assembly was monitored spectrophotometrically at 350 nm, 35°C for 40 min by following changes in turbidity as a measure of polymer mass. Gaskin, F.; Cantor, C.R..; Shelanksi, M.L.J. *Mol. Biol.* **1974,** 89, 737. Drugs were tested at a concentration of 10 μM, in the absence of GTP. Microtubule formation was verified by electron microscopy. To determine the stability of microtubules assembled in the presence of GTP or drug, turbidity was followed for 40 min after the reaction temperature was shifted to 4°C.

Cytotoxicity studies showed drastically reduced activity in the 8-desmethyl series. Compounds **3D** and **16D** were approximately 200 times less active than their corresponding epothilone A counterparts (see Table 1). Recalling earlier SAR findings at both $C_3$ and $C_5$, in conjunction with the findings disclosed herein, the polypropionate sector of the epothilones emerges as a particularly sensitive locus of biological function. Su, D.-S., *et al*., *Angew. Chem. Int. Ed. Engl.* **1997,** *36,* 757; Meng, D., *et al., J. Am. Chem. Soc.* **1997,** *119.*

Table 1.

Relative efficacy of epothilone compounds against drug-sensitive and resistant human leukemic CCRF-CEM cell lines.[a]

| Compound | CCRF-CEM $IC_{50}$ (μM)[b] | CCRF-CEM/VBL $IC_{50}$ (μM)[b] | CCRF-CEM/VM, $IC_{50}$ (μM)[b] |
|---|---|---|---|
| **16D** | 5.00 | 5.75 | 6.29 |
| **3D** | 0.439 | 2.47 | 0.764 |
| epothilone A | 0.003 | 0.020 | 0.003 |
| desoxyepothilone A | 0.022 | 0.012 | 0.013 |
| epothilone B | 0.0004 | 0.003 | 0.002 |
| desoxyepothilone B | 0.009 | 0.017 | 0.014 |
| paclitaxel | 0.002 | 3.390 | 0.002 |

[a]The cytotoxicities of test compounds were determined by the growth of human lymphoblastic leukemic cells CCRF-CEM, or their sublines resistant to vinblastine and Taxol® (CCRF-CEM/VBL) or resistant to etoposide (CCRF-CEM/VM-1). XTT-microculture tetrazolium/formazan assays were used.
[b]The $IC_{50}$ values were calculated from 5-6 concentrations based on the median-effect plot using computer software.

Desoxyepothiline B: an Effective Microtubule-targeted Antitumor Agent with a Promising *in Vivo* Profile Relative to Epothilone b

The epothilones have been synthesized as herein disclosed and evaluated for antitumor potential *in vitro* and *in vivo.* Epothilones and paclitaxel are thought to share similar mechanisms of action in stabilizing microtubule arrays as indicated by binding displacement studies, substitution for Taxol® in Taxol®-dependent cell growth, and electron microscopic examinations. Cell growth inhibitory effects have been determined in two rodent and three human tumor cell lines and their drug resistant sublines. While Taxol® showed as much as 1970-fold cross-resistance to the sublines resistant to Taxol®, adriamycin, vinblastine or adinomycin D, most ephothilones exhibit little or no cross-resistance. In multidrug resistant CCRF-CEM/$VBL_{100}$ cells, the 50% cell growth inhibitory concentrations ($IC_{50}$ values) for epothilone A, epothilone B, desoxyepothilone A, desoxy epothilone B and Taxol® were 0.02, 0.002, 0.012, 0.017 and 4.14 μM, respectively. *In vivo* studies, using i.p. administration, indicate that the parent, epothilone B, is highly toxic to mice with little therapeutic effect when compared with lead compound desoxyepothilone B (25-40 mg/kg, Q2Dx5, i.p.), which showed far superior therapeutic effect and lower toxicity than paclitaxel, doxorubicin, camptothecin or vinblastine (at maximal tolerated doses) in parallel experiments: In nude mice bearing a human mammary carcinoma xenograft (MX-1), marked tumor regression and cures have been obtained with desoxyepothilone B.

The isolation of the naturally occurring macrolides epothilone A and epothilone B from the myoxobaderia *Sorangium cellulosum* (Hoefle, G., *et al*., *Angew.Chem.Int.Ed.Engl.* 1996, *35*, 567-1569; Gerth, K., *et al*. *J. Antibiot*.

1996, 49, 560-563) and the subsequent demonstration of their ability to stabilize microtubule arrays *in vitro* elicited considerable interest in this class of compounds (Bollag, D.M., *et al*., *Cancer Res.* 1995, *55*, 2325-2333; Su, D.-S., *et al*., *Angew. Chem. Int. Ed. Engl*. 1997, *36*, 2093-2096; Meng, D., *et al*., *J.Am*. Chem. *Soc.* 1997, *119*, 2733-2734; Muhlradt, P.F. & Sasse, F. *Cancer Res.* 1997*, 57*, 3344-3346; Service, R.E. Science 1996, 274, 2009). We have recently conducted the total synthesis of these natural products as well as over 45 related analogs (Meng, D., *et al*., *J.* Am. *Chem. Soc*. 1997, *119*, 10073-10092; Su, D-S., *et al*., *Angew. Chem. Int. Ed. Engl.* 1997, *36*, 757-759; Chou, T.-C., Zhang, X.-G., & Danishefsky, S.J. *Proc.* Am. *Assoc. Cancer Res.* **1998,** *39*, 163-164) in order to investigate their chemical structure-biological activity relationships (Su, D.-S, *et al*., *Agnew. Chem. Int. Ed. Engl*. 1997, 36, 2093-2096). The studies disclosed herein allowed the characterization of the epothilone structure in three zones. Thus, in the C-1-8 acyl sector, the present inventors have determined that structural changes are not tolerated in terms of *in vitro* cytoxocity and microtubule stabilizing ability. This stands in contrast to the C-9-15 O-alkyl sector and the C-15 pendant aryl sectors wherein considerable modification of structures is tolerated (Su, D.-S., *et al*., *Angew.* Chem. *Int. Ed. Engl*. **1997,** *36*, 2093-2096; Meng, D., *et al*., 1997, *J.Am.Chem.Soc*. *119*, 10073-10092). Described herein are the results of *in vitro* and *in vivo* experiments on the Z-12,13 desoxy version of epothilone B (desoxyepothilone B).

It has been shown that the natural epothilones A and B have a similar mechanism of action to paclitaxel (Taxol®) although structurally diverse (Su, D.-S., *et al., Angew. Chem. Int. Ed. Engl.* **1997**, *36*, 2093-2096; Meng, D., *et al., J.Am.Chem.Soc.* **1997**, *19*, 2733-2734; Schiff, P.B., Fant, J. & Horwitz, S.B. *Nature* **1979**, *277*, 665-667; Landino, L.M. & MacDonald, T.L., *in: The Chemistry and Pharmacology of Taxol and Its Derivatives,* Favin, V., ed., Elsevier, New York 1995,, Chapter 7, p. 301). Paclitaxel, isolated from the Pacific yew tree (*Taxus brevifolia*), has been widely used clinically to treat a variety of solid cancers including neoplasms of ovary, breast, colon and lung (Landino, L.M. & MacDonald, T.L. *id*.; Rose, W.C. *Anti-Cancer Drugs,* 1992, 3, 311-321; Rowinsky, E.K., *et al*., *Seminars Oncol.* **1993**, *20,* 1-15). Epothilones A and B as well as Taxol® stabilize microtubule assemblies as demonstrated by binding displacement, substitution for paclitaxel in paclitaxel-dependent cell growth, and electron microscopic examinations (Bollag, D.M., *et al., Cancer Res*. **1995**, *55*, 2325-2333). Despite these similarities, the epothilones are more water soluble than paclitaxel, thereby offering potentially distinct advantages for formulation. Epothilones are more potent than paclitaxel in inhibiting cell growth, especially against cells expressing P-glycoprotein (Pgp) that are multidrug resistant (MDR), including cross-resistance to paclitaxel (Bollag, D.M., *id.;* Su, D.-S., *et al., Angew. Chem. Int. Ed. Engl*. **1997**, *36*, 2093-2096).

Materials and Methods

All stock solutions of the above (except VBL in saline) were prepared using dimethylsulfoxide (DMSO) as a solvent and were further diluted to desired concentrations for experimental use. The final concentration of DMSO in tissue culture was 0.25% (v/v) or less to avoid solvent cytotoxicity. For *in vivo* studies, paclitaxel in Cremophor-EtOH was further diluted with DMSO as needed. Vinblastine sulfate (Velban) (Eli Lilly & Co. Indianapolis, IN), and doxorubicin or adriamycin HCl (DX or Adr) (Pharmacia, Columbus, OH) in saline were diluted with DMSO as needed. DMSO was used as a vehicle for epothilones. Each mouse received ≤40μL DMSO in all experiments.

Cell Lines

The CCRF-CEM human T-cell acute lymphoblastic leukemia cell line and its vinblastine-resistant (CCRF-CEM/$VBL_{100}$) and teniposide-resistant (CCRF-CEM/$VM_1$) sublines (Cass, C.E., *et al*., 1989, Cancer *Res. 49*, 5798-5804; Danks, M.K., Yalowich, J.C., & Beck, W.T. *1987, Cancer Res. 47,* 1297-1301) were used. CCRF-CEM/Taxol® was developed by the present inventors following continuous exposure of CCRF-CEM cells with increasing concentrations of paclitaxel (at $IC_{50}$ - $IC_{90}$) for ten months. The fresh medium with paclitaxel was replenished every week. The CCRF-CEM/Taxol® exhibited 57-fold resistance to paclitaxel ($IC_{50}$ = 0.0021 μM, see Table 1 A). The DC-3F hamster lung fibroblast cell line and its actinomycin D-selected sublines (DC-3F/ADII and DC-3F/ADX) were obtained from the Memorial Sloan-Kettering Cancer Center (MSKCC). The murine leukemic P388/0 and its doxorubicin-setected subline (P388/DX) as well as human neuroblastoma SK-N-As and its doxorubicin-seleded subline (SK-N-FUAdr) were obtained from MSKCC.

The drug-resistant cell lines were continuously cultured in the presence of the selecting agent, AD, DX, VBL or VM to maintain the drug resistant phenotypes. Each sub-cell line was cultured for one to two passages in an appropriate concentration (e.g. $IC_{50}$) of the drug, which was then removed from the media and the cells were rested in fresh media for a minimum of 4 days before each assay. All cells were cultured in RPMI 1640-10% FBS at 37°C, 5% $CO_2$ (see below).

Cytotoxicity Assays

The cells were cultured at an initial density of 5 x $10^4$ cells/mL. They were maintained in a 5% $CO_2$-humidified atmosphere at 37°C in RPMI-1640 medium (GIBCO-BRL, Gaithersburg, MD) containing penicillin (100U/mL), streptomycin (100 mg/mL) (GIBCO-BRL) and 10% heat inactivate fetal bovine serum. Culture for cell suspension (such as for CCRF-CEM, P388 and sublines), were performed by the XTT-microculture tetrazonium method (Scudiero, D.A. *et al*., *Cancer Res.* 1988, *48*, 4827-4833) in duplicate in 96-well microtiter plates.

2',3'-Bis(methoxy-4-nitro-5-sufophenyl)-5-[(phenylamino)carbonyl]-2H-tetrazolium hyudroxide (XTT) was prepared at 1 mg/mL in prewarmed (37°C) medium without serum. Phenazine methosulfate (PMS) and fresh XTT were mixed together to obtain 0.025 mM PMS-XTT solution (25µL of the stock 5 mM PMS was added per 5 mL of 1 mg/mL XTT). Following a 72 h incubation, 50 µL of the assay aliquots were added to each well of the cell culture. After incubation at 37°C for 4h, absorbance at 450 nm and 630 nm was measured with a microplate reader (EL340, Bio-Tek Instruments, Inc., Winooski, VT).

The cytotoxicity of the drug toward the monolayer cell cultures (such as DC-3F, MCF-7, SK-N-As and sublines) was determined in 96-well microtiter plates by the SRB method as described by Skehan and co-workers (Skehan, P., *et al., J. Natl. Cancer Inst*. 1990, *82*, 1107-1112) for measuring the cellular protein content. Cultures were fixed with trichloroacetic acid and then stained for 30 min with 0.4% suforhodamine B dissolved in 1% acetic acid. Unbound dye was removed by acetic acid washes, and the protein-bound dye was extracted with an unbuffered Tris base (tris(hydroxy-methyl)aminomethane) for determination of absorbance at 570 nm in a 96-well microtiter plate reader. The experiments were carried out in duplicate. Each run entailed six to seven concentrations of the tested drugs. Data were analyzed with the median-effect plot (Chou, T.-C. & Talalay, P.T. (1984) *Adv. Enzyme Regul*. 22, 27-55) using a previously described computer program (Chou, J., & Chou T.-C. 1987, *Dose-effect* analysis *with microcomputers: Quantitation of* $ED_{50}$*, synergism, antagonism, low-dose risk, reception-ligand binding and enzyme kinetics,* IBM-PC software and manual, Biosoft, Cambridge, U.K.).

Stability of Desoxyepothilone B in Plasma

HPLC Method.

Sample Preparation. To 300 microliters of spiked plasma are added 30 microliters of methanol. The mixture is agitated and allowed to stand for 2 minutes. Then 600 microliters of methanol are added. The mixture is centrifuged. The supematant is removed for analysis by H PLC. Analyses were performed under the following chrmotographic conditions: Column: Nova-Pak C18, 15 cm. Eluant: 50% acetonitrile/water with 0.8% triethylamine, 0.2% phosphoric acid. Detection: UV (250 nm).

Animals

Athymic nude mice (nu/nu) were used for MX-1 and MCF-7/Adr human mammary carcinoma xenografts. Mice were obtained from Taconic Laboratory Animals and Service (Germantown, NY: outbred, Swiss background). Male mice 6-8 weeks old, weighing 20 -25g were used.

RESULTS

Structure-activity Relationships

To determine structure-activity relationships of epothilones (Su, D.-S., *et al*., *Angew. Chem. Int. Ed. Engl*. 1997, *36*, 2093-2096), the susceptibility of CCRF-CEM leukemic cells and the respective drug-resistant sublines CCRF-CEM/$VBL_{100}$ (Pgp-MDR cells) (Cass, C.E., *et al., Cancer Res.* 1989, *49*, 5798-5804) and CCRF/CEM/VM, (cells with a mutated topo II gene) (Danks, M.K., Yalowich, J.C., & Beck, W.T. *Cancer Res.* 1987, *47*, 1297-1301) to epothilones A and B and desoxyepthilone B (Table 1A) were determined. Although/$VBL_{100}$ is 527-fold resistant to VBL and 1970-fold resistance to paclitaxel, the epothilones A and B exhibited only 6.1-7.4-fold resistance, while desoxyepothilones A and B evidenced only 0.6-1.8-fold resistance. Using paclitaxel as the selecting agent, CCRF-CEM/Taxol® was grown 57-fold resistant and found to be 10.9-fold resistance to VBL. By contrast, DX, AD and VP-16 showed only 2.3-4.5 fold resistance, and epothilones A and B showed very little resistance (i.e., 1.4-3.1-fold) and desoxyepothilones A and B displayed almost no resistance (i.e., 0.7-1.7 fold) (Table 1A). CCRF-CEM/VM, cells that were 117-fold resistant to etoposide were sensitive to all epothilones or desoxyepothilones listed in Table 1A with only 0.6-3.6 fold resistance.

Table 1A.

| Susceptibility of CCRF-CEM and its drug resistant sublines to epothilone derivatives. | | | | | | | |
|---|---|---|---|---|---|---|---|
| Compound | (A) CCRF-CEM | (B) CCRF-CEM/ $VBL_{100}$ | (C) CCRF-CEM/ Taxol® | (D) CCRF-CEM/ VM, | (B)(A) | (C)(A) | (D)(A) |
| $IC_{50}$ (μM)* | | | | | | | |
| Epo A | 0.0027 | 0.020 | 0.0037 | 0.0061 | 7.4 | 1.4 | 2.3 |
| Epo B | 0.00035 | 0.0021 | 0.0011 | 0.0013 | 6.1 | 3.1 | 3.6 |
| dEpo A | 0.0220 | 0.012 | 0.0150 | 0.013 | 0.55 | 0.7 | 0.59 |
| dEpo B | 0.0095 | 0.017 | 0.0162 | 0.014 | 1.8 | 1.7 | 1.5 |
| Taxol® | 0.0021 | 4.140 | 0.120 | 0.0066 | 1971 | 57 | 3.1 |
| Vinblastine | 0.0063 | 0.332 | 0.0069 | 0.00041 | 527 | 10.9 | 0.7 |
| Etoposide | 0.290 | 10.30 | 1.32 | 34.4 | 35 | 4.5 | 117 |
| Adriamycin | 0.036 | 1.74 | 0.082 | 0.128 | 48 | 2.3 | 3.6 |
| Actinomycin D | 0.00035 | 0.038 | 0.0013 | 0.00027 | 109 | 3.7 | 0.8 |

*Cell growth inhibition was measured by XTT tetrazonium assay (Scudiero, D.A. *et al., Cancer Res.* 1988, *48,* 4827-4833) following 72 h incubation for cell growth as described previously. The $IC_{50}$ values were determined with 6-7 concentrations of each drug using a computer program (Chou, T.-C. & Talalay, P.T. (1984) *Adv. Enzyme Regul. 22*, 27-55); Chou, J., & Chou T.-C., *Dose-effect analysis with microcomputers: Quantitation of $ED_{50}$, synergism, antagonism, low-dose risk, reception-ligand binding and enzyme kinetics,* 1987, IBM-PC software and manual, Biosoft, Cambridge, U.K.)

Toxicity of dEpoB vs. EpoB

The toxicity of EpoB and dEpoB was compared in normal athymic nude mice on the daily i.p. schedule. EpoB at 0.6 mg/kg, QDX4, i.p. led to lethality in all eight mice. In contrast, in the group treated with dEpoB 25 mg/kg, QDX5, i.p., zero of six mice died. It was also observed that the vehicle treated control group showed a steady increase in body weight and the dEpoB treated mice maintained approximately the same average body weight, whereas the EpoB treated group showed steady decreases in body weight until death. These results indicated a higher toxicity for both EpoB and dEpoB than in tumor bearing nude mice when the treatment schedule was Q2Dx5, i.p. (see Tables 1C and 1D). In the preliminary studies, for the non-tumor bearing nude mice receiving EpoB 0.6 mg/kg or dEpoB 25 mg/kg, QDx4, i.p., there were no apparent changes in hematological cell counts or blood chemistry parameters except for a 43% decrease in lymphocytes. Similar leukopenia was found with paclitaxel. Some obstructive fecal mass in the large intestine was noted following Epo treatments in the preliminary study. No gross pathological abnormalities were observed in other organs.

Table 1B.

| Toxicity of Epothilone B, and Desoxyepothilone B in normal nude mice. | | | |
|---|---|---|---|
| Group | Dose schedule and route of administration | Number of mice | Number of mice died |
| Control | | 4 | 0 |
| Epothilone B | 0.6 mg/kg, QD x4, i.p. | 8 | 8* |
| Desoxyepothilone B | 25 mg/kg, QD x 4, i.p. | 6 | 0 |

* Mice died of toxicity on day 5,6,6,7,7,7,7,7

Comparison of Different Routes of Administration

Nude mice bearing human ovarian adenocarcinoma, SK-OV3, and human mammary adenocarcinoma, MX-1, were treated with dEpoB, both i.p. (DMSO as solvent) and i.v. (cremophor and EtOH, 1:1), with Taxol®, i.p. and i.v. (both with clinical samples in cremophor and EtOH as specified by the manufacturer), and with EpoB, i.v. (used cremophor and EtOH, 1:1). As shown in Table 6, for Q2Dx5 schedule, dEpoB, i.p. (35mg/kg) and Taxol® i.v. (15 mg/kg) both yield potent therapeutic effects against MX-1 with tumor-size on day 19, treated/control = 0.02 and 0.01, respectively (see Table 1F). For the ovarian tumor a lesser therapeutic effect was seen, tumor size on day 21, treated/control = 0.28 for both drugs (see Table 1G). For EpoB i.v., at 0.6 mg/kg there was less therapeutic effect and more toxicity than for dEpoB and Taxol®. In contrast, dEpoB, i.v. (15mg/kg) and Taxol®, i.p. (5mg/kg) showed more toxicity and less

therapeutic effect against both tumors. Thus, dEpoB showed the best results when given i.p. and Taxol® gave better results when given i.v. in cremophor and EtOH.

In Vitro Effect against Various Tumor Sublines

Further susceptibility evaluations were conducted for epothilones A and B and desoxyepothilones A and B in four additional tumor cell lines and four of their drug resistant sublines (Table 1C). Hamster lung tumor cells DC-3F/ADX, that were selected 13,000-fold resistant to AD, were found to be 328-fold resistant to paclitaxel and 124-fold resistant to DX when compared with the parent cell line (DC-3F). In contrast, epothilones A and B and desoxyepothilone A showed only 3.9-28-fold resistance, and epothilones A and B and desoxyepothilone B showed no cross-resistance (0.9-fold resistance).

Murine leukemic P388/Adr cells that were selected 482 -fold resistant to DX, were found to be 111-fold resistant to paditaxel. However, epothilones A and B showed less than 6-fold resistance, and for desoxyepothilone A and B there was no cross-resistance (<0.6-fold resistance).

Human neuroblastoma cells, SK-N-F1, that were selected as 18-fold resistant to DX, were found to be 80-fold resistant to paclitaxel. By contrast, epothilone B was 25-fold resistant, while the resistance of epothilone A and desoxyepothilones A and B was only between 1.9 and 3.1.

Human mammary carcinoma cells, MCF-7/Adr, that were selected 3.8-fold resistant to DX, were found to be 46-fold resistant to paclitaxel. In contrast, compounds epothilones A and B and desoxyepothilone B was 3.1-5.4-fold resistant, and dEpoB showed only 2.4-fold resistant. Overall, dEpoB was the least cross-resistant among epothilones and desoxyepothilones in various drug-resistant tumor sublines. By contrast, paclitaxel suffers from marked cross-resistance in tumor cells that were selected to be resistant to VBL, DX or AD. In three out of five cell lines studied, cross-resistance to paclitaxel was even greater than that of the selecting agents.

Therapeutic Effects against MX-1 Xenografts

Therapeutic effects of compounds epothilone A and desoxyepothilone B, paclitaxel, VBL and CPT were evaluated in athymic nude mice bearing human mammary adenocarcinoma MX-1 xenografts (Table 1D). Desoxyepothilone B at a 15 mg/kg dose i.p. on days 7, 9, 11, 13 and 15 produced a 50-60% tumor volume reduction when compared to the control group. A higher dose of drug, 25 mg/kg, produced as much as 96% average tumor volume reduction measured two days after the last treatment (i.e., on day 17). These effects were achieved with no lethality nor body weight reduction. Furthermore, with a 25 mg/kg dose, one out of six mice was tumor-free on day 35 after tumor implantation (i.e. on day 35). In contrast, after treatment with EpoB (0.3 mg/kg and 0.6 mg/kg, i.p., on days 7, 9, 11, 13 and 15), the average body weight decreased over 1 g and 2 g, respectively. In the case of 0.6 mg/kg treatment, three out of seven mice died of toxicity. Despite the apparent toxicity at these doses, EpoB appeared to have only marginal therapeutic effect, as only 16% to 26% tumor volume reduction was observed (Table 1D). The parallel experiments for paclitaxel led to a lower therapeutic effect. In animals treated with paclitaxel, 5 mg/kg, there was 55% reduction in tumor volume and no decrease in average body weight. At a dose of 10 mg/kg, paclitaxel showed a 89% tumor reduction, however, four out of seven mice died of toxicity. For DX (2-3 mg/kg) and CPT (1.5-3 mg/kg) i.e., near the maximal tolerated doses, inferior results were obtained when compared with dEpoB. Thus, dEpoB even at non-toxic dose had the best therapeutic effect among the five compounds studied under the same experimental conditions.

In a separate experiment, MX-1 xenograft-bearing mice were treated with dEpoB, 35 mg/kg, Q2Dx5, i.p. beginning on day 8 after tumor implantation (Fig. 60). On day 16, two out of ten mice had no detectable tumor. These ten mice were further treated with dEpo B, 40 mg/kg, Q2Dx5 beginning on day 18. At the end of treatment on day 26, five out of ten mice had no detectable tumor, and three remained tumor-free on day 60. There was body weight reduction during treatments but no lethality occurred. In a parallel experiment, ten mice were treated with paclitaxel, 5 mg/kg, Q2Dx5, i.p. from day 8 to day 16, followed by a second cycle of treatment in the same manner from day 18 to day 26. The tumor sizes were reduced but continued to grow during treatment and by day 24, the average tumor size was $2285 \pm 597$ mm$^3$ (n = 10). In a parallel experiment, DX was given 2 mg/kg, Q2D×5, i.p. (Fig. 60), and reduced therapeutic effect was seen compared to dEpoB or paclitaxel. No data after day 18 is shown because the tumor burden in the control group was excessive and the mice in this group were sacrificed.

**Therapeutic Effects against MCF-7/Adr Xenografts**

The therapeutic effects of dEpoB, Taxol®, DX arid CPT were also evaluated in nude mice bearing xenografts of human mammary adenocarcinoma resistant to DX (MCF-7/Adr) (Table 1E). As indicated earlier in Table 18 for the cytotoxicity results *in vitro*, MCF-7/Adr cells selected to be 3.8-fold resistant to DX were found to be 46-fold resistant to paclitaxel, and only 2.4-fold resistant to dEpoB. For *in vivo* studies, each drug was given Q2Dx5, i.p. beginning on

day 8 after tumor implantation. Paclitaxel 12 mg/kg and DX 3 mg/kg were highly toxic to the nude mice with 3/7 and 3/6 lethality, respectively. CPT 3 mg/kg led to moderate toxicity without lethality, and dEpoB 35 mg/kg showed negligible toxicity as shown by minimal body weight changes (Table 1 E). CPT at 3 mg/kg reduced 57% of tumor size on day 17 ($p < 0.05$ when compared with control group). Desoxyepothilone B at 35 mg/kg significantly suppressed tumor size by 66-73% when compared with the control group ($p < 0.005$-0.05), without complete tumor regression. In contrast, paclitaxel 5 mg/kg, and DX 2 mg/kg, produced slight growth suppression of this drug-resistant tumor which was not significantly different from the control group (see Table 1D). Thus, dEpoB stands out as the superior drug among the four tested against this drug-resistant tumor.

Table 1C. Comparison of in vitro growth inhibition potency of epothilone derivatives against various parent and drug resistant tumor cell lines.

| Compound | DC-3F | DC-3F/ADX | P388/0 | P388/Adr | SK-N-As | SK-N-FI | MCF-7 | MCF-7/Adr |
|---|---|---|---|---|---|---|---|---|
| | | | | $IC_{50}$ (μM)[*] | | | | |
| Epo A | 0.0037 | 0.053 (14.5x)' | 0.0018 | 0.0010 (5.3x)' | 0.012 | 0.023 (1.9x)' | 0.0030 | 0.0094 (3.1x)' |
| Epo B | 0.0006 | 0.017 (28x) | 0.00029 | 0.0016 (5.5x) | 0.004 | 0.010 (25x) | 0.0005 | 0.0027 (5.4x) |
| dEpo A | 0.011 | 0.042 (3.9x) | 0.0213 | 0.0125 (0.59x) | 0.073 | 0.223 (3.1x) | 0.032 | 0.144 (4.5x) |
| dEpo B | 0.00097 | 0.00091 (0.9x) | 0.0068 | 0.0042 (0.62x) | 0.021 | 0.046 (2.2x) | 0.0029 | 0.0071 (2.4x) |
| Taxol® | 0.095 | 32.0 (338x) | 0.0029 | 0.326 (111x) | 0.0016 | 0.130 (80x) | 0.0033 | 0.150 (46x) |
| Actinomycin D | 0.00044 | 0.572 (13000x) | 0.00015 | 0.0012 (8x) | 0.00085 | 0.0119 (14x) | 0.00068 | 0.00167 (2.5x) |
| Adriamycin | 0.018 | 2.236 (124x) | 0.0055 | 2.65 (482x) | 0.077 | 1.42 (18.4x) | 0.057 | 0.216 (3.8x) |

[*] Cell growth inhibition was measured by protein staining SRB assay (Skehan, P., *et al., J. Natl. Cancer Inst.* 1990, *82*, 1107-1112) following 72 h incubation as described previously. The $IC_{50}$ values were determined with 6-7 concentrations of each drug using a computer program (Chou, T.-C. & Talalay, P.T., *Adv. Enzyme Regul.* 1984, *22*, 27-55; Chou, J., & Chou T.-C., *Dose-effect analysis with microcomputers: Quantitation of $ED_{50}$, synergism, antagonism, low-dose risk, reception-ligand binding and enzyme kinetics*, 1987, IBM-PC software and manual. Biosoft, Cambridge, U.K.). ' Numbers in parentheses are folds of resistance based on the $IC_{50}$ ratio when compared with the corresponding parent cell lines except for P388/0 and P388/Adr, XTT assay (Scudiero, D.A., *et al., Cancer Res.* 1988, *48*, 4827-4833) was used.

Table 1D. Therapeutic effect of desoxyopothilone B, epothilone B, Taxol®, vinblastine and camptothecin in nude mice bearing human MX-1 zenograft.

| Drug | Dose (mg/kg) | Average Body Weight Change (g) | | | | | Average Tumor Size (T/C) | | | | Toxicity Death | N |
|---|---|---|---|---|---|---|---|---|---|---|---|---|
| | | Day 7 | 11 | 13 | 15 | 17 | Day 11 | 13 | 15 | 17 | | |
| Control | | 27.2 | +0.8 | +1.1 | +1.9 | +0.6 | 1.00 | 1.00 | 1.00 | 1.00 | 0/8 | 8 |
| dEpo B | 15 | 27.1 | +0.8 | +1.1 | +1.6 | +1.5 | 0.65 | 0.46 | 0.49** | 0.41** | 0/6 | 6 |
| | 25' | 27.0 | +0.4 | +0.7 | +1.0 | +0.7 | 0.38* | 0.11** | 0.05*** | 0.04**** | 0/6 | 6 |
| Epo B | 0.3 | 26.9 | +0.5 | +0.4 | -0.3 | -1.2 | 1.00 | 0.71 | 0.71 | 0.84 | 0/7 | 7 |
| | (0.6' | 27.4 | -0.3 | -1.3 | -2.1 | -2.1 | 1.08 | 0.73 | 0.81 | 0.74 | 3/7)'' | 7 |
| Taxol® | 5 | 26.9 | -0.1 | +0.4 | +1.1 | +1.2 | 0.54 | 0.46 | 0.40* | 0.45** | 0/7 | 7 |
| | 10' | 27.6 | -2.7 | -1.1 | -0.3 | +2.2 | 0.43 | 0.37 | 0.12 | 0.11 | 4/7'' | 7 |
| Vinblastine | 0.2 | 25.7 | +0.6 | +1.4 | +2.3 | +2.9 | 0.65 | 0.54 | 0.56 | 0.88 | 0/7 | 7 |
| | (0.4[1] | 26.4 | +0.8 | +0.5 | +1.9 | +2.1 | 0.80 | 0.56 | 0.83 | 0.88 | 1/7)'' | 7 |
| Campothecin | 1.5 | 27.4 | -0.9 | -0.7 | -0.4 | +1.0 | 0.61 | 0.45* | 0.32* | 0.36** | 0/7 | 7 |

MX-1 tissue 50 μl/mouse was implanted s.c. on day 0. Every other day i.p. treatments were given on days 7, 9, 11, 13, 15; The average tumor volumes of the control group on day 11, 13, 15 and 17 were 386±120, 915±245, 1390±324, and 1903±319 $mm^3$ (mean±SEM), respectively; * $P<0.05$, ** $P<0.01$, *** $P<0.005$, **** $P<0.001$; 'One out of six mice with no detectable tumor on day 35; ' Three mice died of drug toxicity on day 17; ' Four mice died of drug toxicity on day 13, 13, 13, 15; [1]One mouse died of drug toxicity on day 15; ''P values were not shown due to toxic lethality.

Table 1E. Therapeutic effects of desoxyopothilone B, epothilone B, taxol, adriamycin and camptothecin in nude mice bearing MDR human MCF-7/Adr tumor.

| Drug | Dose (mg/kg) | Average Body Weight Change (g) | | | | | Average Tumor Size (T/C) | | | | Toxicity Death | N |
|---|---|---|---|---|---|---|---|---|---|---|---|---|
| | | Day 8 | 11 | 13 | 15 | 17 | Day 11 | 13 | 15 | 17 | | |
| Control | 0 | 25.0 | +2.0 | +2.6 | +3.1 | +3.7 | 1.00 | 1.00 | 1.00 | 1.00 | 0/8 | 8 |
| dEpo B | 35 | 25.0 | 0.3 | +0.7 | +0.6 | +0.8 | 0.31** | 0.27*** | 0.30*** | 0.34* | 0/8 | 8 |
| Taxol® | 6 | 25.3 | +1.7 | +1.8 | +0.8 | +0.9 | 0.57 | 0.66 | 0.85 | 0.90 | 0/7 | 7 |
| | (12 | 24.5 | -0.7 | -1.3 | -2.4 | 0 | 0.50 | 0.51 | 0.32 | 0.40 | 3/7 | 7)' |
| Adriamycin | 2 | 25.6 | +0.2 | -0.4 | -0.6 | -0.4 | 0.70 | 0.68 | 0.84 | 0.78 | 0/8 | 8 |
| | (3 | 24.6 | +0.5 | -1.3 | -3.2 | -1.6 | 0.66 | 0.83 | 0.57 | 0.53 | 3/6 | 6)' |
| Campothecin | 1.5 | 24.4 | +1.1 | +0.9 | +1.7 | +1.4 | 1.08 | 0.72 | 0.61 | 0.72 | 0/8 | 8 |
| | (3 | 24.5 | -0.6 | -0.4 | -0.8 | -0.9 | 0.95 | 0.76 | 0.61 | 0.43* | 0/6 | 6 |

MCF-7/Adr cell 3 x $10^6$/mouse was implanted s.c. on day 0. Every other day ip treatments were given on days 8, 10, 12, 14 and 16. The average tumor size of control group on day 11, 13, 15 and 17 was 392"±84, 916±210, 1499±346, and 2373±537$mm^3$ respectively (mean±SEM). * <P0.05, **P≤0.01, ***P<0.005; ' P values were not shown due to lethality.

Table 1F. Therapeutic effects of desoxyopothilone B, Epo B and Taxol® in nude mice bearing MX-1 tumors using different vehicles and different routes of administration.[a]

| Drug/Route | Dose (mg/kg) | Average Body Weight Change (g) | | | | | Average Tumor Size (T/C) | | | | Tumor Disapp. | Toxicity Death |
|---|---|---|---|---|---|---|---|---|---|---|---|---|
| | | Day 9 | 13 | 15 | 17 | 19 | Day 13 | 15 | 17 | 19 | | |
| Control | 0 | 26.4 | -0.2 | -0.4 | +0.2 | +0.8 | 1.00 | 1.00 | 1.00 | 1.00 | 0/6 | 0/6 |
| dEpo B/i.p. | 35 | 27.8 | -1.7 | -2.1 | -2.1 | -2.4 | 0.35 | 0.14 | 0.04 | 0.02 | 3/6 | 0/6 |
| dEpo B/i.v. | 15 | 27.0 | 0 | -0.6 | -1.1 | -2.6 | 0.47 | 0.30 | 0.10 | 0.04 | 0/6 | 4/6[b] |
| Epo B/i.p. | 0.6 | 27.0 | -0.9 | -0.5 | -3.3 | -3.4 | 0.67 | 0.63 | 0.61 | 0.51 | 0/6 | 0/6 |
| Taxol®/i.p. | 5 | 27.4 | -1.1 | -2.0 | -1.0 | -0.2 | 0.59 | 0.72 | 0.59 | 0.55 | 0/6 | 0/6 |
| Taxol®/i.v. | 15 | 27.2 | -0.6 | -0.8 | -0.8 | -0.9 | 0.36 | 0.13 | 0.04 | 0.01 | 2/6 | 0/6 |

a: 50 μg tumor tissue was implanted s.c. on day 0. Every other day i.p. or i.v. treatments were given on days 9, 11, 13 , 15 and 17. The average tumor size of control group on day 13, 15, 17 and 19 was 274, 378, 677, 1139mm³ respectively.

b: 4/6 mice died of drug toxicity on day 23, 23, 23, 25.

Table 1G: Therapeutic effects of desoxyopothilone B, Epo B and Taxol® in nude mice bearing SK-OV-3 tumors using different vehicles and different routes of administration.[a]

| Drug/Route | Dose (mg/kg) | Average Body Weight Change (g) | | | | | Average Tumor Size (T/C) | | | | Tumor Disapp. | Toxicity Death |
|---|---|---|---|---|---|---|---|---|---|---|---|---|
| | | Day 9 | 13 | 15 | 17 | 19 | Day 15 | 17 | 19 | 21 | | |
| Control | 0 | 26.4 | -0.2 | -0.4 | +0.2 | +0.8 | 1.00 | 1.00 | 1.00 | 1.00 | 0/6 | 0/6 |
| dEpo B/i.p. | 35 | 27.8 | -1.7 | -2.1 | -2.1 | -2.4 | 0.57 | 0.33 | 0.35 | 0.28 | 0/6 | 0/6 |
| dEpo B/i.v. | 15 | 27.0 | 0 | -0.6 | -1.1 | -2.6 | 0.86 | 0.56 | 0.50 | 0.44 | 0/6 | 4/6[b] |
| Epo B/i.p. | 0.6 | 27.0 | -0.9 | -0.5 | -3.3 | -3.4 | 0.75 | 0.69 | 0.88 | 0.77 | 0/6 | 0/6 |
| Taxol®/i.p. | 5 | 27.4 | -1.1 | -2.0 | -1.0 | -0.2 | 0.69 | 0.60 | 0.49 | 0.40 | 0/6 | 0/6 |
| Taxol®/i.v. | 15 | 27.2 | -0.6 | -0.8 | -0.8 | -0.9 | 0.97 | 0.67 | 0.42 | 0.28 | 0/6 | 0/6 |

a: 50 µg tumor tissue was implanted s.c. on day 0. Every other day i.p. or i.v. treatments were given on days 9, 11, 13, 15 and 17. The average tumor size of control group on day 13, 15, 17 and 19 was 274, 378, 677, 1139mm³ respectively.

b: 4/6 mice died of drug toxicity on day 23, 23, 23, 25.

## DISCUSSION

Two classes of naturally occurring compounds, epothilones and paclitaxel, which are apparently structurally dissimilar, show similar modes of action in stabilizing microtubule assemblies. These similarities include binding tubulin, substitution for paclitaxel in maintaining paclitaxel-dependent cell growth in a resistant cell line, and similar morphologic changes as determined by electron microscopic examination of the drug-microtubule complex. There are differences, however, between the two classes of compounds. These differences are most prominently exhibited by the lack of cross-resistance in cytotoxicity between the epothilones and paclitaxel even in CCRF-CEM/Taxol® cells (Table 1A). Furthermore, in CCRF/CEM/$VBL_{100}$, the cells were 527-fold resistant to vinblastine and 1971-fold resistance to paclitaxel, but were only 6.1-fold resistant to EpoB and 1.8-fold resistant to dEpoB (Table 1A). In DC-3F/ADX cells, there was 13,000-fold resistance to actinomycin D and 338-fold resistance to paclitaxel. However, these cells were only 28-fold resistance to EpoB and had no resistance to dEpoB (i.e., 0.9-fold resistance or collateral sensitivity) (Table 1B). Paclitaxel showed a higher degree of cross-resistance in these cell lines than other MDR-drugs such as doxorubicin, actinomycin D, vinblastine or etoposide. In some cases the degrees of resistance to paclitaxel were even greater than those of the resistance-selecting agent (e.g., CCRF-CEM/$VBL_{100}$ in Table 1A, and SK-N-FI and MCF/7-Adr in Table 1B). In contrast, among all compounds tested, dEpoB showed the least cross-resistance in several drug-resistant cell lines (e.g. DC-3F/Adr) and even showed slight collateral sensitivity (e.g. DC-3F/ADX and P388/Adr in Table 1B). Parallel cancer chemotherapeutic studies for EpoB, dEpoB, Taxol® and other drugs were performed under the same experimental conditions (i.e., treatment schedule, Q2D; solvent vehicle, DMSO; and route of administration, i.p.) in animals.

The i.p. route of other formulations for administration of dEpo B is far better tolerated than the i.v. method. Even though EpoB is the most potent, it is by no means the best candidate for cancer therapy in terms of therapeutic index (i.e. the therapeutic efficacy at tolerable dosage, or the ratio of toxic dose vs the therapeutic dose). Desoxyepothilone B, lacking the epoxide functionality, exhibited far superior therapeutic results *in vivo* as compared to the more potent EpoB. Similarly, the present therapeutic results for dEpoB in MX-1 xenografts were far better than those for EpoB, paclitaxel, doxorubicin, vinblastine or camptothecin. In addition, the effects of dEpo B on MCF-7/Adr xenografts were significantly better than those for paclitaxel, doxorubicin and camptothecin. In view of the finding that the epothilones have little or no cross-resistance against MDR tumor cells *in vitro*, the special therapeutic advantage of such compounds might be in their use against MDR-resistant tumors.

Biological Results

In the tables which follow, model system I is desoxyepothilone. Model system 2 has the structure:

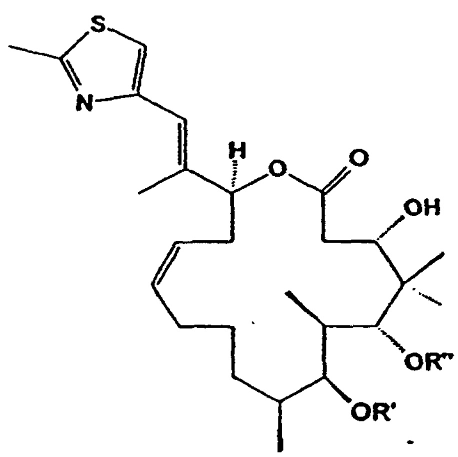

wherein R' and R" are H.

Model system 3 has the structure:

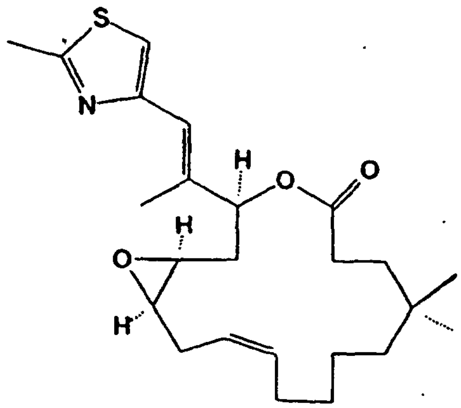

As shown in Table 2A, CCRF-CEM is the parent cell line. CCRF-CEM/VBL (MDR cell line) is 1143-fold resistant to Taxol®. CCRF-CEM/VM (Topo II mutated cell line) only 1.3-fold resistant to Taxol®.

In terms of relative potency, synthetic Epothilone is roughly the same as natural Epothilone A. For CCRF-CEM cells, the ordering is:

Taxol® ≈ Epothilone A > Desoxy Epothilone A > > Triol Analog > > Model System I

For CCRF-CEM/VBL, the relative potency ordering is:

Desoxy Epothilone A ≥ Epothilone A>> Taxol® >Triol Analog >Model System I

For CCRF-CEM/VM, the relative potency ordering is:

Taxol® = Epothilone A > Desoxy Epothilone A > > Model System I > Triol Analog

It is concluded that CCRF-CEM/VM cells are collaterally sensitive to certain epothilone compounds.

**Table 2.** Relative Efficacy of Epothilone Compound Against HumanLeukemic CCRF-CEM Cell Growth and Against CCRF-CEM MDR Sublines Resistant to Taxol® or Etoposide

| COMPOUND | $IC_{50}$ in μM | | |
|---|---|---|---|
| | CCRF-CEM | CCRF-CEM/VLB | CCRF-CEM/VM-1 |
| EPOTHILONE A NATURAL | 0.0035 | 0.0272 | 0.0034 |
| EPOTHILONE A SYNTHETIC | 0.0029 | 0.0203 | 0.0034 |
| MODEL SYSTEM I [3] | 271.7 | 22.38 | 11.59 |
| TRIOL ANALOG [2] | 14.23 | 6.28 | 43.93 |
| DESOXY EPOTHILONE [1] | 0.002 | 0.012 | 0.013 |
| Taxol® | 0.0023 | 2.63 | 0.0030 |
| VINBLASTINE | 0.00068 | 0.4652 | 0.00068 |
| VP-16 (ETOPOSIDE) | 0.2209 | 7.388 | 34.51 |

Table 2A. Relative Potency of Epothilone Compounds Against Human Leukemic CCRF Sublines

| COMPOUND | CCRF-CEM (Parent Cell Line) | CCRF-CEM/VBL (MDR Cell Line) (Taxol® Resistant)-(1143 fold) (Vinblastine Resistant) | CCRF-CEM/VM, (Topo II gene mutated cell line) (Taxol® Sensitive) (VP-16 resistant) |
|---|---|---|---|
| | $IC_{50}$ [ $IC_{50}$ (μM) relative to (A) Epothilone A ] | $IC_{50}$ [$IC_{50}$ (μM) relative to (B) Epothilone A (B)/(A)] | $IC_{50}$ [$IC_{50}$ (μM) relative to (C) Epothilone A (C)/(A)] |
| Taxol® | 0.0023 [0.72] | 2.63 [109.6] (1143)[a] | 0.0030 [0.88] (1.30)[a] |
| MODEL SYSTEM I | 271.7 [84906] | 22.38 [932.5] (0.082)[b] | 11.59 [3409] (0.043)[b] |
| TRIAL ANALOG | 14.23 [4447] | 6.28 [261.7] (0.44)[b] | 43.93 [12920] (3.09)[a] |
| DESOXYEPO-THILONE A | 0.022 [6.9] | 0.012 [0.5] (0.55)[b] | 0.013 [3.82] (0.59)[b] |
| EPOTHILONE A | 0.0032 [1] | 0.024 [1] (7.5)[a] | 0.0034 [1] (1.06)[a] |

a. (B)/(A) or (C)/(A) ratio > 1 indicates fold of resistance when compared with the parent cell line. b. (B)/(A) or (C)/(A) ratio < 1 indicates fold of collateral sensit when compared with the parent cell line.

**Table 3.** Relative Efficacy of Epothilone Compounds Against The DC-3F Hamster Lung Cell Growth and Against DC-3F MDR Sublines Resistant Actinomylin D

| COMPOUNDS | $IC_{50}$ in $\mu M$ | | |
|---|---|---|---|
| | DC-3F | DC-3F/ADII | DC-3F/ADX |
| EPOTHILONE A NATURAL | 0.00368 | 0.01241 | 0.0533 |
| EPOTHILONE A SYNTHETIC | 0.00354 | 0.0132 | 0.070 |
| MODEL SYSTEM I [3] | 9.52 | 3.004 | 0.972 |
| TRIOL ANALOG [2] | 10.32 | 4.60 | 4.814 |
| DESOXY EPOTHILONE [1] | 0.01061 | 0.0198 | 0.042 |
| Taxol® | 0.09469 | 3.205 | 31.98 |
| VINBLASTINE | 0.00265 | 0.0789 | 1.074 |
| VP-16 (Etoposide) | 0.03386 | 0.632 | 12.06 |
| ACTINOMYCIN-D | 0.000058 (0.05816nm) | 0.0082 | 0.486 |

Concerning Table 3, experiments were carried out using the cell lines DC-3F (parent hamster lung cells), DC-3F/ADII (moderate multidrug-resistant (MDR) cells) and DC-3F/ADX (very strong MDR cells).
The relative potency of the compounds are as follows:

| | |
|---|---|
| DC-3F: | Actinomycin D > Vinblastine ≥Epothilone A (0.0036μM) > Desoxy epothilone >VP-16> Taxol® (0.09μM) > Model system I and triol analog |
| DC-3F/ADX: | Desoxyepothilone ≥ Epothilone A (0.06 μM)> Actinomycin D >Model system I > Vinblastine >triol analog >viablastine > Taxol® (32.0 μM) |

DC-3F/ADX cells (8379-fold resistant to actinomycin D) are > 338 fold (ca. 8379 fold) resistant to Taxol®, VP-16, Vinblastine and Actinomycin D but < 20 fold resistant to epothilone compounds.
In general, these results are similar to those for CCRF-CEM cells.

**Table 4.** Three Drug Combination Analysis

(Based on the Mutually Exclusive Assumption – Classical Isobologram Method)

Drug A: EPOTHILONE B (#8) (μM)

Drug B: Taxol® (μM)

Drug C: VINBLASTINE (μM)

Conditions: CCRF-CEM, 3 DRUG COMBINATION, RATIO (A:B:C: 1:5:1); EPOTHILONE + Taxol® + VINBLASTINE; EXPOSURE TIME 72 HRS; XTT ASSAY.

| Drug | Combination Index* Values at: | | | | Parameters | | |
|---|---|---|---|---|---|---|---|
| | ED50 | ED75 | ED90 | ED95 | Dm ($IC_{50}$) (μM) | m | r |
| A | | | | | -00061 | 1.71561 | .98327 |
| B | | | | | -00109 | 2.14723 | .98845 |
| C | | | | | -00061 | 1.76186 | .9919 |
| A+B | 1.51545 | 1.38631 | 1.27199 | 1.20162 | -00146 | 2.41547 | .97168 |
| B+C | 1.43243 | 1.33032 | 1.23834 | 1.18091 | .00138 | 2.35755 | .95695 |
| A+C | .74395 | .68314 | .62734 | .59204 | .00045 | 2.0098 | .96232 |
| A+B+C | 1.37365 | 1.32001 | 1.27285 | 1.24412 | .00122 | 2.11202 | .93639 |

VBL → microtubule depolymerization

Taxol® → microtubule polymerization

Epo-B → microtubule polymerization

Epothilone B and Taxol® have a similar mechanism of action (polymerization) but Epothilone B synergizes VBL whereas Taxol® antagonizes VBL.

Taxol®+VBL → Antagonism

EpoB + Taxol® → Antagonism

EpoB + VBL → Synergism

EpoB + Taxol® + VBL → Antagonism

*Combination index values <1, =1, and >1 indicate synergism, additive effect, and antagonism, respectively.

**Table 5.** Relative cytotoxicity of epothilone compounds *in vitro*.

| Compounds | $IC_{50}$ in $\mu M$ | | |
|---|---|---|---|
| | CCRF-CEM | CCRF-CEM/VLB | CCRF-CEM/VM-1 |
| VINBLASTINE | **** 0.0008<br>0.0006 (0.00063<br>0.0005 ±0.00008) | 0.44<br>0.221 (0.332<br>0.336 ±0.063<br>(52.7X)§ | 0.00049<br>0.00039 (0.00041<br>0.00036 ±0.00004)<br>(0.7X) |
| VP-16 | 0.259<br>0.323 (0.293<br>0.296 ± 0.019) | 6.02<br>9.20 (10.33<br>15.76 ± 2.87)<br>(35.3X) | 35.05<br>42.24 (34.39<br>25.89 ± 4.73)<br>(117.4X) |
| Taxol® | *** 0.0021 | 4.14 | 0.0066 |
| #17 | * 0.090 | 0.254 | |
| #18 | 1157.6 | >>1 | |
| #19 | 0.959 | >>1 | |
| #20 | * 0.030 | 0.049 | |
| #21 | | | |
| #22 | * 0.098 | 0.146 | |
| #23 | | | |
| #24 | *** 0.0078 | 0.053 | |
| #25 | * 0.021 | 0.077 | |
| #26 | * 0.055 | 0.197 | |
| #27 | **** 0.0010 | 0.0072 | |
| Epothilone A (Syn) | *** 0.0021 | 0.015 | |
| Epothilone B (Syn) | **** 0.00042 | 0.0017 | |

* Number of asterisks denotes relative potency.

§ Number in parentheses indicates relative resistance (fold) when compared with parent cell line.

**Table 6.** Relative potency of epothilone compounds *in vitro*.

| Compounds | | $IC_{50}$ in $\mu M$ | | |
|---|---|---|---|---|
| | | CCRF-CEM | CCRF-CEM/VBL | CCRF-CEM/VM-1 |
| Desoxy Epo. A | 1 | * 0.022 | 0.012 | 0.013 |
| | 2 | 14.23 | 6.28 | 43.93 |
| | 3 | 271.7 | 22.38 | 11.59 |
| | 4 | 2.119 | 43.01 | 2.76 |
| | 5 | >20 | 35.19 | 98.04 |
| Trans- A | 6 | 0.052 | 0.035 | 0.111 |
| | 7 | 7.36 | 9.82 | 9.65 |
| Syn-Epo.-B | 8 | **** 0.00082 | 0.0029 | 0.0044 |
| Natural B | 9 | **** 0.00044 | 0.0026 | 0.0018 |
| Desoxy Epo. B | 10 | *** 0.0095 | 0.017 | 0.014 |
| Trans. Epo. B | 11 | * 0.090 | 0.262 | 0.094 |
| | 12 | 0.794 | >5 | >5 |
| | 13 | 11.53 | 5.63 | 14.46 |
| 8-desmethyl desoxy-Epo | 14 | 5.42 | 5.75 | 6.29 |
| 8-desmethyl Mix-cis Epo | 15 | 0.96 | 5.95 | 2.55 |
| 8-desmethyl β-Epo | 15 | 0.439 | 2.47 | 0.764 |
| 8-demethyl α-Epo | 16 | 7.47 | 16.48 | 0.976 |
| EPOTHILONE A (Natural) | | *** 0.0024 (0.0027<br>0.0031 ±0.0003) | 0.0211 (0.020<br>0.0189 ±0.001)<br>(7.4X) | 0.006 (0.00613<br>0.00625 ±0.0001)<br>(2.3X) |
| EPOTHILONE B (Natural) | | **** 0.00017 | 0.0017 (7.0X) | 0.00077 |
| EPOTHILONE B (Synthetic) | | 0.00055 | 0.0031 | 0.0018 |
| EPOTHILONE B (Synthetic, larger quantity synthesis) (25.9mg) | | (0.00035<br>± 0.0003)<br>0.00033 | (0.00213<br>±0.00055)<br>0.0021 (6.1X) | (0.00126<br>± 0.0003)<br>0.0012 (3.6X) |

**Table 7.** Relative cytotoxicity of epothilone compounds *in vitro*.

| | $IC_{50}$ | |
|---|---|---|
| | CEM | CEM/VBL |
| epothilone A | 0.0029 μM | 0.0203 μM |
| desoxyepothilone | 0.022 | 0.012 |
| 2 | 14.2 | 6.28 |
| 3 | 271.7 | 22.4 |
| 4 | 2.1 | 43.8 |
| 5 | >20 | 35.2 |
| 6 | 0.052 | 0.035 |
| 7 | 7.4 | 9.8 |
| synthetic epothilone B | 0.00082 | 0.00293 |
| natural epothilone B | 0.00044 | 0.00263 |
| desoxyepothilone B | 0.0095 | 0.0169 |
| 11 | 0.090 | 0.262 |
| 12 | 0.794 | >5 |
| 13 | 11.53 | 5.63 |
| 14 | 5.42 | 5.75 |
| 15 | 0.439 | 2.47 |
| 16 | 7.47 | 16.48 |
| 17 | 0.090 | 0.254 |
| 18 | 1157.6 | >>1 |
| 19 | 0.959 | >>1 |
| 20 | 0.030 | 0.049 |
| 21 | Not Available | - |
| 22 | 0.098 | 0.146 |
| 23 | Not Available | - |
| 24 | 0.0078 | 0.053 |
| 25 | 0.0212 | 0.077 |
| 26 | 0.0545 | 0.197 |
| 27 | 0.0010 | 0.0072 |

Table 8. Chemotherapeutic Effect of Epothilone B, Taxol® & Vinblastine in CB-17 Scid Mice Bearing Human CCRF-CEM and CCRF-CEM/VBL Xenograft[1]

| Tumor | Drug[2] | Dose | Average weight change | | | | | Average tumor volume | | | |
|---|---|---|---|---|---|---|---|---|---|---|---|
| | | | Day 0 | Day 7 | Day 12 | Day 17 | Day 22 | Day 7 | Day 12 | Day 17 | Day 22 |
| CCRF-CEM | | 0 | 24.4 | +0.2 | +0.4 | +0.1 | +0.5 | 1.0[3] | 1.00 | 1.00 | 1.00 |
| | Epo B | 0.7[4] | 24.7 | -0.1 | -0.7 | -1.4 | +0.3 | 1.0 | 0.53 | 0.48 | 0.46 |
| | | 1.0[5] | 25.0 | +0.1 | -1.5 | -2.4 | +0.1 | 1.0 | 0.46 | 0.35 | 0.43 |
| | Taxol® | 2.0 | 25.1 | -0.1 | -1.1 | -1.5 | -0.3 | 1.0 | 0.39 | 0.29 | 0.28 |
| | | 4.0 | 25.1 | -0.2 | -1.7 | -1.9 | -0.3 | 1.0 | 0.37 | 0.23 | 0.19 |
| | VBL | 0.2 | 25.9 | +0.2 | -0.8 | -1.5 | -0.3 | 1.0 | 0.45 | 0.25 | 0.29 |
| CCRF-CEM /VBL | | 0 | 26.3 | -0.3 | +0.1 | -0.3 | +0.4 | 1.0 | 1:00 | 1.00 | 1.00 |
| | Epo B | 0.7 | 25.8 | +0.1 | -0.7 | -1.0 | -0.2 | 1.0 | 0.32 | 0.40 | 0.33 |
| | | 1.0[6] | 26.0 | -0.2 | -1.3 | -2.1 | -0.5 | 1.0 | 0.41 | 0.27 | 0.31 |
| | Taxol® | 2.0 | 26.1 | 0 | -0.9 | -1.5 | -0.1 | 1.0 | 0.60 | 0.58 | 0.70 |
| | | 4.0 | 26.0 | 0 | -1.4 | -1.6 | -0.9 | 1.0 | 0.79 | 0.55 | 0.41 |
| | VBL | 0.2 | 25.9 | -0.3 | -0.8 | -1.4 | -0.3 | 1.0 | 0.86 | 0.66 | 0.67 |
| | | 0.4 | 25.9 | 0 | -1.2 | -1.8 | -0.5 | 1.0 | 1.02 | 0.57 | 0.62 |

1. CCRF-CEM and CCRF-CEM/VBL tumor tissue 50ul/mouse implanted S.C. on day 0, Treatments i.p., QD on day 7, 8, 9, 10, 14 and 15. There were seven CB-17 scid male mice in each dose group and control.
2. Epo B, epothilone B; VBL, vinblastine.
3. The tumor volumes for each group on day 7 was about 1 $mm^3$. The average volumes of CCRF-CEM control group on day 12, 17 and 22 were 19, 76 and 171 $mm^3$, and of CCRF-CEM/VBL control group were 35, 107 and 278 $mm^3$, respectively.
4. Two mice died of drug toxicity on day 19 & 20.
5. Three mice died of drug toxicity on day 18, 19 and 21.
6. One mouse died of drug toxicity on day 17.

In summary, epothilones and Taxol® have similar modes of action by stabilizing polymerization of microtubules. However, epothilones and Taxol® have distinct novel chemical structures.

MDR cells are 1500-fold more resistant to Taxol® (CCRF-CEM/VBL cells), epothilone A showed only 8-fold resistance and epothilone B showed only 5-fold resistance. For CCRF-CEM cells, Epo B is 6-fold more potent than Epo A and 10-fold more potent than Taxol®. Desoxyepothilone B and compd #24 are only 3-4-fold less potent than Taxol® and compound #27 is > 2-fold more potent than Taxol®. Finally, Taxol® and vinblastine showed antagonism against CCRF-CEM tumor cells, whereas the combination of Epo B + vinblastine showed synergism.

Relative Cytotoxicity of Epothilones against Human Leukemic Cells *in Vitro* is in the order as follows:

CCRF-CEM Leukemic Cells

Epo B ($IC_{50}$ = 0.00035μM; Rel. Value = 1) > VBL(0.00063;1/1.8) > #27(0.0010;1/2.9) > Taxol® (0.0021; 1/6) > Epo A (0.0027; 1/7.7) > #24(0.0078; 1/22.3) > #10(0.0095; 1/27.1) > #25 (0.021; 1/60) > #1 (0.022; 1/62.8) > #20 (0.030; 1/85.7) > #6 (0.052; 1/149) >#26 0.055; 1/157) > #17 (0.090; 1/257) >VP-16 10.29; 1/8.29) > #15 (0.44; 1/1257) > #19 (0.96; 1/2943)

CCRF-CEM/VBL MDR Leukemic Cells

Epo B (0.0021; 1/6* [1]**) > #27 (0.0072; 1/20.6) > #1 (0.012; 1/34.3) > #10 (0.017; 1/48.6) > Epo A (0.020; 1/57.1 [1/9.5]) > #6 (0.035) > #20 (0.049) > #24 (0.053) > #25 (0.077) > #22 (0.146) > #26 (0.197) > #17 (0.254) > #11 (0.262) > VBL (0.332; 1/948.6 [1/158.1]) > Taxol® (4.14; 1/11828 [1/1971.4]) > VP-16 (10.33; 1/29514 [1/4919])

*Potency in parentheses is relative to Epo B in CCRF-CEM cells.

**Potency in square brackets is relative to Epo B in CCRF-CEM/VBL MDR cells.

As shown in Table 9, treatment of MX-1 xenograft-bearing nude mice with desoxyepothilone B (35mg/kg, 0/10 lethality), Taxol® (5mg/kg, 2/10 lethality; 10mg/kg, 2/6 lethality) and adriamycin (2mg/kg, 1/10 lethality; 3mg/kg, 4/6 lethality) every other day, i.p. beginning day 8 for 5 doses resulted in a far better therapeutic effect for desoxyepothilone B at 35 mg/kg than for Taxol® at 5 mg/kg and adrimycin at 2mg/kg with tumor volume reduction of 98%, 53% and 28%, respectively. For the desoxyepothilone B-treated group, 3 out of 10 mice were found with tumor non-detectable on day 18. (See Fig. 46)

Extended treatment with desoxyepothitone B (40mg/kg, i.p.) beginning day 18 every other day for 5 more doses resulted in 5 out of 10 mice with tumor disappearing on day 28 (or day 31). See Table 10. By contrast, the extended treatment with Taxol® at 5mg/kg for five more doses resulted in continued tumor growth at a moderate rate, and 2 out of 10 mice died of toxicity.

Toxicity studies with daily i.p. doses of desoxyepothilone B (25mg/kg, a very effective therapeutic dose as indicated in earlier experiments) for 4 days to six mice resulted in no reduction in average body weight. (Table 13; Fig. 47) By contrast, epothilone B (0.6mg/kg, i.p.) for 4 days to eight mice resulted in 33% reduction in average body weight; all eight mice died of toxicity between day 5 and day 7.

As evident from Table 15, desoxyepothilone B performs significantly better than Taxol®, vinblastine, adriamycin and camptothecin against MDR tumor xenografts (human mammary adeoncarcinoma MCF-7/Adr xenografts). This drug-resistant tumor grows very aggressively and is refractory to Taxol® and adriamycin at half their lethal doses. Taxol® at 6mg/kg i.p. Q2Dx5 reduced tumor size only 10% while adriamycin resulted in only a 22% reduction on day 17. Whereas, desoxyepothilone B at 35 mg/kg reduced tumor size by 66% on day 17 and yet showed no reduction in body weight or apparent toxicity. Even at the $LD_{50}$ dosage for Taxol® (12mg/kg) or adriamycin (3mg/kg), desoxyepothilone B still performed more effectively. By comparison, camptothecin at 1.5 and 3.0 mg/kg reduced tumor size by 28% and 57%, respectively. Overall, in comparison with the four important anticancer drugs in current use, i.e., Taxol®, adriamycin, vinblastine and camptothecin, desoxyepothilone B showed superior chemotherapeutic effect against MDR xenografts.

*In vivo* therapeutic results in nude mice for dEpoB and Taxol® are reported in Tables 19-21. As shown in the Tables, 6hr i.v. infusion via tail vein provided a good therapeutic profile with remarkably low toxicity.

For mammary MX-1 xenograft (non-MDR), desoxyepothilone B was as effective as Taxol®. Both drugs were administered by 6hr i.v. infusion and both achieved full cure.

For the MDR-mammary MCF-7/Adr xenograft, the therapeutic effect of desoxyepothilone B was far better than Taxol®, although Q2D×5 did not achieve a cure.

For CCRF-CEM/Taxol® (57-fold resistant to Taxol® *in vitro,* in-house developed cell line), Taxol® did not show significant therapeutic effect in this nude mice xenograft whereas desoxyepothilone B achieved a full cure.

Prolonged (6 hr.) i.v. infusion allowed higher doses (e.g., 30 mg/kg, Q2Dx5) to be administered (without lethality) than i.v. bolus injection of desoxyepothilone B,and yet reduced drug toxicity.

Accordingly, the present inventors have found desoxyepothilone B to have excellent properties for therapeutic application as an MDR agent and moreover as a general anticancer agent.

**Table 9.** Therapeutic Effect of Desoxyepothilone B, Taxol®, and Adriamycin in Nude Mice Bearing Human MX-1 Xenograft[a]

| Drug | Dose (mg/kg) | Average Body Weight Change (g) | | | | | | Average Tumor Volume (T/C) | | | | | Tumor Disappear-ance | # Mice Died |
|---|---|---|---|---|---|---|---|---|---|---|---|---|---|---|
| | | Day 8 | 10 | 12 | 14 | 16 | 18 | Day 10 | 12 | 14 | 16 | 18 | | |
| Control | 0 | 24.6 | -0.1 | +1.0 | +1.0 | +1.3 | +1.8 | 1.00 | 1.00 | 1.00 | 1.00 | 1.00 | 0/10 | 0/10 |
| Desoxyepothilone B | 35 | 23.0 | -0.1 | +0.7 | -0.3 | -1.7 | -1.6 | 0.42 | 0.28 | 0.07 | 0.04 | 0.02 | 0/10 | 3/10 |
| Taxol® | 5 | 24.0 | -1.3 | -0.8 | -1.4 | -1.9 | -1.8 | 0.58 | 0.36 | 0.34 | 0.42 | 0.47 | 2/10 | 0/10 |
| | 10 | 24.3 | -1.0 | -1.0 | -2.3 | -3.5 | -3.8 | 0.85 | 0.40 | 0.21 | 0.20 | 0.12 | 2/6 | 1/6 |
| Adriamycin | 2[b] | 23.9 | +0.3 | 0 | -1.4 | -1.9 | -2.0 | 0.94 | 0.88 | 1.05 | 0.69 | 0.72 | 1/10 | 0/10 |
| | 3[c] | 22.4 | +1.3 | -0.2 | -1.5 | -2.1 | -2.3 | 0.72 | 0.54 | 0.56 | 0.51 | 0.36 | 4/6 | 0/6 |

a. MX-1 tissue 100 μl/mouse was implanted s.c on day 0. Every other day i.p. treatments were given on day 8, 10, 12, 14 and 16. The average tumor volume of control group on day 10, 12, 14, 16 and 18 were 78, 151, 372, 739 and 1257 $mm^3$, respectively.

b. One mouse died of toxicity on day 22.

c. Four mice died of toxicity on day 24.

**Table 10.** Extended Experiment of Desoxyepothilone B, Taxol®, Cisplatin and Cyclophosphamide in Nude Mice Bearing Human MX-1 Xenograft[a]

| Drug | Dose (mg/kg) | Average Body Weight Change (g) | | | | | | Tumor Disappearance | | | | | Average Tumor Disappearance Duration (Day) | # Died |
|---|---|---|---|---|---|---|---|---|---|---|---|---|---|---|
| | | Day 8 | 20 | 22 | 24 | 26 | 28 | Day 20 | 22 | 24 | 26 | 28 | | |
| Desoxyepo B | 40 | 23.0 | -1.7 | -2.4 | -2.4 | -1.4 | -1.2 | 2/10[b] | 2/10 | 3/10 | 5/10 | 5/10 | 44(5/10) | 0/10 |
| Taxol® | 5 | 24.0 | -1.6 | -0.3 | +0.1 | -0.6 | -0.4 | 0/10 | 0/10 | 0/10 | 0/10 | 0/10 | | 2/10 |
| | 10 | No extended test | | | | | | 1/6 on day 16 | | | | | Reappeared on day 38 | 2/6 |

a. Extended experiment was carried out after 5 times injection (on day 8, 10, 12, 14 and 16). Every other day i.p. treatments were given continuously: Desoxyepothilone B and Taxol® on day 18, 20, 22, 24 and 26; control group mice were sacrificed.
b. One of the mice tumor reappeared on day 20.

**Table 11.** Toxicity of Epothilone B and Desoxyepothilone B in normal nude mice.

| Group Duration | Dose and Schedule (mg/kg) | Number of mice | Died | Disappearance |
|---|---|---|---|---|
| Control | | 4 | 0 | |
| Epothilone B[a] | 0.6 QD x 4 | 8 | 8 | |
| Desoxyepothilone B | 25 QD x 4 | 6 | 0 | |

a. Mice died of toxicity on day 5, 6, 6, 7, 7, 7, 7, 7

**Table 12.** Therapeutic Effect of Epothilone B, Desoxyepothilone B and Taxol® in $B6D2F_1$ Mice Bearing B16 Melanoma[a]

| Drug | Dose (mg/kg) | Average Weight Change (g) | | | | | | Average Tumor Volume (T/C) | | | | # Mice Died |
|---|---|---|---|---|---|---|---|---|---|---|---|---|
| | | Day 0 | 3 | 5 | 7 | 9 | 11 | Day 5 | 7 | 9 | 11 | |
| Control | 0 | 26.5 | -0.2 | 0 | -0.2 | 0 | +1.0 | 1.00 | 1.00 | 1.00 | 1.00 | 0/15 |
| Epothilone B | 0.4 QDx6[b] | 27.1 | -0.2 | -0.6 | -1.1 | -3.4 | -3.9 | 1.08 | 1.07 | 1.27 | 1.07 | 1/8 |
| | 0.8 QDx5[c] | 27.0 | 0 | -0.8 | -3.1 | -4.7 | -4.7 | 0.57 | 0.89 | 0.46 | 0.21 | 5/8 |
| Desoxyepothilone B | 10 QDx8 | 27.0 | -0.7 | -0.9 | -1.1 | -1.5 | -0.3 | 0.23 | 0.22 | 0.51 | 0.28 | 0/6 |
| | 20 QD1-4,7-8 | 26.9 | -1.3 | -2.2 | -1.3 | -1.6 | -0.8 | 0.59 | 0.63 | 0.58 | 0.33 | 0/6 |
| Taxol® | 4 QDx8 | 26.7 | +0.1 | +0.2 | +0.3 | +0.4 | +0.8 | 0.62 | 0.39 | 0.56 | 0.51 | 0/8 |
| | 6.5 QDx8 | 26.7 | +0.1 | +0.3 | +0.3 | +0.4 | +1.7 | 0.19 | 0.43 | 0.20 | 0.54 | 0/8 |

a. B16 melanoma cells $1.2 \times 10^6$/mouse was implanted S.C. on day 0. Daily treatments start on day 1 after inoculation. Number of mice in each group: Control, 15; Epothilone B, 8; Desoxythilone B, 5 and Taxol®, 8. The average tumor volume of control group on day 5, 7, 9 and 11 were 16, 138, 436 and 1207 $mm^3$, respectively. See Figs. 44(a) and (b).

b. One mouse died of toxicity on day 10.

c. Five mice died of toxicity on day 8, 10, 10, 11, 12. One moribund mouse was sacrificed for toxicological examinations on day 11.

**Table 13.** Therapeutic Effect of Desoxyepothilone B, Epothilone B, Taxol® and Vinblastine in Nude Mice Bearing Human MX-1 Xenograft[a].

| Drug | Dose (mg/kg) | Average Body Weight Change (g) Day 7 | 11 | 13 | 15 | 17 | Average Tumor Volume (T/C) Day 11 | 13 | 15 | 17 | Note |
|---|---|---|---|---|---|---|---|---|---|---|---|
| Control died | | 27.9 | +0.8 | +1.1 | +1.9 | +0.6 | 1.00 | 1.00 | 1.00 | 1.00 | 0/8 |
| Desoxyepothilone B | 15 | 27.1 | +0.8 | +1.1 | +1.6 | +1.5 | 0.65 | 0.46 | 0.49 | 0.41 | 0/6 died |
| | 25[b] | 27.0 | +0.4 | +0.7 | +1.0 | +0.7 | 0.38 | 0.11 | 0.05 | 0.04 | 0/6 died (1/6 cured on day 35) |
| Epothilone B | 0.3 | 26.9 | +0.5 | +0.4 | -0.3 | -1.2 | 1.00 | 0.71 | 0.71 | 0.84 | 0/7 died |
| | 0.6[c] | 27.4 | -0.3 | -1.3 | -2.1 | -2.1 | 1.08 | 0.73 | 0.81 | 0.74 | 3/7 died |
| Taxol® | 5 | 26.9 | -0.1 | +0.4 | +1.1 | +1.2 | 0.54 | 0.46 | 0.40 | 0.45 | 0/7 died |
| | 10[d] | 27.6 | -2.7 | -1.1 | -0.3 | +2.2 | 0.43 | 0.37 | 0.12 | 0.11 | 4/7 died |
| Vinblastine | 0.2 | 25.7 | +0.6 | +1.4 | +2.3 | +2.9 | 0.65 | 0.54 | 0.56 | 0.88 | 0/7 died |
| | 0.4[e] | 26.4 | +0.8 | +0.5 | +1.9 | +2.1 | 0.80 | 0.56 | 0.83 | 0.88 | 1/7 died |

a. MX-1 tissue 50 μl/mouse was implanted s.c. on day 0. Every other day i.p. treatments were given on day 7, 9, 11, 13 and 15. Number of mice in each group: Control, 8; Desoxyepothilone B, 6; Epothilone B, 7; Taxol®, 7 and Vinblastine, 7. The average tumor volume of control group on day 11, 13, 15 and 17 were 386, 915, 1390 and 1903 $mm^3$, respectively. See Fig. 45.

b. One out of six mice with no detectable tumor on day 35.

c. Three mice died of drug toxicity on day 17. Every other day i.p. treatments were given except day 15.

d. Four mice died of drug toxicity on day 13, 13, 13, 15.

e. One mouse died of drug toxicity on day 15.

**Table 14.** Toxicity of Hematology and Chemistry of Desoxyepothilone B, and Taxol® in Nude Mice Bearing Human MX-1 Xenograft[a]

| Drug | Dose | Hematology[b] | | | | | Chemistry[b] | |
|---|---|---|---|---|---|---|---|---|
| | (mg/kg ip) | WBC | | | RBC | PLT | GOT | GPT |
| | | Total ($10^3/mm^3$) | Neutrophils (%) | Lymph (%) | ($10^3/mm^3$) | ($10^6/mm^3$) | (U/L) | (U/L) |
| Control | | 12.9 | 38 | 61 | 8.1 | 800 (n=4) | 203 | 45 (n=4) |
| Desoxyepo-thilone B | 25 and 35[c] | 11.8 | 48 | 48 | 8.4 | 700 (n=6) | 296 | 55 (n=3) |
| Taxol® | 5 and 6[d] | 10.9 | 51 | 48 | 6.1 | 1083 (n=5) | 438 | 79 (n=5) |
| Normal range[e] | | 6.91~12.9 | 8.25~40.8 | 62~90 | 10.2~12.0 | 190~340 | 260 | 138.7 |

a. Minced MX-1 tumor tissue 50 μl/mouse was implanted s.c. on day 0.
b. All assays were determined on day 30; averaged values were given.
c. Desoxyepothilone B 25 mg/kg was given i.p on day 7, 9, 11, 13, 15; 35mg/kg on day 17, 19, 23, 24, 25.
d. Taxol® 5mg/kg was given i.p. on day 7, 9, 11, 13, 15; 6mg/kg on day 17, 19, 23, 24, 25.
e. Normal ranges are for wild type deer mice and $C_3$/HeJ mice (obtained from clinical, biochemical and hematological Reference values in *Normal Experimental Animals*, Brtjm Mitruka, ed., Masson Publishing USA, Inc., N.Y., 1977, and from *Clinical Chemistry of Laboratory Animals*, Weter F. Loeb, ed., Pergamon Press, 1989)

**Table 15.** Therapeutic Effect of Desoxyepothilone B, Taxol®, Adriamycin, and Camptothecin in Nude Mice Bearing MDR Human MCF-7/Adr Tumor.

| Drug | Dose (mg/kg) | Average Body Weight Change (g) | | | | | Average Tumor Volume (T/C) | | | | Died |
|---|---|---|---|---|---|---|---|---|---|---|---|
| | | Day 8 | 11 | 13 | 15 | 17 | Day 11 | 13 | 15 | 17 | |
| Control | 0 | 25.0 | +2.0 | + 2.6 | +3.1 | +3.7 | 1.00 | 1.00 | 1.00 | 1.00 | 0/8 |
| DesoxyEpoB | 35 | 25.0 | +0.3 | +0.7 | +0.6 | +0.8 | 0.31 | 0.27 | 0.30 | 0.34 | 0/8 |
| Taxol® | 6 | 25.3 | +1.7 | +1.8 | +0.8 | +0.9 | 0.57 | 0.66 | 085 | 0.90 | 0/8 |
| | 12 | 24.5 | +0.7 | -1.3 | -2.4 | 0 | 0.50 | 0.51 | 0.32 | 0.40 | 3/6 |
| Adriamycin | 1.8 | 25.6 | +0.2 | -0.4 | -0.6 | -0.4 | 0.70 | 0.68 | 0.84 | 0.78 | 0/8 |
| | 3 | 24.6 | +0.5 | -1.5 | -3.2 | -1.6 | 0.66 | 0.83 | 0.57 | 0.53 | 3/6 |
| Camptothecin | 1.5 | 24.4 | +1.1 | +0.9 | +1.7 | +1.4 | 1.08 | 0.72 | 0.61 | 0.72 | 0/8 |
| | 3.0 | 24.5 | -0.6 | -0.4 | -0.8 | -0.9 | 0.95 | 0.76 | 0.61 | 0.43 | 0/6 |

a. MCF-7/Adr cell 3 x $10^6$/mouse was implanted s.c. on day 0. Every other day i.p. treatments were given on day 8, 10, 12, 14 and 16. The average tumor volume of control group on day 11, 13, 15 and 17 were 392, 919, 1499 and 2372mm$^3$, respectively.

**Table 16.** Extended Experiment of Desoxyepothilone B, Taxol® in Nude Mice Bearing Human MX-1 Xenograft[a]

| Drug | Dose (mg/kg) | Average Body Weight Change (g) | | | | | | Tumor Disappearance | | | | | Average Tumor Disappearance | Died |
|---|---|---|---|---|---|---|---|---|---|---|---|---|---|---|
| | | Day 8 | 20 | 22 | 24 | 26 | 28 | Day 20 | 22 | 24 | 26 | 28 | Duration (Day) | |
| Desoxyepo B | 40 | 23.0 | -1.7 | -2.4 | -2.4 | -1.4 | -1.2 | 2.10[b] | 2/10 | 3/10 | 5/10 | 5/10 | 44 (5/10) | 0/10 |
| Taxol® | 5 | 24.0 | -1.6 | -0.3 | +0.1 | -0.6 | -0.4 | 0/10 | 0/10 | 0/10 | 0/10 | 0/10 | | 2/10 |
| | 10 | No Extended Test | | | | | | 1/6 on day 16, | | | | | Reappear on day 38 | $2/6_{(0/6)}$ |

a. Extended experiment was going on after 5 times injection (on day 8, 10, 12, 14 and 16). Every other day i.p. treatments were given continuously: Desoxyepothilone B and Taxol® on day 18, 20, 22, 24 and 26; Control group mice were sacrificed.

b. In one of the mice, a tumor reappeared on day 20.

As evident from Table 16, extended treatment of nude mice bearing human MX-1 xenografts with desoxyepothilone B results in complete tumor disappearance, with no mortality in any test animals. In conclusion, treatment with desoxyepothilone B shows remarkable specificity with respect to tumor toxicity, but very low normal cell toxicity.

**Table 17.** Therapeutic Effects of Desoxyepothilone B, Taxol® in Nude Mice Bearing MX-1 Xenograft.

| | | | | | | | | | | | | | | | | | |
|---|---|---|---|---|---|---|---|---|---|---|---|---|---|---|---|---|---|
| | Treatment Schedule | | | | | | | | | | | | | | | | # Died of toxicity |
| **CONTROL** | | | | | | | | | | | | | | | | | 0/10 |
| Day | 8 | 10 | 12 | 14 | 16 | 18 | 20 | | | | | | | | | | |
| Tumor Size | 19 | 78 | 151 | 372 | 739 | 1257 | 1991 | Sacrificed (n = 10) | | | | | | | | | |
| $(mm^3)$ | ±2 | ±8 | ±15 | ±55 | ±123 | ±184 | ±331 | | | | | | | | | | |
| **DESOXYEPOTHILONE B** | | | | | | | | | | | | | | | | | 0/10 |
| Dose | 35mg/kg on day | | | | | 40mg/kg on day | | | | No Treatment | | | | | | | |
| Schedule | | | | | | | | | | | | | | | | | |
| Day | 8 | 10 | 12 | 14 | 16 | 18 | 20 | 22 | 24 | 26 | 28 | 30 | 45 | 47 | 50 | 60 | |
| Tumor Size | | | | | | | | | | | | | | | | | |
| Mouse 1 | 15 | 15 | 40 | 40 | 15 | 32 | 30 | 30 | 30 | 30 | 0 | 0 | 0 | 24 | S* | --- | |
| Mouse 2 | 23 | 23 | 15 | 15 | 15 | 15 | 30 | 48 | 48 | 0 | 30 | 48 | 900 | 1200 | S | --- | |
| Mouse 3 | 15 | 60 | 90 | 105 | 105 | 126 | 96 | 150 | 180 | 0 | 48 | 64 | 600 | 600 | S | --- | |
| Mouse 4 | 21 | 38 | 38 | 0 | 0 | 10 | 8 | 8 | 8 | 8 | 0 | 0 | 0 | 0 | 0 | 0 | |
| Mouse 5 | 12 | 23 | 50 | 12 | 0 | 4 | 0 | 0 | 0 | 0 | 0 | 0 | 0 | 0 | 0 | 0 | |
| Mouse 6 | 15 | 40 | 32 | 8 | 8 | 8 | 8 | 12 | 12 | 12 | 12 | 30 | 120 | 120 | S | --- | |
| Mouse 7 | 21 | 30 | 15 | 15 | 8 | 8 | 8 | 8 | 8 | 8 | 8 | 8 | 180 | 280 | S | --- | |
| Mouse 8 | 20 | 48 | 70 | 15 | 15 | 8 | 8 | 0 | 0 | 0 | 0 | 0 | 0 | 8 | S | --- | |
| Mouse 9 | 25 | 50 | 40 | 15 | 8 | 0 | 0 | 0 | 0 | 0 | 0 | 0 | 0 | 0 | 4 | 4 | |
| Mouse 10 | 20 | 38 | 38 | 38 | 38 | 25 | 25 | 25 | 0 | 0 | 15 | 15 | 100 | 100 | S | --- | |
| **Taxol®** | | | | | | | | | | | | | | | | | 2/10 |
| Dose | 5mg/kg on day | | | | | 5mg/kg on day | | | | | | | | | | | |
| Schedule | | | | | | | | | | | | | | | | | |
| Day | 8 | 10 | 12 | 14 | 16 | 18 | 20 | 22 | 24 | 26 | 28 | 30 | 45 | 47 | 50 | 60 | |
| Tumor Size | 17 | 45 | 54 | 128 | 311 | 596 | 1114 | 1930 | 2285 | S | (n = 10) | | | | | | |
| | ±2 | ±7 | ±13 | ±42 | ±115 | ±151 | ±346 | ±569 | ±597 | | | | | | | | |
| | | | | | | | Extended studies | | | - | Extended observations | | | - | Experiment ended | | |

*S: Sacrificed due to tumor burden

**Table 18.** Toxicity of Epothilone B and Desoxyepothilone B in normal nude mice

| Group | Dose and Schedule (mg/kg) | Number of mice | Died |
|---|---|---|---|
| Control | | 4 | 0 |
| Epothilone B[a] | 0.6 QD x 4 | 8 | 8 |
| Desoxyepothilone B | 25 QD x 4 | 6 | 0 |

a. Mice died of toxicity on day, 5,6,6,7,7,7,7,7

**Table 19.** Therapeutic effects of desoxyepothilone B (dEpo B) and Taxol® in nude mice bearing MX-1 xenograft[a].

| Drugs | Dose (mg/kg) | Route[b] i.v. infusion | Average Body Weight (g) | | | | | Average Tumor Size (T/C) | | | | Tumor Disappearance |
|---|---|---|---|---|---|---|---|---|---|---|---|---|
| | | | Day 8 | 14 | 16 | 18 | 20 | Day14 | 16 | 18 | 20 | |
| Control | 0 | | 30.2 | +0.8 | +1.7 | +2.6 | +3.2 | 1.00 | 1.00 | 1.00 | 1.00 | 0/5 |
| dEpo B | 30 | Q2Dx5 | 30.3 | -2.7 | -4.0 | -4.5 | -6.8 | 0.19 | 0.10 | 0.03 | TD[c] | 3/3 |
| Taxol® | 15 | Q2Dx5 | 30.8 | 0 | -1.1 | -1.6 | -1.4 | 0.06 | 0.01 | TD | TD | 4/4 |
| | 24 | Q2Dx5 | 28.5 | -4.8 | -5.3 | -6.0 | -6.2 | 0.03 | TD | TD | TD | 4/4 |

[a] MX-1 human mammary carcinoma tissue 50 µg was implanted s.c. into mice on day 0. Every other day i.v. infusion were given on day 8, 10, 12, 14 and 16. The average tumor volume of control group on day 14, 16, 18 and 20 were 170 ± 10, 246 ± 29, 345 ± 42 and 529±65 $mm^3$ (mean "SEM), respectively.

[b] The i.v. infusion was for 6 hrs. The vehicle used was in 100 Fl (Cremophor + EtOH = 1:1) + 4ml saline.

[c] TD: Tumor disappearance.

**Table 20.** Therapeutic effects of desoxyepoB (dEpo B) and Taxol® in nude mice bearing MCF-7 Adr xenograft[a]

| Drug | Dose (mg/kg) | Route[b] i.v. Infusion | Average Body Weight (g) Day 8 | 14 | 16 | 18 | Average Tumor Size (T/C) Day 14 | 16 | 18 | Toxicity death |
|---|---|---|---|---|---|---|---|---|---|---|
| Control | 0 | | 30.2 | +0.8 | +1.7 | +2.6 | 1.00 | 1.00 | 1.00 | 0/5 |
| dEpo B | 30 | Q2Dx5 | 30.3 | -2.7 | -4.0 | -4.5 | 0.16*** | 0.15 | 0.13*** | 0/3 |
| Taxol® | 15 | Q2Dx5 | 30.8 | 0 | -1.1 | -1.6 | 0.81 | 0.89 | 0.76 | 0/4 |
| | 24 | Q2Dx5 | 28.5 | -4.8 | -5.3 | -6.0 | 0.73 | 0.71* | 0.73* | 0/4 |

[a] MCF-7/Adr human mammary adenocarcinoma tissue 50 $\mu g$ was implanted s.c. into mice on day 0. Every other day i.v. infusion were given on a day 8, 10, 12, 14 and 16. The average tumor volume of control group on day 14, 16, and 18 were 1281 ± 145, 1767 ± 161 and 2381 ± 203 $mm^3$ (mean ± SEM), respectively.

[b] The i.v. infusion n was for 6 hrs. The vehicle used was in 100 $\mu l$ (Cremophor + EtOH = 1:1) + 4ml saline.

In further tests, desoxyepothilone B showed similar anticancer efficacy as Taxol® in regular human tumor xenographs in nude mice,represented in Table 21. However, in drug-resistant tumors, desoxyepothilone B is by far better cancer therapeutic agent when compared with Taxol® in all tumors tested. Desoxyepothilone B is not only superior to Taxol® in many respects but is also a better therapeutic agent than epothilone B. camptothecin, vinblastine,

adriamycin or VP-16 (etoposide) against many other tumors. See Table 21.

Stability of Desoxyepothilone B in Plasma

As shown in Figure 75, desoxyepothilone B is surprisingly and unexpectedly significantly more stable in human serum plasma than in mouse plasma. Desoxyepothilone B is relatively unstable in mouse plasma with a short half-life of about 15 or 20 min, but is very stable in human plasma with a half-life of about 75 hours. It is therefore likely that desoxyepothilone B can be particularly favorable in treating patients in view of the long lasting effects and the absence of a need to use prolonged i.v. infusions.

Table 20A.

| Therapuetic effects of desoxyepoB (dEpo B) and Taxol® in nude mice bearing human ovarian UL3-C tumor.[a] | | | | | | | | | | |
|---|---|---|---|---|---|---|---|---|---|---|
| Drug | Dose (mg/kg) | Average Body Weight (g) | | | | | Average Tumor Size (T/C) | | | |
| | | Day 19 | 27 | 29 | 31 | 33 | Day 27 | 29 | 31 | 33 |
| Control | 0 | 30.9 | +0.9 | +0.8 | +0.4 | +0.1 | 1.00 | 1.00 | 1.00 | 1.00 |
| dEpo B | 30 | 30.0 | -1.5 | -4.0 | -3.6 | -2.7 | 0.19 | 0.13 | 0.03 | 0.03 |
| Taxol® | 20 | 31.3 | -2.9 | -3.3 | -2.4 | -2.7 | 0.10 | 0.08 | 0.03 | 0.04 |

[a] UL3-C tissue (50 mg) was implanted s.c. into mice on day 0. I.V. infusions were given on day 19, 21, 23, 25 and 27. The average tumor volume of the control group on day 27, 29, 31 and 33 were 349, 378, 488 and 66 $mm^3$, respectively.

**Table 21.** Comparison Between dEpoB and Taxol® Chemotherapy in Nude Mice

| Tumor | Drugs | Doses Schedule | Lowest Average Tumor Size (T/C) | Tumor Disappearance | Therapeutic Effects dEpoB vs Taxol® |
|---|---|---|---|---|---|
| A549 Lung | dEpoB<br>Taxol® | 40mg/kg Q2D x 3<br>15mg/kg Q2D x 3 | 0.01<br>0.01 | 1/3<br>2/4 | ≅ |
| MX-1 | dEpoB<br>Taxol® | 30mg/kg Q2D x 6<br>15mg/kg Q2D x 6 | 0<br>0 | 5/5<br>5/5 | ≅ |
| HT-29 Colon | dEpoB<br>Taxol® | 30mg/kg Q2D x 6<br>15mg/kg Q2D x 6 | 0.02<br>0.01 | 0<br>0 | ≤ |
| UL3-C Ovary | dEpoB<br>Taxol® | 30mg/kg Q2Dx5<br>15mg/kg Q2Dx5 | 0.03<br>0.04 | 0<br>0 | ≥ |
| PC-3 Prostate | dEpoB<br>Taxol® | 40mg/kg Q2D x 3<br>15mg/kg Q2D x 3 | 0.12<br>0.02 | 0<br>0 | < < |
| SK-OV-3 Ovary | dEpoB<br>Taxol® | 30mg/kg Q2D x 6<br>15mg/kg Q2D x 6 | 0.17<br>0.02 | 0<br>1/4 | < < |
| MCF-7/Adr Breast | dEpoB<br>Taxol® | 30mg/kg Q2D x 5<br>24mg/kg Q2D x 5 | 0.11<br>0.71 | 0<br>0 | > > |
| CCRF/Taxol Leukemia | dEpoB<br>Taxol® | 30mg/kg Q2D x 6<br>15mg/kg Q2D x 6 | 0<br>1.09 | 3/3<br>0 | > > > |
| CCRF/VBL Leukemia | dEpoB<br>Taxol® | 30mg/kg Q2D x 5<br>15mg/kg Q2D x 5 | 0<br>0.76 | 2/2<br>0 | > > > |
| CCRF/CEM Leukemia | dEpoB<br>Taxol® | 30mg/kg Q2D x 5<br>15mg/kg Q2D x 5 | 0<br>0 | 2/2<br>2/2 | ≅ |

**Table 22.** Comparison of Cancer Chemotherapeutic Effects

| Tumor | Route of Adminlstation | Therapeutic effect Rank Order |
|---|---|---|
| B16 Melanoma | i.p. | dEpoB>Taxol®>>EpoB |
| MX-1 | i.p. | dEpoB>Camptothecin>Taxol®>VBL, EpoB |
| MX-1 | i.p. | dEpoB>Adriamycin>Taxol® |
| SK-OV-3 | i.v./i.p. | Taxol® ≈ dEpoB |
| PC-3 | i.v./i.p. | Taxol®>dEpoB>>Adriamycin |
| MCF-7/Adr | i.p. | dEpoB>>Camptothecin>Adriamycin>Taxol® |
| MX-1 | i.v. infusion | Taxol®≈dEpoB |
| MCF-7/Adr | i.v. infusion | dEpoB>>VP-16, Taxol®>VBL, Adriamycin |
| CCRF-CEM | i.v. infusion | dEpoB ≈ Taxol® |
| CCRF-CEM/Taxol® | i.v. infusion | dEpoB>>>Taxol® |
| CCRF-CEM/VBL | i.v. infusion | dEpoB>>>Taxol® |
| SK-OV-3 | i.v. infusion | Taxol®>dEpoB |
| HT-29 | i.v. infusion | Taxol® ≥ dEpoB |
| A549 | i.v. infusion | Taxol®, dEpoB, VBL > VP-16 |
| PC-3 | i.v. infusion | Taxol®, VBL ≥ dEpoB >> VP-16 |

**Table 23.** Therapeutic Effects of DesoxyepoB and Taxol® in Nude Mice Bearing Human Lymphoblastic Leukemia CCRF-CEM[a].

| Drug | Dose | | Average Body Weight Changes (g) | | | | | | | | | | |
|---|---|---|---|---|---|---|---|---|---|---|---|---|---|
| | | Day | 21 | 23 | 25 | 27 | 29 | 31 | 33 | 35 | 37 | 39 | 41 |
| Contro l | 0 | | 29.0 | +0.1 | -1.1 | -0.2 | +0.8 | +0.1 | End | End | End | End | End |
| DesoxyepoB | 40 | | 26.6 | -1.4 | -3.6 | -3.9 | -5.2 | -4.2 | -3.1 | -2.3 | -1.2 | -0.2 | +0.9 |
| Taxol® | 20 | | 29.0 | -0.1 | -1.9 | -2.9 | -3.0 | -3.6 | -2.6 | -0.5 | +1.2 | +1.3 | +2.1 |

| | | | Average Tumor Size (T/C)<br>Proportion of Tumor Disappearance (n/total) | | | | | | | | | | |
|---|---|---|---|---|---|---|---|---|---|---|---|---|---|
| | | Day | 21 | 23 | 25 | 27 | 29 | 31 | 33 | 35 | 37 | 39 | 41 |
| Control | 0 | | 1.00<br>0/5 | 1.00<br>0/5 | 1.00<br>0/5 | 1.00<br>0/5 | 1.00<br>0/5 | End | End | End | End | End | End |
| DesoxyepoB | 40 | | 1.10<br>0/3 | 0.73<br>0/3 | 0.31<br>0/3 | 0.09<br>0/3 | 0<br>3/3 | 0<br>3/3 | 0<br>3/3 | 0<br>3/3 | ND[b]<br>1/3 | ND<br>1/3 | ND<br>0/3 |
| Taxol® | 20 | | 1.17<br>0/4 | 0.78<br>0/4 | 0.44<br>0/4 | 0.11<br>1/4 | 0<br>4/4 | 0<br>4/4 | 0<br>4/4 | 0<br>1/4 | ND<br>0/4 | ND<br>0/4 | ND<br>0.4 |

[a]: CCRF-CEM tissue (50 μg) was implanted s.c. into mice on day 0. I.V. infusion 6hr were given on day 21, 23, 25 and 27. The average tumor volumes of the control group on day 23, 25, 27, 29 and 31 were 376±100 427±130, 685±144, 850±155, and 1062±165 $mm^3$ (mean±SEM), respectively.

[b]: Tumor reappeared.

"End": Test ended because of tumor burden.

**Table 24.** Therapeutic Effects of dEpoB, Taxol®, VBL and VP-16 in Nude Mice Bearing Human Lung Carcinoma A549

| Drugs | Dose (mg/kg) Q2D x 3 | i.v. infusion (hrs) | Average Body Weight (g) Day 7 | 11 | 13 | 15 | Average Tumor Size ($mm^3$) Day 11 | 13 | 15 | 17 | Tumor Disappearance |
|---|---|---|---|---|---|---|---|---|---|---|---|
| Control | 0 | 6 | 26.4 | +1.3 | +0.6 | +0.2 | 96 | 128 | 306 | 553 | 0/4 |
| dEpoB | 30 | 18 | 29.3 | -2.0 | -5.8 | -5.6 | 17 | 21 | 26 | 21 | 1/3 |
| | 40 | 6 | 27.8 | -1.6 | -3.2 | -2.4 | 31 | 19 | 4 | 4 | 1/3 |
| | 50 | 6 | 27.1 | -2.0 | -3.2 | -2.5 | 33 | 19 | 6 | 6 | 1/2 |
| Taxol® | 15 | 6 | 27.6 | -1.2 | -0.8 | +0.5 | 49 | 32 | 7 | 6 | 2/4 |
| | 24 | 6 | 28.5 | -2.1 | -2.7 | -0.9 | 14 | 8 | 0 | 0 | 4/4 |
| VBL | 2 | i.v. inj | 25.5 | +13.4 | +2.3 | +3.2 | 11 | 11 | 11 | 7 | 2/3 |
| VP-16 | 25 | i.v. inj | 27.2 | +0.1 | -0.2 | -2.2 | 59 | 150 | 275 | 536 | 0/3 |

**Table 25.** Therapeutic Effects of dEpoB, Taxol®, VBL and VP-16 in Nude Mice Bearing Human Prostate Adenocarcinoma PC-3.

| Drugs | Dose (mg/kg) | i.v. infusion (hrs) | Average Body Weight (g) Day 5 | 9 | 11 | 13 | Average Tumor Size (TC) Day 9 | 11 | 13 | Tumor Disappearance |
|---|---|---|---|---|---|---|---|---|---|---|
| Control | 0 | 6 | 26.5 | +1.4 | +0.5 | +0.1 | 1.00 | 1.00 | 1.00 | 0/5 |
| dEpoB | 30 | 18 | 29.4 | -2.2 | -5.9 | -5.8 | 0.17 | 0.06 | 0.06 | 0/3 |
| | 40 | 6 | 28.3 | -1.7 | -3.4 | -2.7 | 0.40 | 0.19 | 0.12 | 0/3 |
| | 50 | 6 | 28.1 | -2.1 | -3.0 | -2.6 | 0.37 | 0.13 | 0.03 | 0/3 |
| Taxol® | 16 | 6 | 26.6 | -1.1 | -0.9 | +0.4 | 0.34 | 0.09 | 0.02 | 0/4 |
| | 24 | 6 | 28.8 | -2.2 | -2.8 | -0.8 | 0.18 | 0.05 | 0.06 | 2/4 |
| VBL | 2 | i.v. inj | 23.5 | +1.4 | +2.1 | +3.0 | 0.37 | 0.13 | 0.03 | 1/3 |
| VP-16 | 25 | i.v. inj | 24.9 | +0.3 | -0.1 | -2.4 | 1.00 | 1.12 | 0.88 | 0/3 |

PC-3 tissue (50 μg) was implanted s.c into mice on day 0. I.V. infusions were given on day 5, 7 and 9. The average tumor volumes of control group subjects on 9, 11 and 13 day were 766±50, 1457±180 and 2441±221 $mm^3$ (mean ±SEM), respectively.

**Table 26.** Therapeutic Effects of DesoxyepoB and Taxol® in Nude Mice Bearing Human Lymphoblastic Leukemia CCRF-CEM.

| Drug | Dose | Average Body Weight Change (g) | | | | | | | Average Tumor Size (T/C) | | | | | Tumor Disappearance |
|---|---|---|---|---|---|---|---|---|---|---|---|---|---|---|
| Q2Dx4 | | Day 21 | 23 | 25 | 27 | 29 | 31 | Day | 23 | 25 | 27 | 29 | 31 | |
| Control | 0 | 29.0 | +0.1 | -1.1 | -0.2 | +0.8 | +0.1 | | 1.00 | 1.00 | 1.00 | 1.00 | 1.00 | 0/5 |
| DesoxyepoB | 40 | 26.6 | -1.4 | -3.6 | -3.9 | -5.2 | -4.2 | | 0.68 | 0.30 | 0.09 | 0 | 0 | 3/3 |
| Taxol | 20 | 29.0 | -0.1 | -1.9 | -2.9 | -3.0 | -3.6 | | 0.78 | 0.44 | 0.11 | 0 | 0 | 4/4 |

CCRF-CEM tissue (50 μg) was implanted s.c into mice on day 0. I.V. infusion 6hr were given on day 21, 23, 25 and 27. The average tumor volume of the control group on day 23, 25, 27, 29 and 31 were 376 ± 100 427 ± 130, 685 ± 144, 850 ± 155, and 1062 ± 165 $mm^3$ (mean ± SEM), respectively.

**Table 27.** Comparison of Therapeutic Effects and Toxicity of dEpoB and Paclitaxel with Different Solvents, Routes and Schedules of Administration Using a Non-MDR MX-1 Xenograft[a]

| Route | Solvent[b] | Dose (mg/kg)[c] | Therapeutic Effect against MX-1 Xenograft[d] | | Toxicity toward Nude Mice[e] | |
|---|---|---|---|---|---|---|
| | | | dEpoB | Paclitaxel | DepoB | Paclitaxel |
| i.p. | DMSO | 35 Q2Dx5 | + + + + + | + + + + + | + + | + + |
| | Cremophor -EtOH | 15 Q2Dx5 | + | + + | + + + + + | + + + + |
| i.v. | DMSO | 15 Q2Dx5 | + + | + + + + | + + + | + + |
| injection | | 24 Q2Dx5 | + + | + + + + | + + + | + + |
| i.v. infusion | Cremophor -EtOH | | | | | |
| | 6 hr | 30 Q2Dx5 | + + + + + | + + + + + | + + | + + |
| | 24 hr | 60 Q4Dx2 | + + + + + | ND[f] | + + + | ND[f] |

a. Approximate relative scale: +, marginal; + +, little; + + +, moderate; + + + +, substantial; + + + + +, marked.
b. DMSO: dimethylsulfoxide; Cremophor: EtOH (1:1).
c. The dose of dEpoB.
d. Based on tumor volume reduction when compared with the untreated control.
e. Based on body weight decrease or lethality.
f. Not done.

**Claims**

1. A method of preparing a desoxyepothilone having the structure:

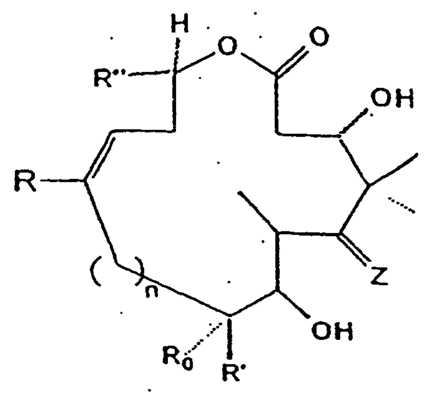

wherein R, $R_0$ and R' are independently H, linear or branched chain alkyl, optionally substituted by hydroxy, alkoxy, carboxy, carboxaldehyde, linear or branched alkyl or cyclic acetal, fluorine, $NR_1R_2$, N-hydroximino, or N-alkoxyimino, wherein $R_1$ and $R_2$ are independently H, phenyl, benzyl, linear or branched chain allcyl; wherein R" is -CY=CX-, or H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein X is H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein Y is H or linear or branched chain alkyl; wherein Z is O, $N(OR_3)$ or $N-NR_4R_5$, wherein $R_3$, $R_4$ and $R_5$ are independently H or a linear or branched alkyl; and wherein n is 0, 1, 2, or 3; which comprises treating an epothilone having a structure:

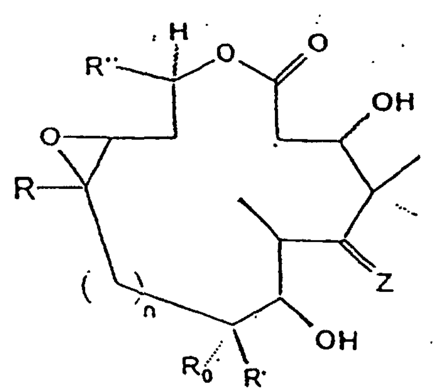

wherein R, $R_0$, $R_1$, $R_2$, $R_3$, $R_4$, $R_5$, R', R", X, Y, Z and n are defined as for the desoxyepothilone, wherein the epothilone is deoxygenated using a zinc/copper couple thereby to provide the desoxyepothilone.

2. The method of claim 1 wherein desoxyepothilone has the structure:

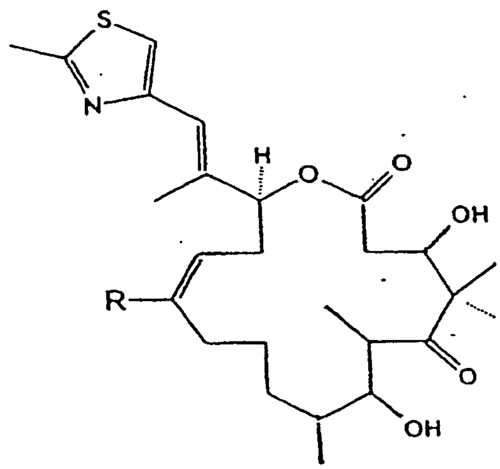

wherein R is H, methyl, ethyl, n-propyl, n-butyl, n-hexyl,

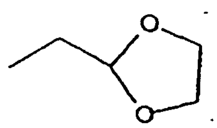

or $(CH_2)_3$-OH.

**3.** A method of preparing a desoxyepothilone having the structure:

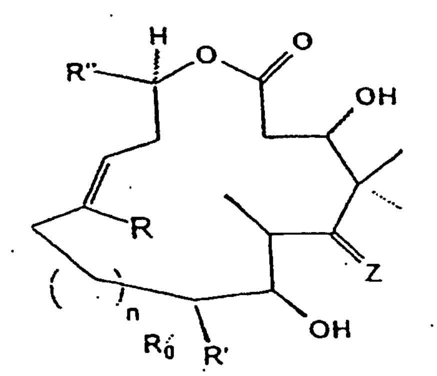

wherein R, $R_0$ and R' are independently H, linear or branched chain alkyl, optionally substituted by hydroxy, alkoxy, carboxy, carboxaldehyde, linear or branched alkyl or cyclic acetal, fluorine, $NR_1R_2$, N-hydroximino, or N-alkoxyimino, wherein $R_1$ and $R_2$ are independently H, phenyl, benzyl, linear or branched chain alkyl; wherein R" is -CY=CX-, or H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein X is H, linear or branched chain alkyl, phenyl, 2-methyl-1,3-thiazolinyl, 2-furanyl, 3-furanyl, 4-furanyl, 2-pyridyl, 3-pyridyl, 4-pyridyl, imidazolyl, 2-methyl-1,3-oxazolinyl, 3-indolyl or 6-indolyl; wherein Y is H or linear or branched chain alkyl; wherein Z is O, $N(OR_3)$ or $N-NR_4R_5$, wherein $R_3$, $R_4$ and $R_5$ are independently H or a linear or branched alkyl; and wherein n is 0, 1, 2, or 3; which comprises treating an epothilone having a structure:

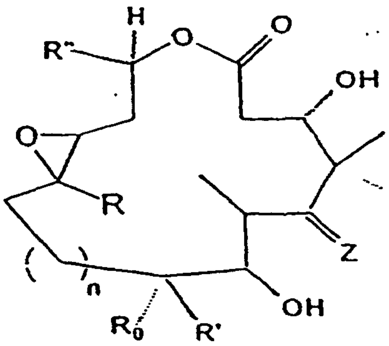

wherein R, $R_0$, $R_1$, $R_2$, $R_3$, $R_4$, $R_5$, R', R", X, Y, Z and n are defined as for the desoxyepothilone, wherein the epothilone is deoxygenated using a zinc/copper couple thereby to provide the desoxyepothilone.

**4.** The method of claim 3 wherein the desoxyepothilone has the structure:

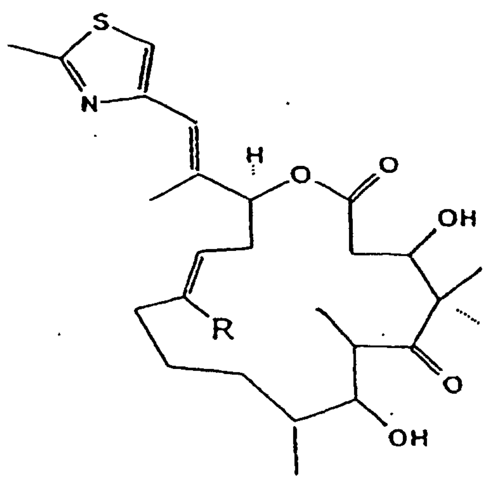

wherein R is H, methyl, ethyl, n-propyl, n-butyl, n-hexyl or hydroxypropyl.

**5.** The method of claim 1 or claim 3 wherein the epothilone is deoxygenated in the presence of a polar solvent comprising isopropanol and water.

**6.** A method of preparing a desoxyepothilone having the structure

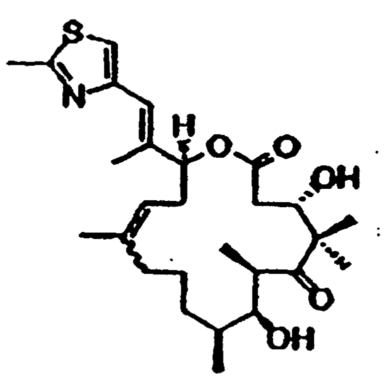

which comprises treating an epothilone having the structure

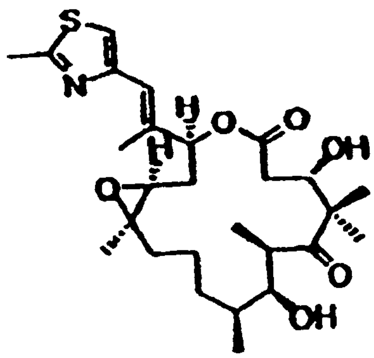

wherein the epothilone is deoxygenated using a zinc/copper couple thereby to provide the desoxyepothilone.

## Patentansprüche

**1.** Verfahren zur Herstellung von Desoxyepothilon folgender Struktur:

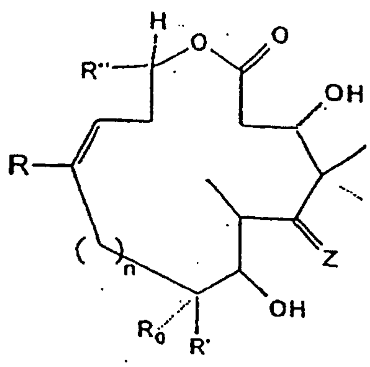

wobei R, $R_0$ und R' unabhängig Wasserstoff, lineare oder verzweigte Alkylketten sind, gegebenenfalls substituiert sind durch Hydroxy, Alkoxy, Carboxy, Carboxaldehyd, lineares oder verzweigtes Alkyl oder zyklisches Acetal, Fluor, $NR_1R_2$, N-Hydroximino, oder N-Alkoxyimino, $R_1$ und $R_2$ unabhängig Wasserstoff, Phenyl, Benzyl, lineare oder verzweigte Alkylketten bedeuten; wobei R" -CY=CX- ist, oder Wasserstoff, lineare oder verzweigte Alkylketten, Phenyl, 2-Methyl-1,3-Thiazolinyl, 2-Furanyl, 3-Furanyl, 4-Furanyl, 2-Pyridyl, 3-Pyridyl, 4-Pyridyl, Imidazolyl, 2-Methyl-1,3-Oxazolinyl, 3-Indolyl oder 6-Indolyl; wobei X Wasserstoff, lineare oder verzweigte Alkylketten, Phenyl, 2-Methyl-1,3-Thiazolinyl, 2-Furanyl, 3-Furanyl, 4-Furanyl, 2-Pyridyl, 3-Pyridyl, 4-Pyridyl, Imidazolyl, 2-Methyl-1,3-Oxazolinyl, 3-Indolyl oder 6-Indolyl ist; und Y Wasserstoff oder lineare oder verzweigte Alkylketten bedeutet; Z O, $N(OR_3)$ oder $N-NR_4R_5$ ist, $R_3$, $R_4$ und $R_5$ unabhängig Wasserstoff oder ein lineares oder verzweigtes Alkyl bedeuten; und n 0,1,2 oder 3 ist; welches Verfahren die Behandlung eines Epothilon folgender Struktur aufweist,

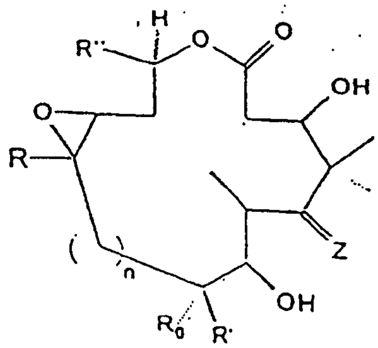

wobei R, $R_0$, $R_1$, $R_2$, $R_3$, $R_4$, $R_5$, R', R", X, Y, Z und n wie für das Desoxyepothilon definiert sind, und das Epothilon mit einem Zink/Kupfer Paar deoxygeniert wird und **dadurch** das Desoxyepothilon bereitstellt.

**2.** Verfahren nach Anspruch 1, wobei Desoxyepothilon folgende Struktur aufweist:

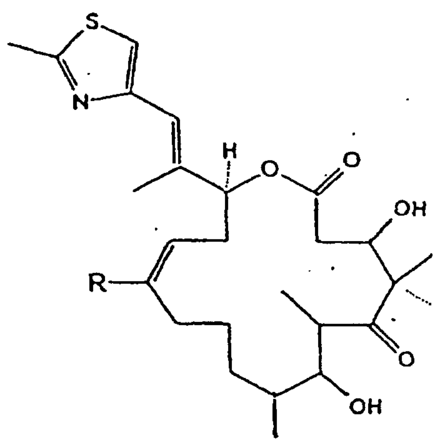

wobei R Wasserstoff, Methyl, Ethyl, n-Propyl, n-Butyl, n-Hexyl,

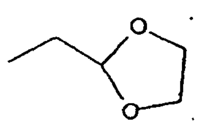

, oder $(CH_2)_3$-OH bedeutet.

**3.** Verfahren zur Herstellung von Desoxyepothilon folgender Struktur:

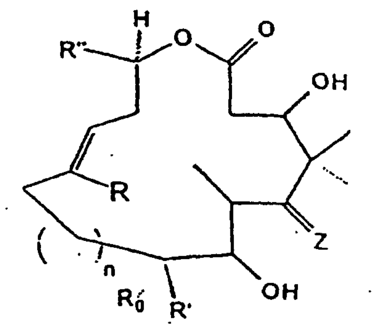

wobei R, $R_0$ und R' unabhängig Wasserstoff lineare oder verzweigte Alkylketten sind, gegebenenfalls substituiert durch Hydroxy, Alkoxy, Carboxy, Carboxaldehyd, lineares oder verzweigtes Alkyl oder zyklisches Acetal, Fluor, $NR_1R_2$, N-Hydroximino, oder N-Alkoxyimino, $R_1$ und $R_2$ sind unabhängig Wasserstoff, Phenyl, Benzyl, lineare oder verzweigte Alkylketten bedeuten; R" -CY=CX- ist, oder Wasserstoff, lineare oder verzweigte Alkylketten, Phenyl, 2-Methyl-1,3-Thiazolinyl, 2-Furanyl, 3-Furanyl, 4-Furanyl, 2-Pyridyl, 3-Pyridyl, 4-Pyridyl, Imidazolyl, 2-Methyl-1,3-Oxazolinyl, 3-Indolyl oder 6-Indolyl; wobei X Wasserstoff, lineare oder verzweigte Alkylketten, Phenyl, 2-Methyl-1,3-Thiazolinyl, 2-Furanyl, 3-Furanyl, 4-Furanyl, 2-Pyridyl, 3-Pyridyl, 4-Pyridyl, Imidazolyl, 2-Methyl-1,3-Oxazolinyl, 3-Indolyl oder 6-Indolyl ist, und Y Wasserstoff oder lineare oder verzweigte Alkylketten bedeutet; Z O, $N(OR_3)$ oder $N-NR_4R_5$ ist, $R_3$, $R_4$ und $R_5$ unabhängig Wasserstoff oder ein lineares oder verzweigtes Alkyl bedeuten; und n 0,1,2 oder 3 ist; welches Verfahren die Behandlung eines Epothilon folgender Struktur aufweist,

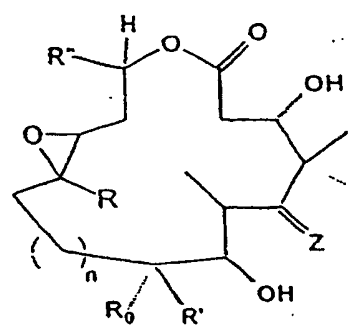

wobei R, $R_0$, $R_1$, $R_2$, $R_3$, $R_4$, $R_5$, R', R", X, Y, Z und n wie für Desoxyepothilon definiert sind, und das Epothilon mit einem Zink/Kupfer Paar deoxygeniert wird und **dadurch** das Desoxyepothilon bereitstellt.

**4.** Verfahren nach Anspruch 3, wobei das Desoxyepothilon folgende Struktur aufweist:

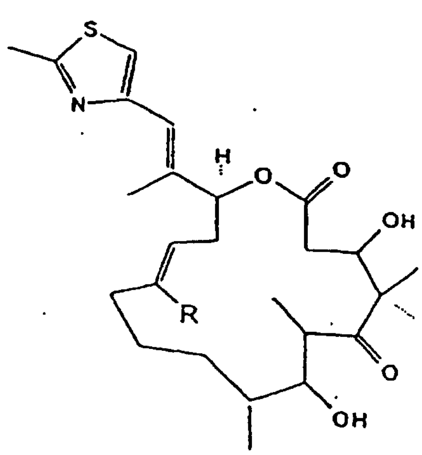

worin R Wasserstoff, Methyl, Ethyl, n-Propyl, n-Butyl, n-Hexyl, oder Hydroxypropyl ist.

5. Verfahren nach Anspruch 1 oder 3, wobei das Epothilon in der Gegenwart eines polaren Lösungsmittels, welches Isopropanol und Wasser aufweist, deoxygeniert wird.

6. Verfahren zur Herstellung von Desoxyepothilon folgender Struktur

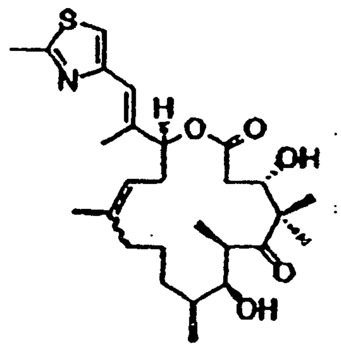

wobei das Verfahren die Behandlung von Epothilon mit der Struktur

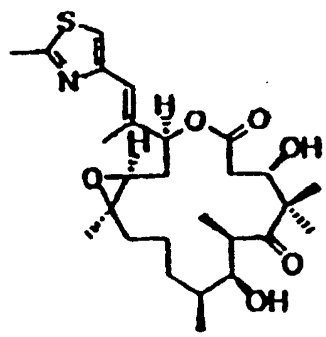

aufweist, wobei das Epothilon mit einem Zink/Kupfer Paar deoxygeniert wird um das Desoxyepothilon bereitzustellen.

## Revendications

1. Procédé de préparation d'une désoxyépothilone présentant la structure :

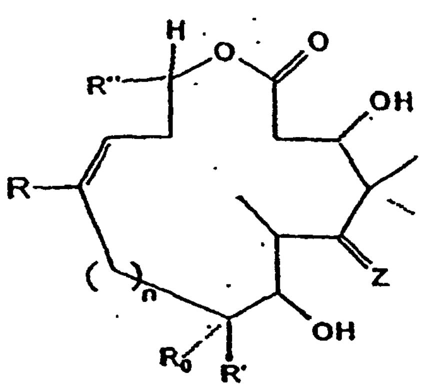

dans laquelle R, $R_0$ et R' sont indépendamment H, alkyle à chaîne linéaire ou ramifiée, facultativement substitué par hydroxy, alkoxy, carboxy, carboxaldéhyde, alkyle linéaire ou ramifié ou acétale cyclique, fluor, $NR_1R_2$, N-hydroximino, ou N-alkoxyimino, $R_1$ et $R_2$ étant indépendamment H, phényle, benzyle, alkyle à chaîne linéaire ou ramifiée ; dans laquelle R" est -CY=CX-, ou H, alkyle à chaîne linéaire ou ramifiée, phényle, 2-méthyl-1,3-thiazolinyle, 2-furanyle, 3-furanyle, 4-furanyle, 2-pyridyle, 3-pyridyle, 4-pyridyle, imidazolyle, 2-méthyl-1,3-oxazolinyle, 3-indolyle ou 6-indolyle; X étant H, alkyle à chaîne linéaire ou ramifiée, phényle, 2-méthyl-1,3-thiazolinyle, 2-furanyle, 3-furanyle, 4-furanyle, 2-pyridyle, 3-pyridyle, 4-pyridyle, imidazolyle, 2-méthyl-1,3-oxazolinyle, 3-indolyle ou 6-indolyle ; Y étant H ou un alkyle à chaîne linéaire ou ramifiée ; dans laquelle Z est O, N ($OR_3$) ou N-$NR_4R_5$, $R_3$, $R_4$ et $R_5$ étant indépendamment H ou un alkyle à chaîne linéaire ou ramifiée ; et dans laquelle n est 0, 1, 2 ou 3 ; procédé qui comprend le traitement d'une épothilone présentant une structure :

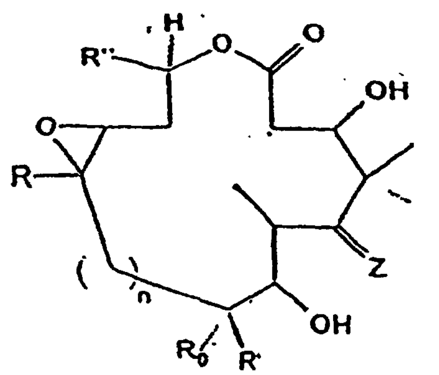

dans laquelle R, Ro, $R_1$, $R_2$, $R_3$, $R_4$, $R_5$ R, R, X, Y, 2 et n sont définis comme pour la désoxyépothilone, l'épothilone étant désoxygénée en utilisant un couple zinc/cuivre pour donner ainsi la désoxyépothilone.

**2.** Procédé selon la revendication 1 dans lequel la désoxyépothilone présente la structure :

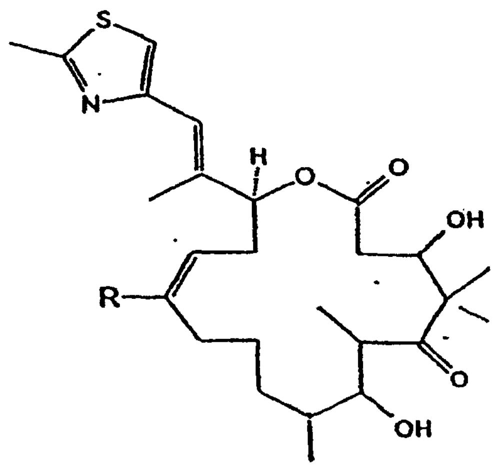

dans laquelle R est H, méthyle, éthyle, n-propyle, n-butyle, n-hexyle,

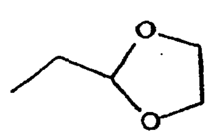

ou $(CH_2)_3$-OH.

**3.** Procédé de préparation d'une désoxyépothilone présentant la structure :

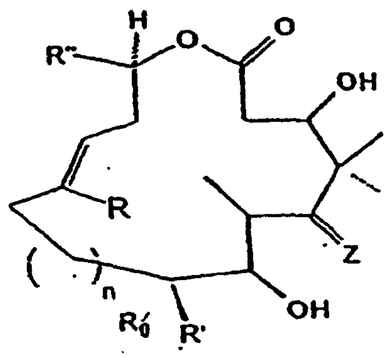

dans laquelle R, $R_0$ et R' sont indépendamment H, alkyle à chaîne linéaire ou ramifiée, facultativement substitué par hydroxy, alkoxy, carboxy, carboxaldéhyde, alkyle linéaire ou ramifié ou acétale cyclique, fluor, $NR_1R_2$, N-hydroximino, ou N-alkoxyimino, $R_1$ et $R_2$ étant indépendamment H, phényle, benzyle, alkyle à chaîne linéaire ou ramifiée ; dans laquelle R" est -CY=CX-, ou H, alkyle à chaîne linéaire ou ramifiée, phényle, 2-méthyl-1,3-thiazolinyle, 2-furanyle, 3-furanyle, 4-furanyle, 2-pyridyle, 3-pyridyle, 4-pyridyle, imidazolyle, 2-méthyl-1,3-oxazolinyle, 3-indolyle ou 6-indolyle; X étant H, alkyle à chaîne linéaire ou ramifiée, phényle, 2-méthyl-1,3-thiazolinyle, 2-oxazolinyle, 3-indolyle ou 6-indolyle; Y étant H ou un alkyle à chaîne linéaire ou ramifiée ; dans laquelle Z est O, N ($OR_3$) ou N-$NR_4R_5$, $R_3$, $R_4$ et $R_5$ étant indépendamment H ou un alkyle à chaîne linéaire ou ramifiée ; et dans laquelle n est 0, 1, 2 ou 3 ; procédé qui comprend le traitement d'une épothilone présentant une structure :

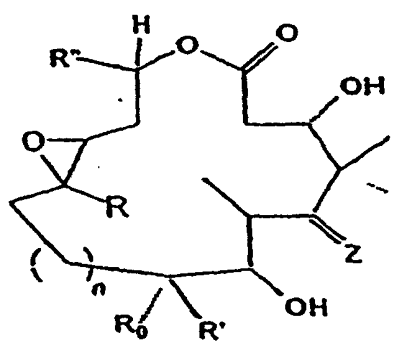

dans laquelle R, $R_0$, $R_1$, $R_2$, $R_3$, $R_4$, $R_5$ R, R, X, Y, Z et n sont définis comme pour la désoxyépothilone, l'épothilone étant désoxygénée en utilisant un couple zinc/cuivre pour donner ainsi la désoxyépothilone.

**4.** Procédé selon la revendication 3 dans lequel la désoxyépothilone présente la structure :

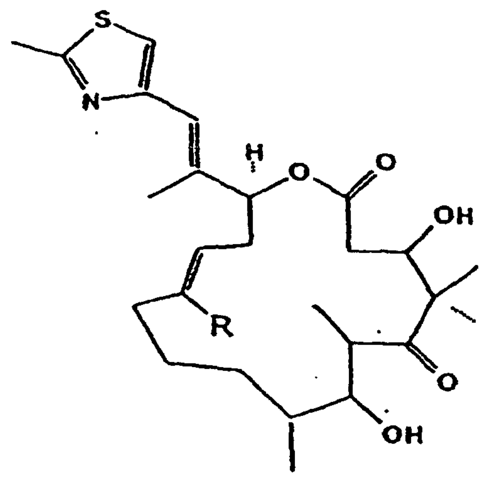

dans laquelle R est H, méthyle, éthyle, n-propyle, n-butyle, n-hexyle, ou hydroxypropyle.

**5.** Procédé selon la revendication 1 ou la revendication 3 dans lequel l'épothilone est désoxygénée en présence d'un solvant polaire comprenant de l'isopropanol et de l'eau.

**6.** Procédé de préparation d'une désoxyépothilone présentant la structure :

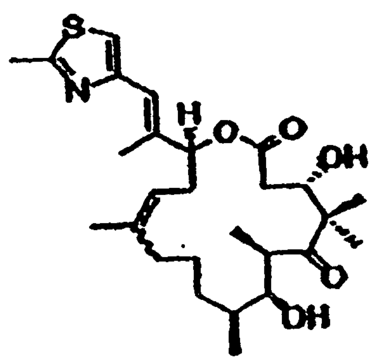

qui comprend le traitement d'une épothilone présentant la structure :

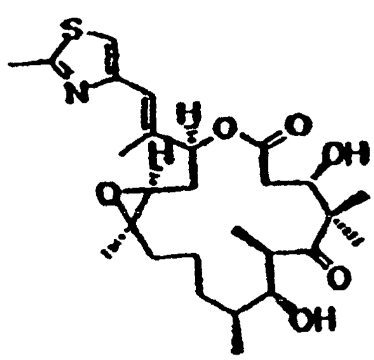

dans laquelle l'épothilone est désoxygénée en utilisant un couple zinc/cuivre pour donner ainsi la désoxyépothilone.

FIG. 1A

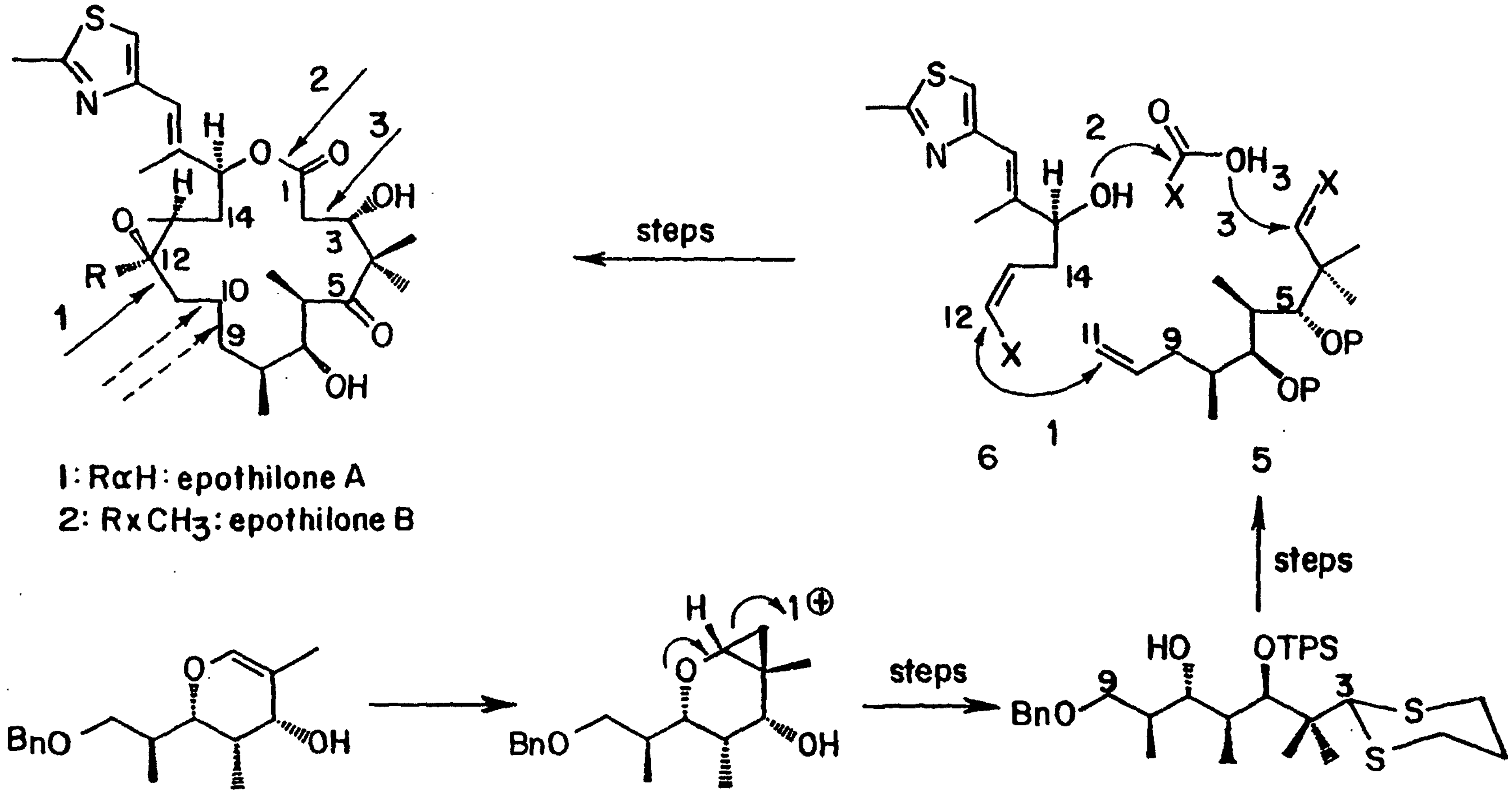


1: RαH: epothilone A
2: RxCH3: epothilone B

6 1 5

FIG. 1B

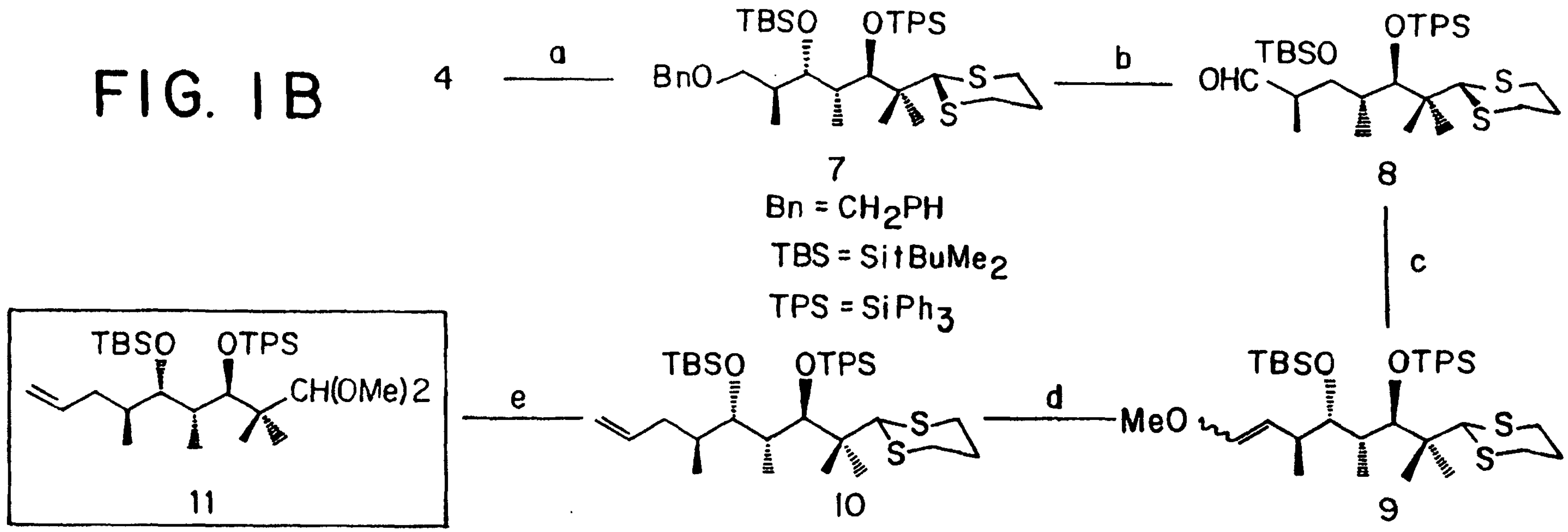


FIG. 12A

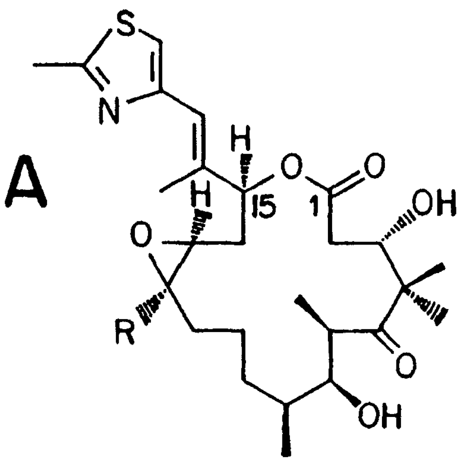

R= H: epothilone A
R= $CH_3$: epothilone B

FIG. 12B

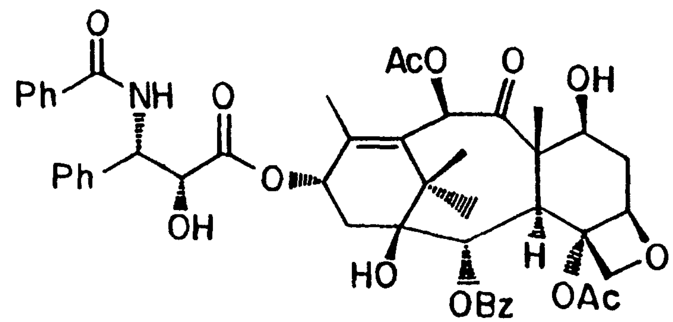

I A: taxol[TM]

# FIG. 2

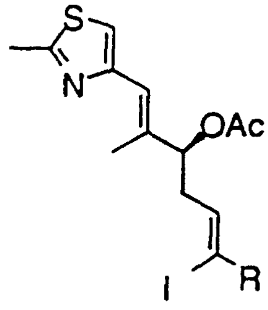

R = $CH_2OTBS$
$(CH_2)_3OTBDPS$

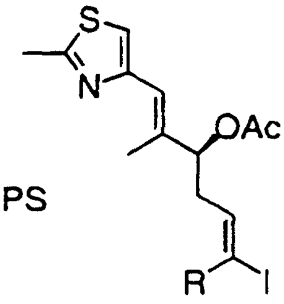

R = $CH_2OTBS$
$(CH_2)_3OTBDPS$

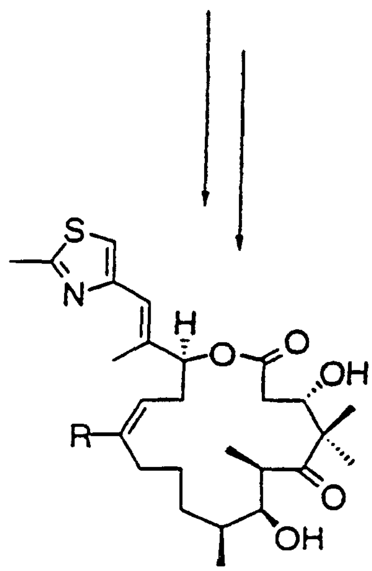

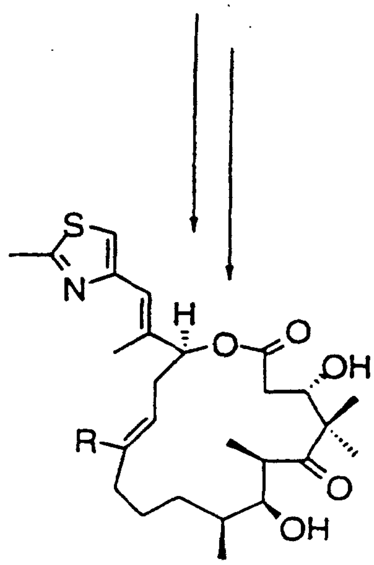

R = $CH_2OH$

$(CH_2)_3OH$

$(CH_2)_3OBz$

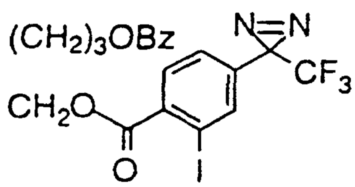

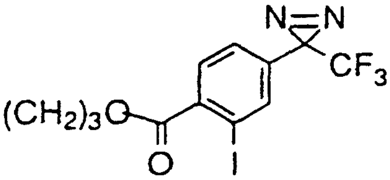

R = $CH_2OH$
$(CH_2)_3OH$

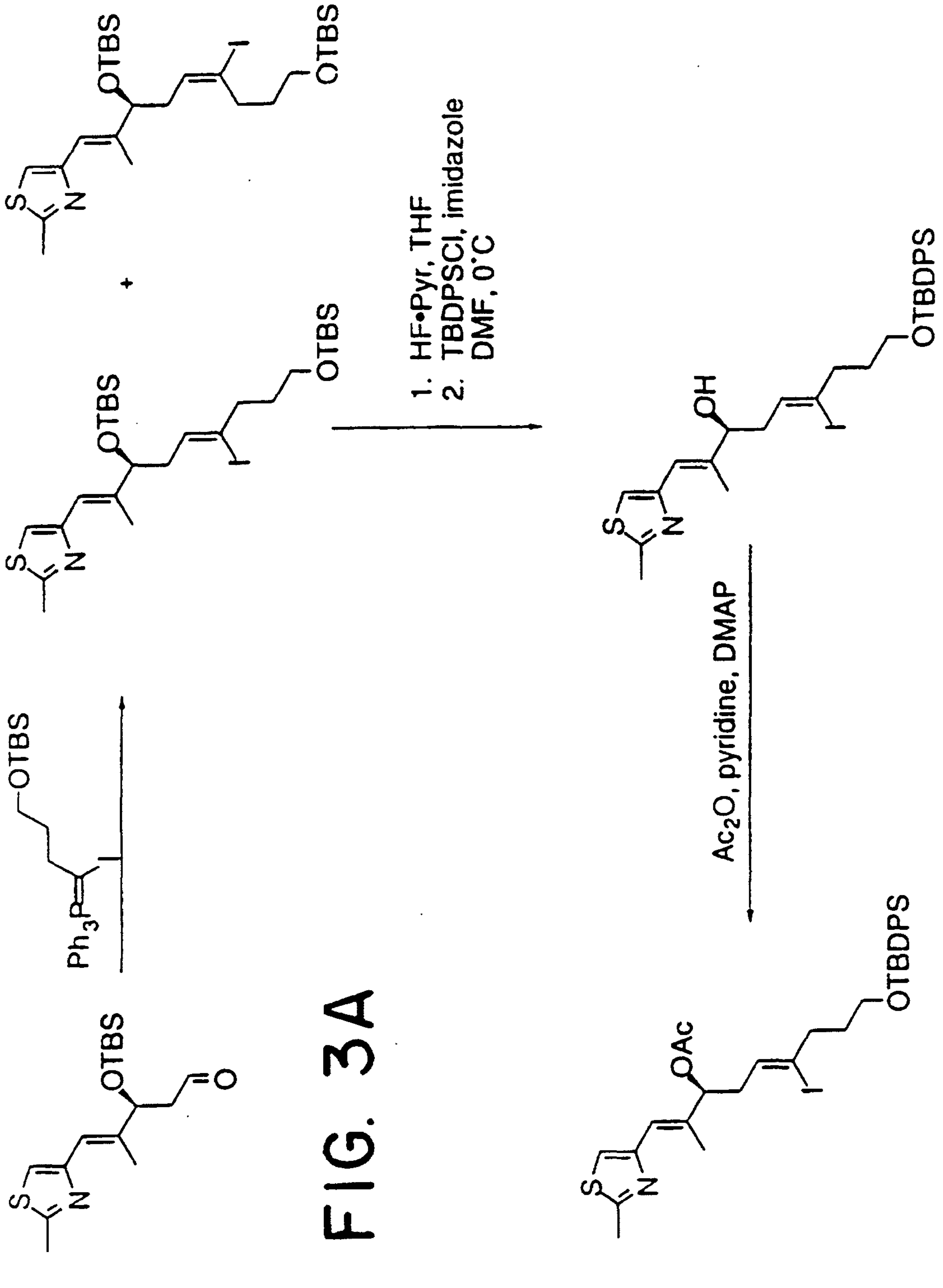


FIG. 3A

FIG. 3B

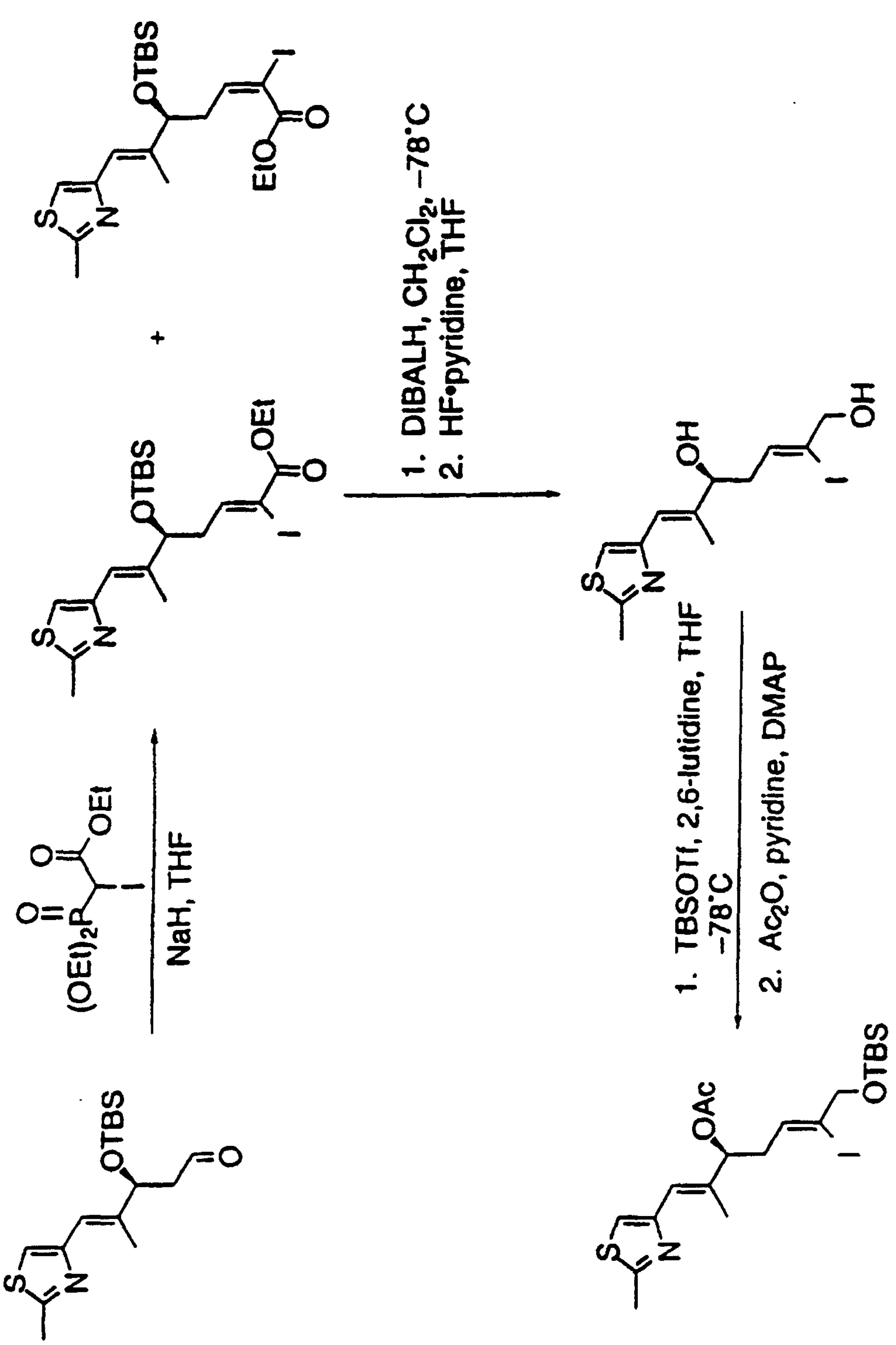
NaH, THF
1. DIBALH, $CH_2Cl_2$, −78°C
2. HF•pyridine, THF
1. TBSOTf, 2,6-lutidine, THF −78°C
2. $Ac_2O$, pyridine, DMAP

# FIG. 3C

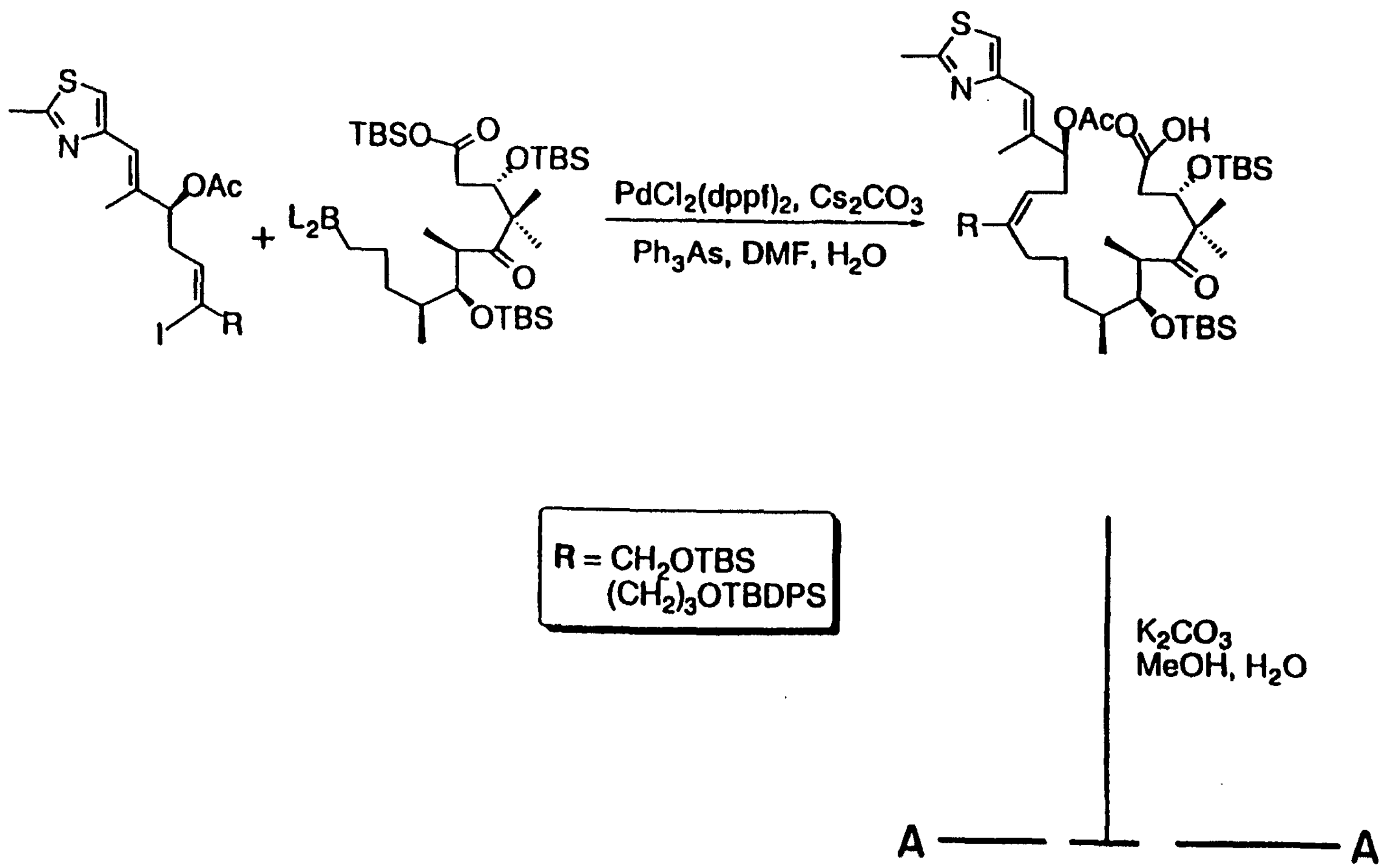
PdCl2(dppf)2, Cs2CO3
Ph3As, DMF, H2O
R = CH2OTBS
(CH2)3OTBDPS
K2CO3
MeOH, H2O
A
A

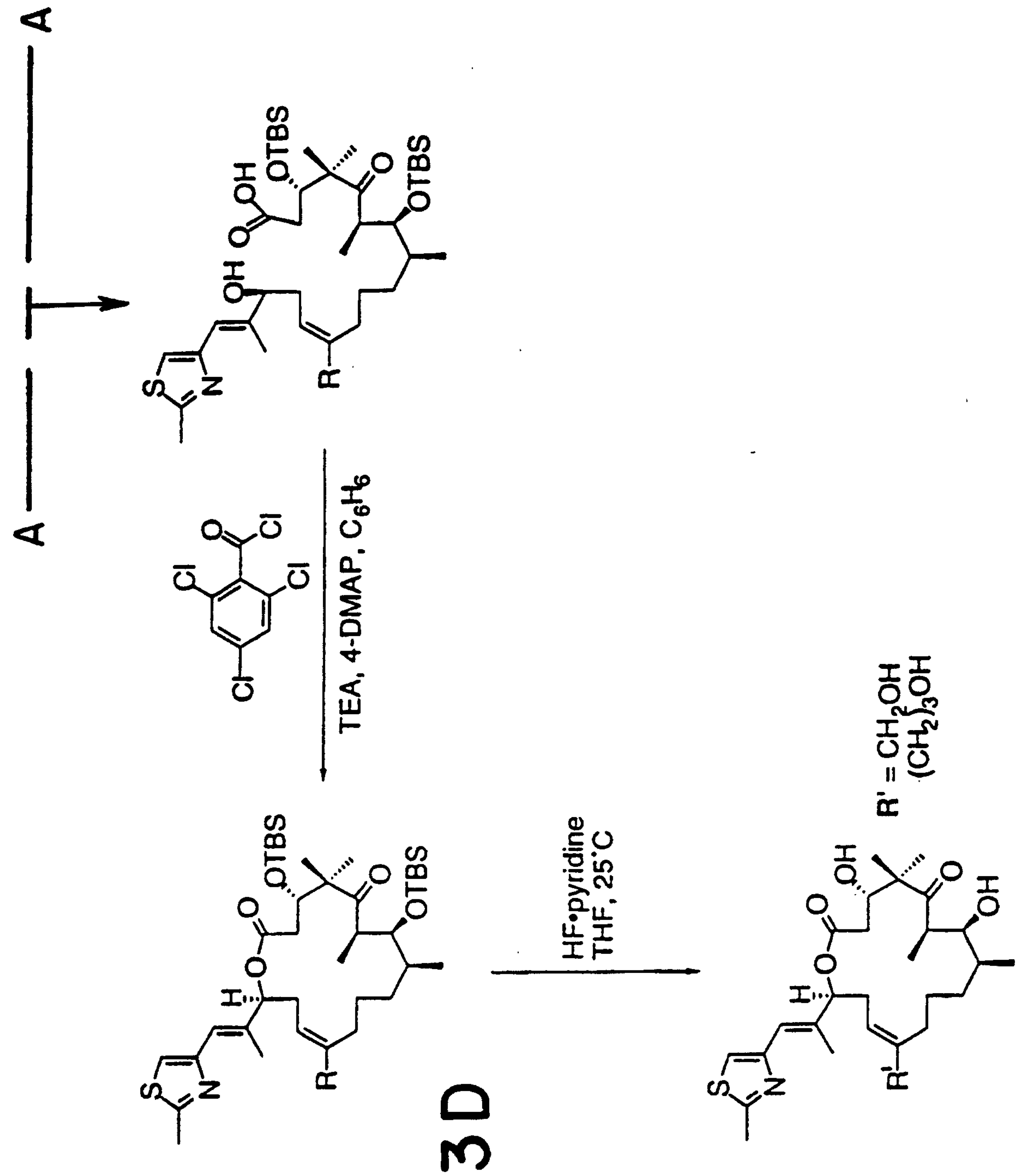


FIG. 3D

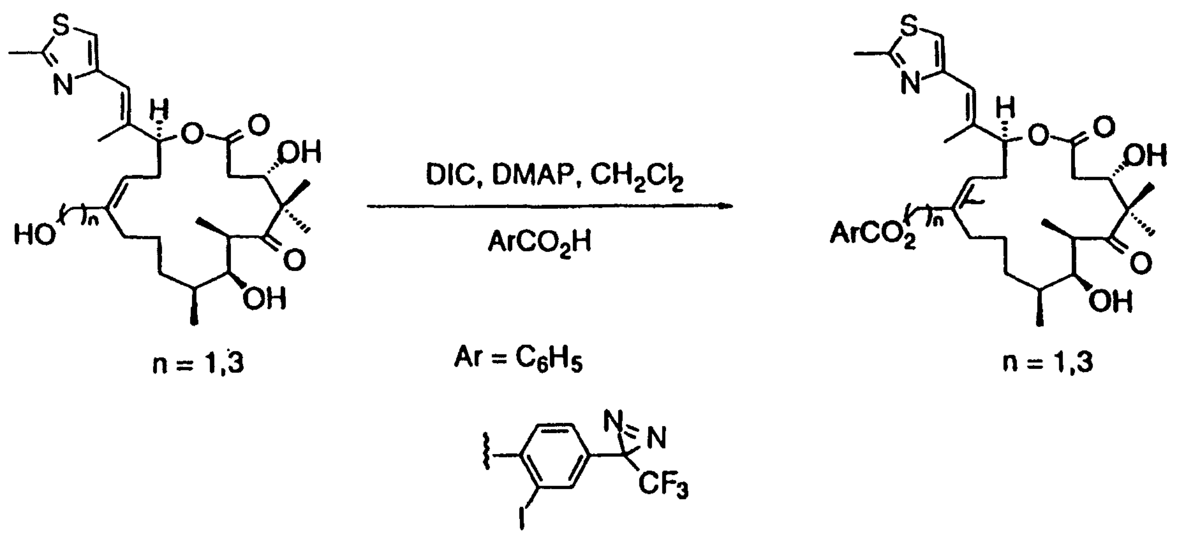


FIG. 3E

FIG. 3F

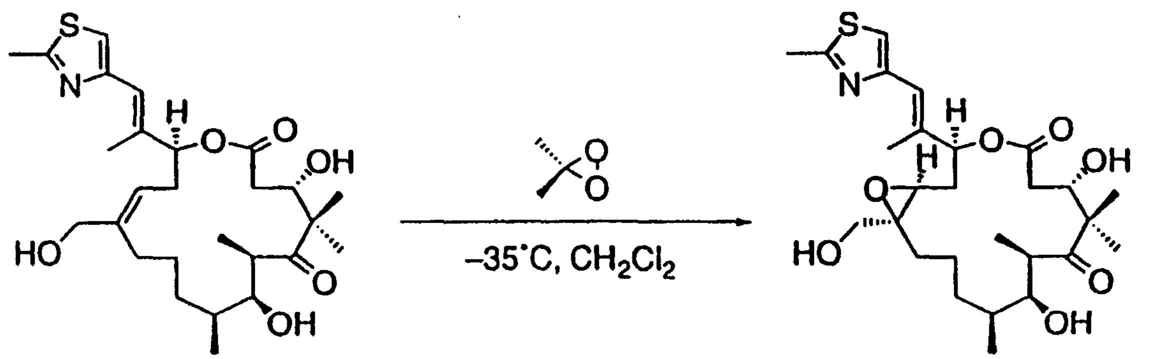

FIG. 4A

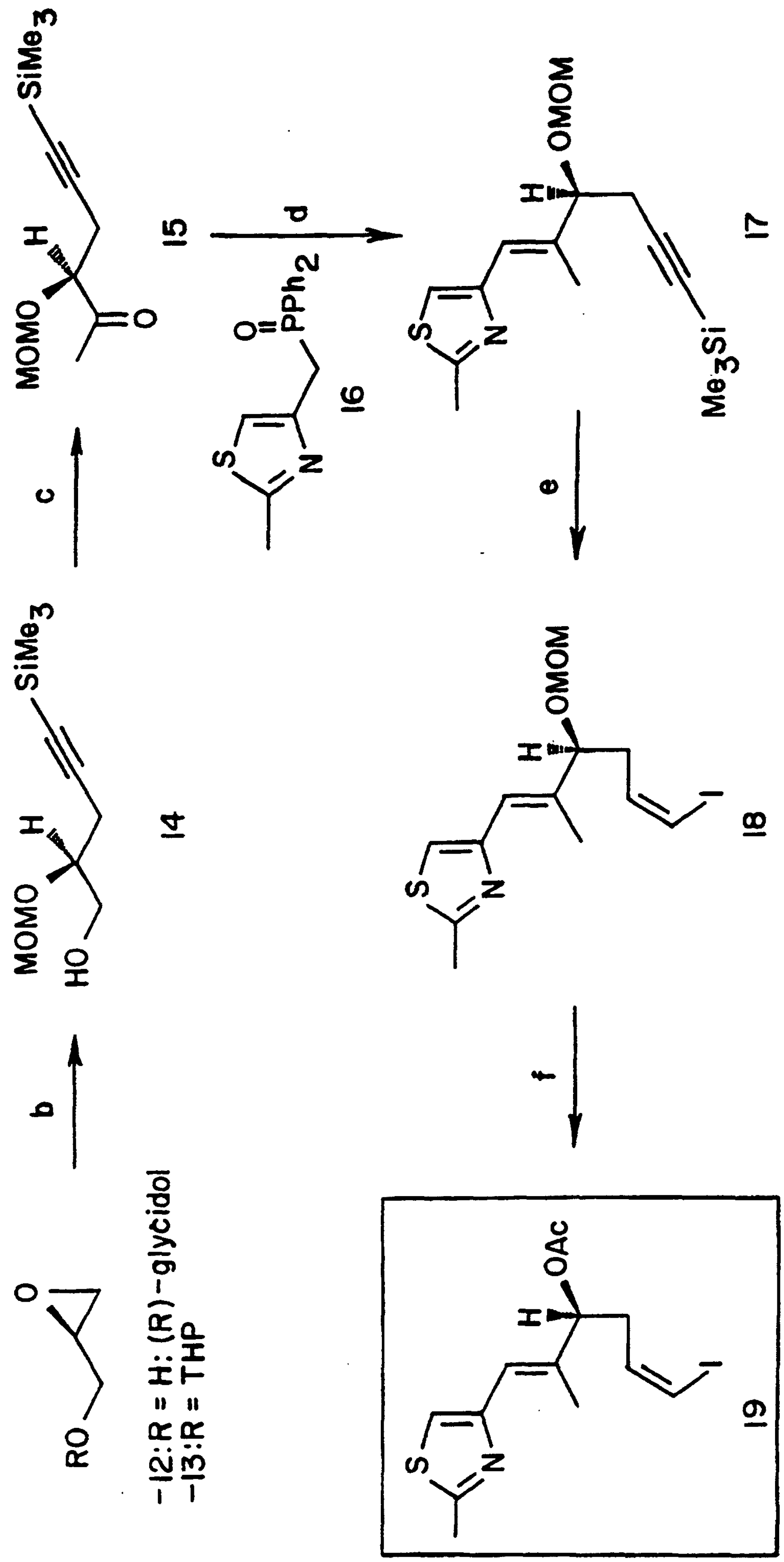


-12:R = H: (R)-glycidol
-13:R = THP

14

15

16

17

18

19

FIG. 4B

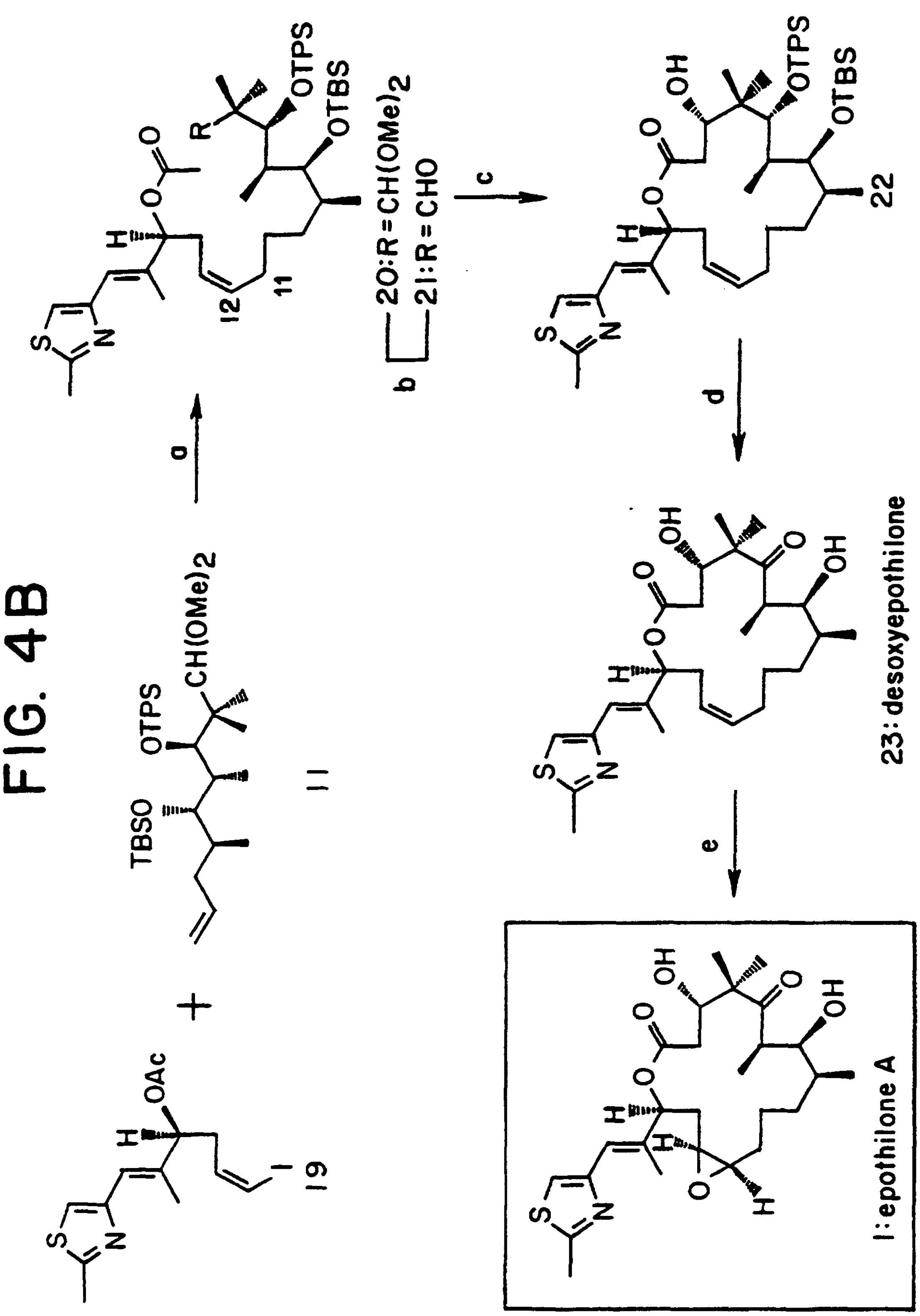


19 + 11

20: R = CH(OMe)$_2$
21: R = CHO

22

23: desoxyepothilone

I: epothilone A

FIG. 5

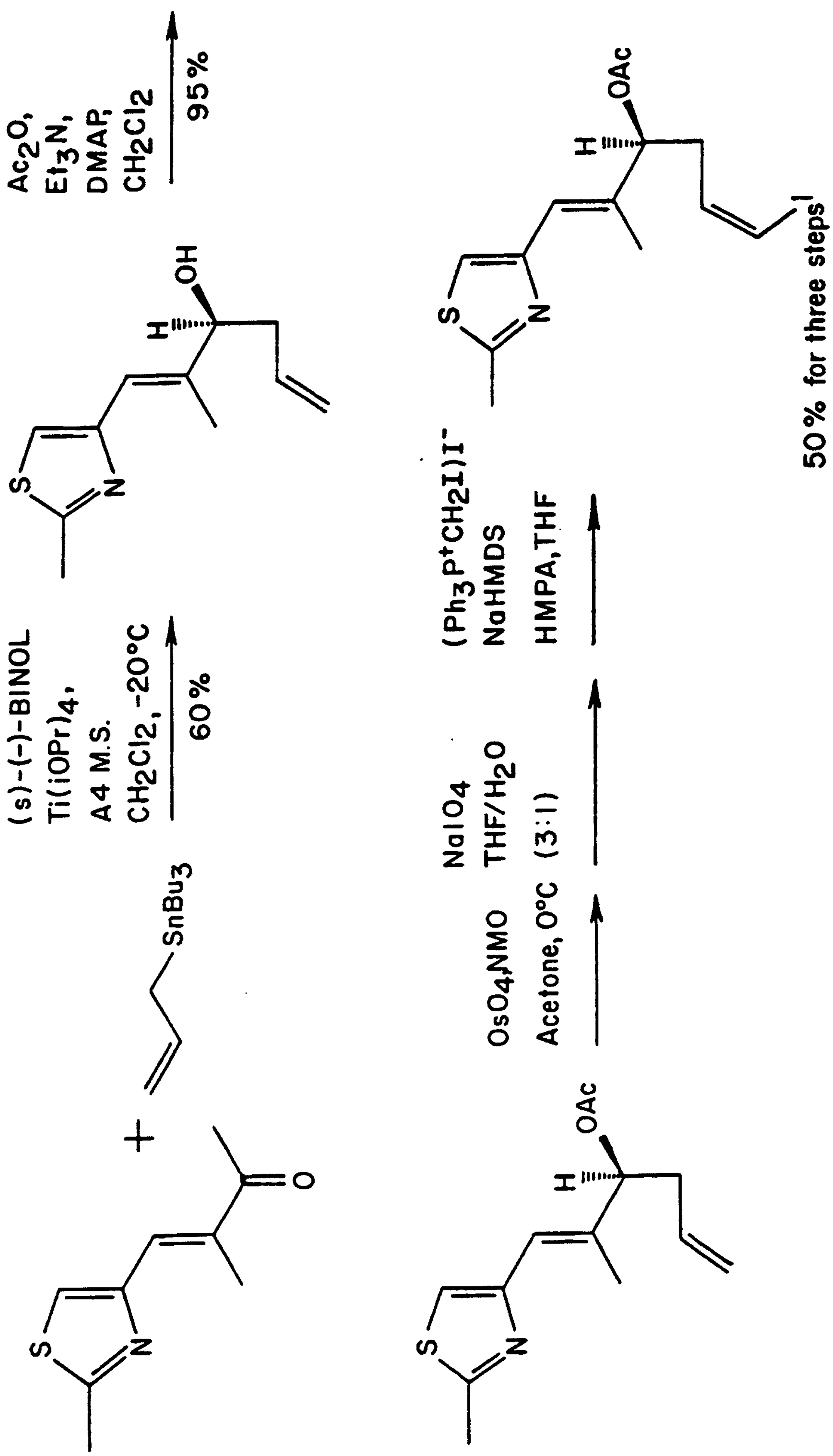
(s)-(-)-BINOL
$Ti(iOPr)_4$,
A4 M.S.
$CH_2Cl_2$, -20°C
60%
$Ac_2O$,
$Et_3N$,
DMAP,
$CH_2Cl_2$
95%
$OsO_4$,NMO
Acetone, 0°C
$NaIO_4$
$THF/H_2O$ (3:1)
$(Ph_3P^+CH_2I)I^-$
NaHMDS
HMPA,THF
50% for three steps

# FIG. 6A

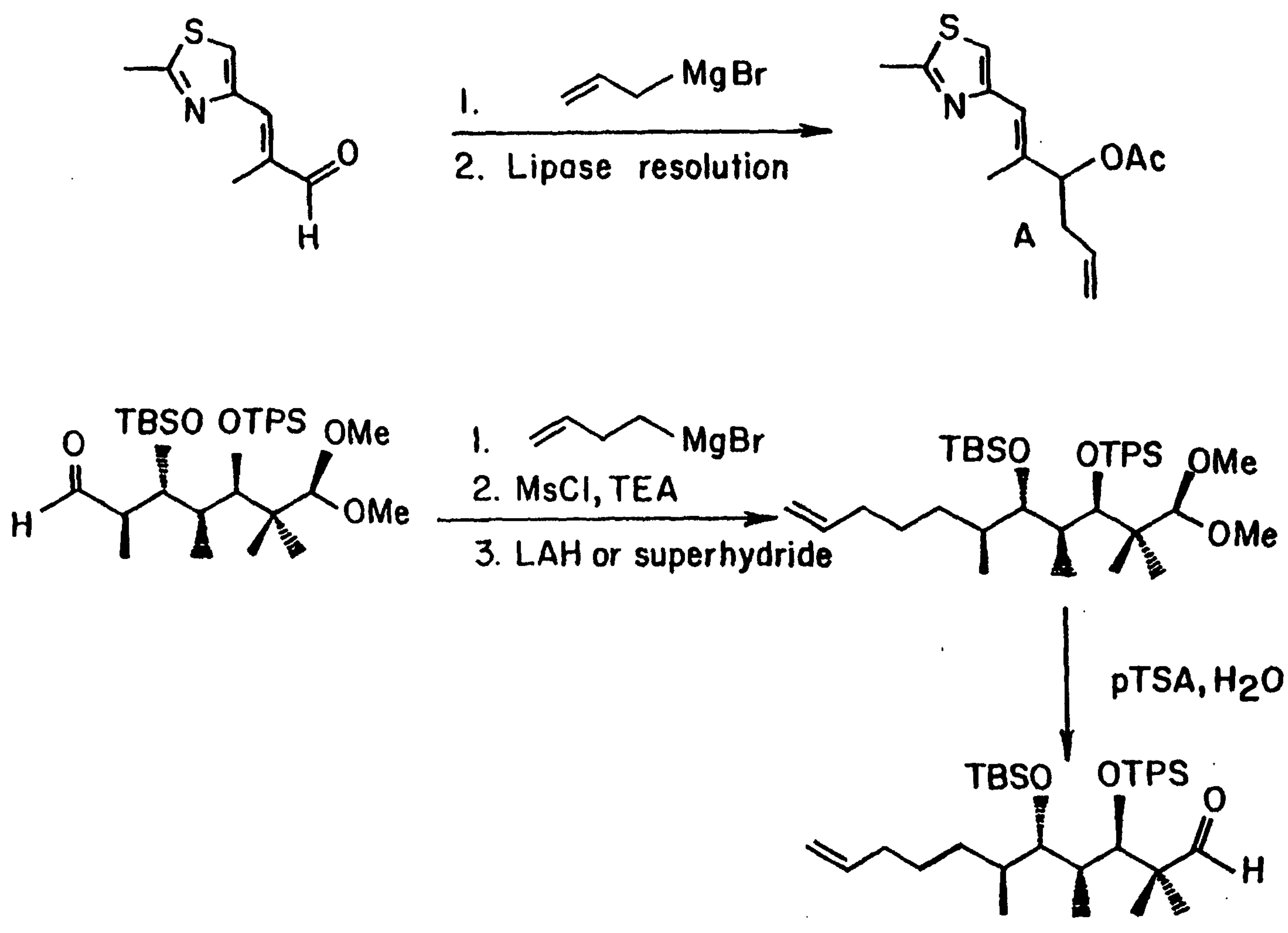
1. MgBr
2. Lipase resolution
A
1. MgBr
2. MsCl, TEA
3. LAH or superhydride
pTSA, $H_2O$

B

# FIG. 6B

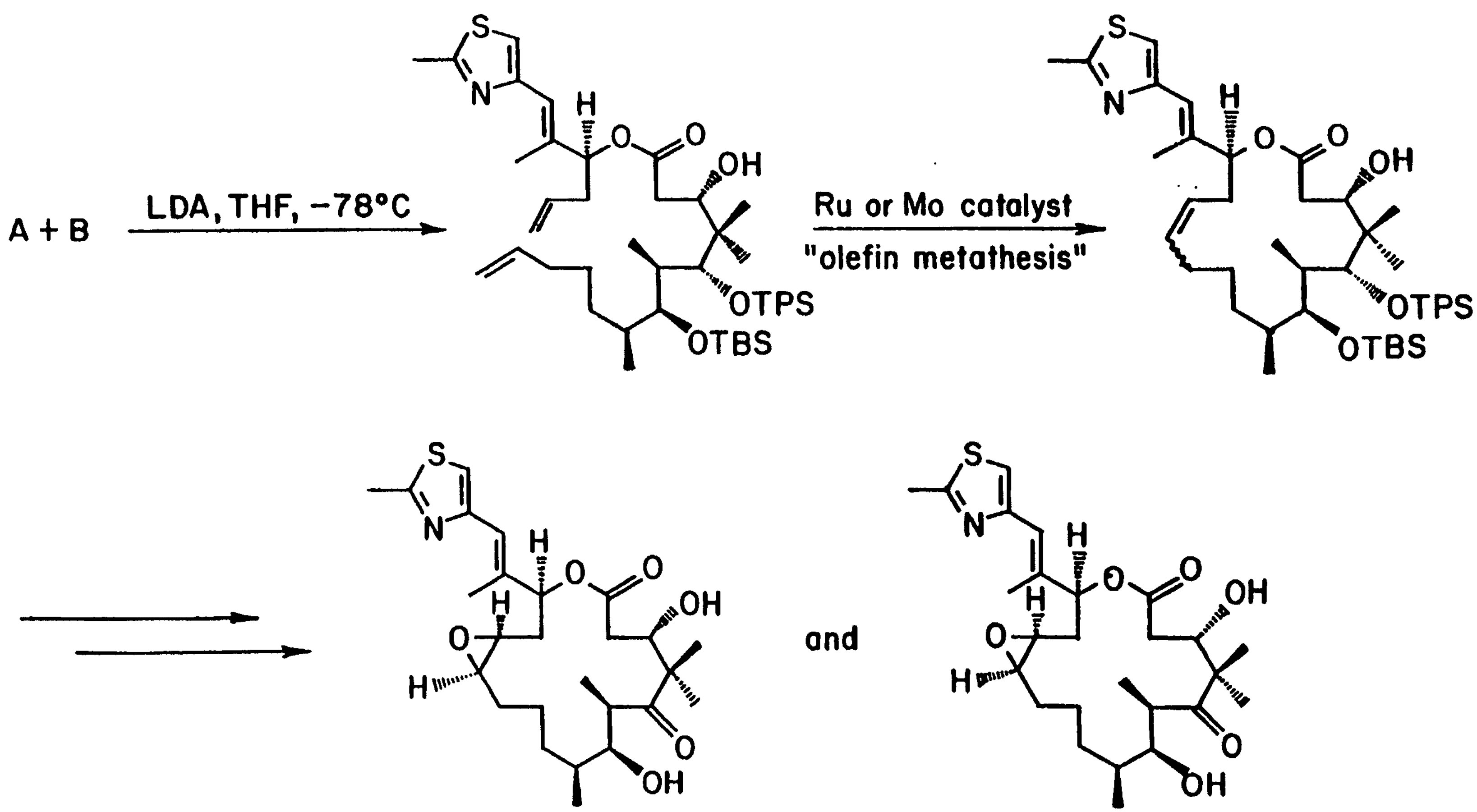


and

cis epoxide (epothilone A)

trans epoxide (epothilone analog)

*17 steps from known starting materials vs. 27 steps for aldol macrocyclization

# FIG. 7

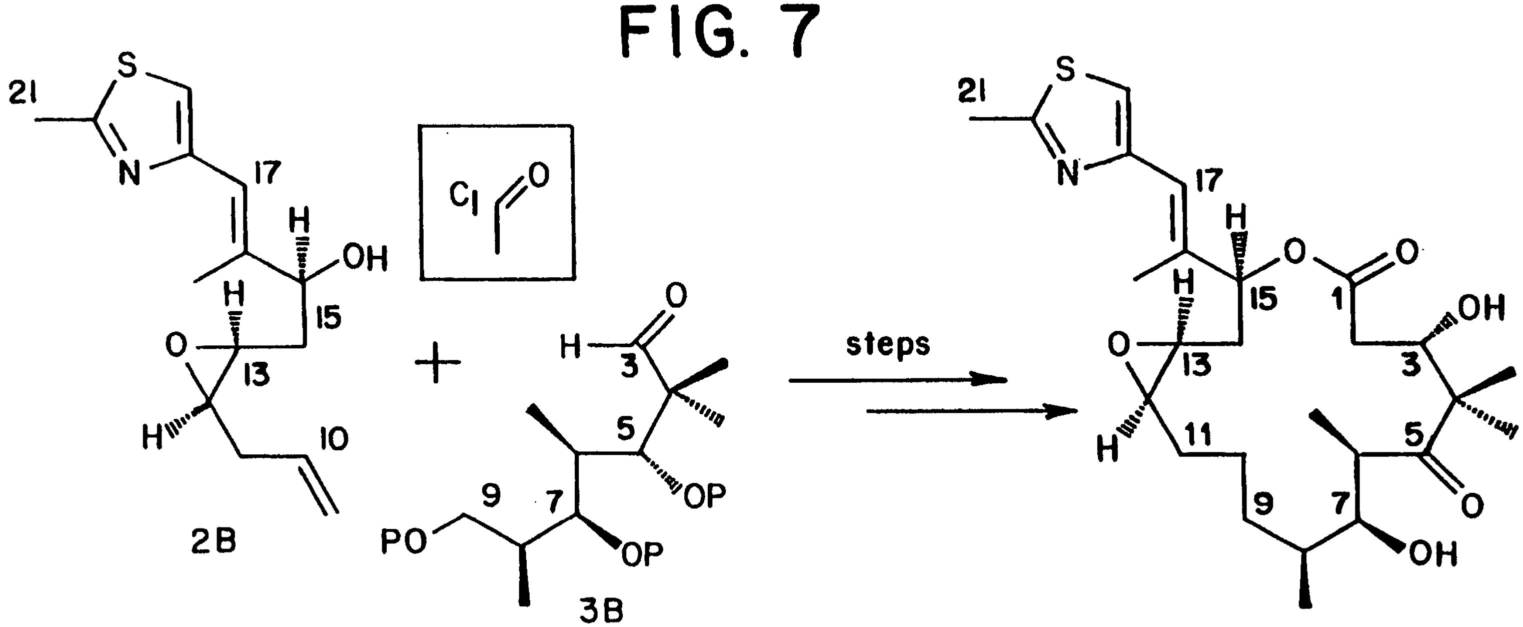


2B

3B

P=unspecified protecting group

I: epothilone A

Convergent strategy for a total synthesis of epothilone A (I).

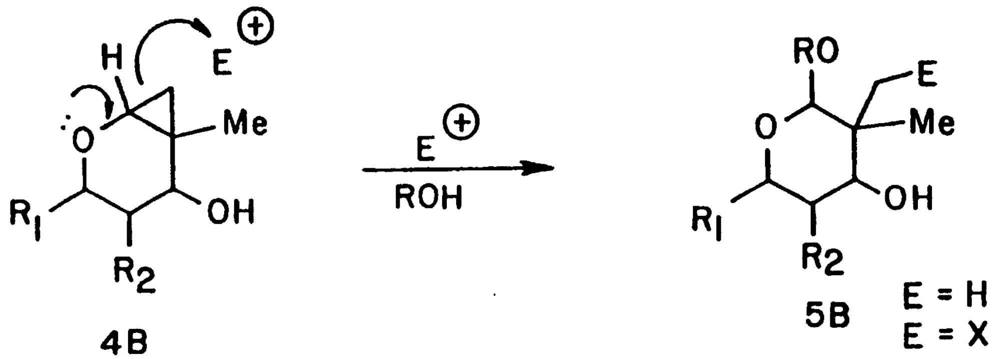


4B

5B

E = H or
E = X→H

FIG. 8

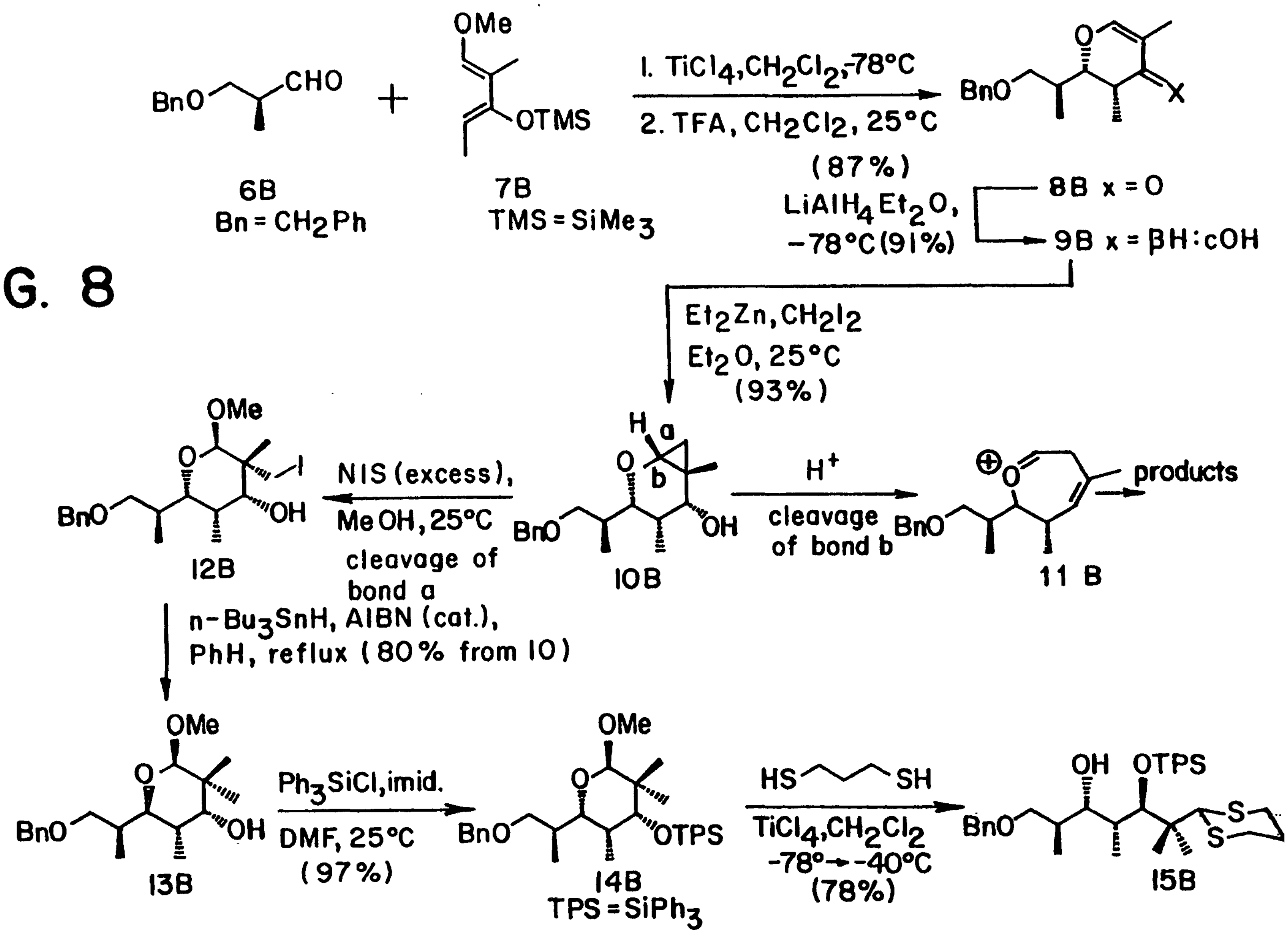
6B
Bn = CH2Ph
7B
TMS = SiMe3
1. TiCl4, CH2Cl2, -78°C
2. TFA, CH2Cl2, 25°C
(87%)
LiAlH4 Et2O,
-78°C (91%)
8B x = O
9B x = βH:cOH
Et2Zn, CH2I2
Et2O, 25°C
(93%)
10B
H+
cleavage of bond b
11 B
products
NIS (excess),
MeOH, 25°C
cleavage of bond a
12B
n-Bu3SnH, AIBN (cat.),
PhH, reflux (80% from 10)
13B
Ph3SiCl, imid.
DMF, 25°C
(97%)
14B
TPS = SiPh3
TiCl4, CH2Cl2
-78° → -40°C
(78%)
15B

FIG. 9

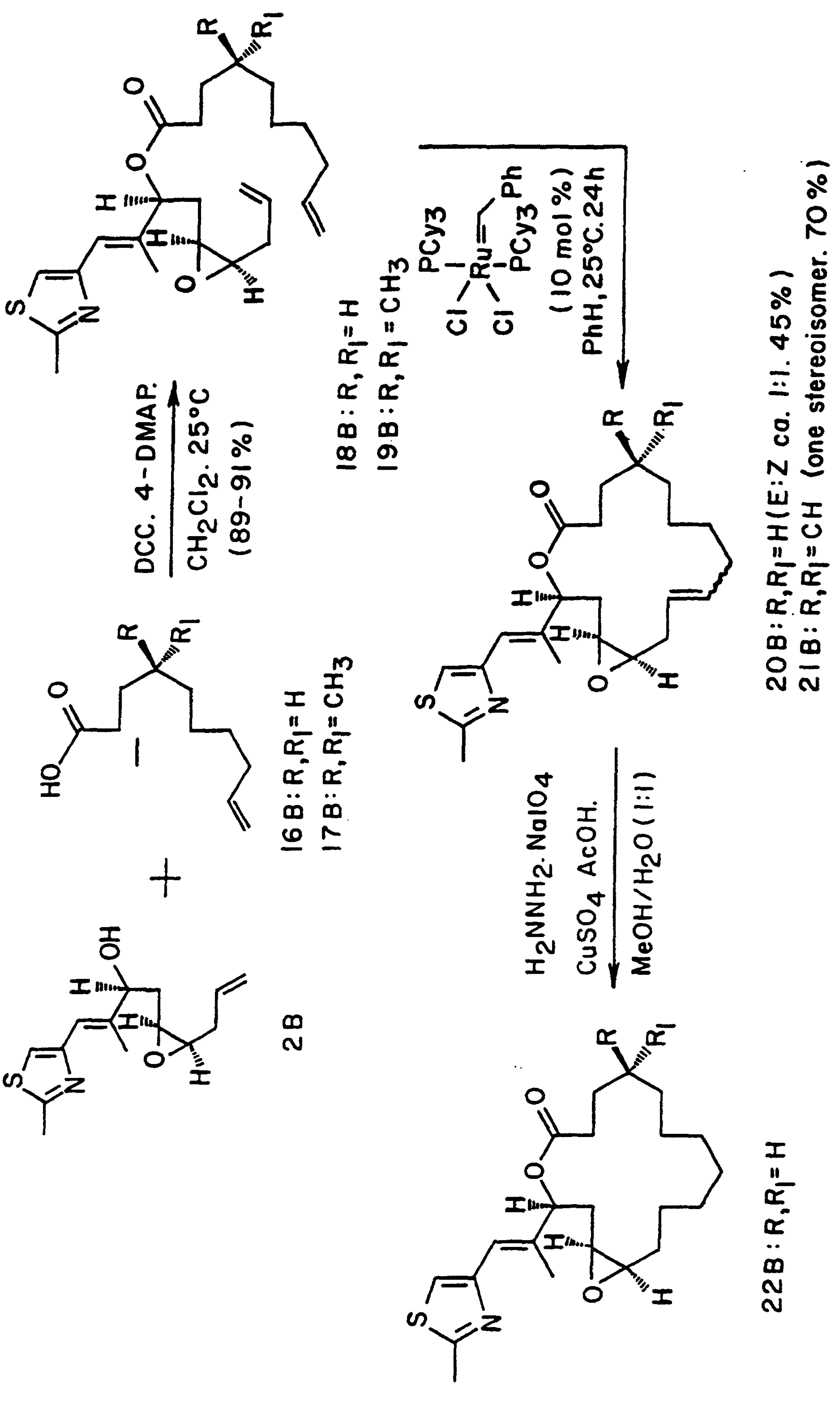


2B

16B: R, $R_1$ = H
17B: R, $R_1$ = $CH_3$

18B: R, $R_1$ = H
19B: R, $R_1$ = $CH_3$

20B: R, $R_1$ = H (E:Z ca. 1:1. 45%)
21B: R, $R_1$ = CH (one stereoisomer. 70%)

22B: R, $R_1$ = H

FIG. 10

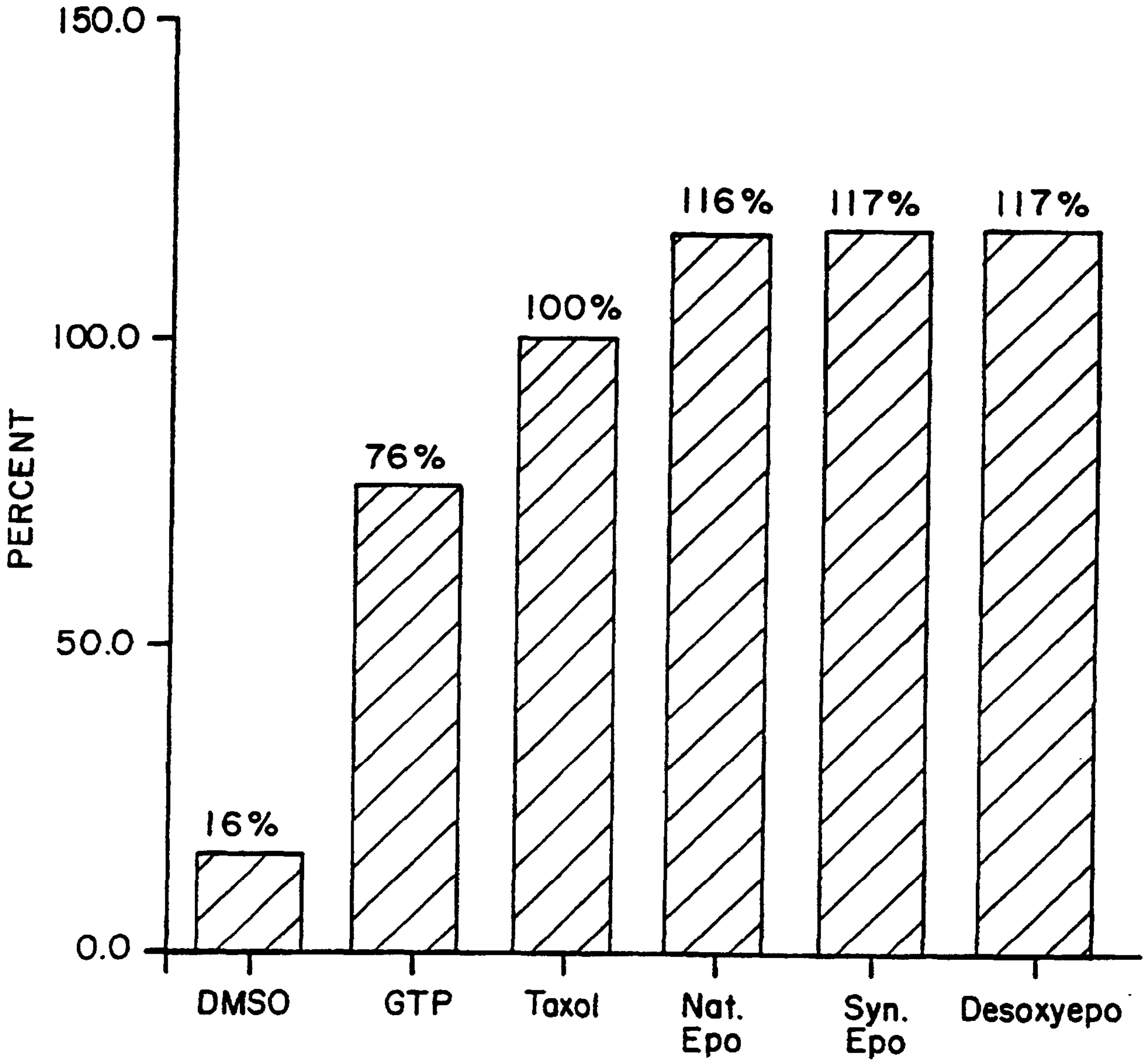
150.0
100.0
50.0
0.0
PERCENT
16%
76%
100%
116%
117%
117%
DMSO
GTP
Taxol
Nat. Epo
Syn. Epo
Desoxyepo

FIG. 11

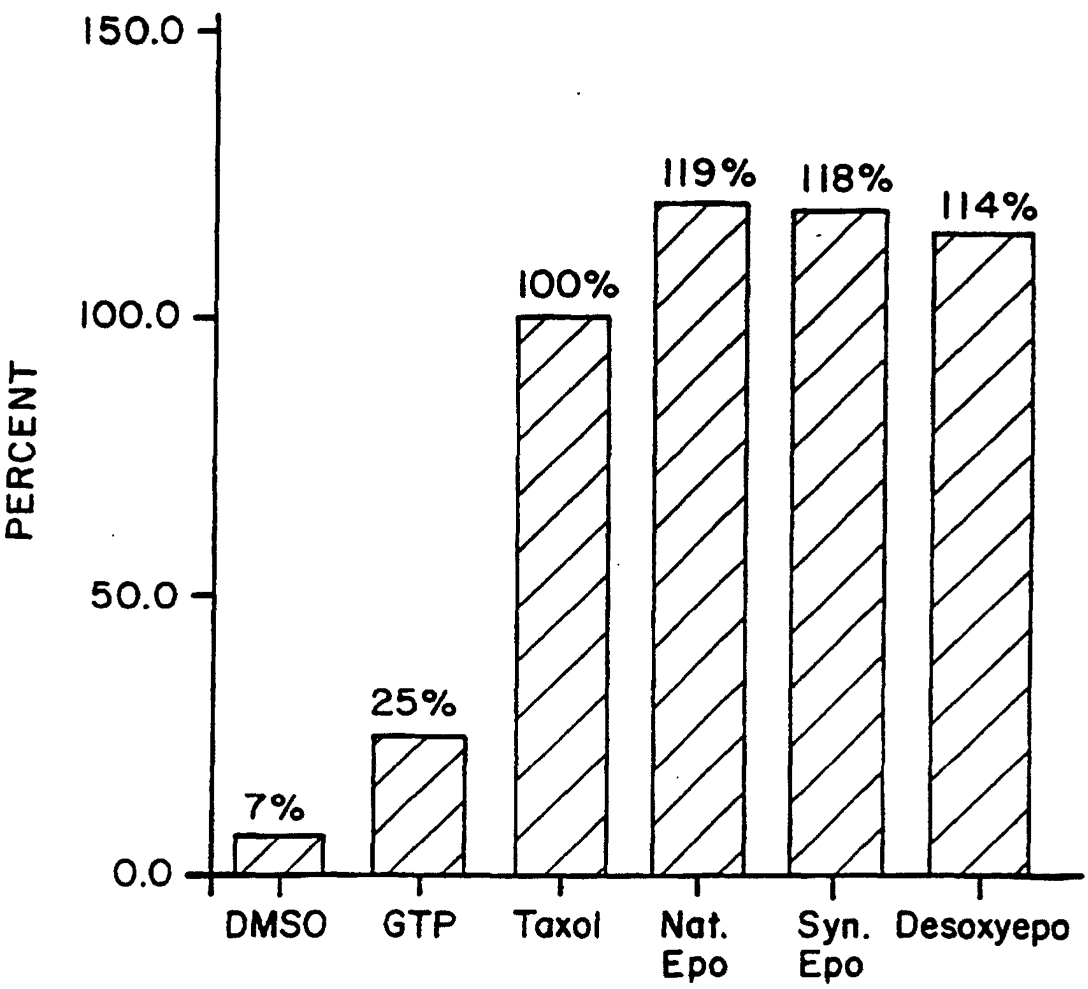
150.0
100.0
50.0
0.0
PERCENT
7%
25%
100%
119%
118%
114%
DMSO
GTP
Taxol
Nat. Epo
Syn. Epo
Desoxyepo

# FIG. 13

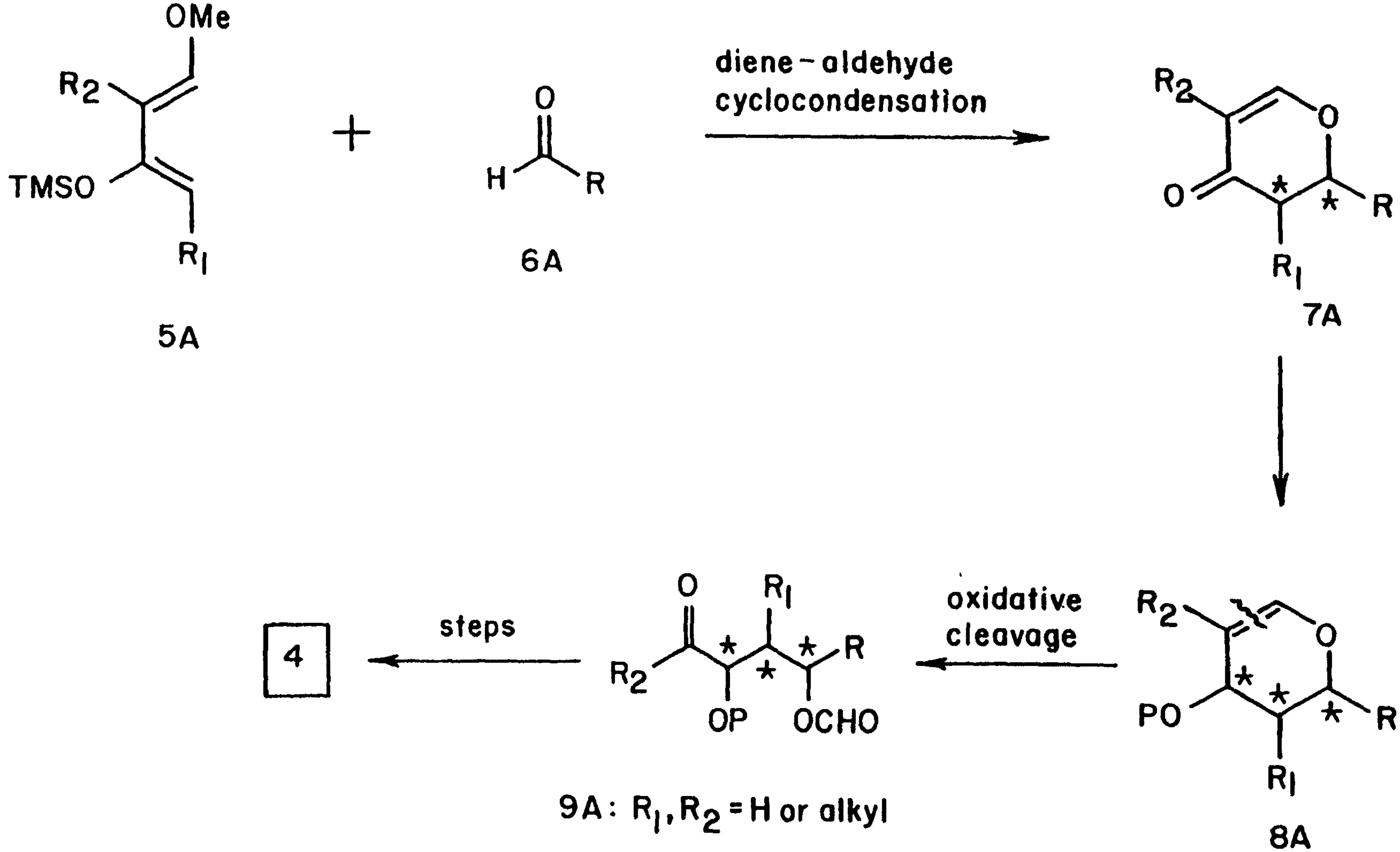


5A

6A

7A

8A

9A: $R_1, R_2$ = H or alkyl

4

# FIG. 14A

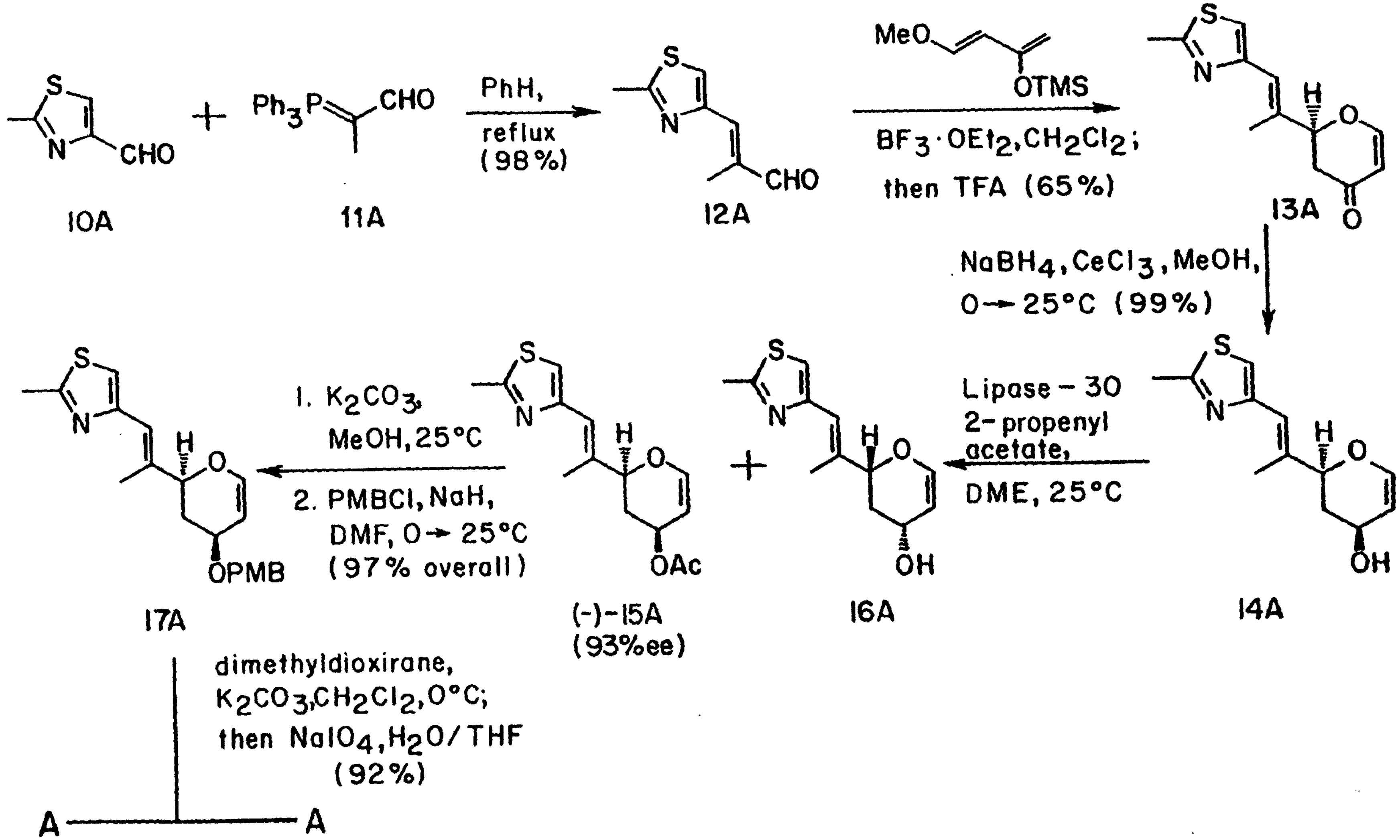


10A
11A
12A
13A
14A
16A
(-)-15A
(93%ee)
17A

# FIG. 14B

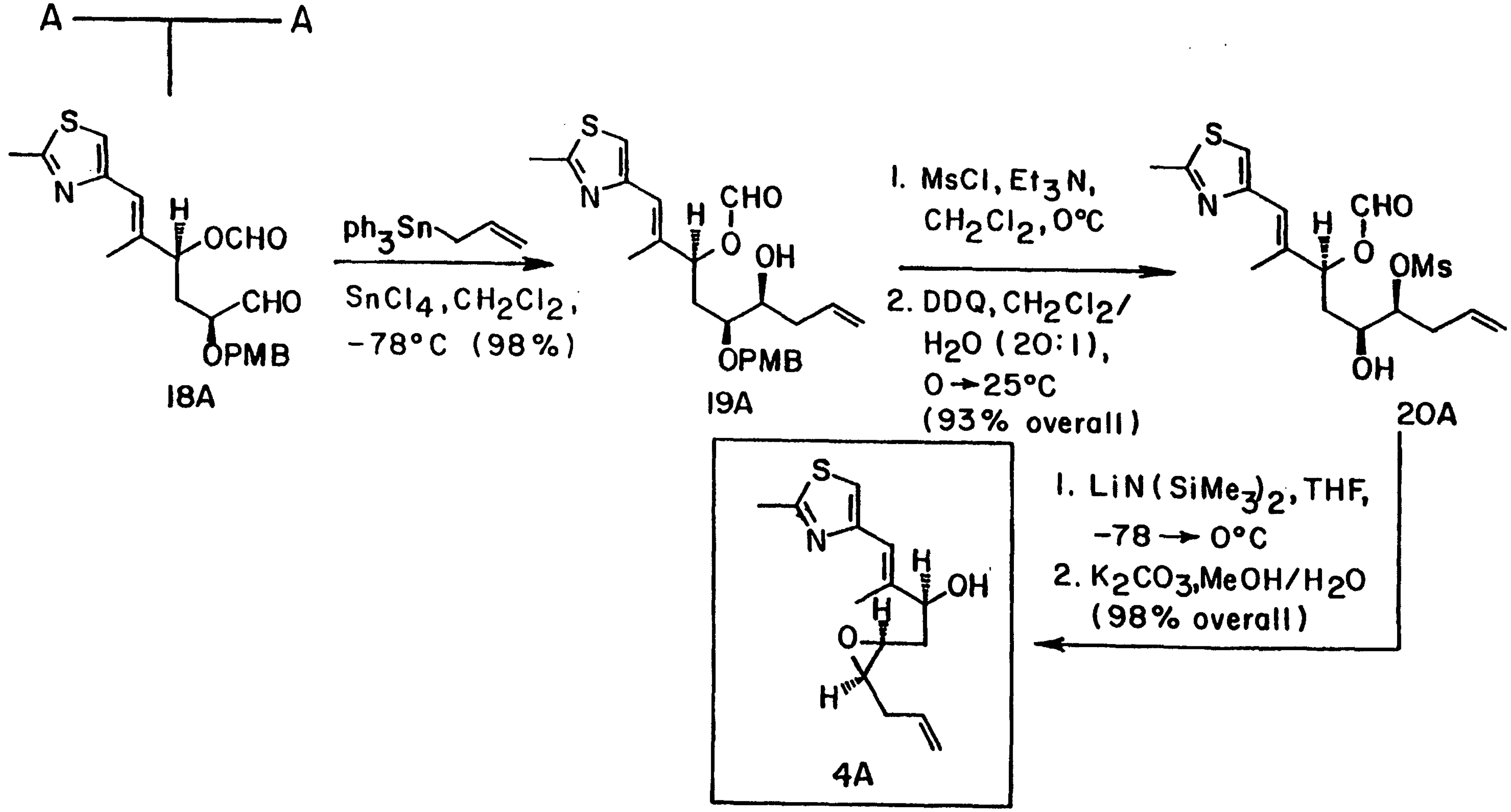
A
A
18A
ph3Sn
SnCl4, CH2Cl2,
-78°C (98%)
19A
1. MsCl, Et3N,
CH2Cl2, 0°C
2. DDQ, CH2Cl2/
H2O (20:1),
0→25°C
(93% overall)
20A
1. LiN(SiMe3)2, THF,
-78 → 0°C
2. K2CO3, MeOH/H2O
(98% overall)
4A

FIG. 15

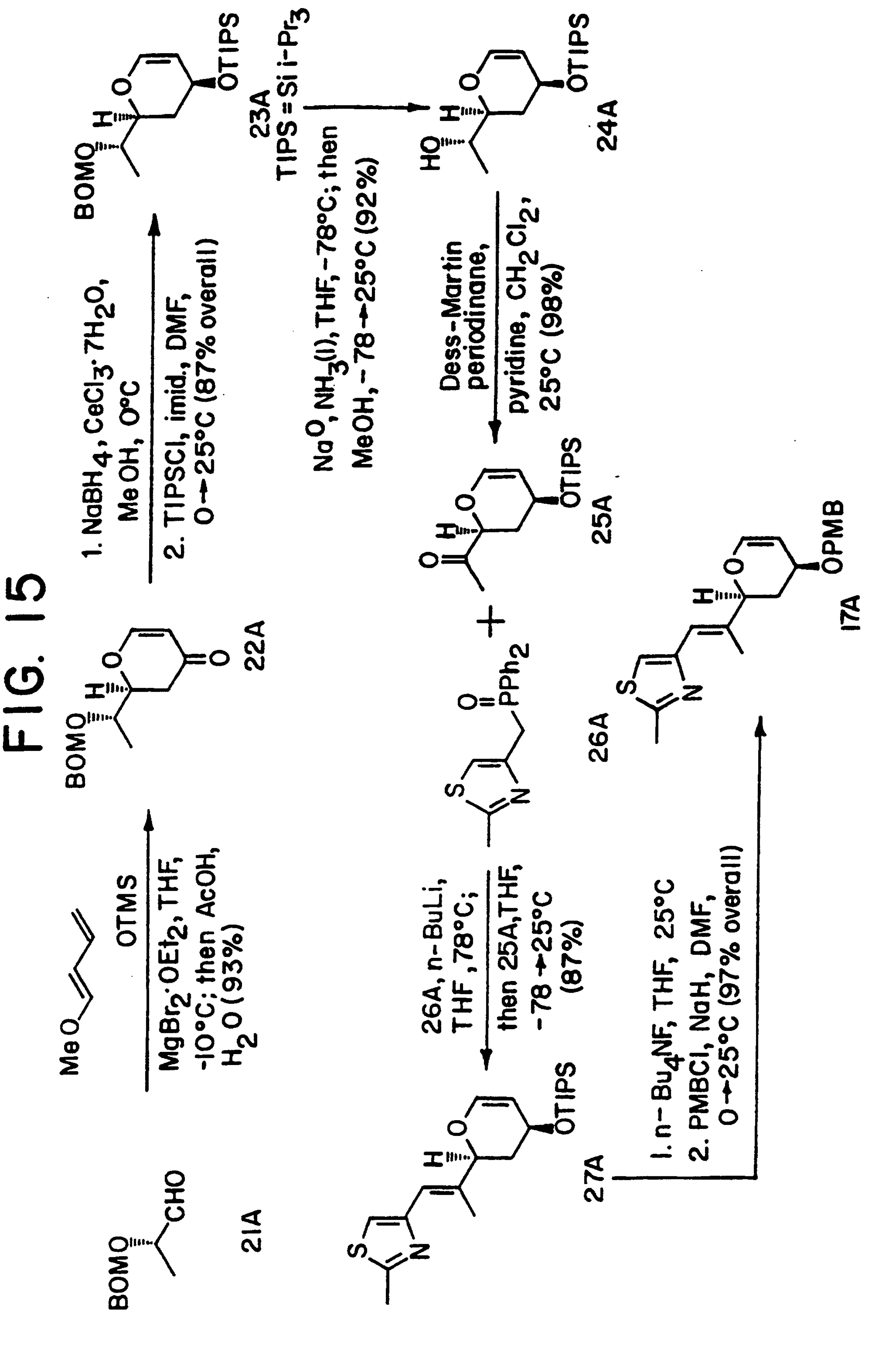


21A, 22A, 23A, 24A, 25A, 26A, 27A, 17A

# FIG. 16

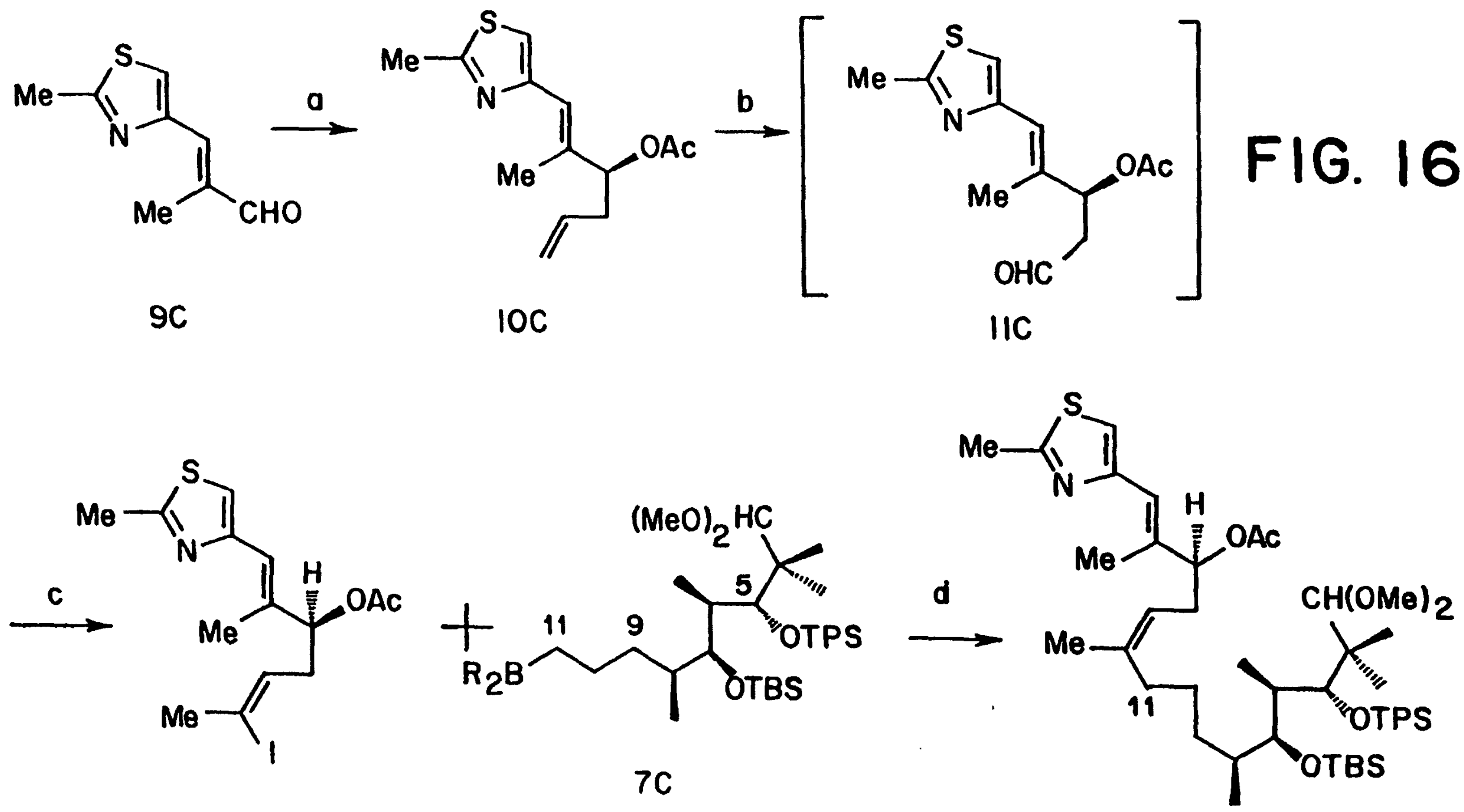


9C

10C

11C

8C

7C

13C

[(Ph$_3$P)CH$_2$CH$_3$]I

nBuLi

THF, rt

I$_2$, THF, −78°C

NaHMDS, THF, −20°C

Ph$_3$P=C(I)CH$_3$

12C

FIG. 17

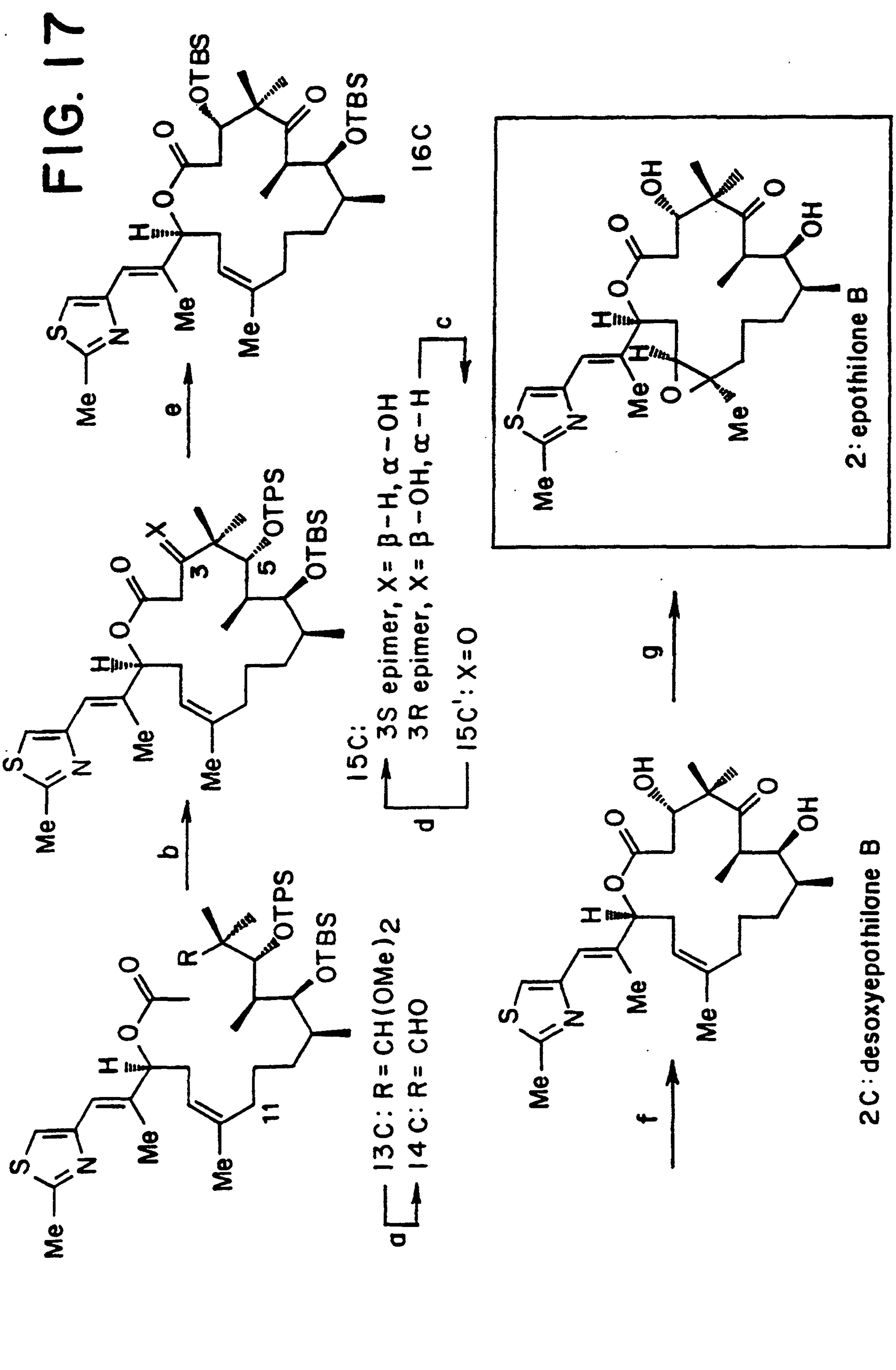


13C: R = $CH(OMe)_2$
14C: R = CHO

15C:
3S epimer, X = β-H, α-OH
3R epimer, X = β-OH, α-H
15C': X=O

16C

2C: desoxyepothilone B

2: epothilone B

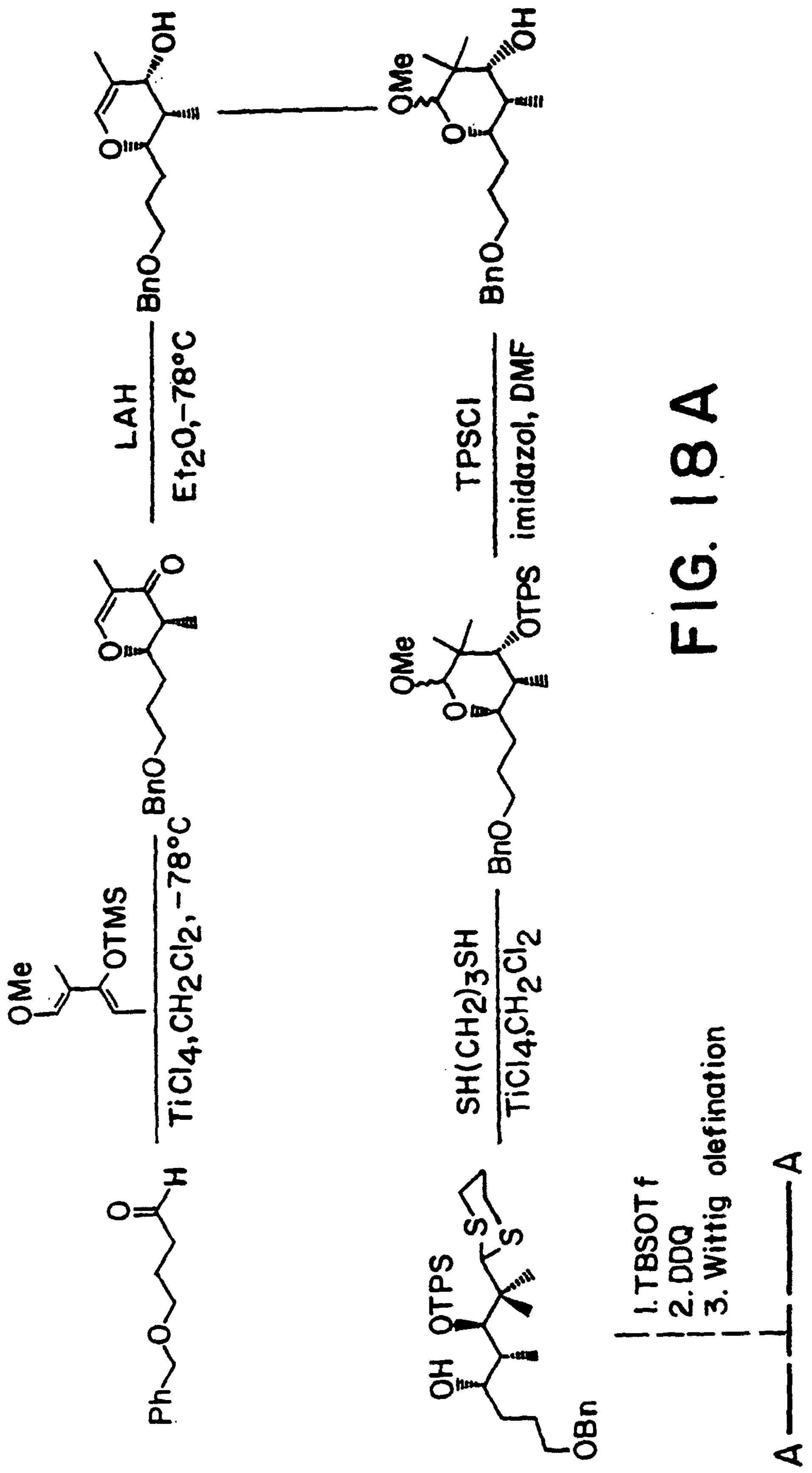
OMe
OTMS
TiCl4,CH2Cl2,-78°C
LAH
Et2O,-78°C
SH(CH2)3SH
TiCl4,CH2Cl2
TPSCl
imidazol, DMF
1. TBSOTf
2. DDQ
3. Wittig olefination

FIG. 18A

FIG. 18B

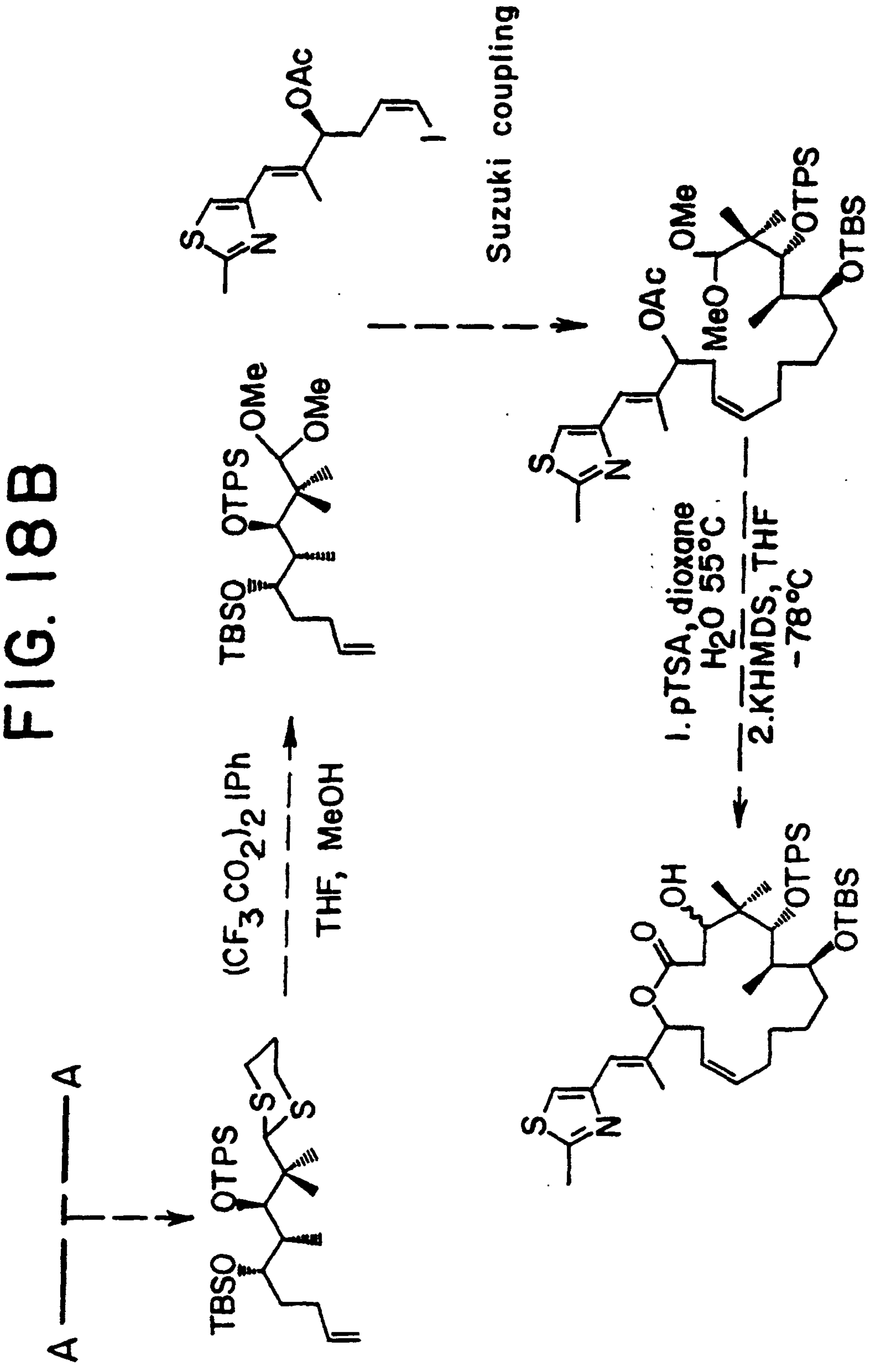
(CF3CO2)2IPh
THF, MeOH
Suzuki coupling
1. pTSA, dioxane
H2O 55°C
2. KHMDS, THF
-78°C

FIG. 19A

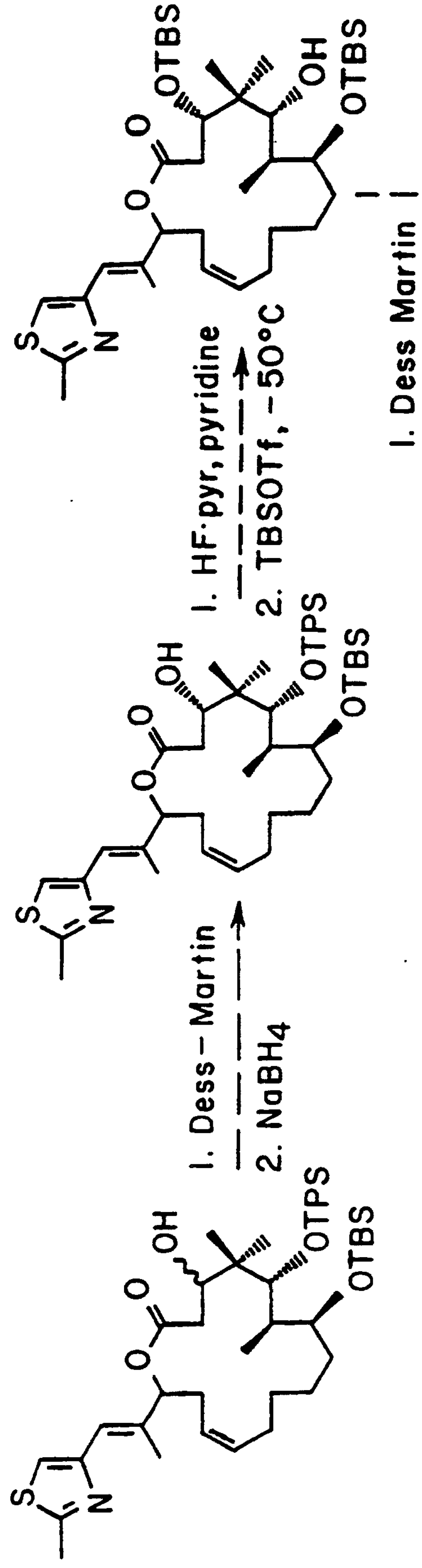


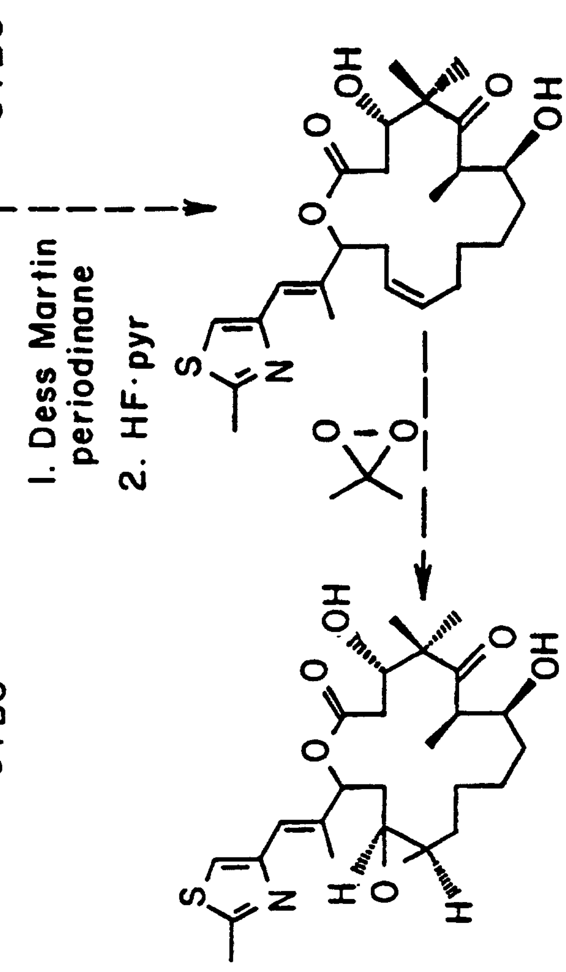


FIG. 19B

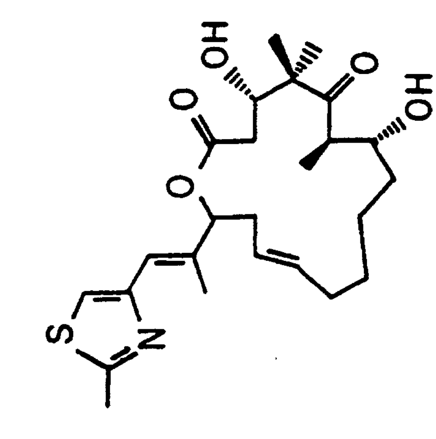

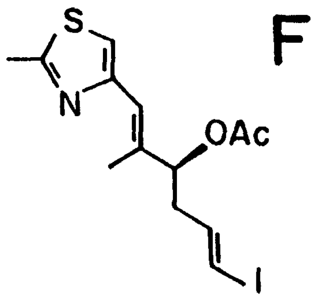

FIG. 19C

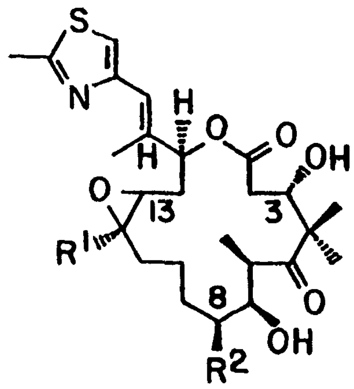

FIG. 20A

$R^1 = H, R^2 = Me$; epothilone A

$R^1 = R^2 = Me$; epothilone B

$R^1 = R^2 = H$; $C_8$-desmethyl-epothilone A (3D)

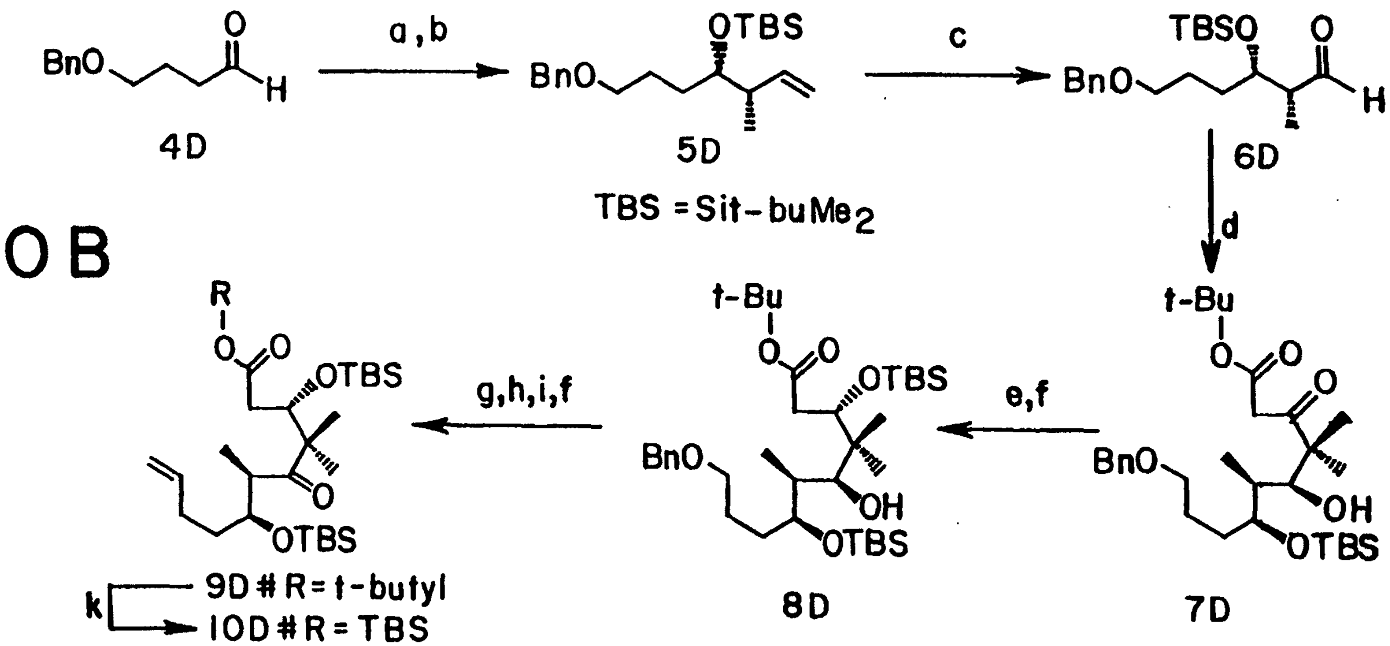


4D

5D

TBS = Sit-buMe$_2$

6D

7D

8D

9D # R = t-butyl

10D # R = TBS

FIG. 20B

# FIG. 21

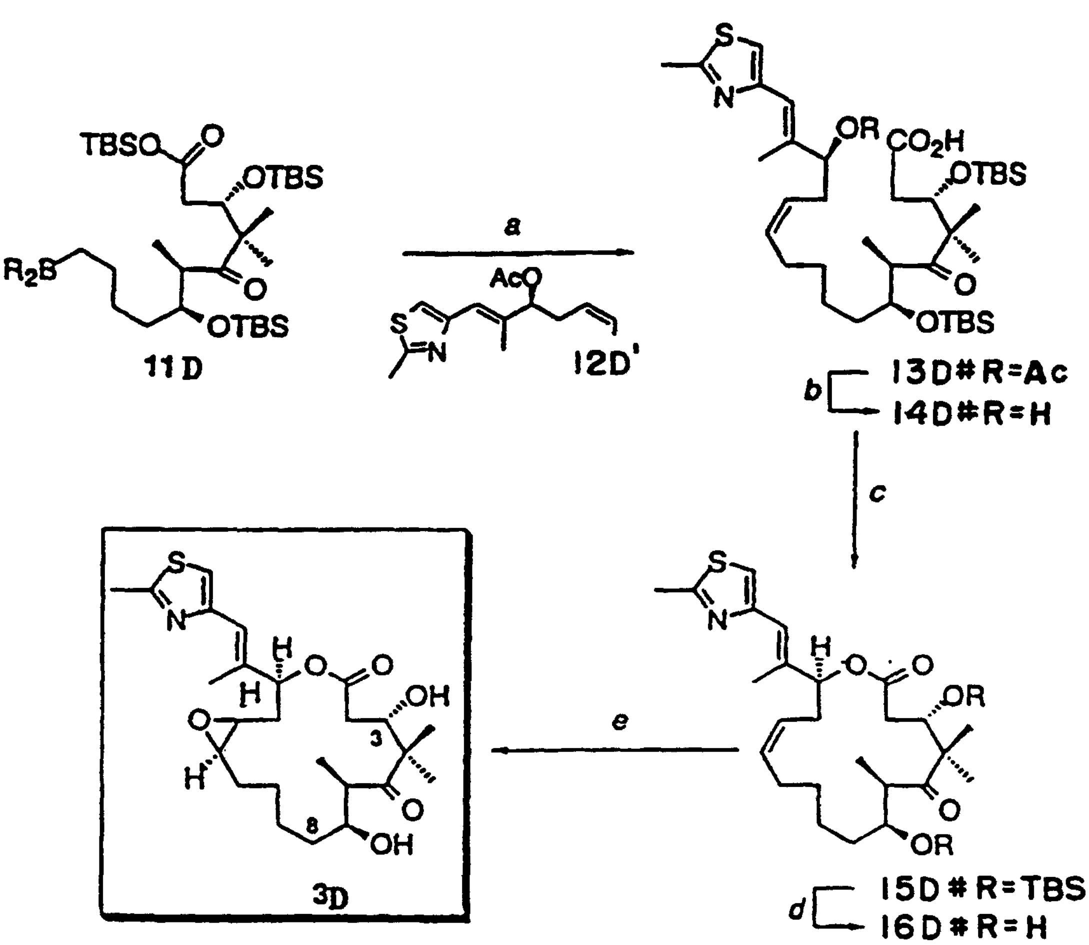


11D

12D'

13D # R=Ac

14D # R=H

15D # R=TBS

16D # R=H

3D

FIG. 22 A

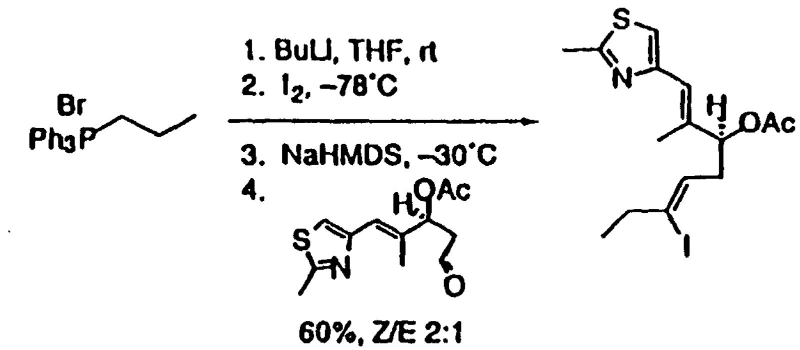


FIG. 22 B

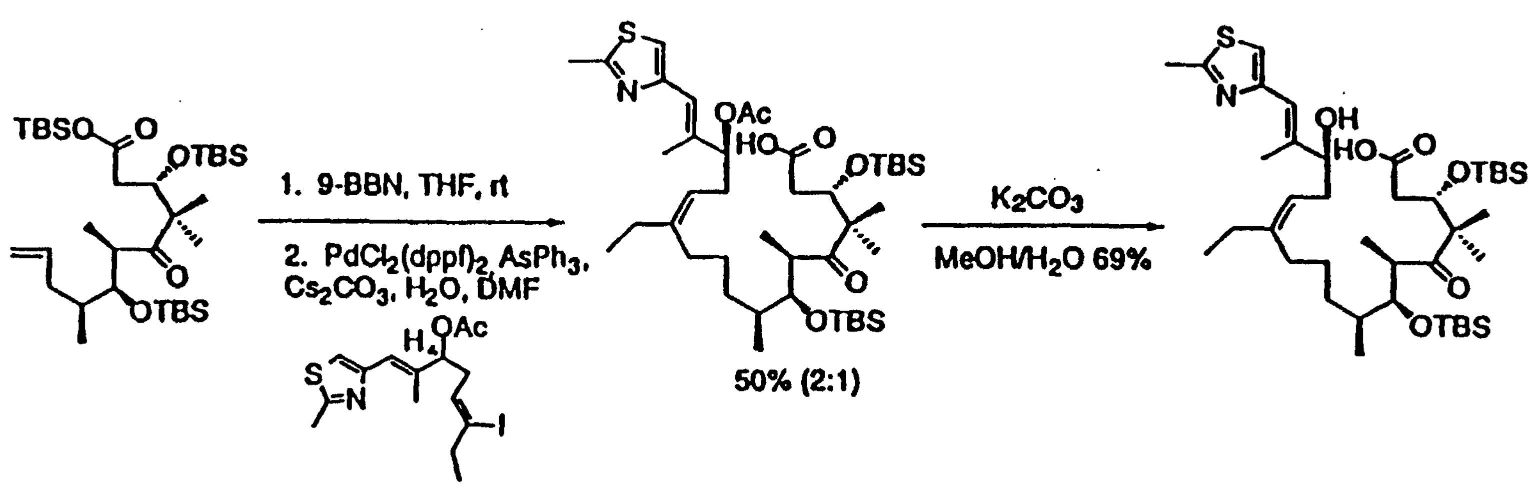

FIG. 22C

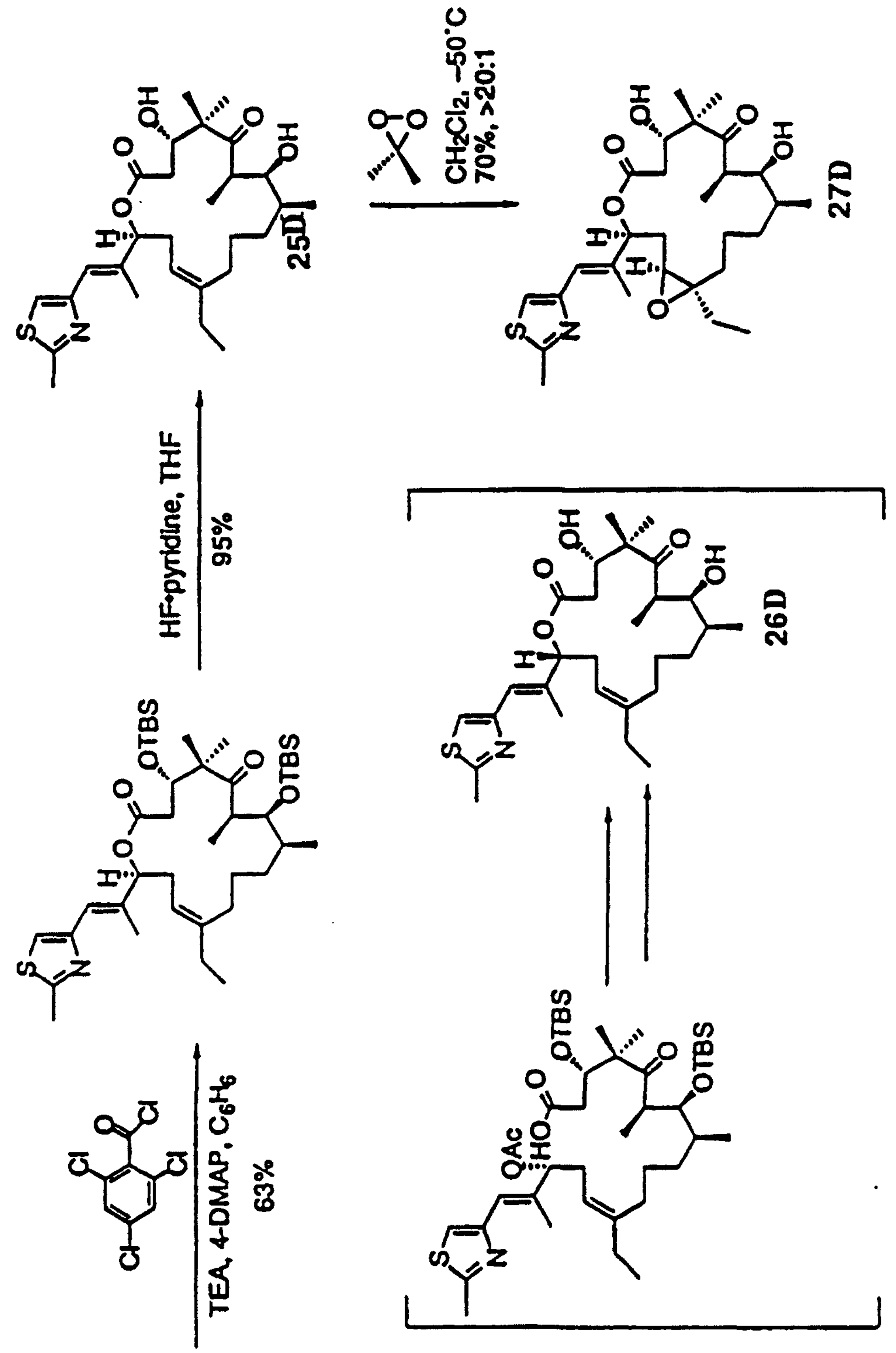


25D

26D

27D

# FIG. 23A

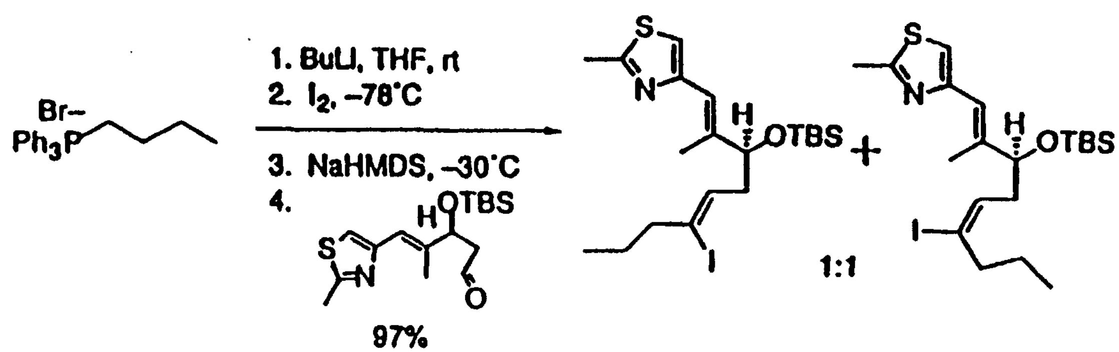


# FIG. 23B

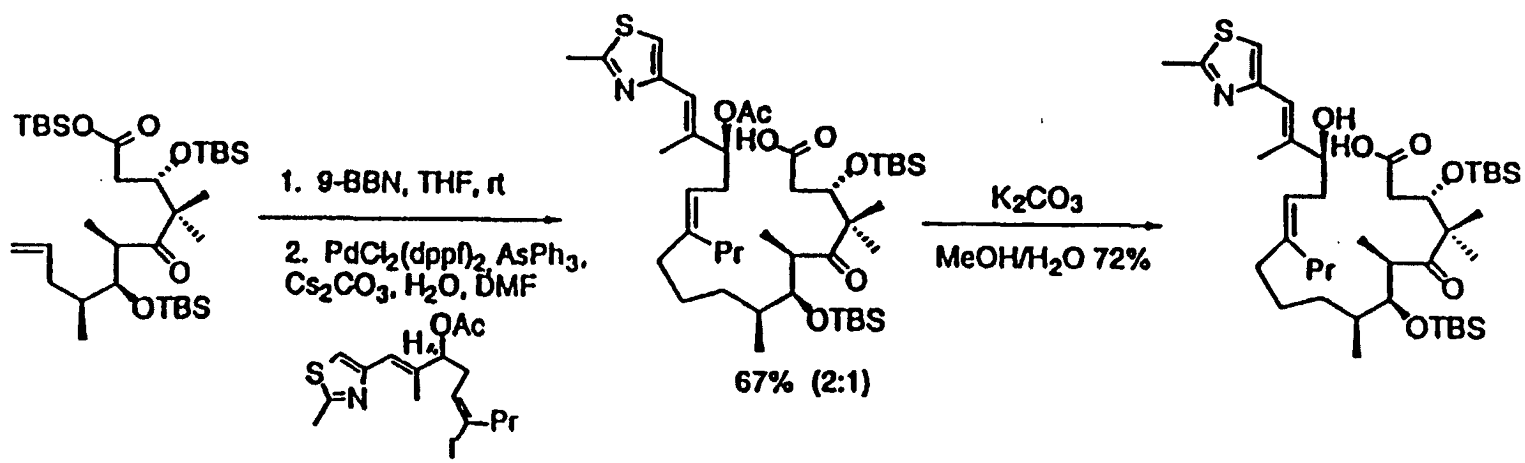

FIG. 23C

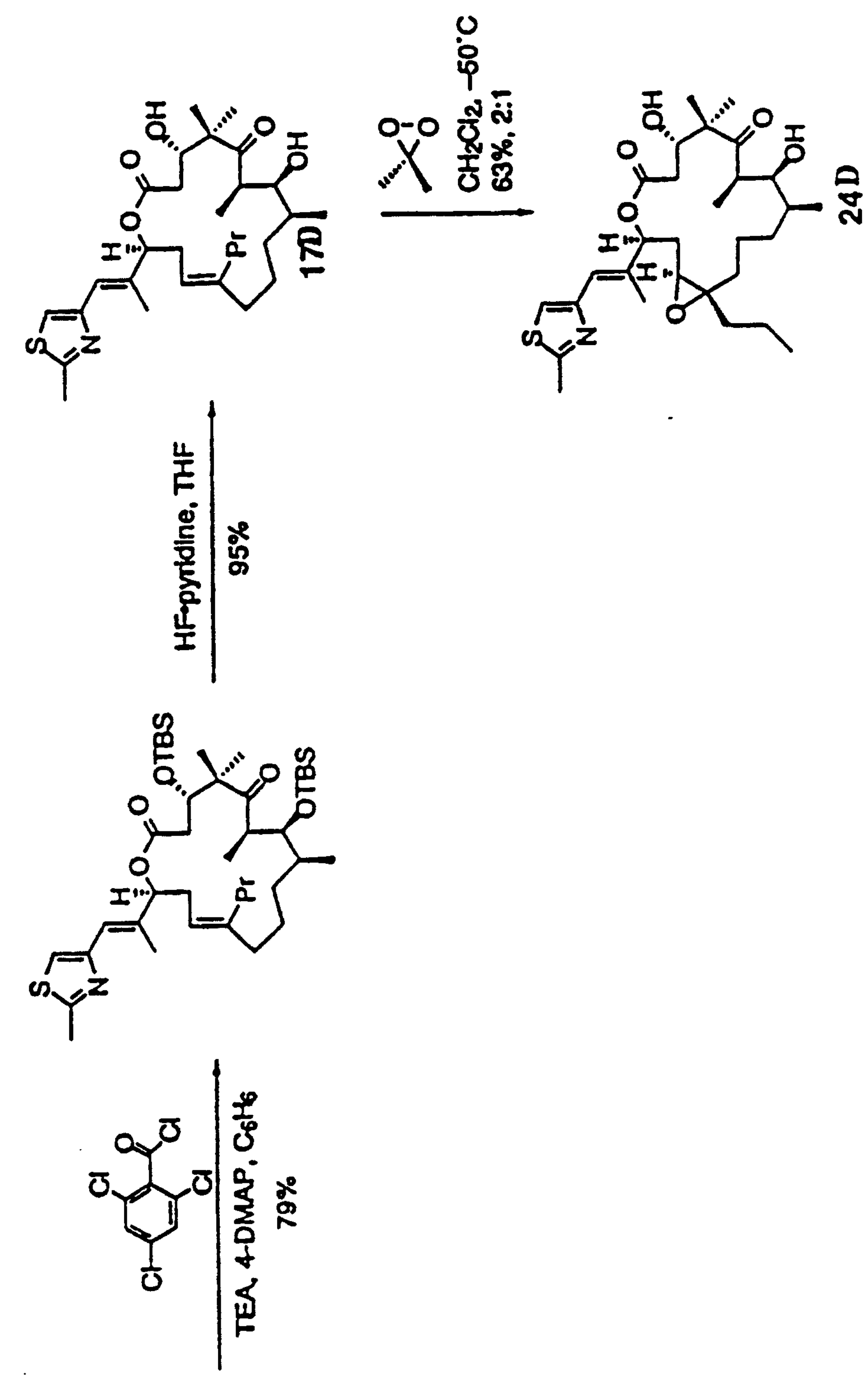


17D

24D

FIG. 24A

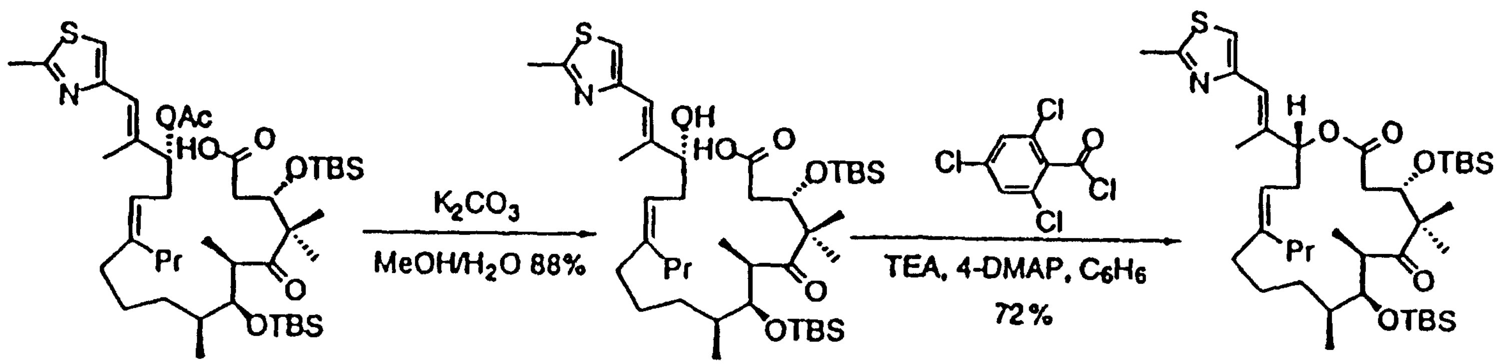


minor product from suzuki coupling reaction

FIG. 24B

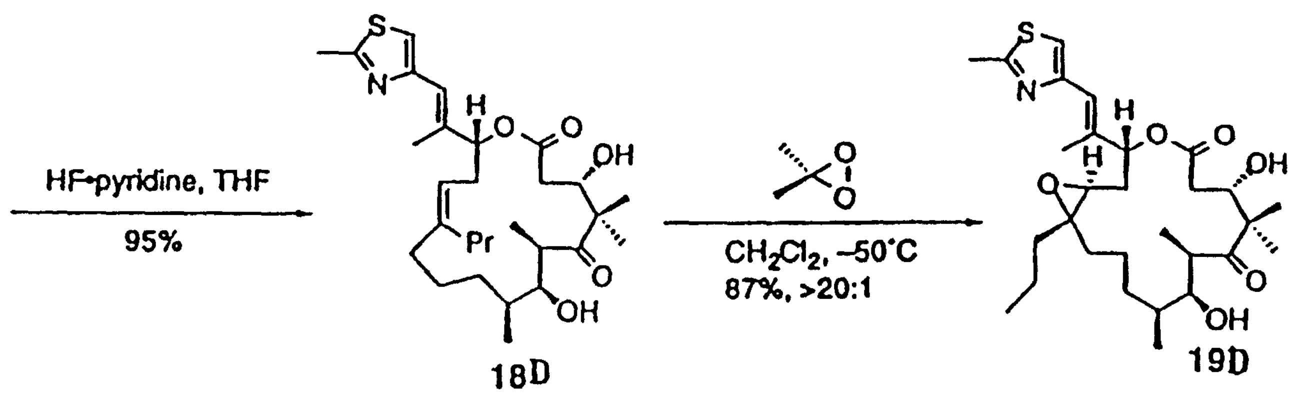


18D

19D

FIG. 25A

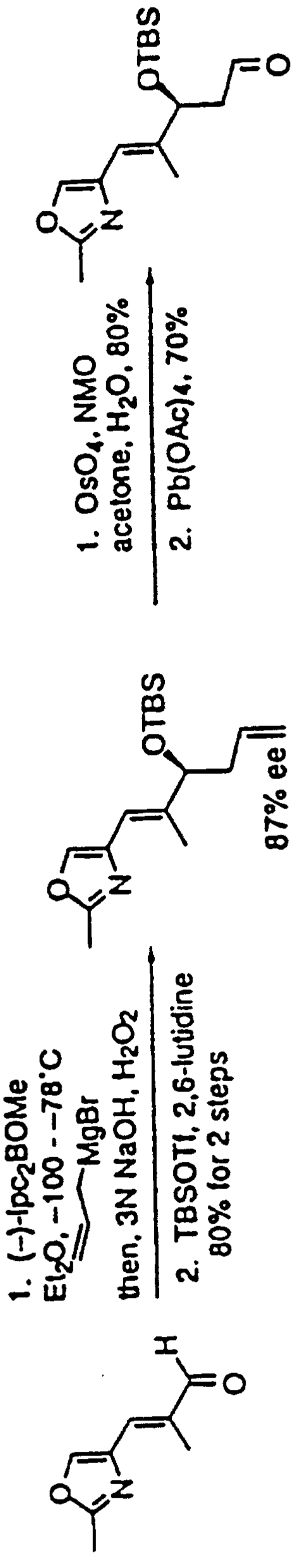


FIG. 25B

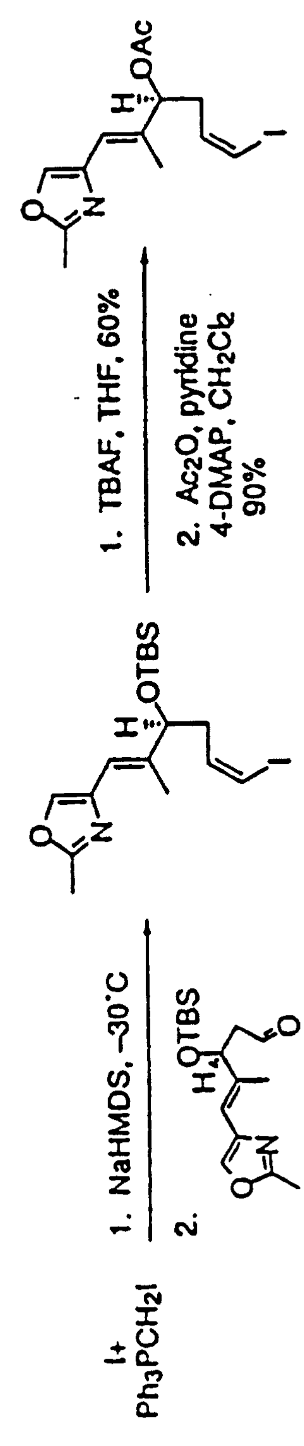

# FIG. 25C

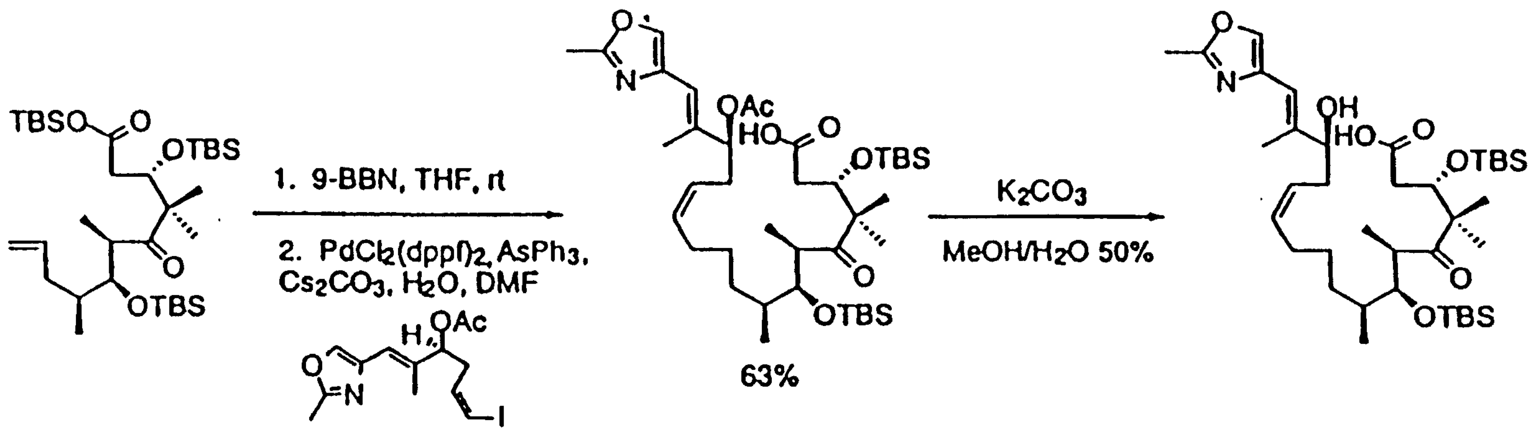


# FIG. 25D

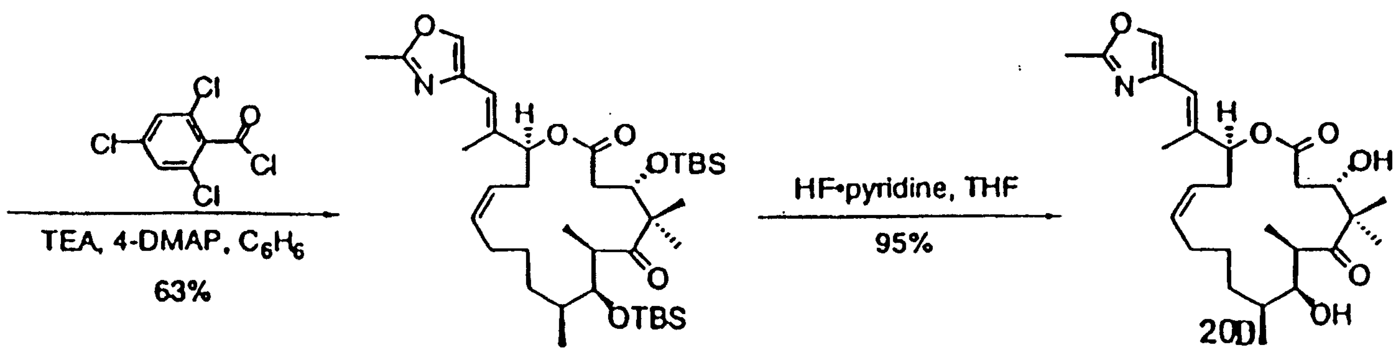


20D

# FIG. 26A

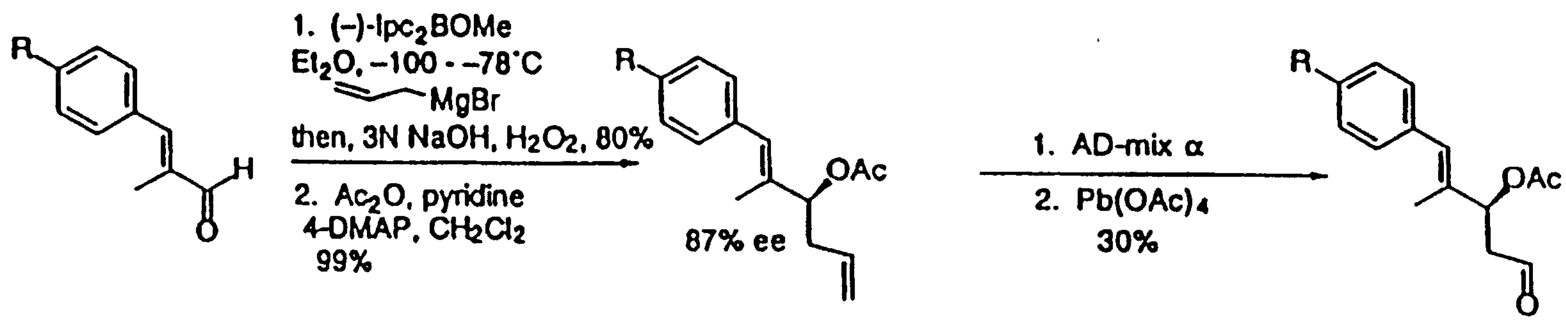


# FIG. 26B

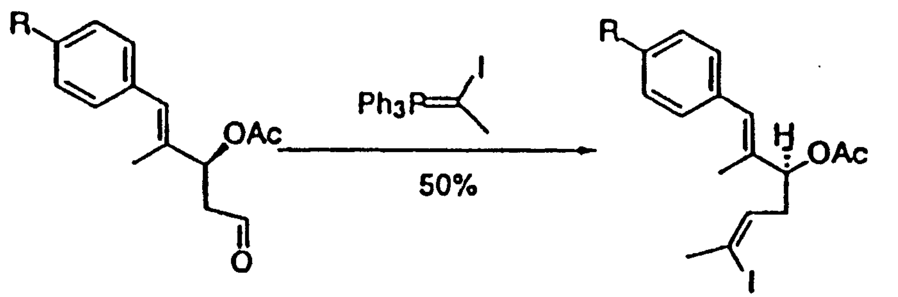


R = H, F, $CF_3$
R=H is the only compound completed, F and $CF_3$ are nearly completed

FIG. 26C

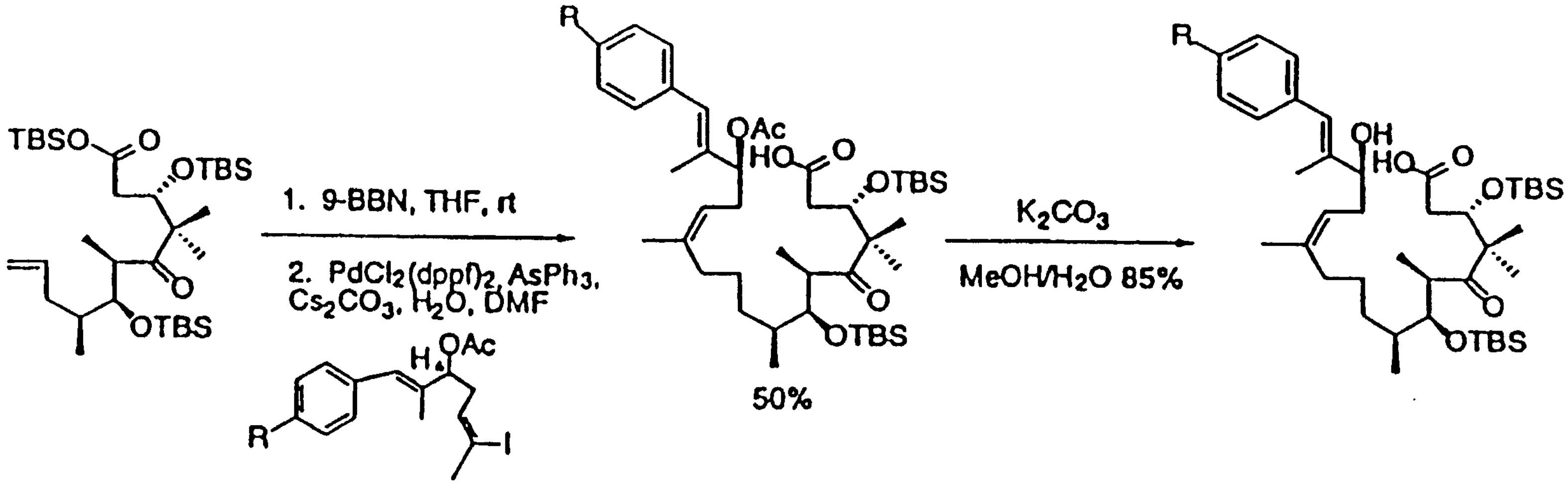


FIG. 26D

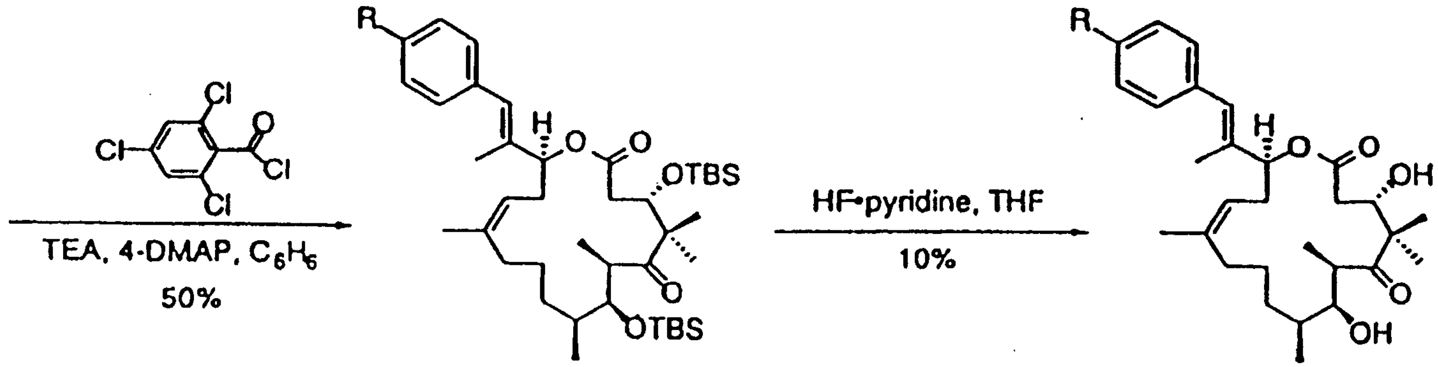


22D (R = H)

FIG. 27A

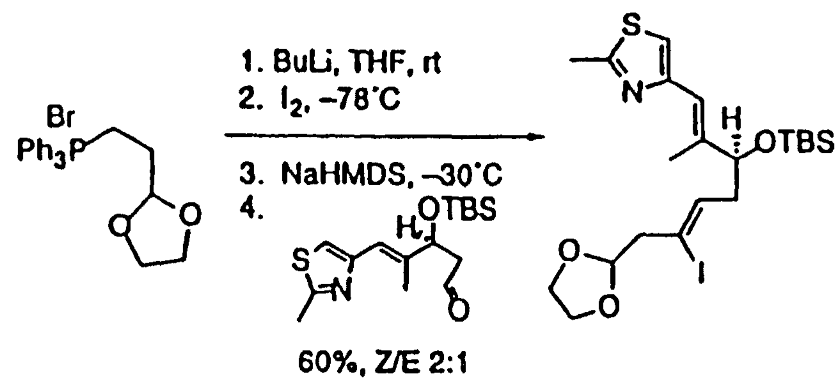


FIG. 27B

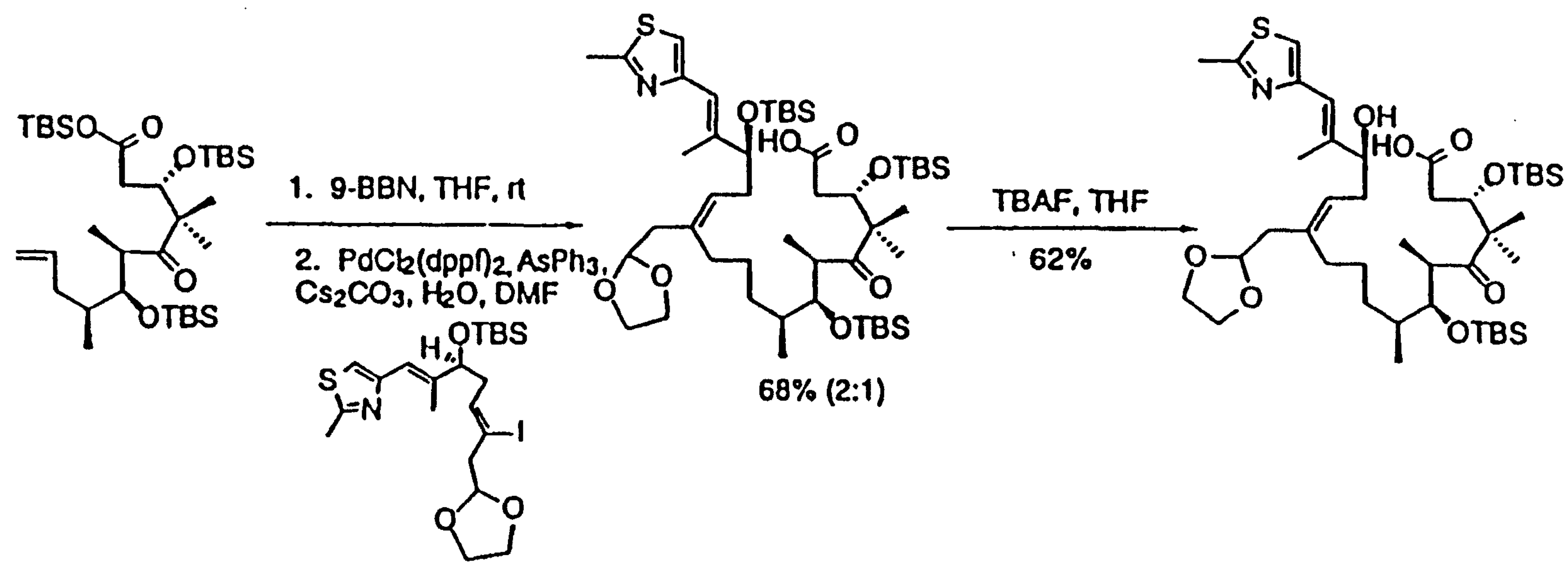

# FIG. 27C

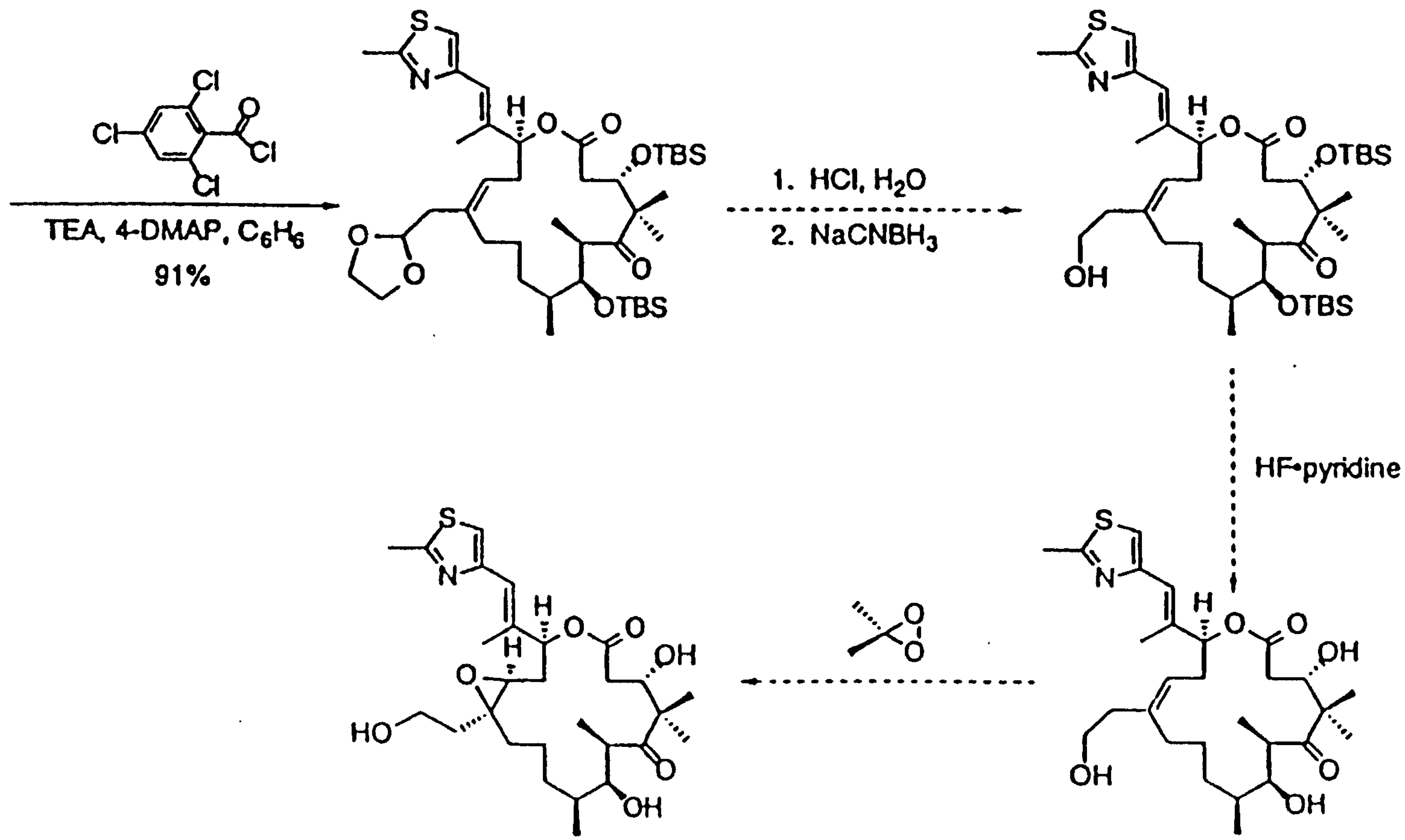

FIG. 28A

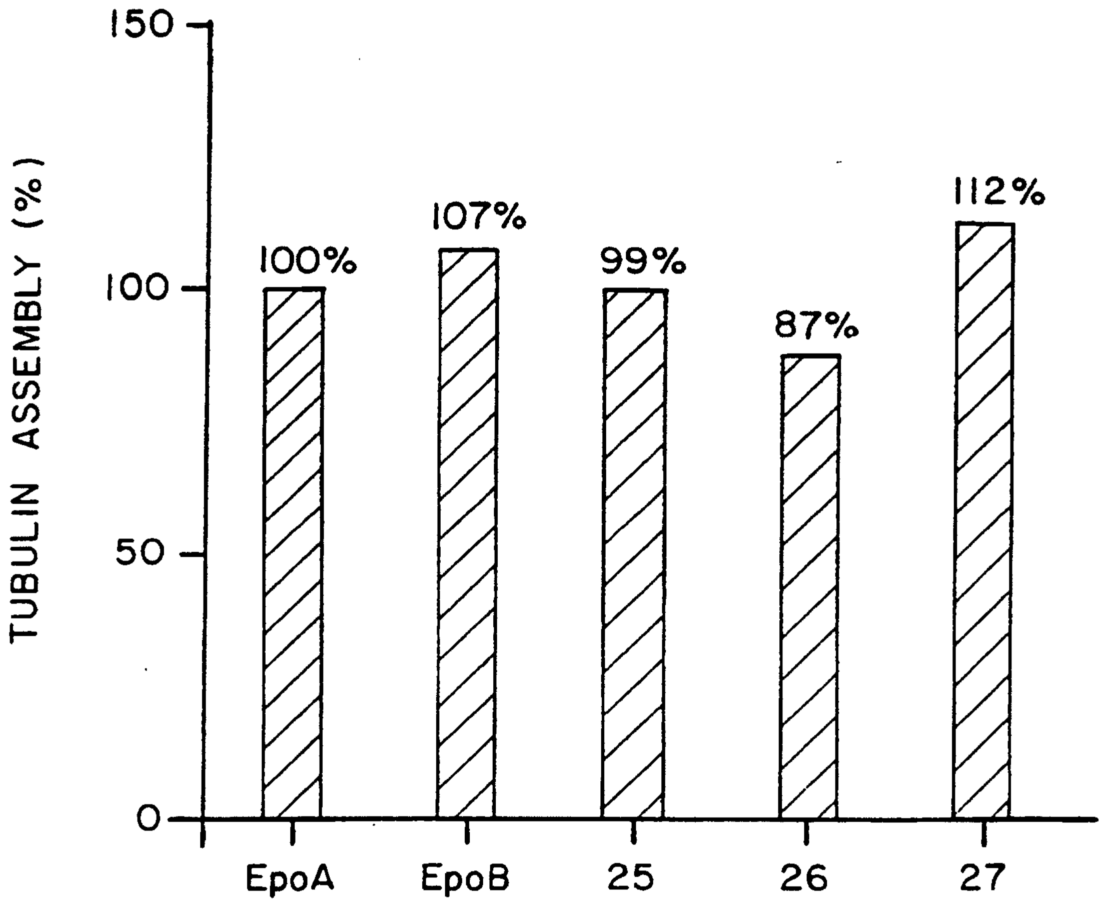
150
100
50
0
TUBULIN ASSEMBLY (%)
100%
107%
99%
87%
112%
EpoA
EpoB
25
26
27

FIG. 28B

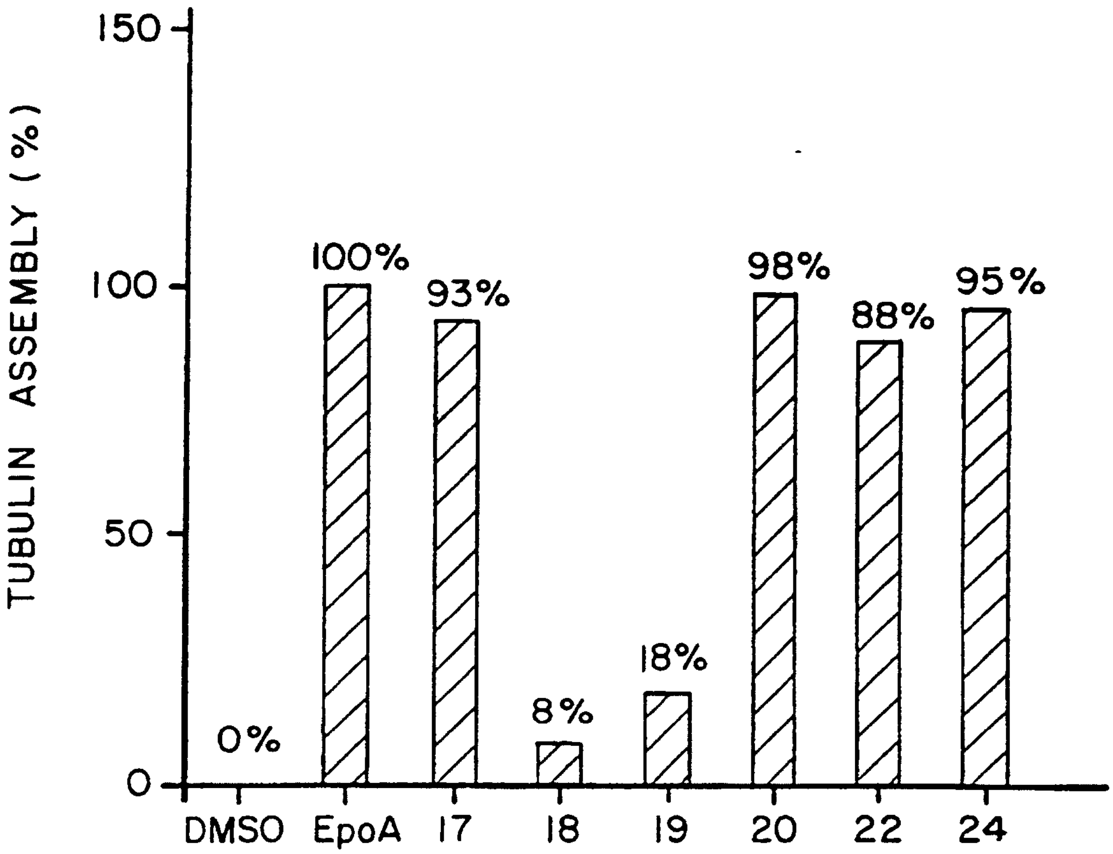
TUBULIN ASSEMBLY (%)
150
100
50
0
0%
100%
93%
8%
18%
98%
88%
95%
DMSO
EpoA
17
18
19
20
22
24

FIG. 29

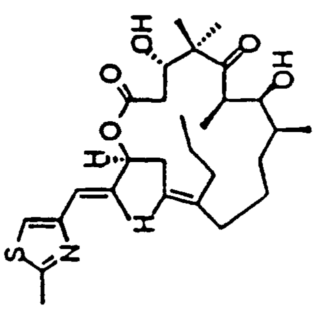

17

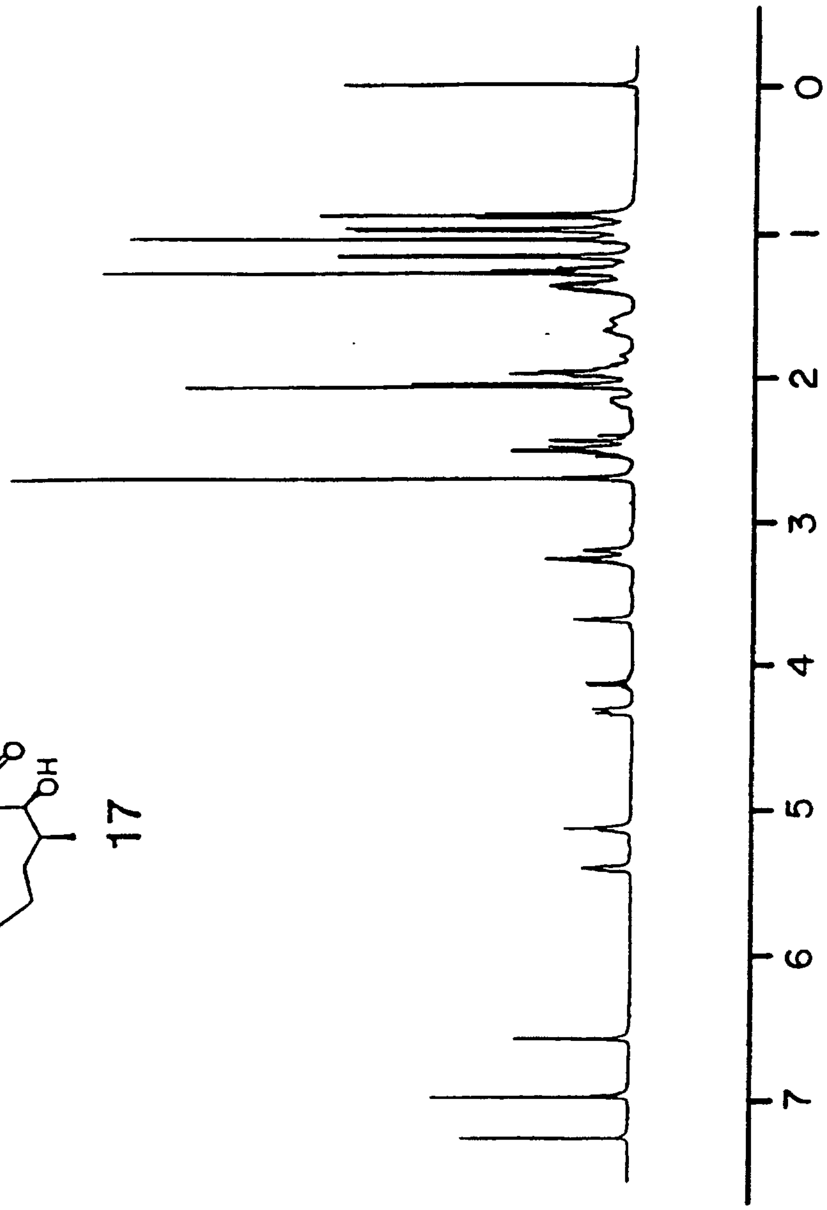

FIG. 30

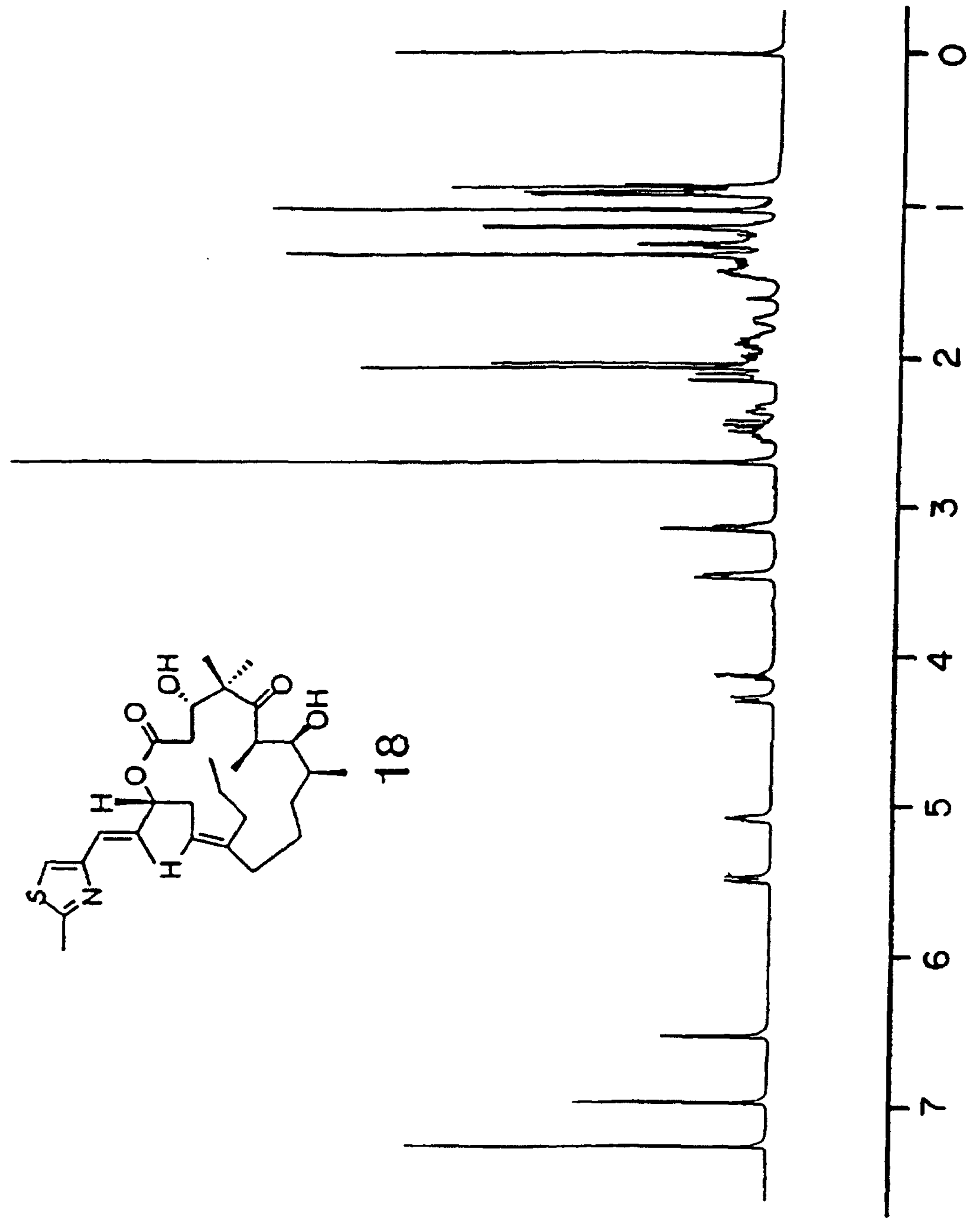


18

FIG. 31

19

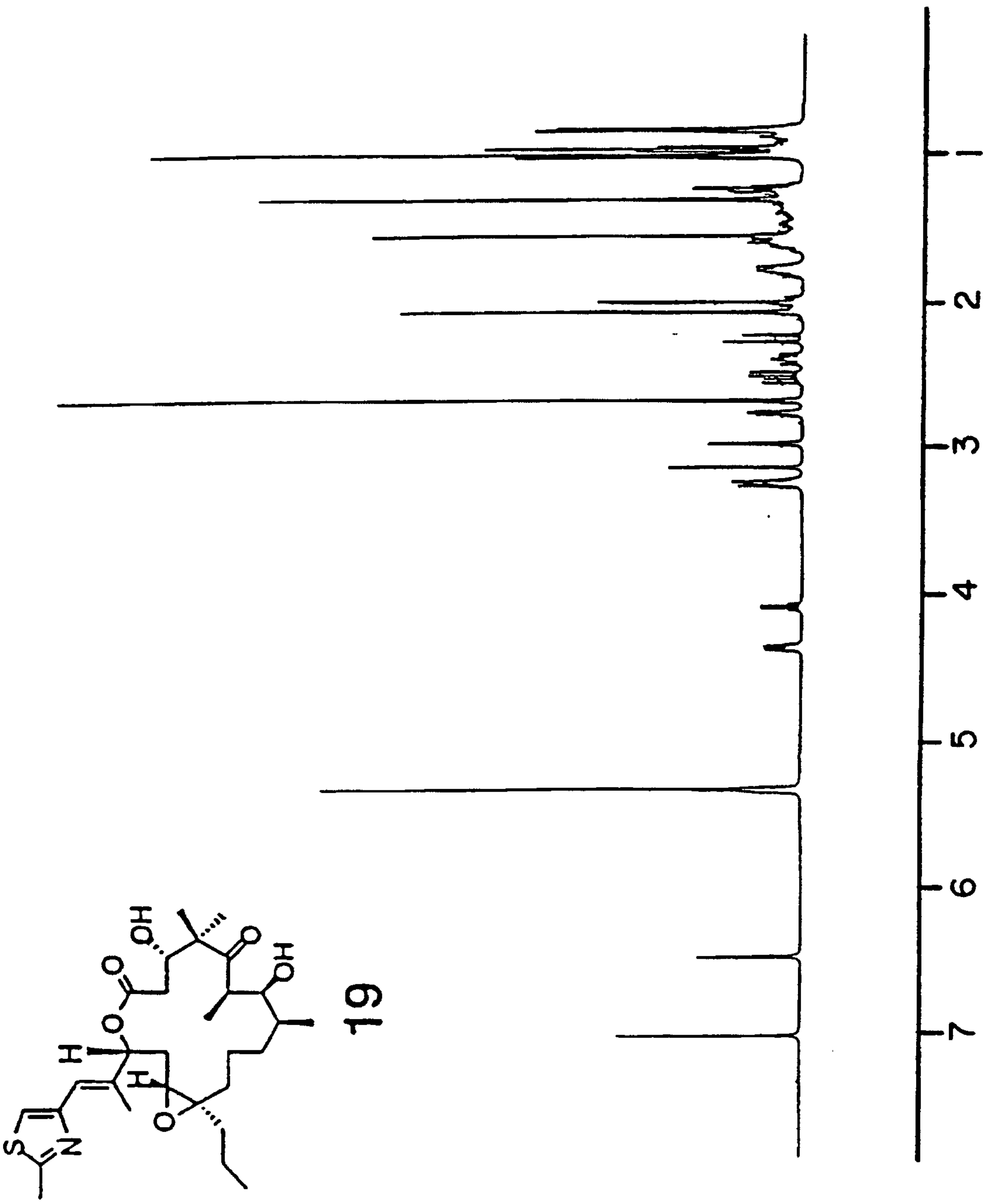

FIG. 32

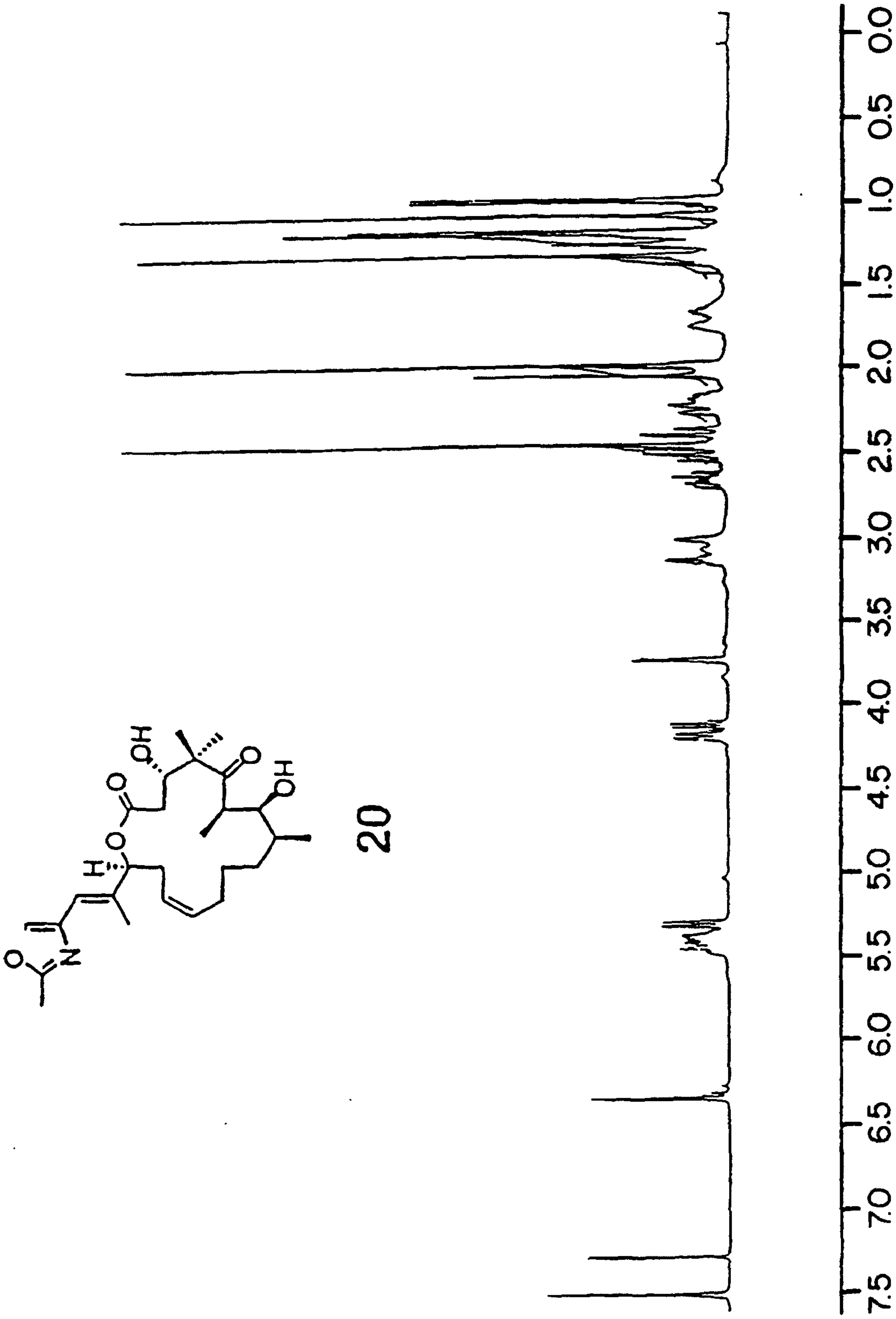

20

FIG. 33

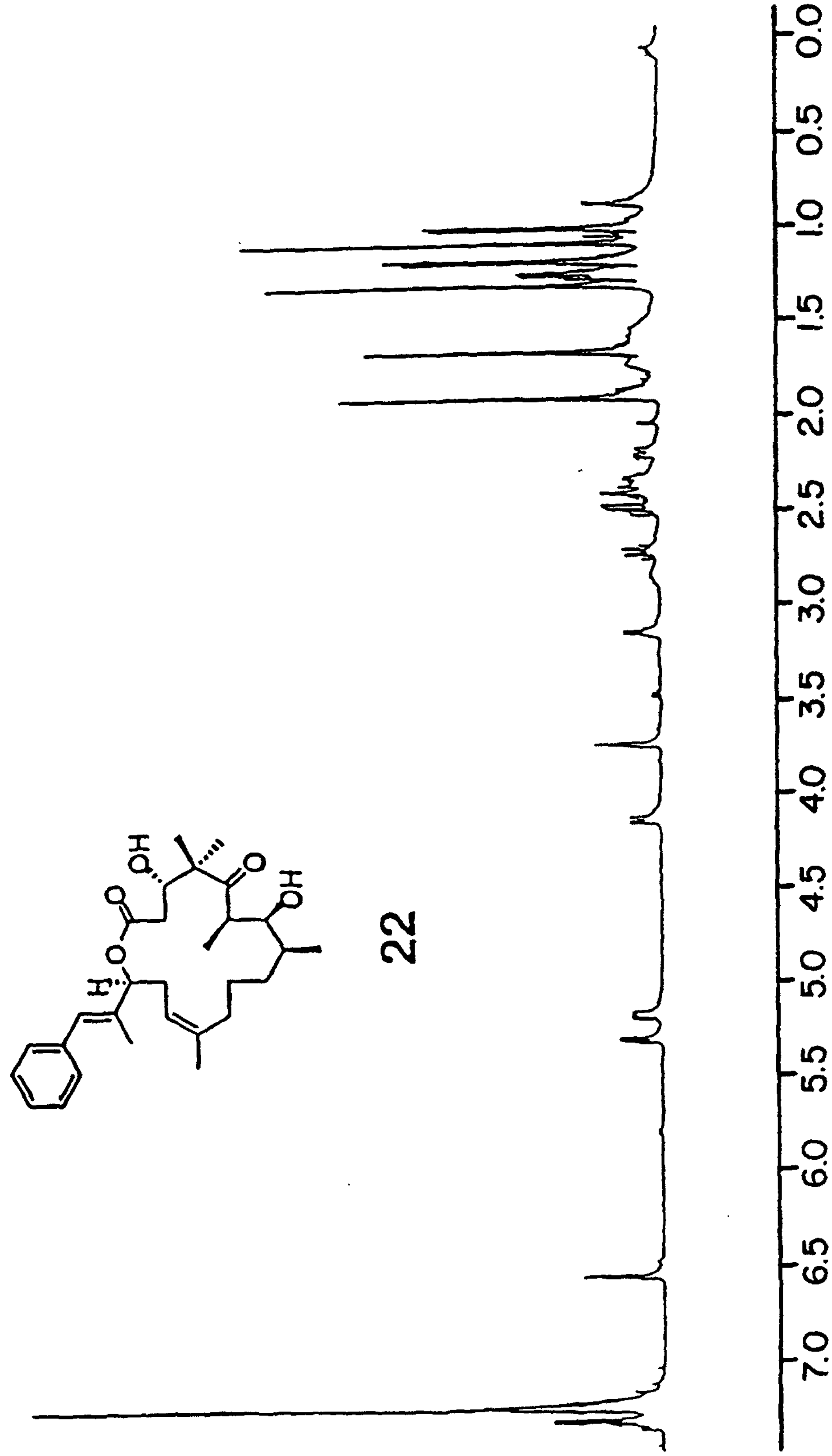

22

FIG. 34

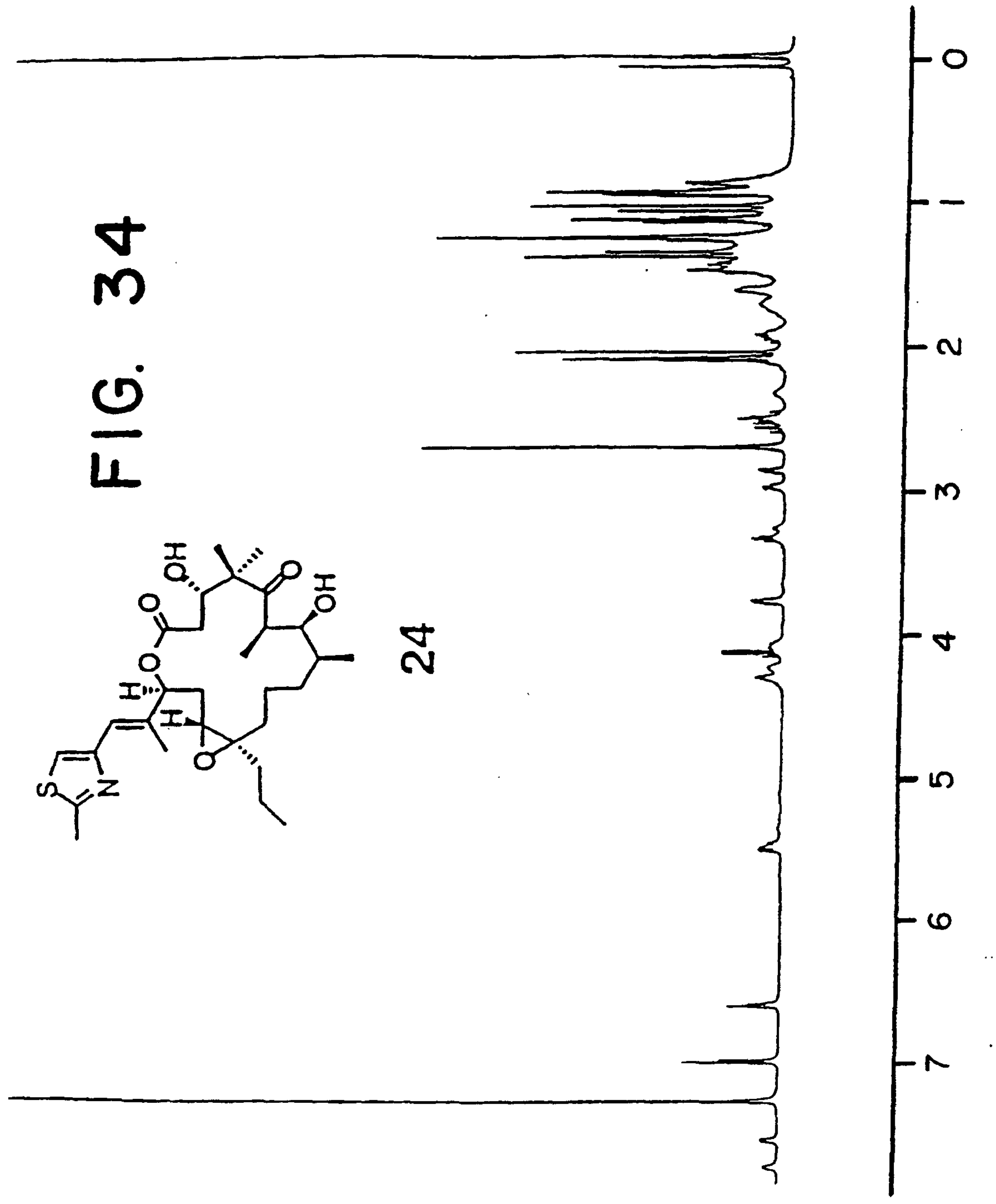


24

FIG. 35

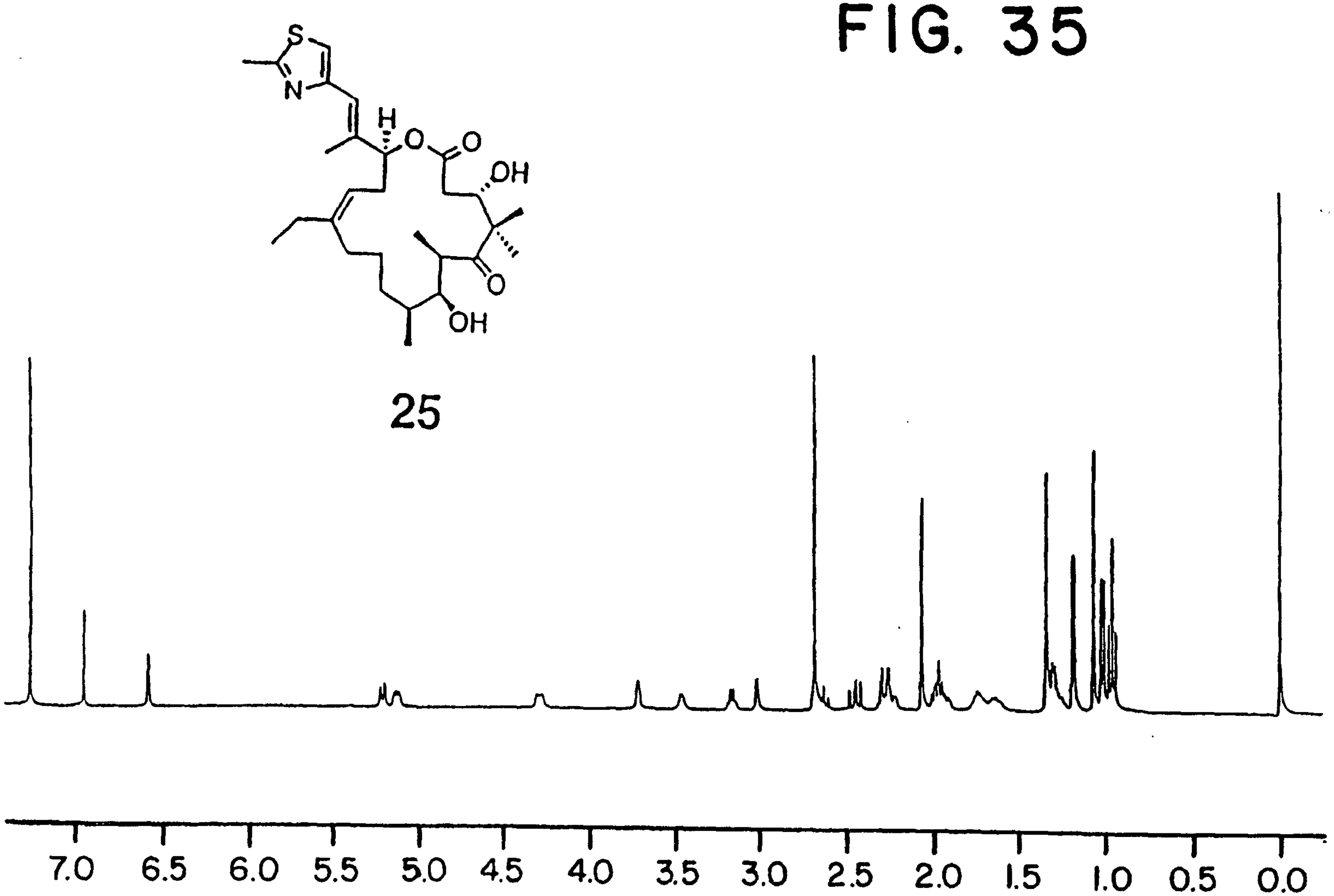


25

FIG. 36

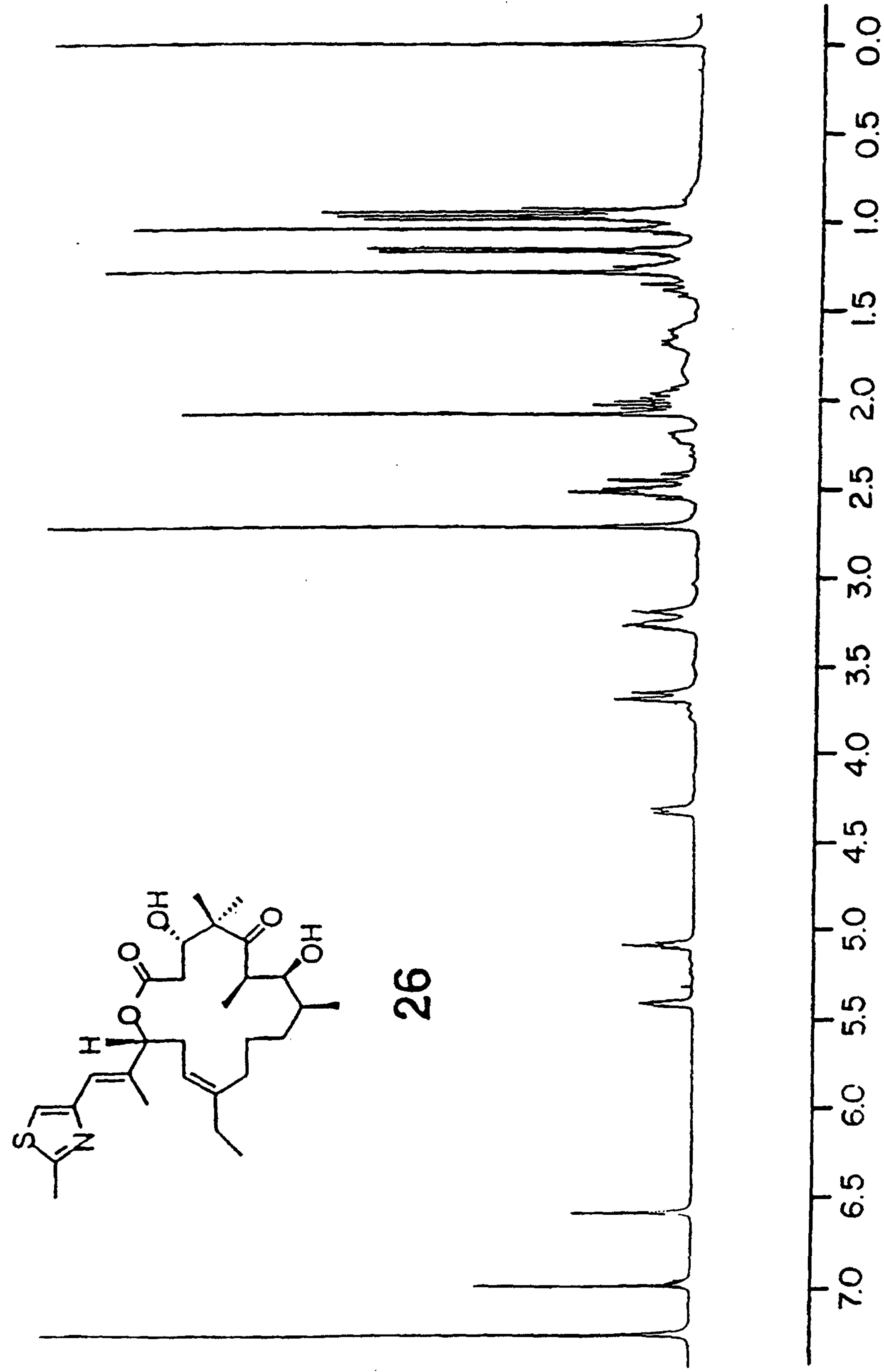


26

FIG. 37

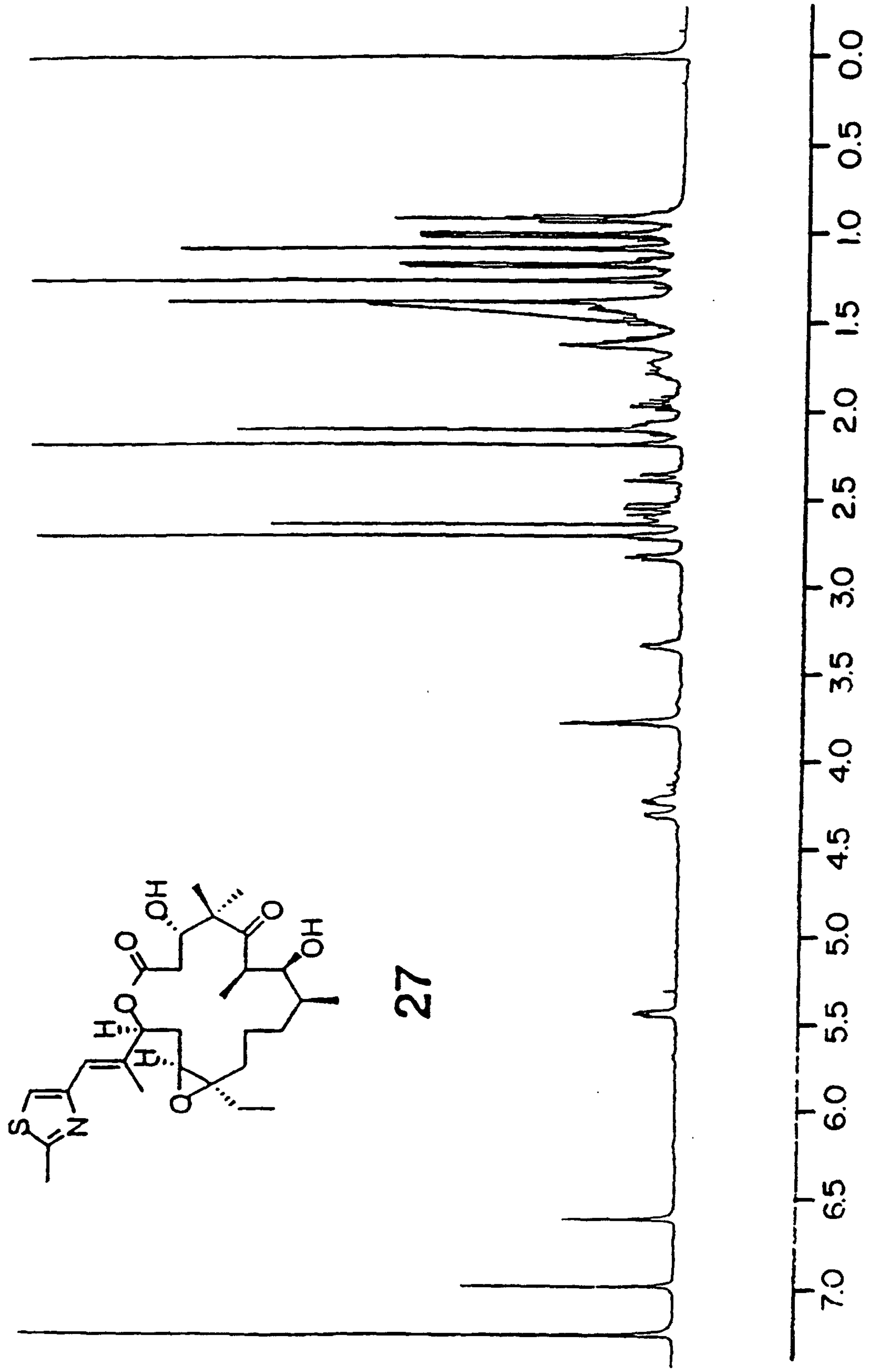


27

FIG. 38

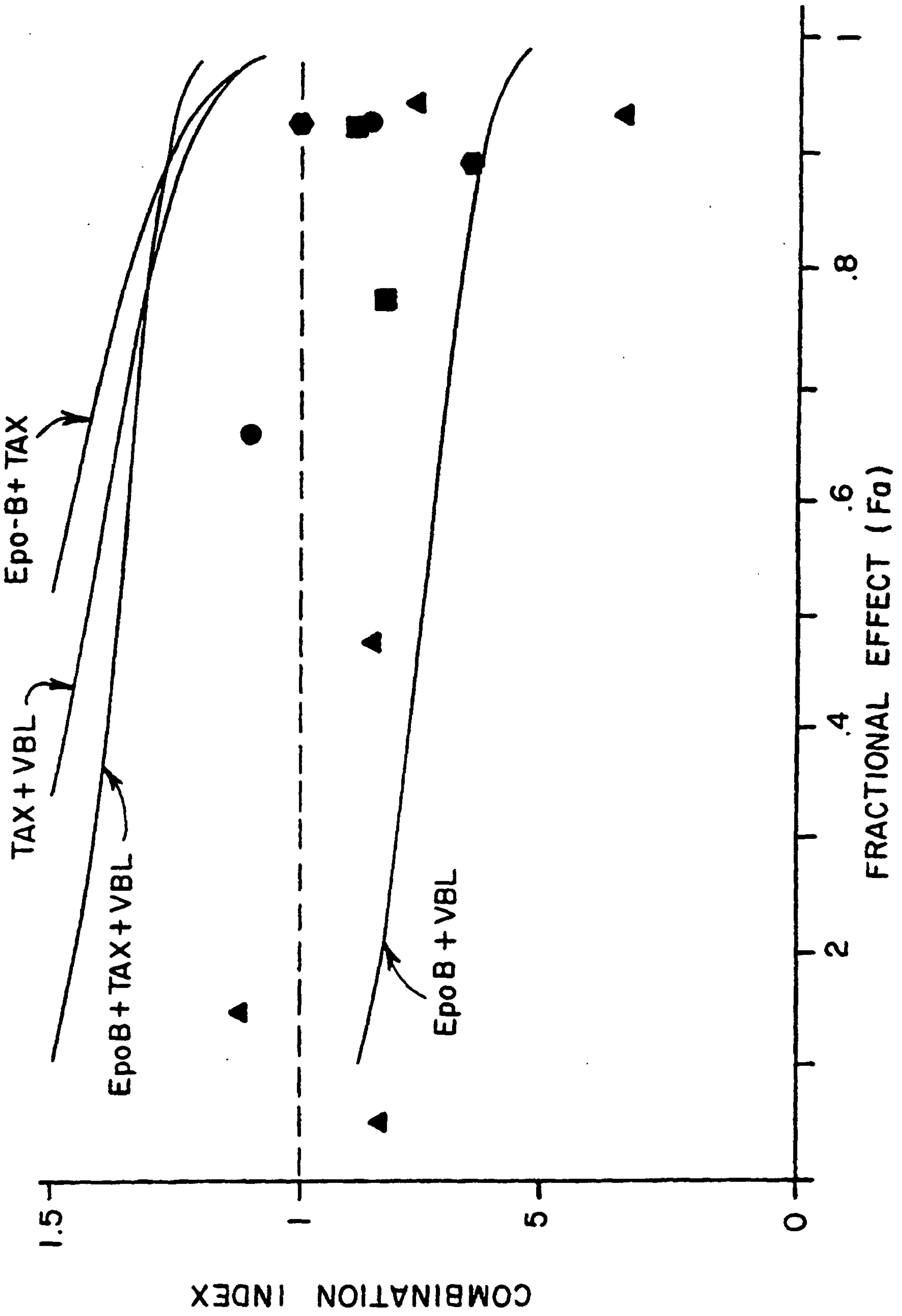
Epo-B+ TAX
TAX + VBL
EpoB + TAX + VBL
EpoB + VBL
1.5
1
5
0
COMBINATION INDEX
2
.4
.6
.8
1
FRACTIONAL EFFECT (Fa)

# FIG. 39A

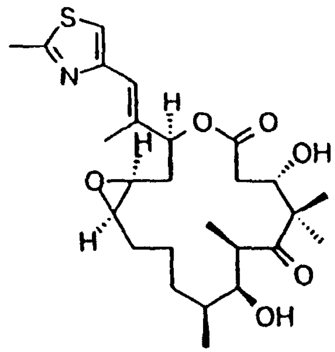

epothilone A
(0.0027)
[0.020]

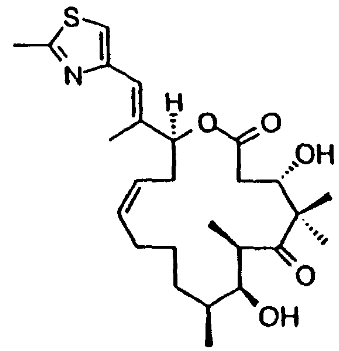

desoxyepothilone A
**1**
(0.022)
[0.012]

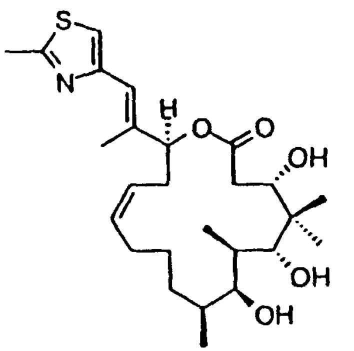

**2**
(14.23)
[6.28]

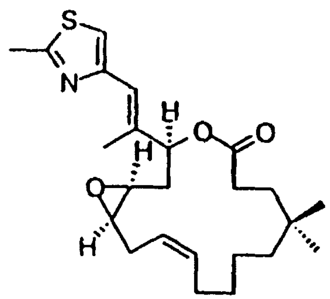

**3**
(271.1)
[22.4]

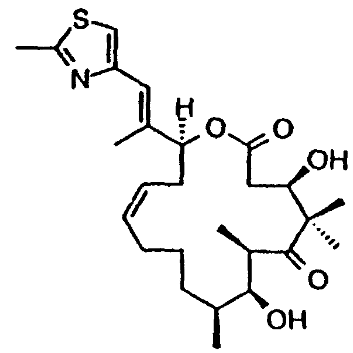

**4**
(2.12)
[43.0]

# FIG. 39B

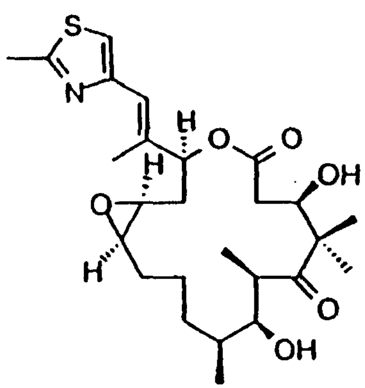

5
(>20)
[35.2]

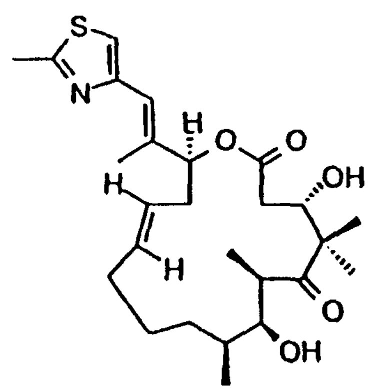

6
(0.052)
[0.035]

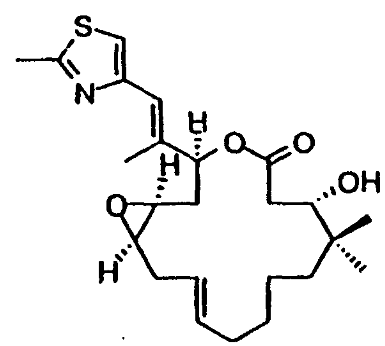

7
(7.36)
[9.82]

# FIG. 40A

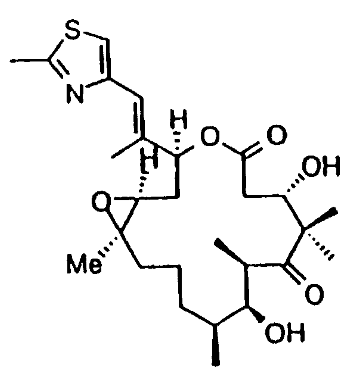

synthetic epothilone B

**8**
(0.00044)
[0.0026]

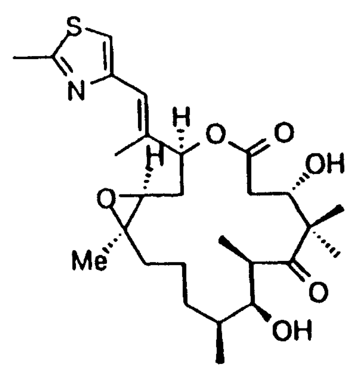

natural epothilone B

**9**
(0.00017)
[0.0012]

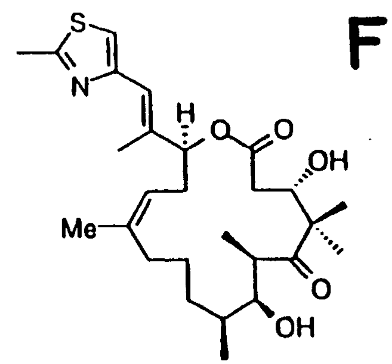

desoxyepothilone B

**10**
(0.0095)
[0.017]

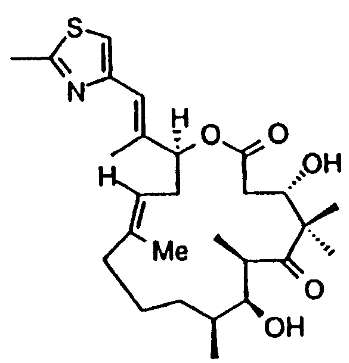

**11**
(0.090)
[0.262]

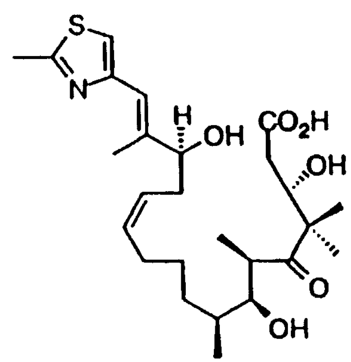

**12**
(0.79)
[>5]

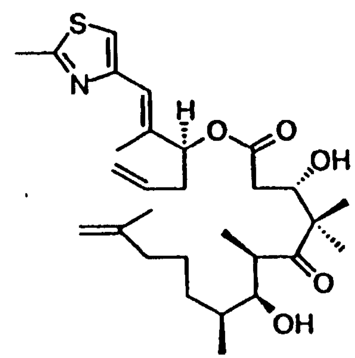

**13**
(11.53)
[5.63]

# FIG. 40B

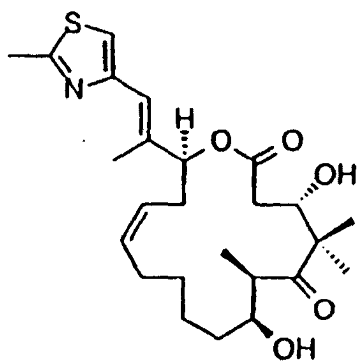

**14**
(5.42)
[5.75]

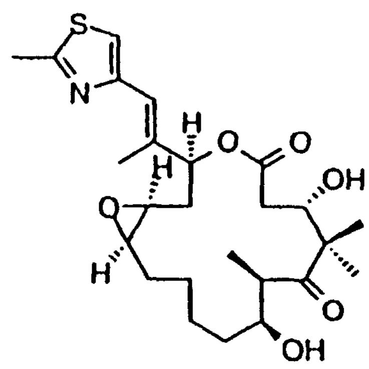

**15**
(0.96)
[5.95]

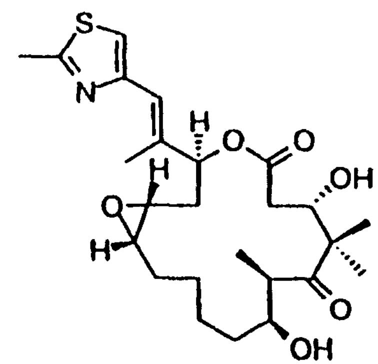

**16**
(7.47)
[16.48]

# FIG. 41A

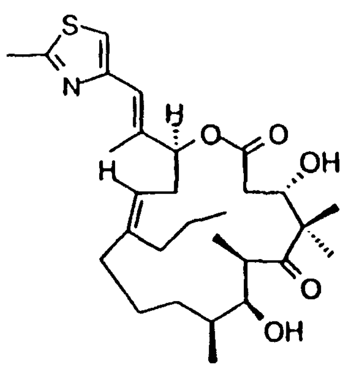

**17**
(0.090)
[0.254]

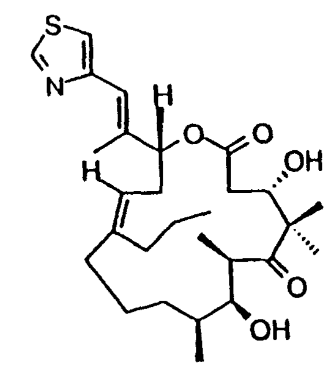

**18**
(1158)
[>720]

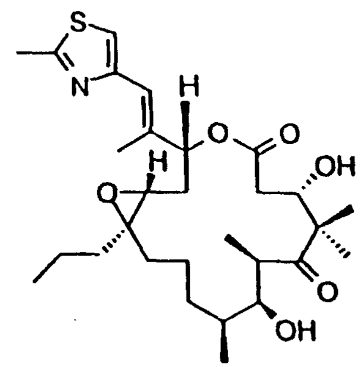

**19**
(0.96)
[>1.0]

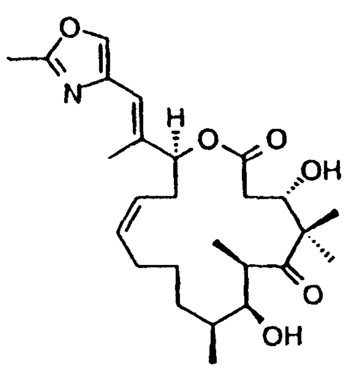

**20**
(0.030)
[0.049]

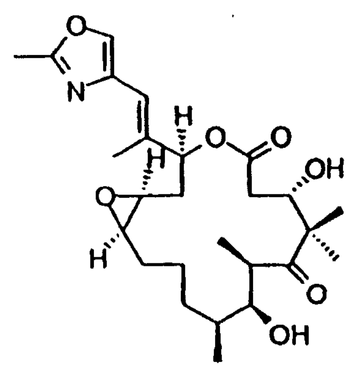

**21**

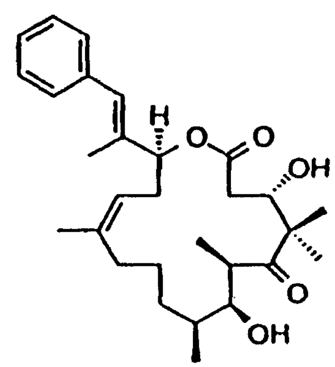

**22**
(0.098)
[0.146]

FIG. 41B

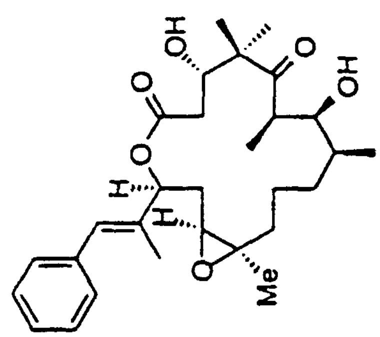

23

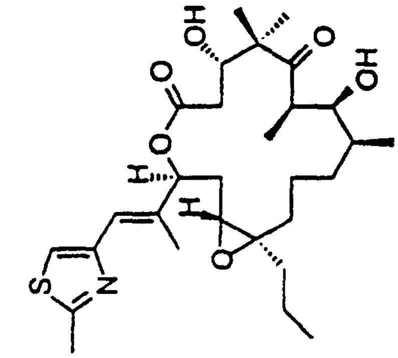

24
(0.0043)
[0.032]

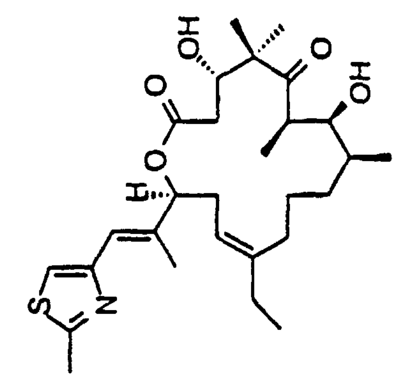

25
(0.021)
[0.077]

# FIG. 42A

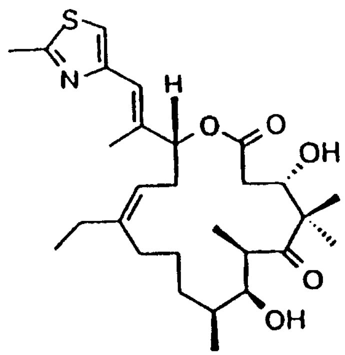

**26**
(0.055)
[0.197]

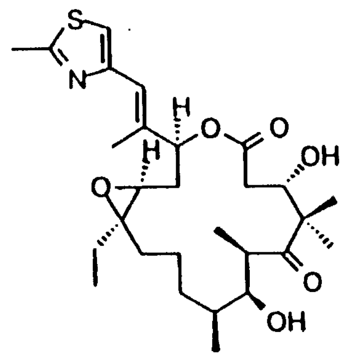

**27**
(0.0010)
[0.0072]

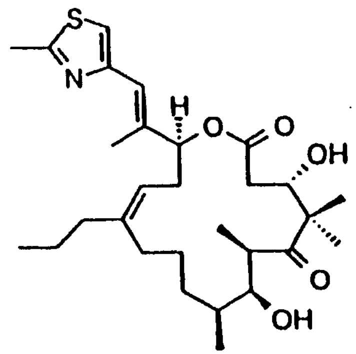

**28**
(0.039)
[0.067]

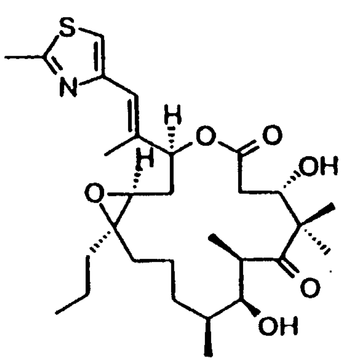

**29**
(0.0038)
[0.0064]

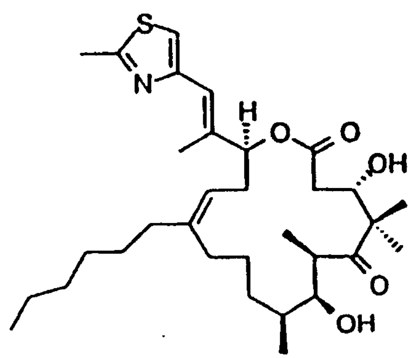

**30**
(0.044)
[0.108]

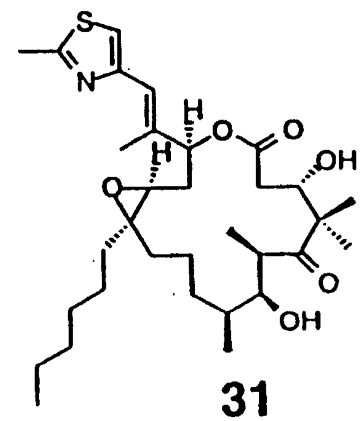

**31**
(0.027)
[0.049]

# FIG. 42B

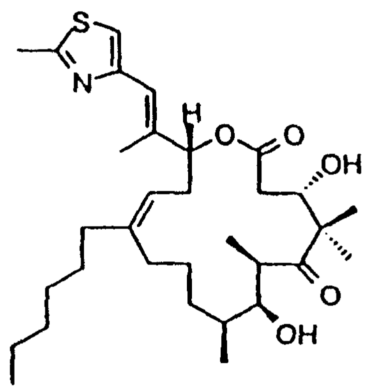

**32**
(0.063)
[0.380]

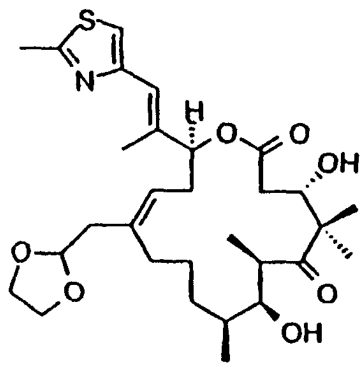

**33**
(0.0031)
[0.0093]

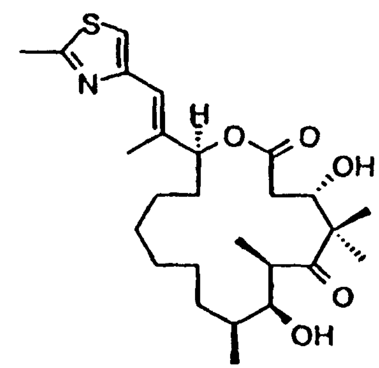

**34**
(0.143)
[0.276]

# FIG. 42C

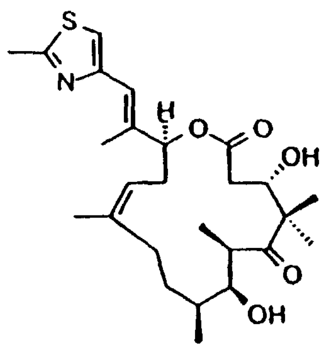

**35**
(>10)
[8.95]

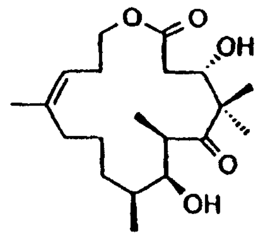

**36**
(234.5)
[>10]

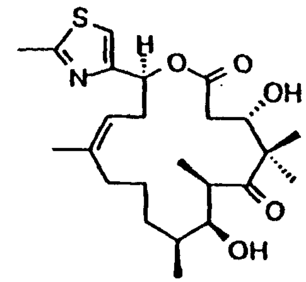

**37**
(3.25)
[1.20]

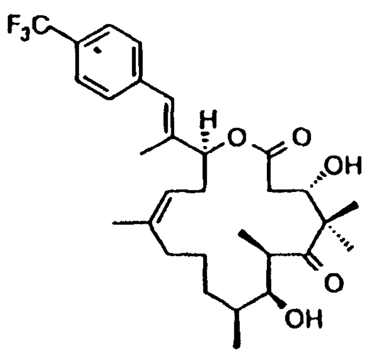

**38**
(0.254)
[>5.0]

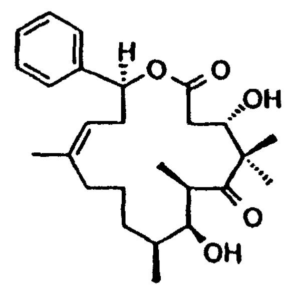

**39**
(1.80)
[>5.0]

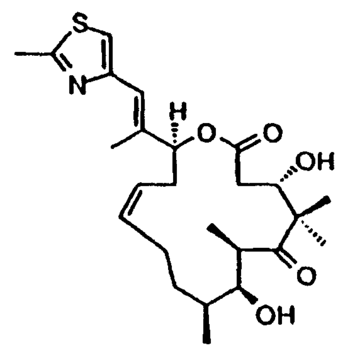

**40**
(36.9)
[47.3]

# FIG. 42D

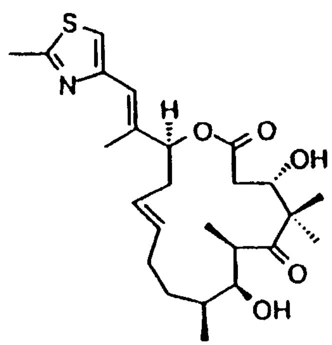

41
(60.1)
[59.2]

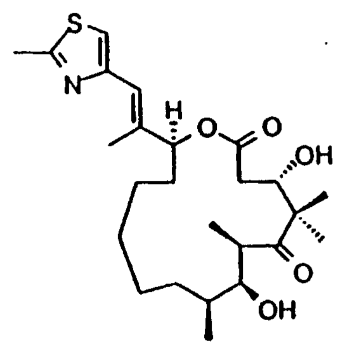

42
(7.41)
[12.9]

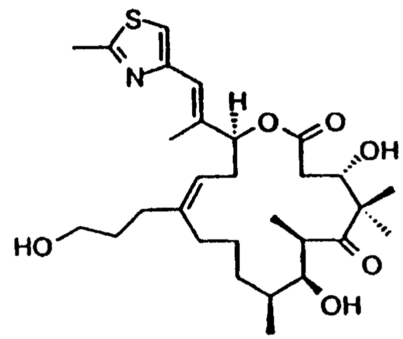

43
(0.0095)
[0.167]

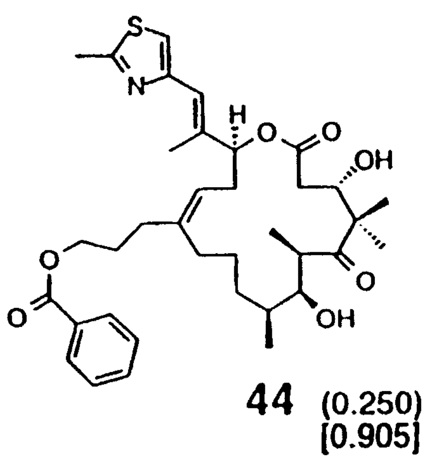

44 (0.250)
[0.905]

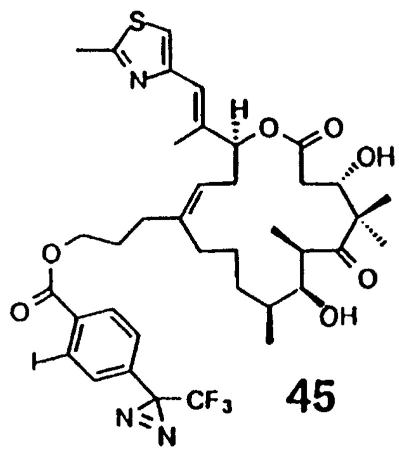

45

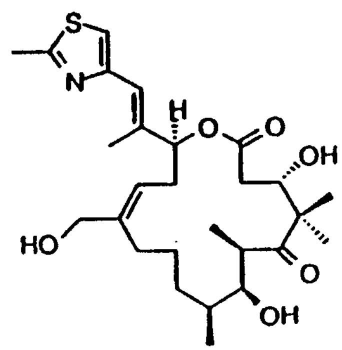

46 (0.049)
[>1.0]

# FIG. 42E

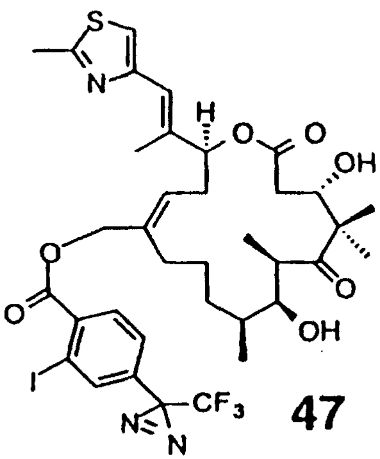

47

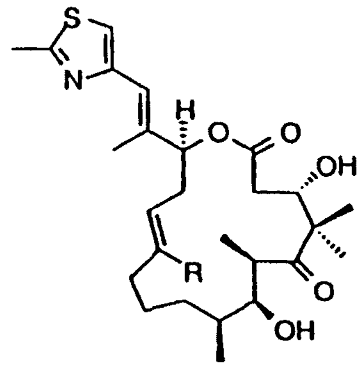

48 R = $(CH_2)_3OH$

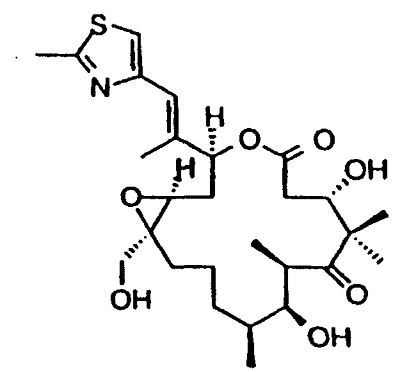

49

FIG. 43A

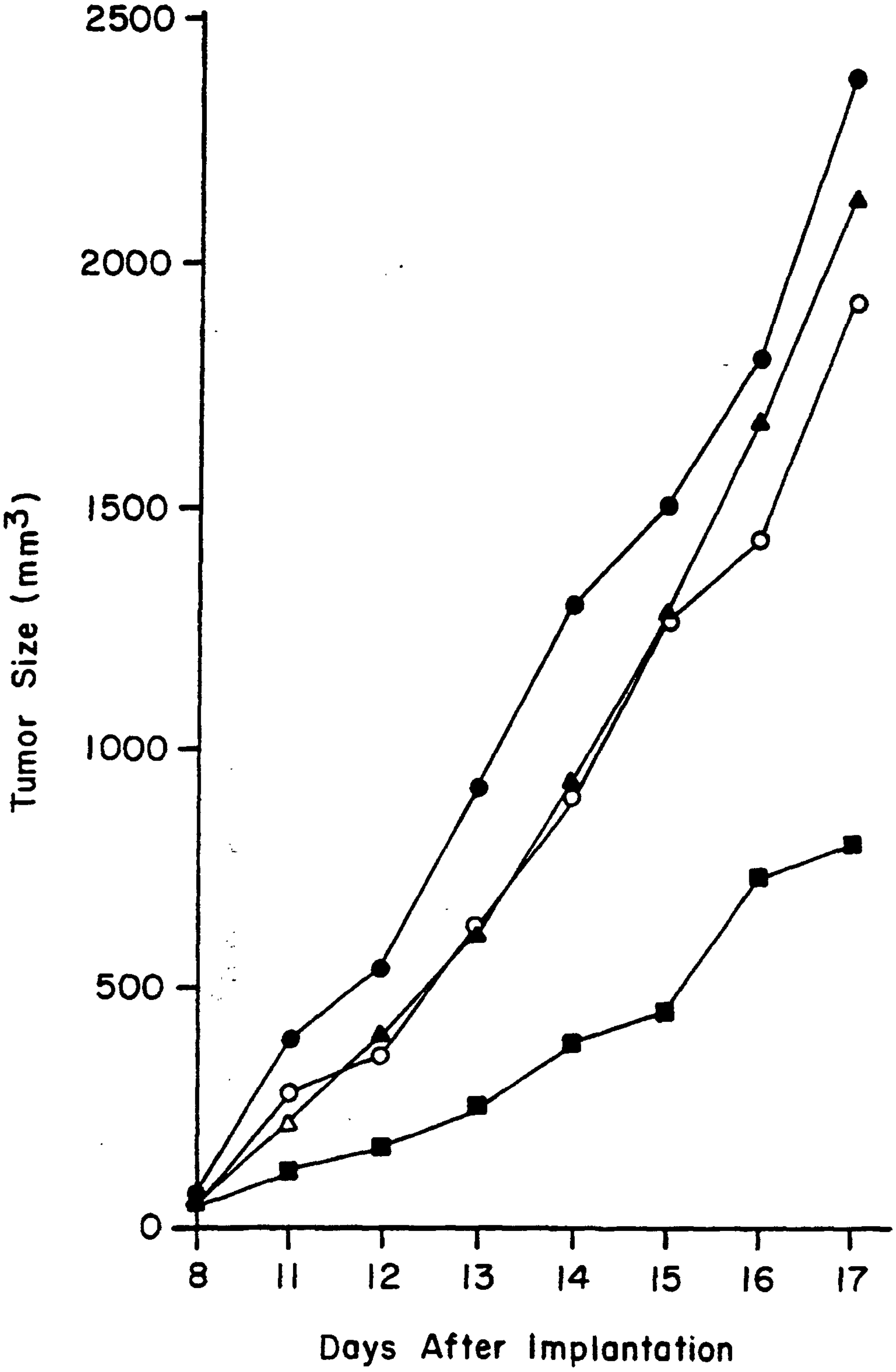
2500
2000
1500
1000
500
0
Tumor Size (mm3)
8
11
12
13
14
15
16
17
Days After Implantation

FIG. 43B

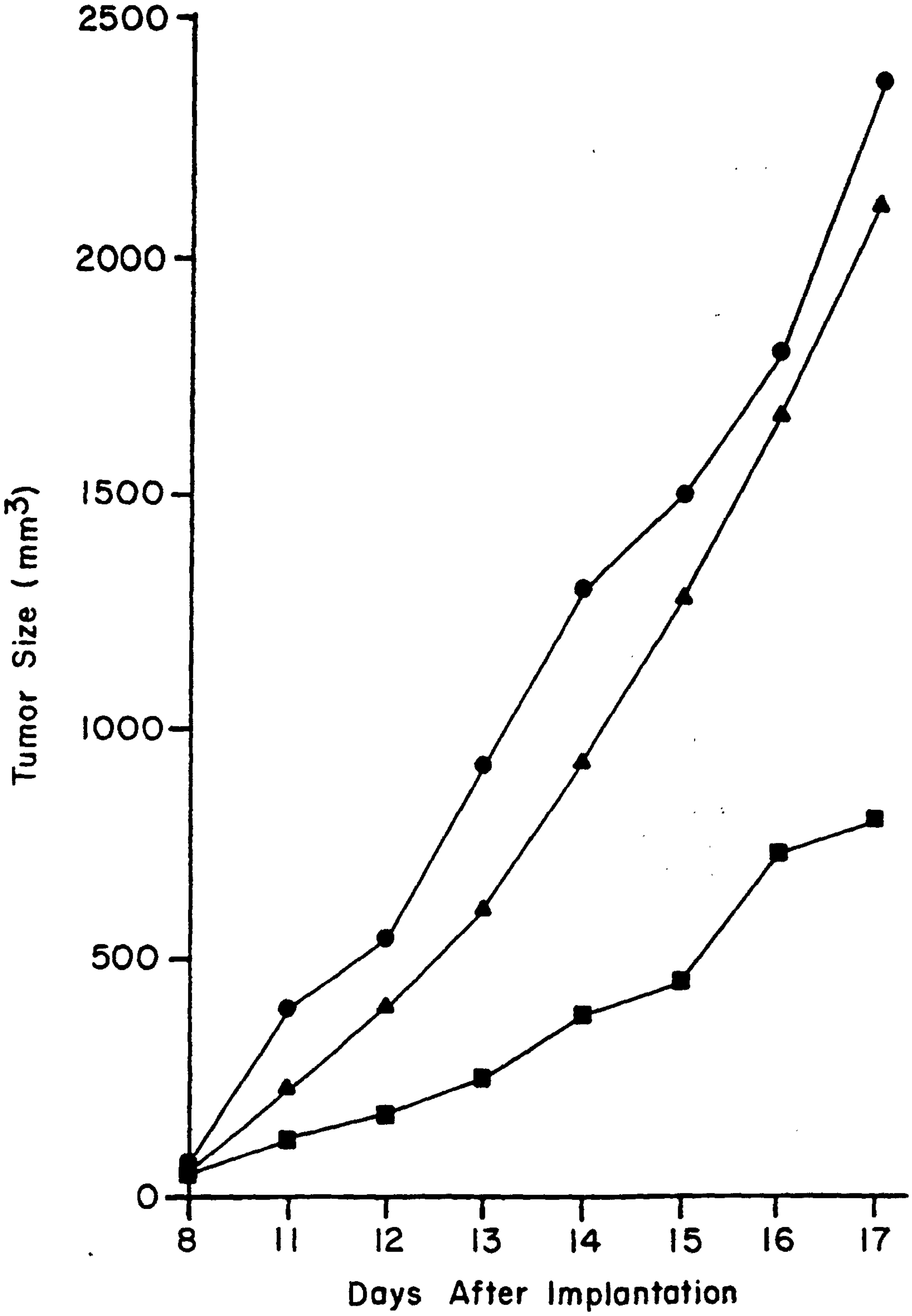
2500
2000
1500
1000
500
0
Tumor Size (mm3)
8
11
12
13
14
15
16
17
Days After Implantation

FIG. 44A

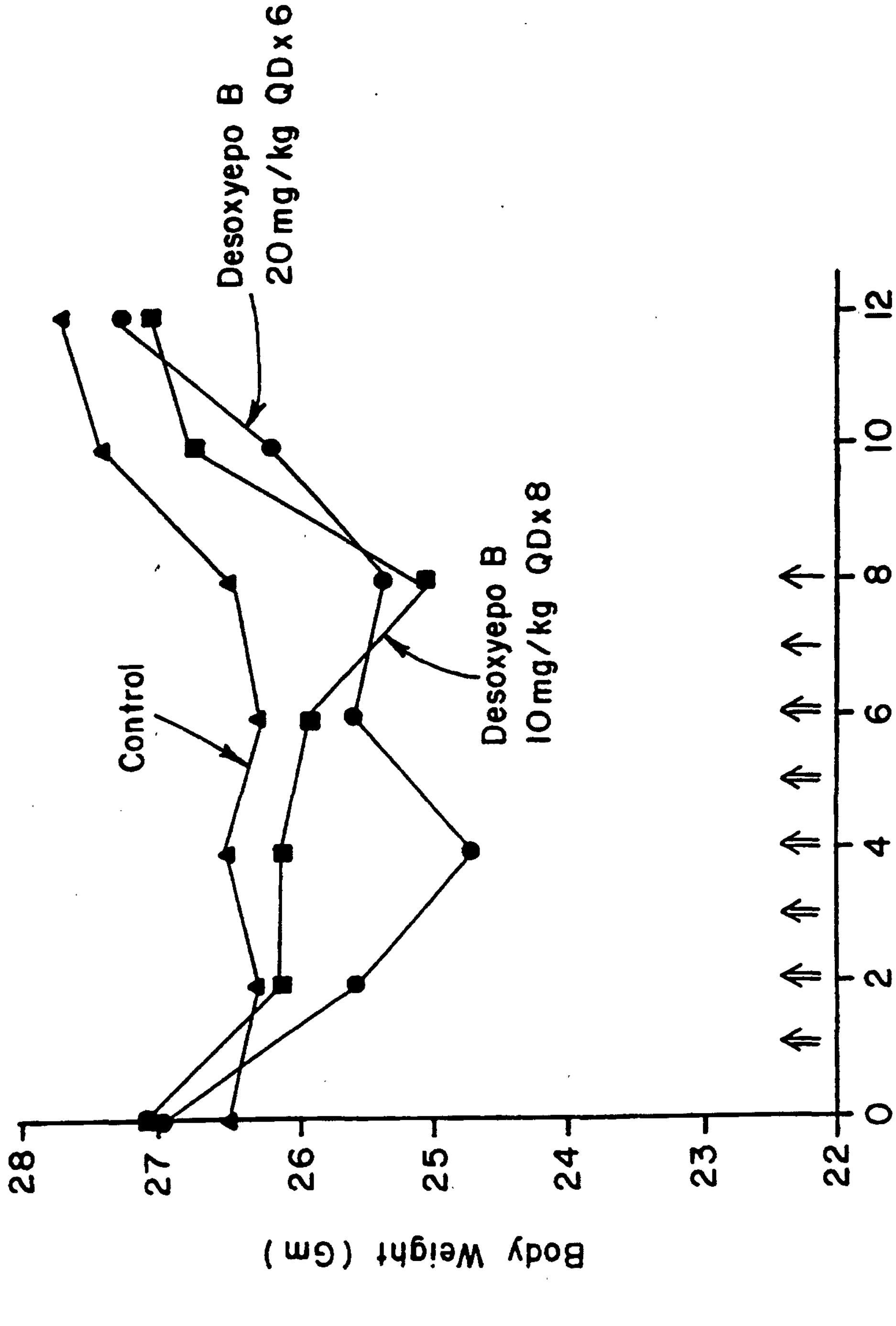
Control
Desoxyepo B
20mg/kg QDx6
Desoxyepo B
10mg/kg QDx8
Body Weight (Gm)
28
27
26
25
24
23
22
0
2
4
6
8
10
12
Day

FIG. 44B

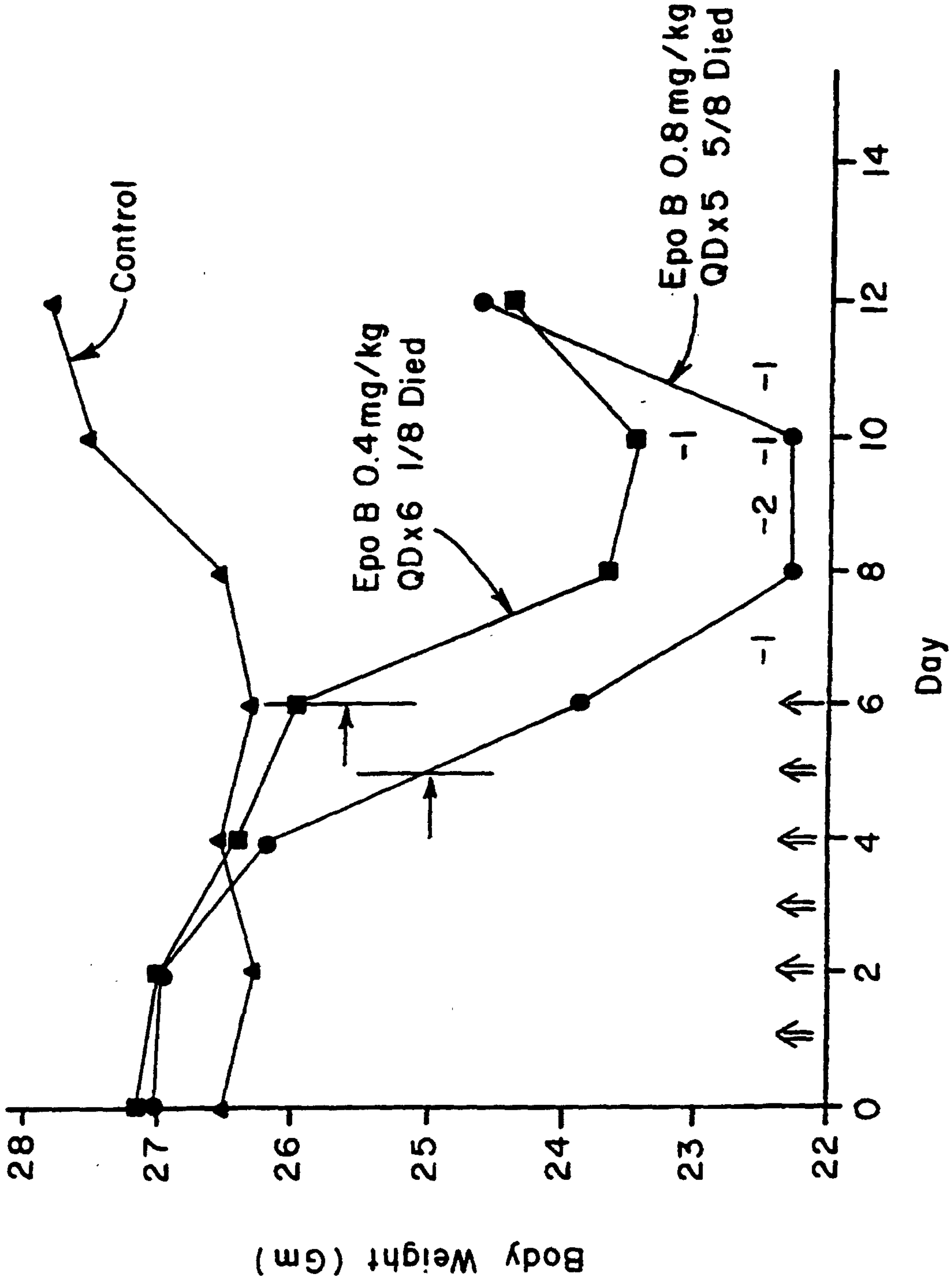
Control
Epo B 0.4 mg/kg
QDx6 1/8 Died
Epo B 0.8 mg/kg
QDx5 5/8 Died
-1
-2
-1
-1
-1
Body Weight (Gm)
28
27
26
25
24
23
22
0
2
4
6
8
10
12
14
Day

FIG. 45A

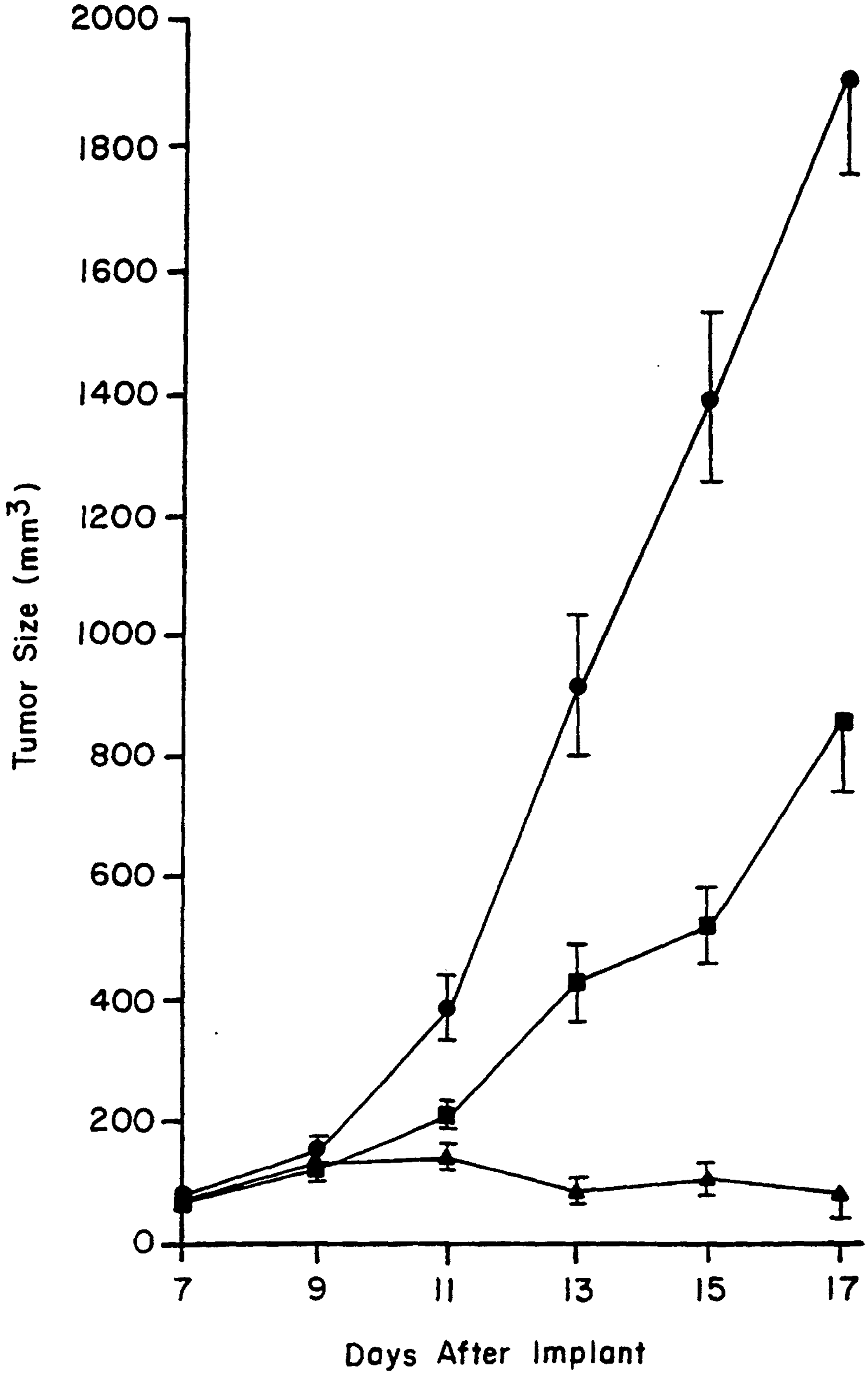
2000
1800
1600
1400
1200
1000
800
600
400
200
0
Tumor Size (mm3)
7
9
11
13
15
17
Days After Implant

FIG. 45B

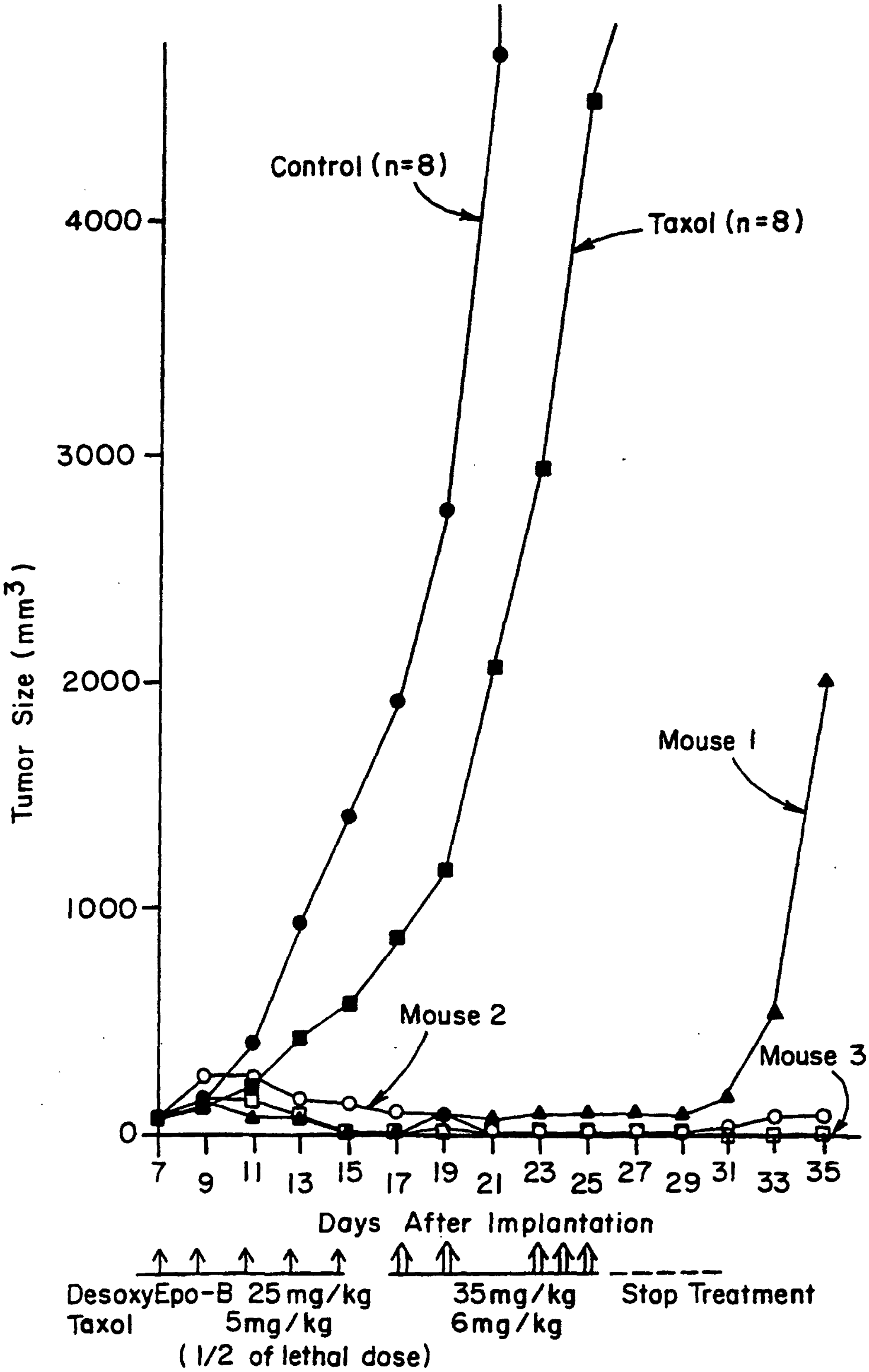
Control (n=8)
Taxol (n=8)
Mouse 1
Mouse 2
Mouse 3
Tumor Size (mm3)
0
1000
2000
3000
4000
7 9 11 13 15 17 19 21 23 25 27 29 31 33 35
Days After Implantation
DesoxyEpo-B 25 mg/kg
Taxol 5mg/kg
(1/2 of lethal dose)
35mg/kg
6mg/kg
Stop Treatment

FIG. 46

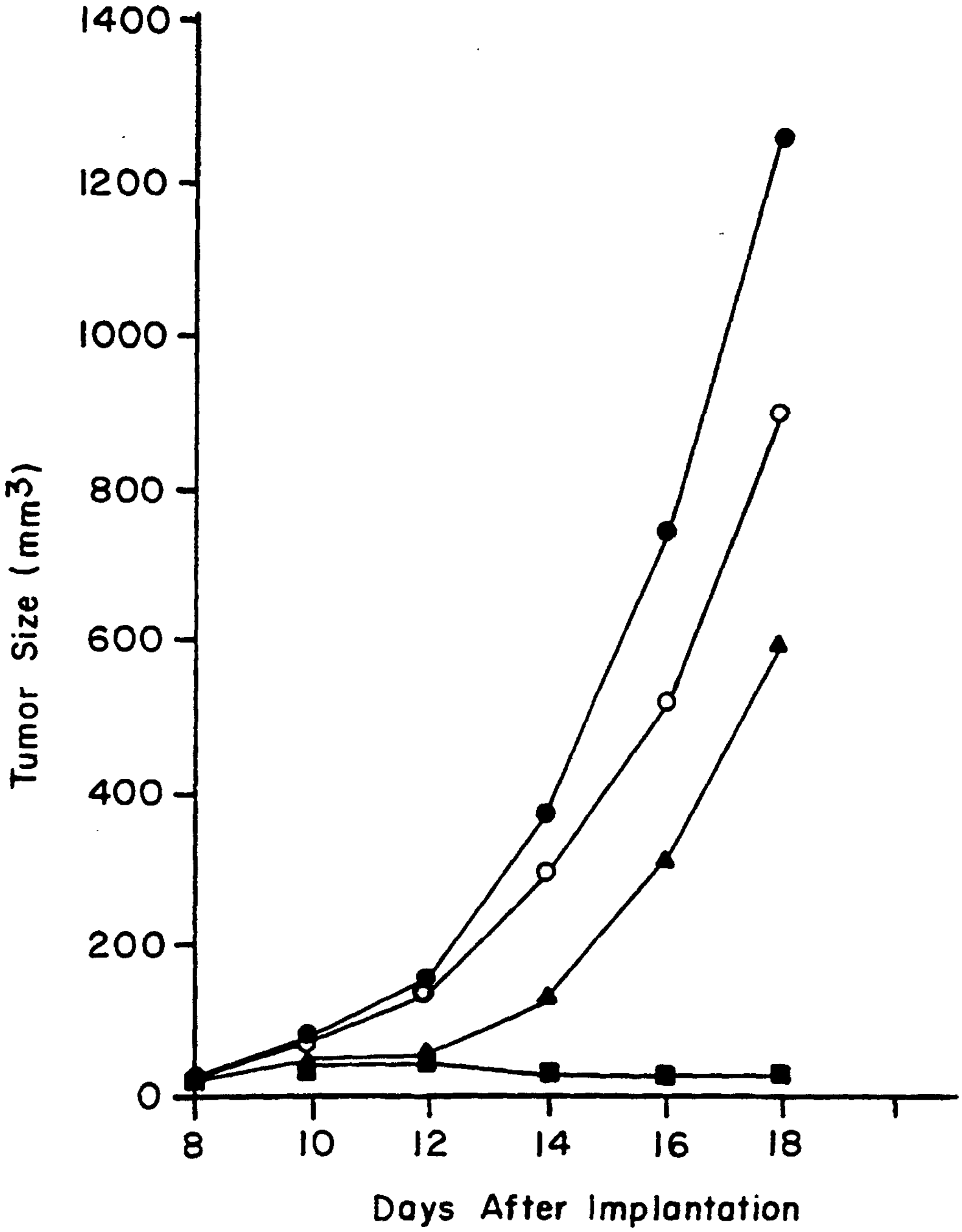
1400
1200
1000
800
600
400
200
0
Tumor Size (mm3)
8
10
12
14
16
18
Days After Implantation

FIG. 47

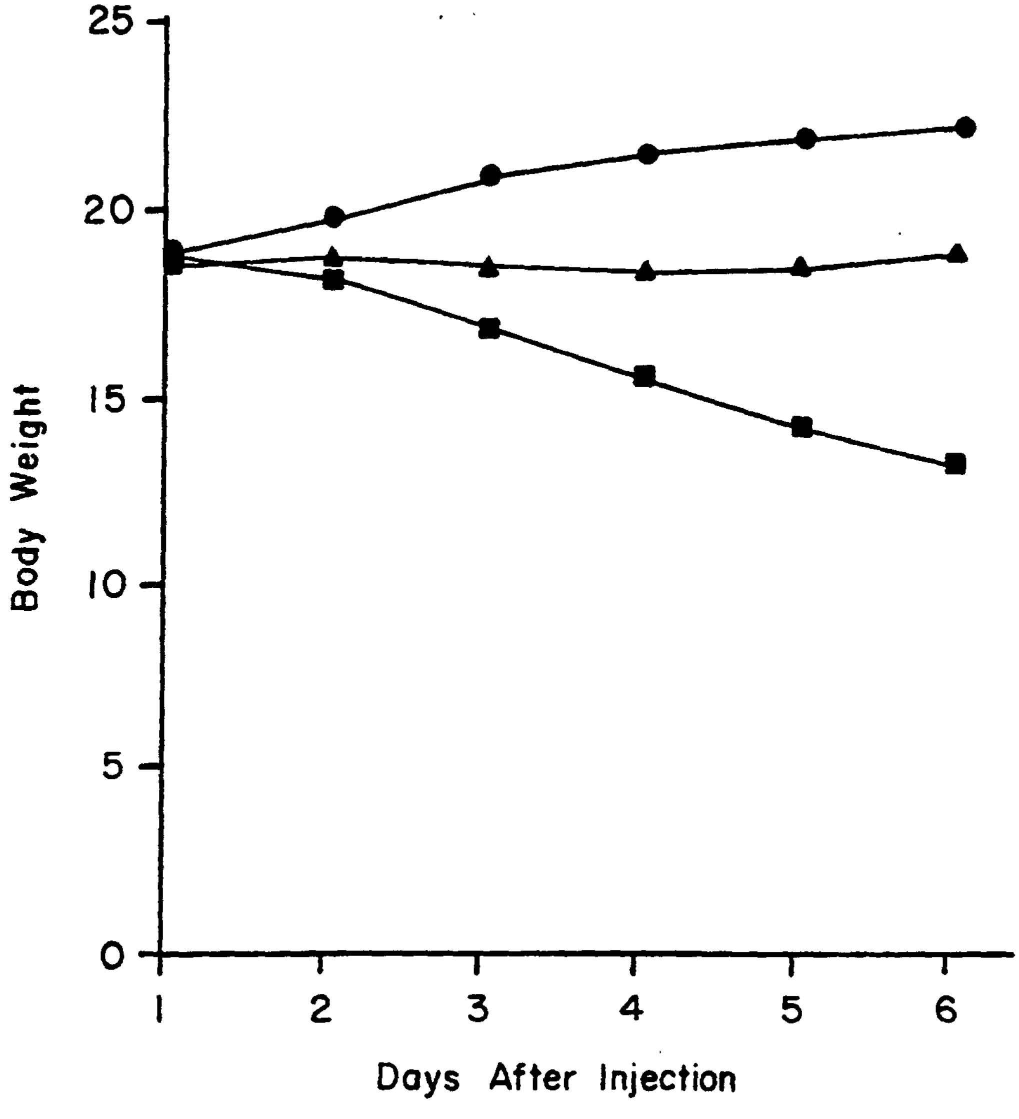

FIG. 48

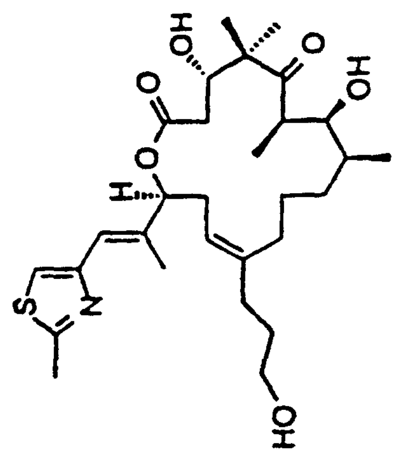

43

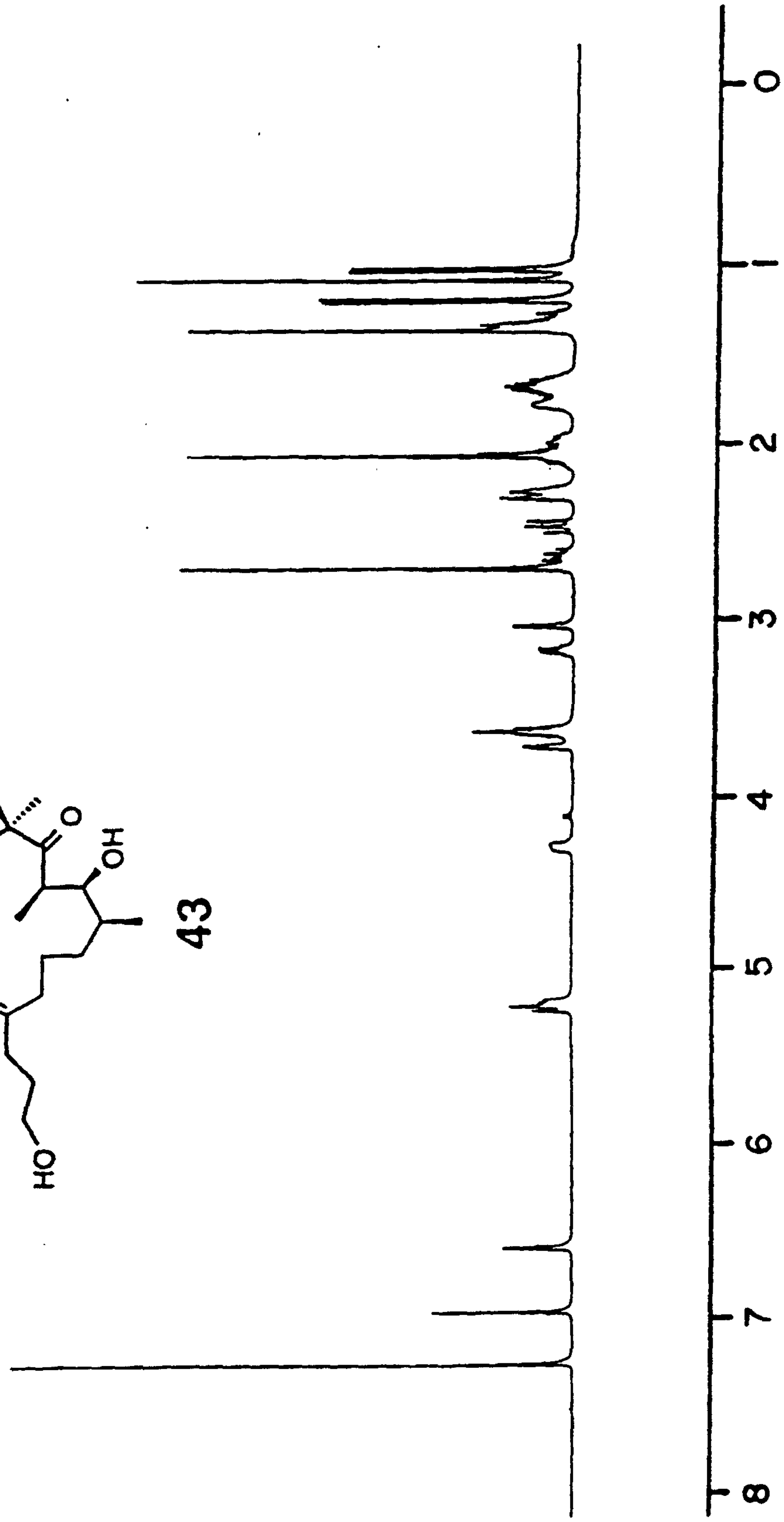

FIG. 49

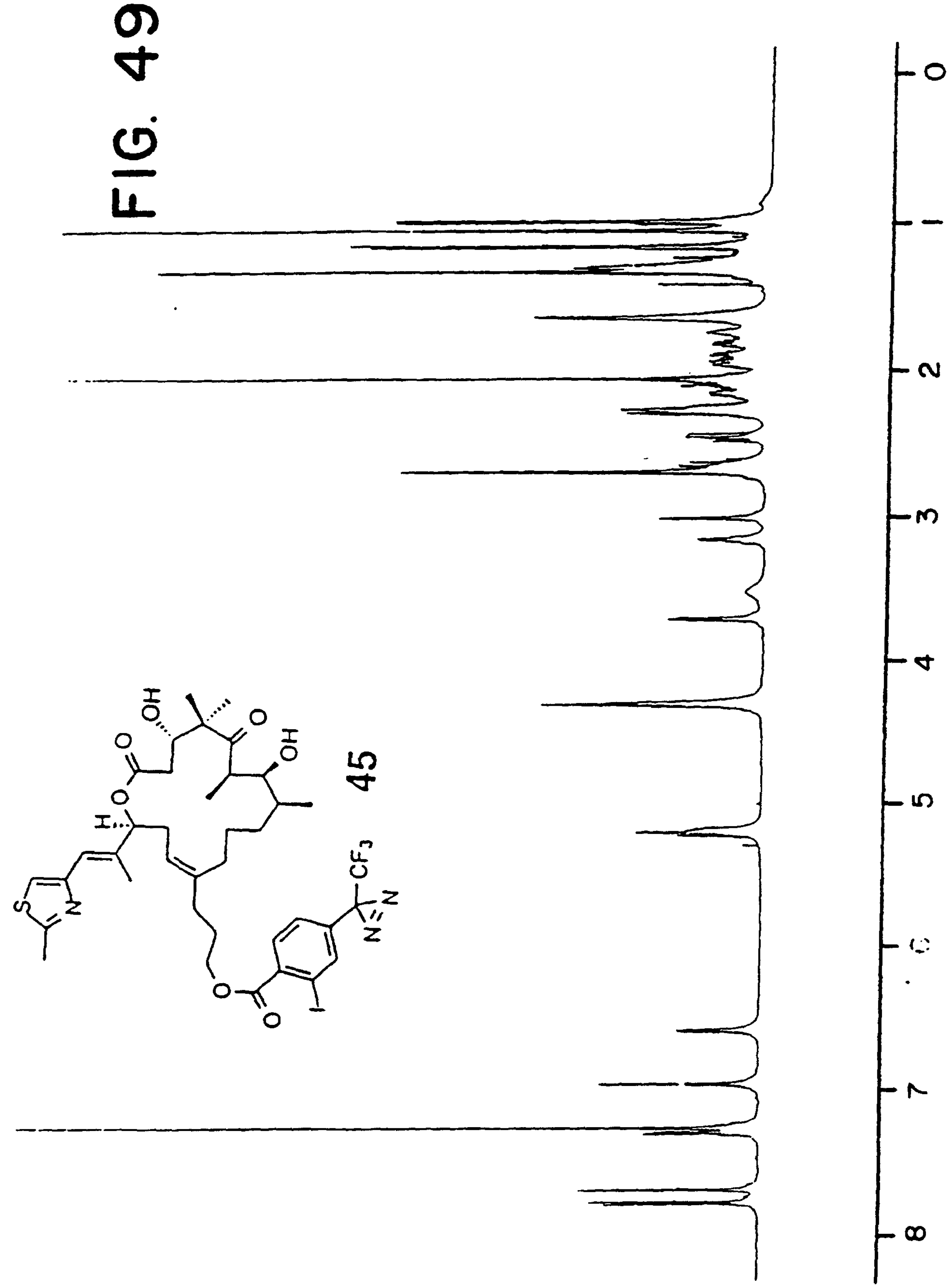


45

FIG. 50

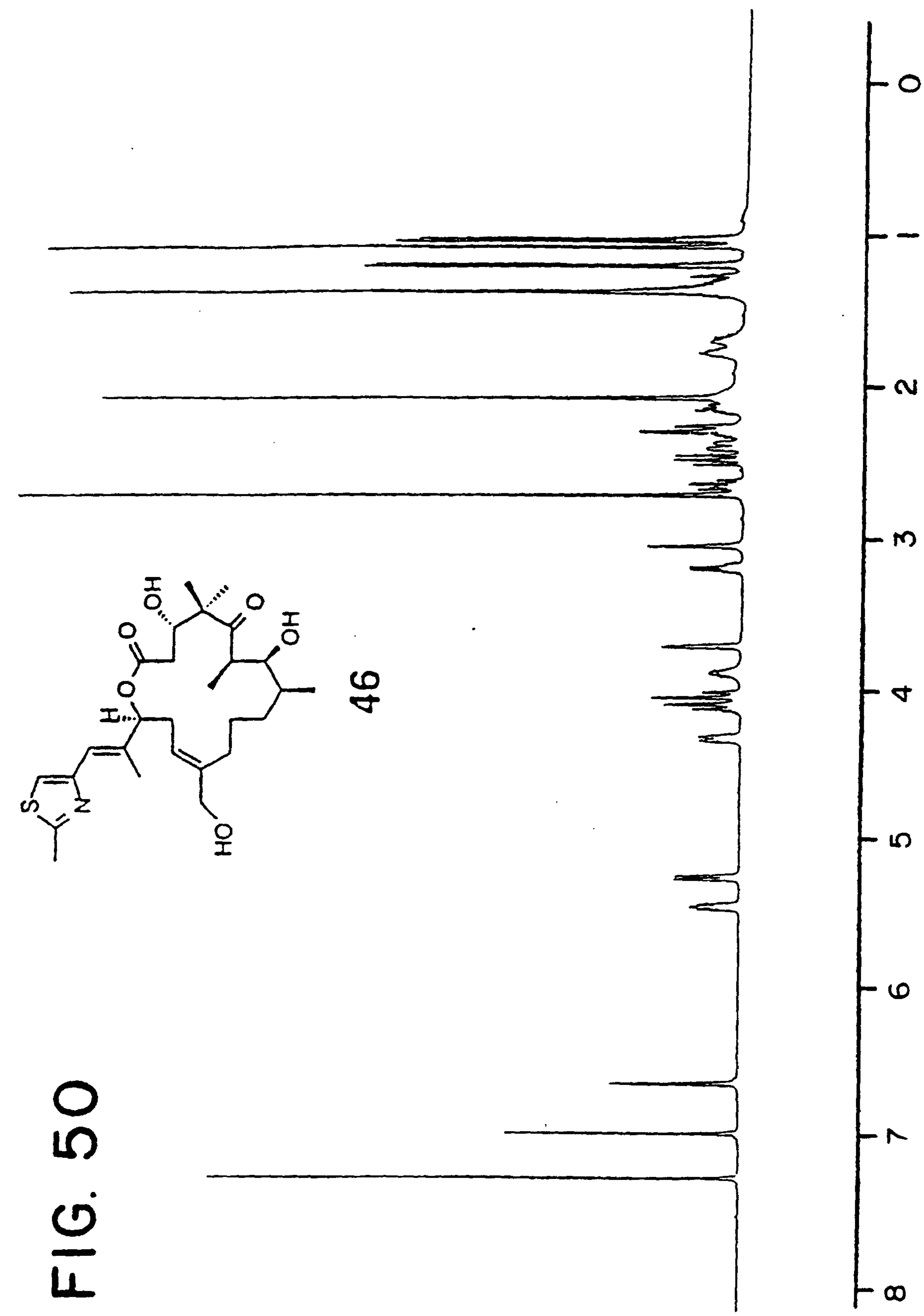

46

FIG. 51

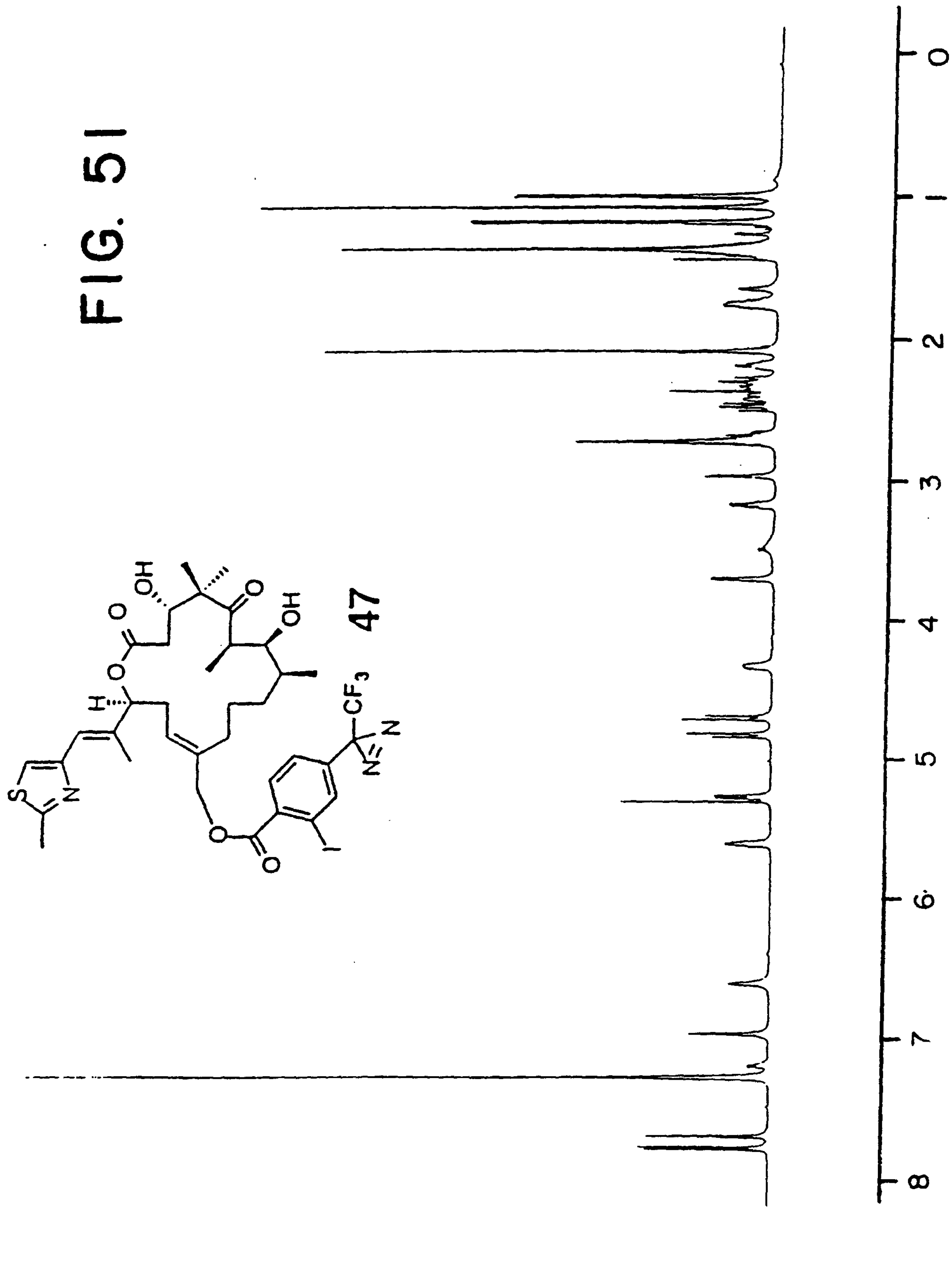


47

# FIG. 52

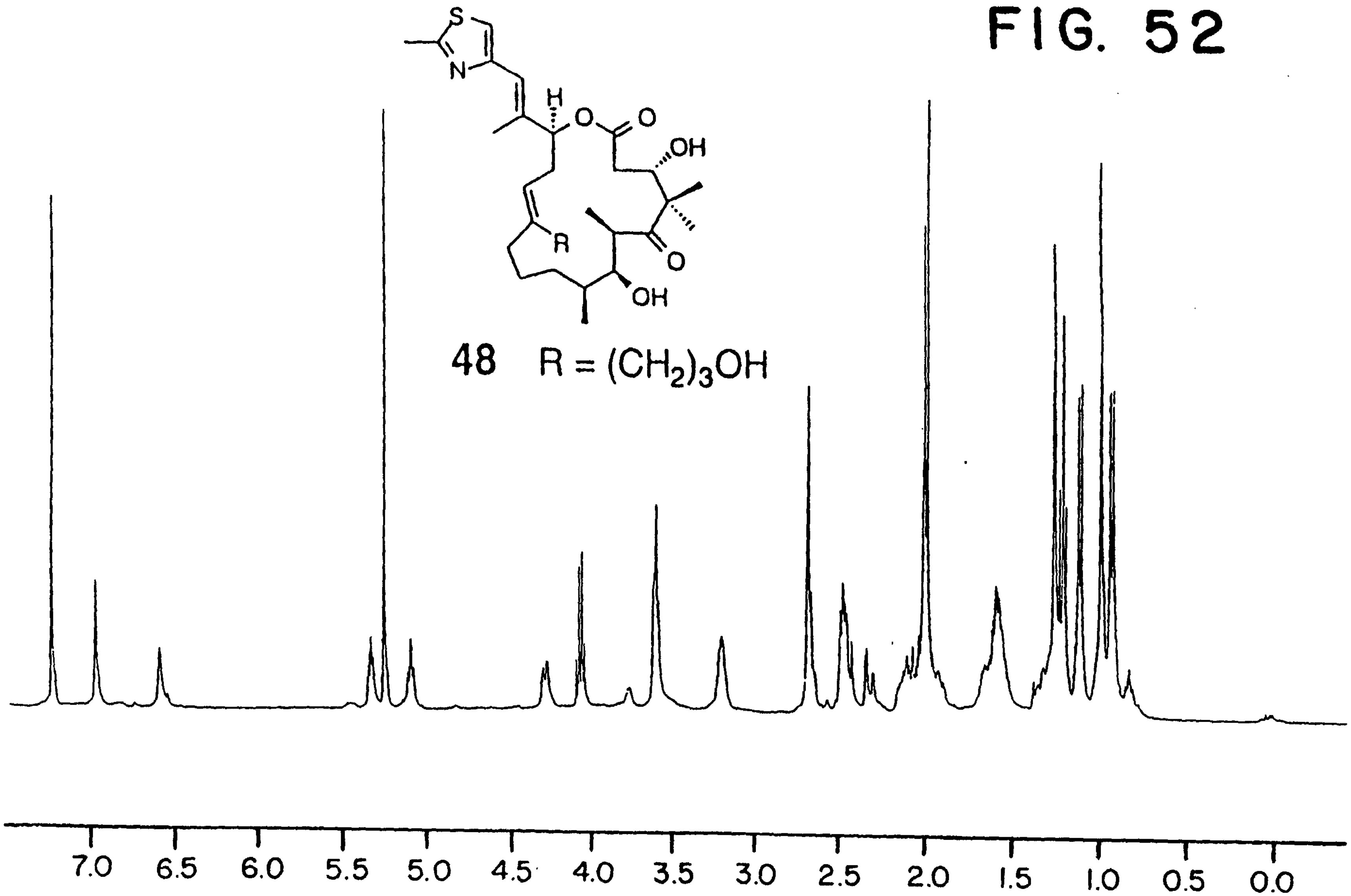


**48** R = $(CH_2)_3OH$

FIG. 53A

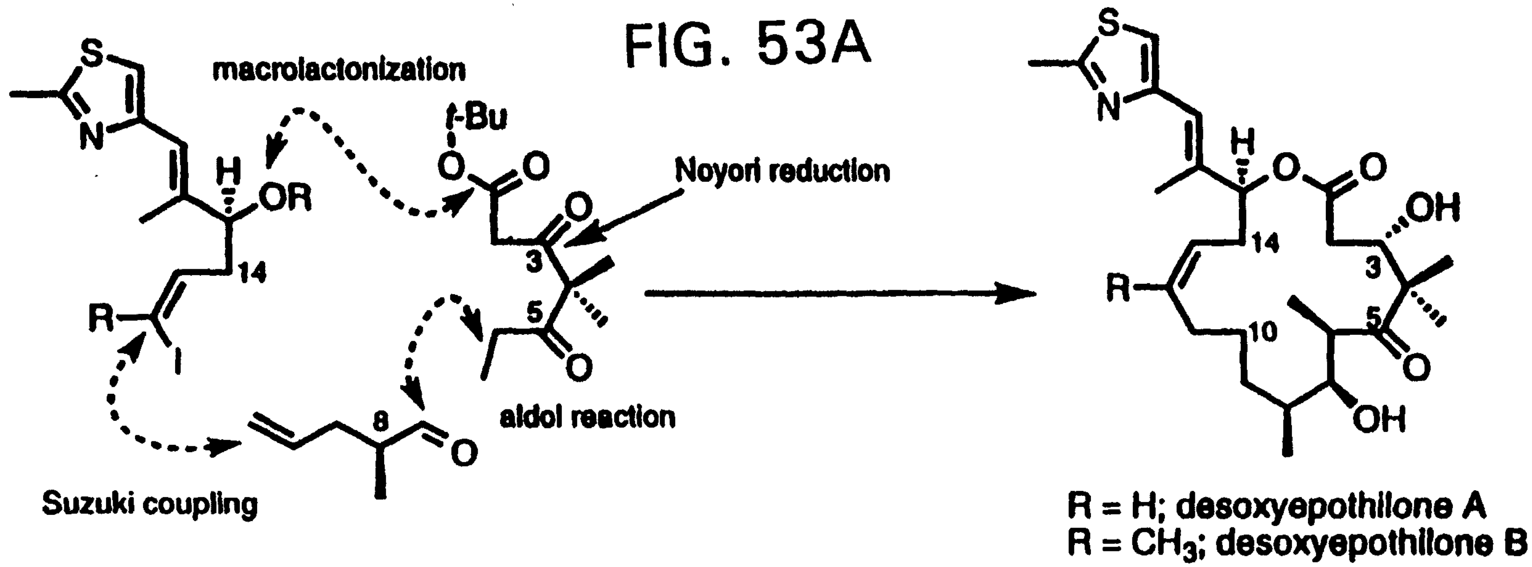


R = H; desoxyepothilone A
R = $CH_3$; desoxyepothilone B

25-steps total

FIG. 53B

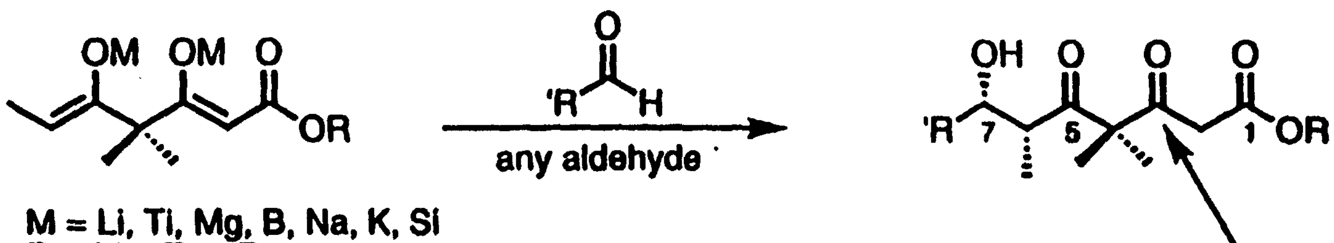


M = Li, Ti, Mg, B, Na, K, Si
R = Me, Et, *t*-Bu etc

Noyori asymmetric reduction of the C-3 carbonyl is another key step

FIG. 54

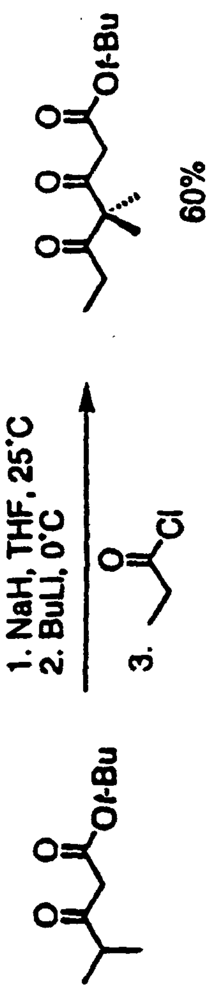

FIG. 55

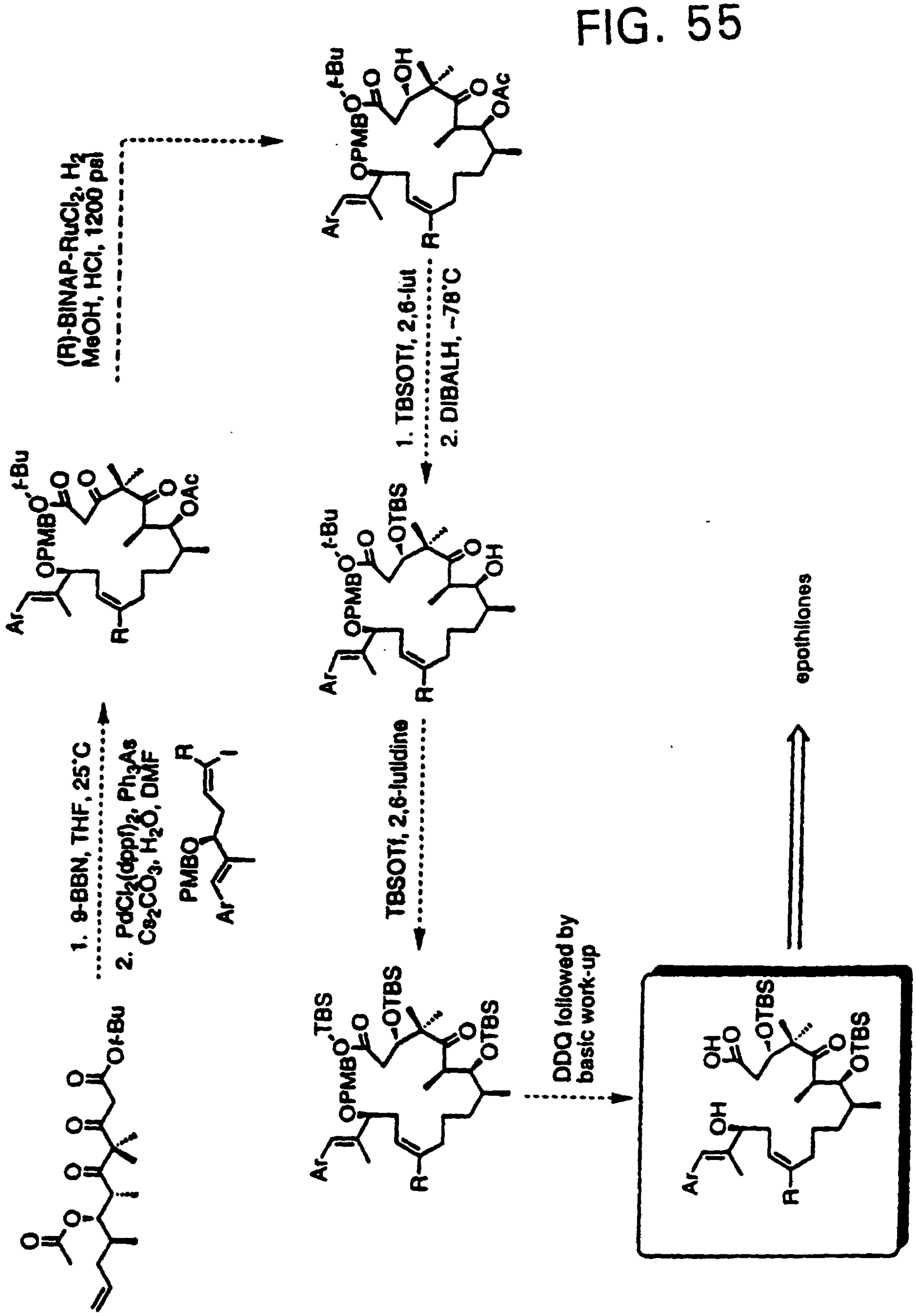

1. 9-BBN, THF, 25°C
2. PdCl$_2$(dppf)$_2$, Ph$_3$As
Cs$_2$CO$_3$, H$_2$O, DMF
(R)-BINAP-RuCl$_2$, H$_2$
MeOH, HCl, 1200 psi
1. TBSOTf, 2,6-lut
2. DIBALH, -78°C
TBSOTf, 2,6-lutidine
DDQ followed by
basic work-up
epothilones

FIG. 56

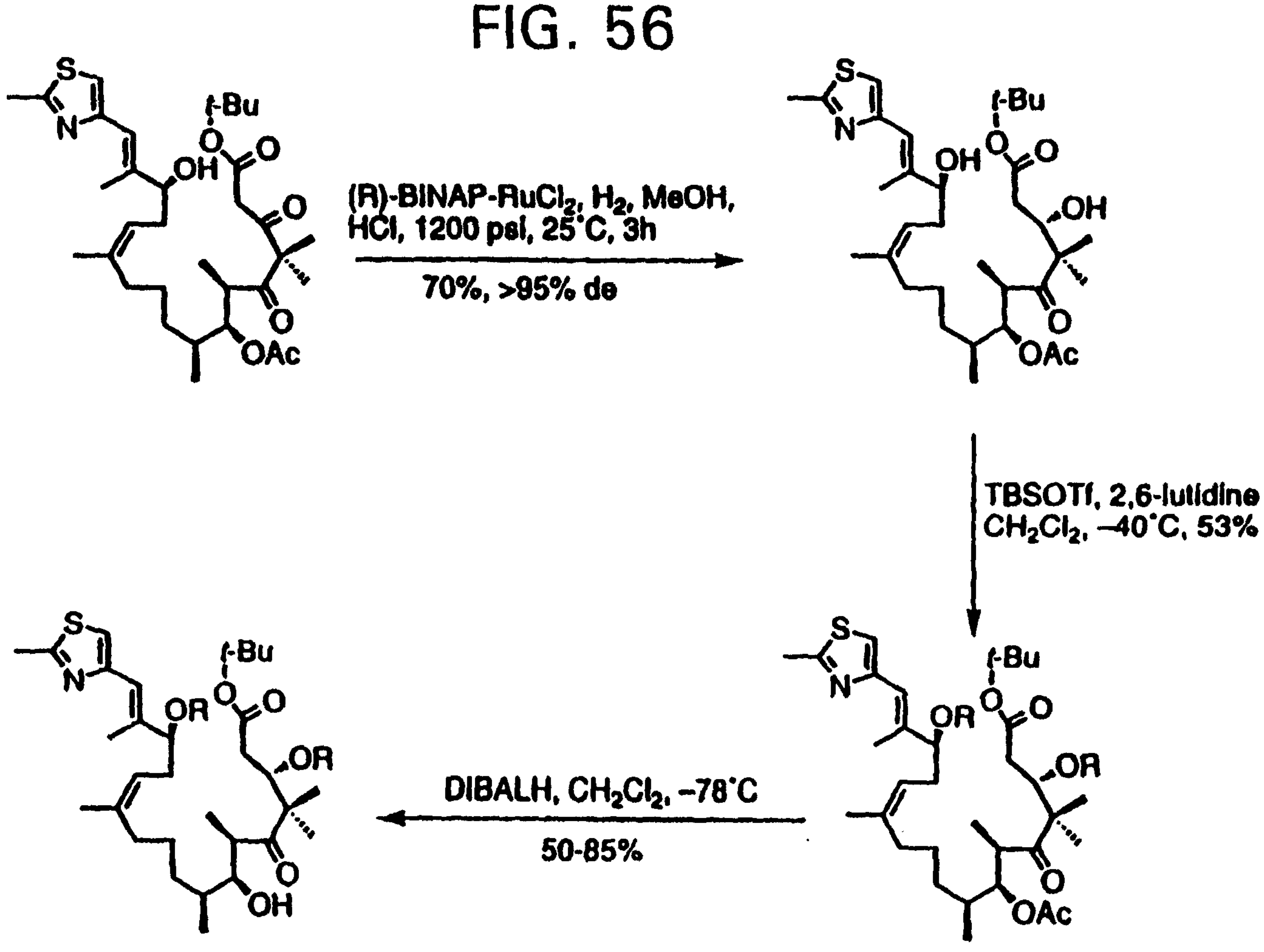


R = TBS

R = TBS

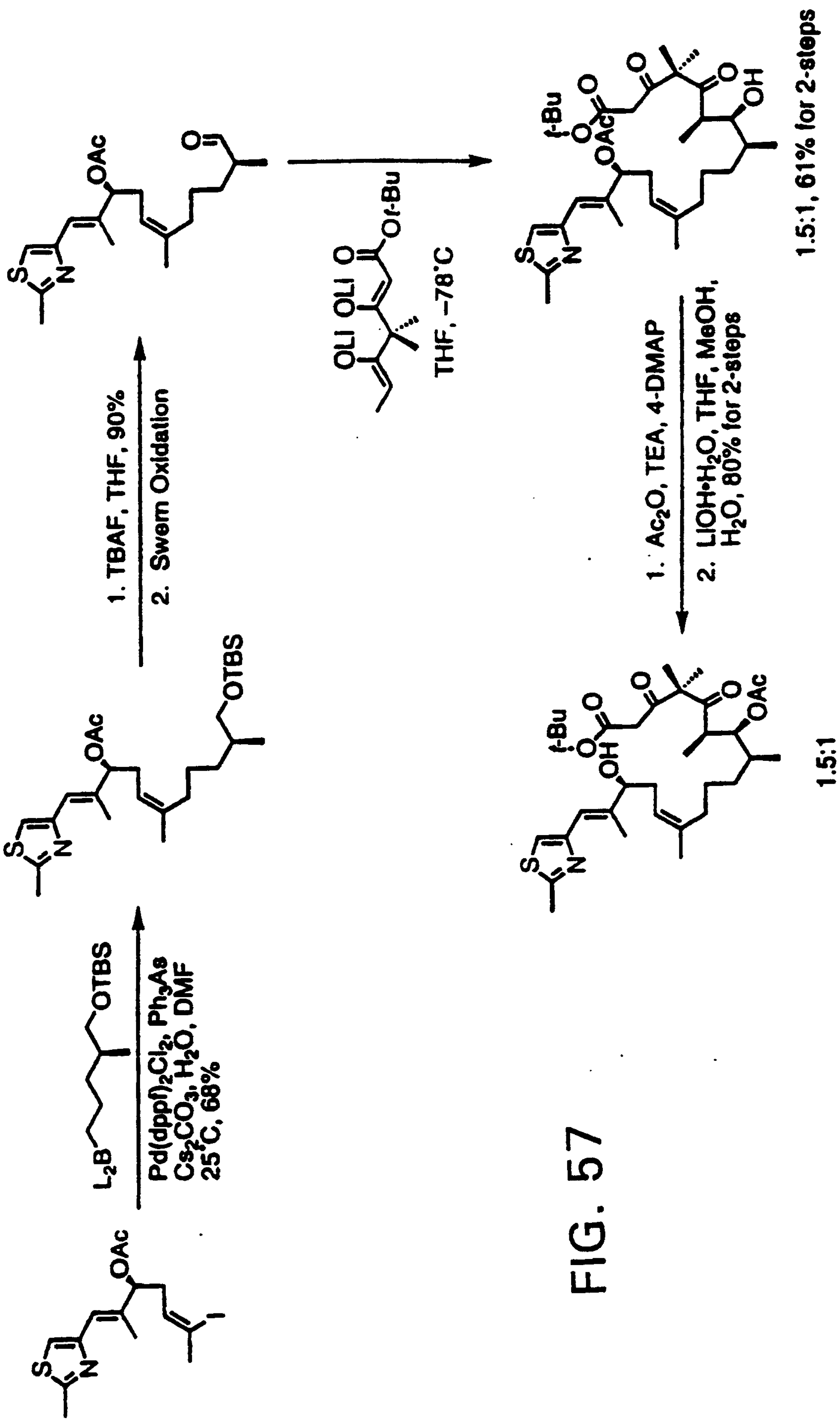
Pd(dppf)$_2$Cl$_2$, Ph$_3$As
Cs$_2$CO$_3$, H$_2$O, DMF
25°C, 68%
1. TBAF, THF, 90%
2. Swern Oxidation
THF, –78°C
1. Ac$_2$O, TEA, 4-DMAP
2. LiOH•H$_2$O, THF, MeOH, H$_2$O, 80% for 2-steps
1.5:1, 61% for 2-steps
1.5:1

FIG. 57

FIG. 58

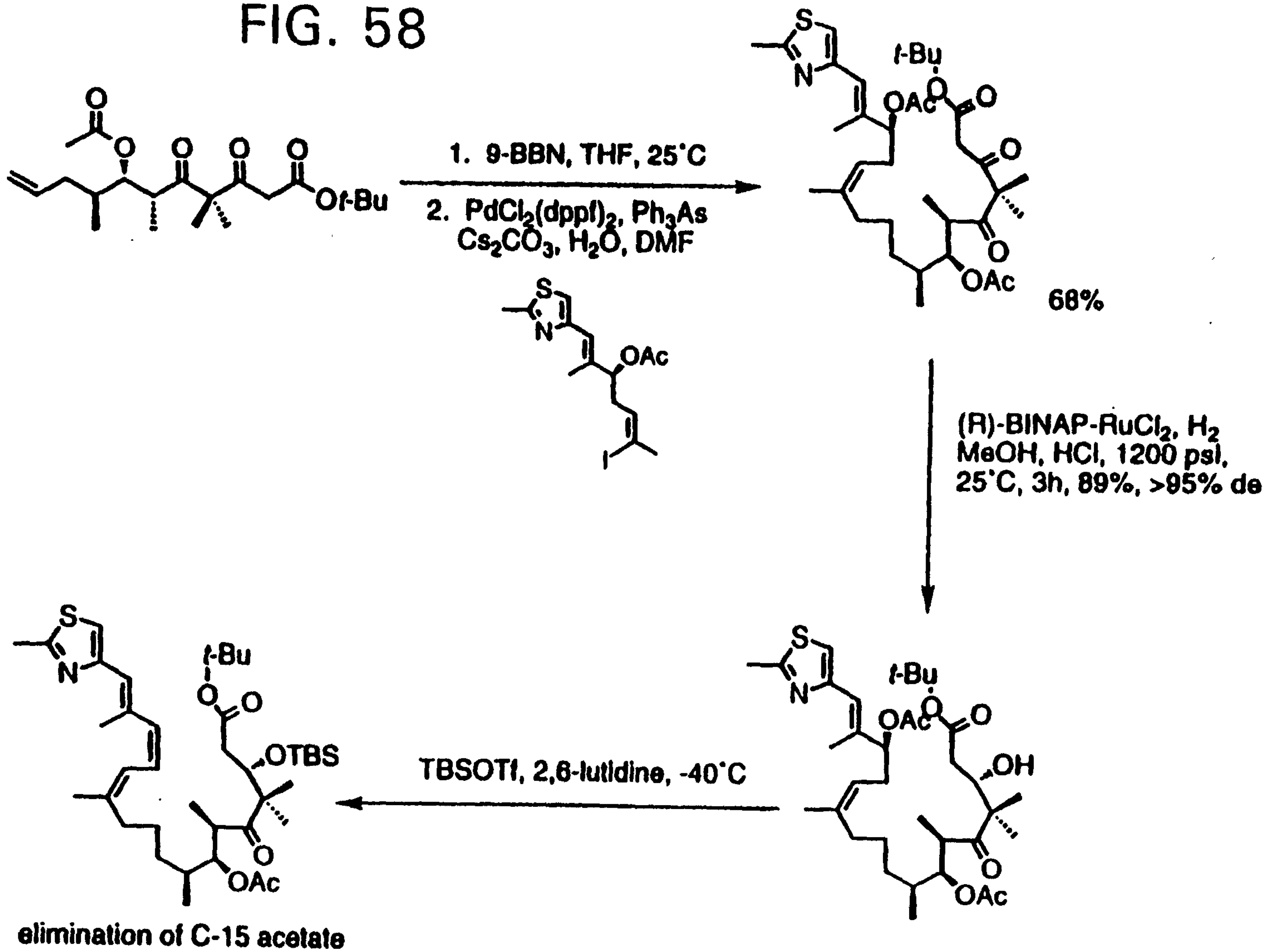


elimination of C-15 acetate

FIG. 59

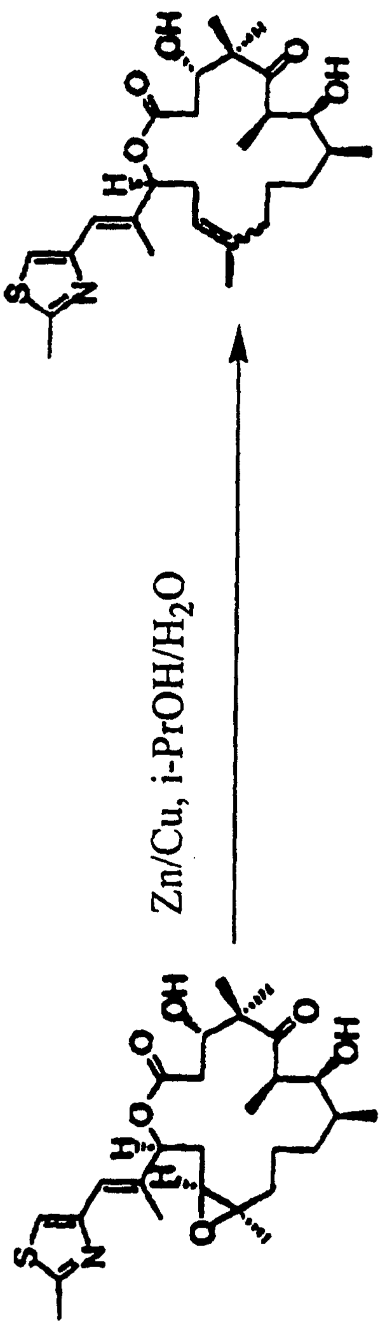

FIG. 60

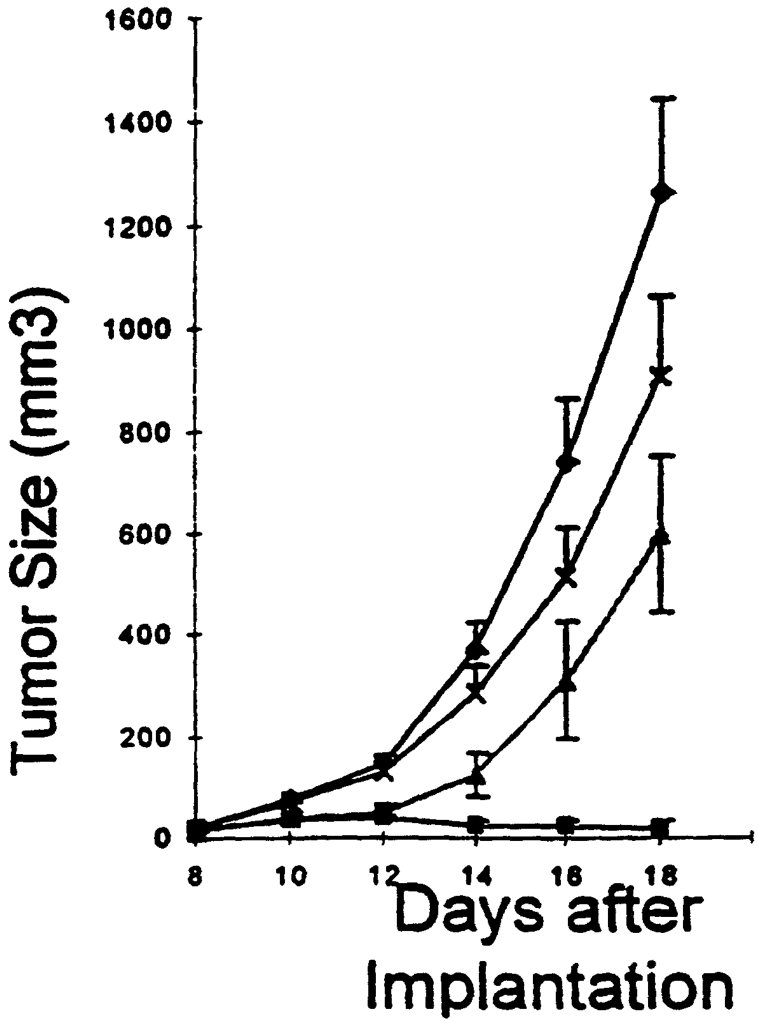
1600
1400
1200
1000
800
600
400
200
0
Tumor Size (mm3)
8
10
12
14
16
18
Days after Implantation

# FIG. 61A

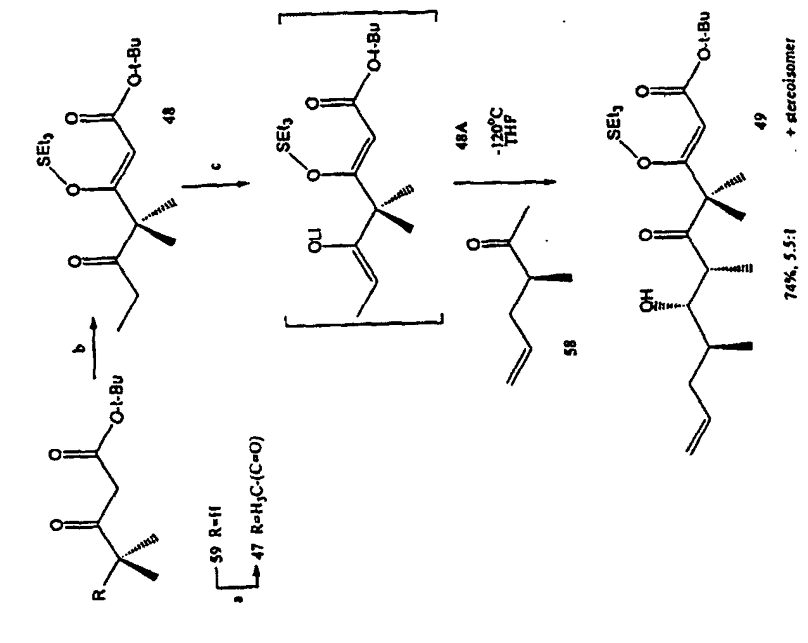
a
59 R=H
47 R=H3C-C(C=O)
b
48
c
48A
-120°C
THF
58
49
74%, 5.5:1
+ stereoisomer

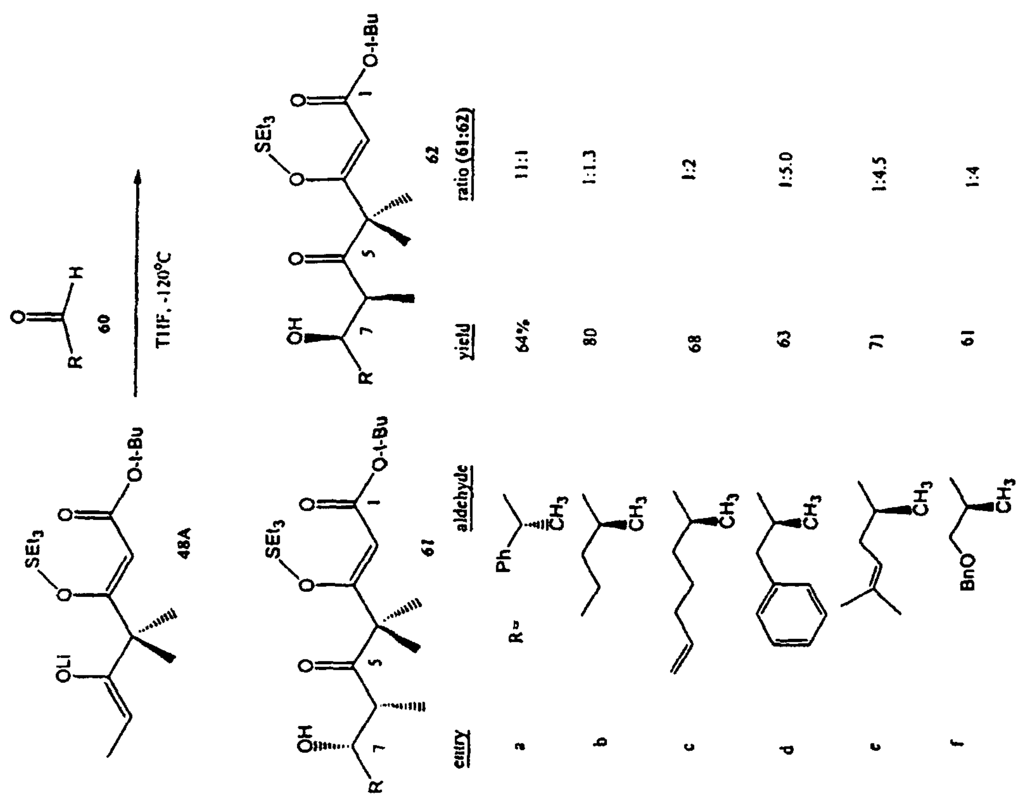
48A
60
THF, -120°C
61
62
entry
aldehyde
yield
ratio (61:62)
a 64% 1:1
b 80 1:1.3
c 68 1:2
d 63 1:5.0
e 71 1:4.5
f 61 1:4

# FIG. 61B

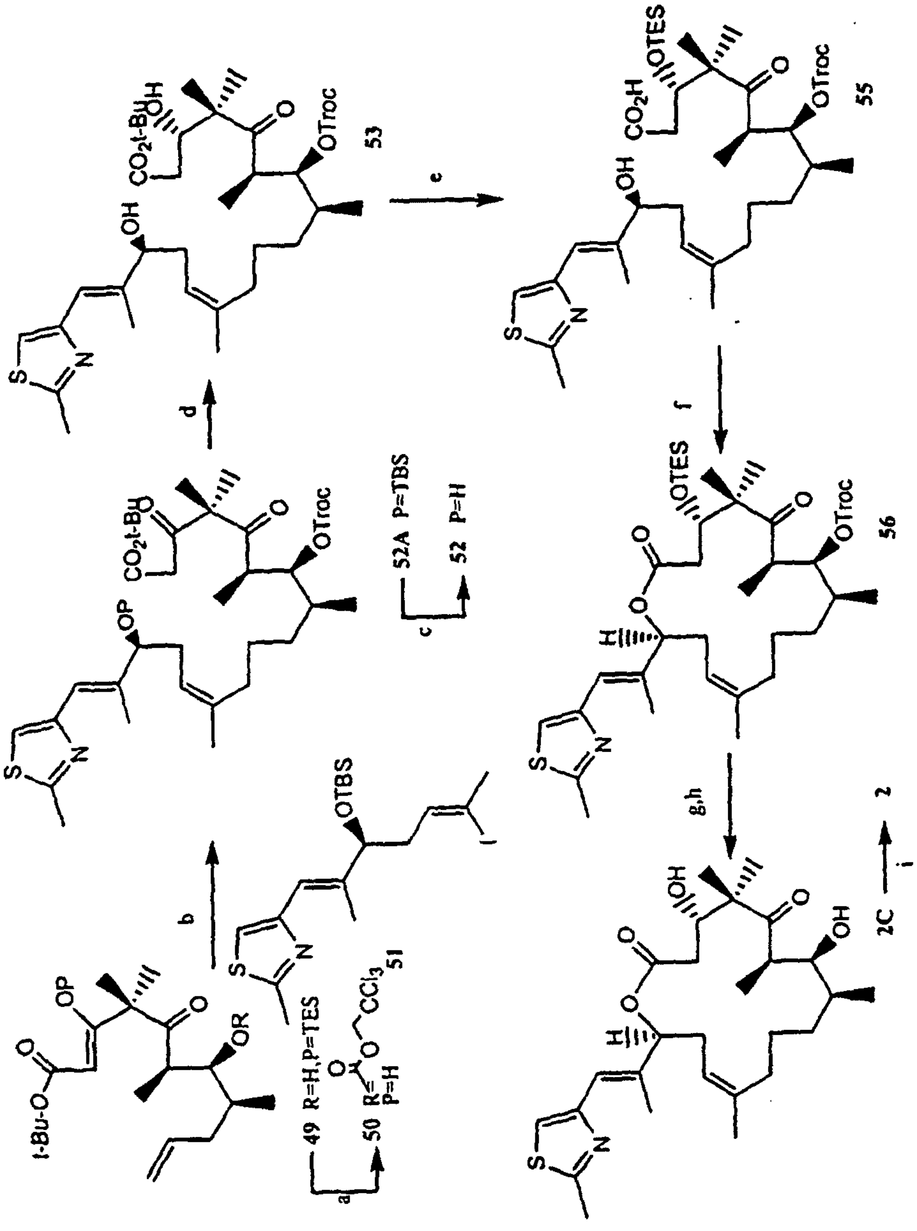
49 R=H, P=TES
50 R=C(O)OCH2CCl3, P=H
51
a
b
52A P=TBS
52 P=H
c
d
53
e
55
f
56
g,h
2C
i
2

FIG. 62

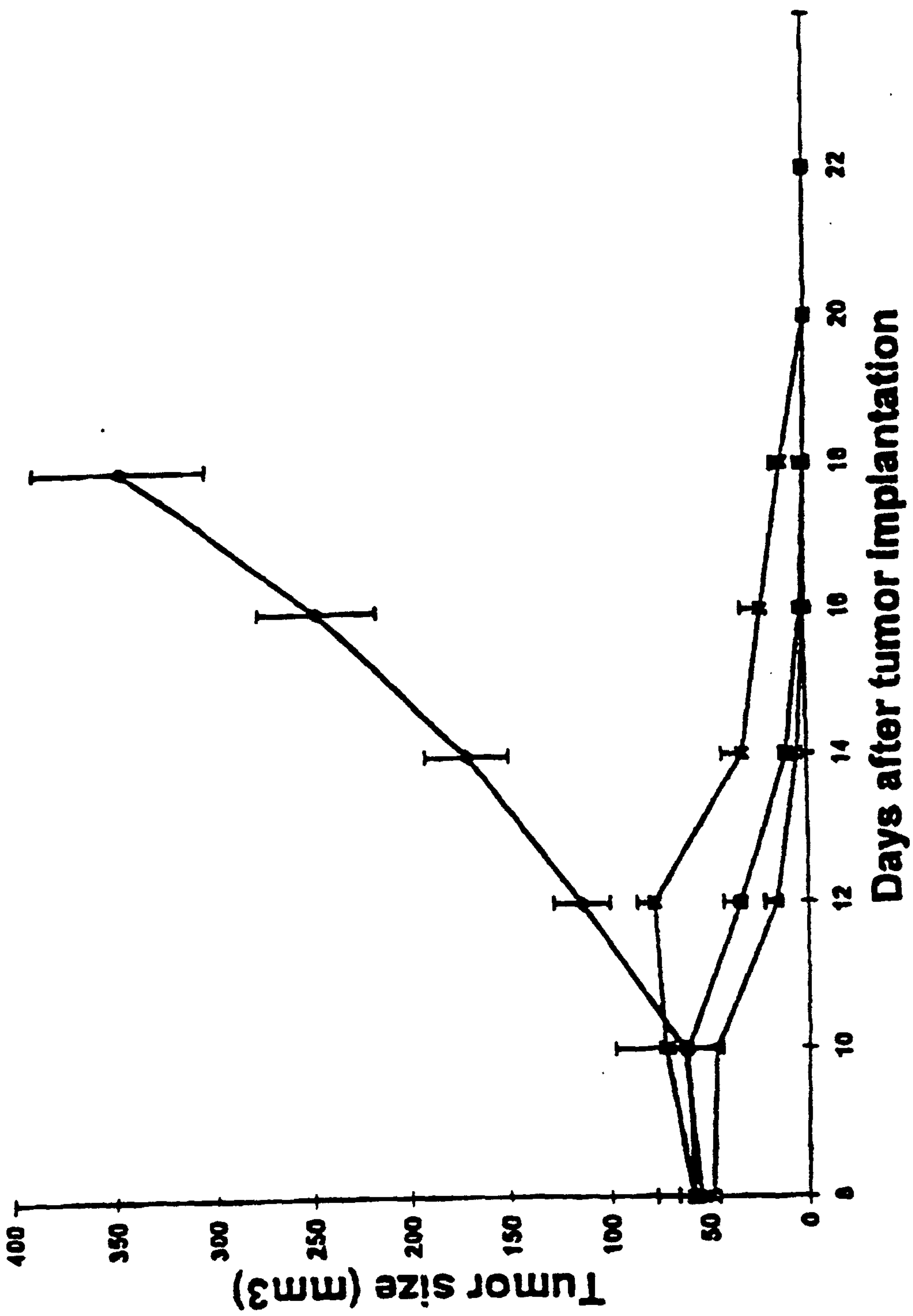
Tumor size (mm3)
0
50
100
150
200
250
300
350
400
Days after tumor Implantation
8
10
12
14
16
18
20
22

FIG. 63

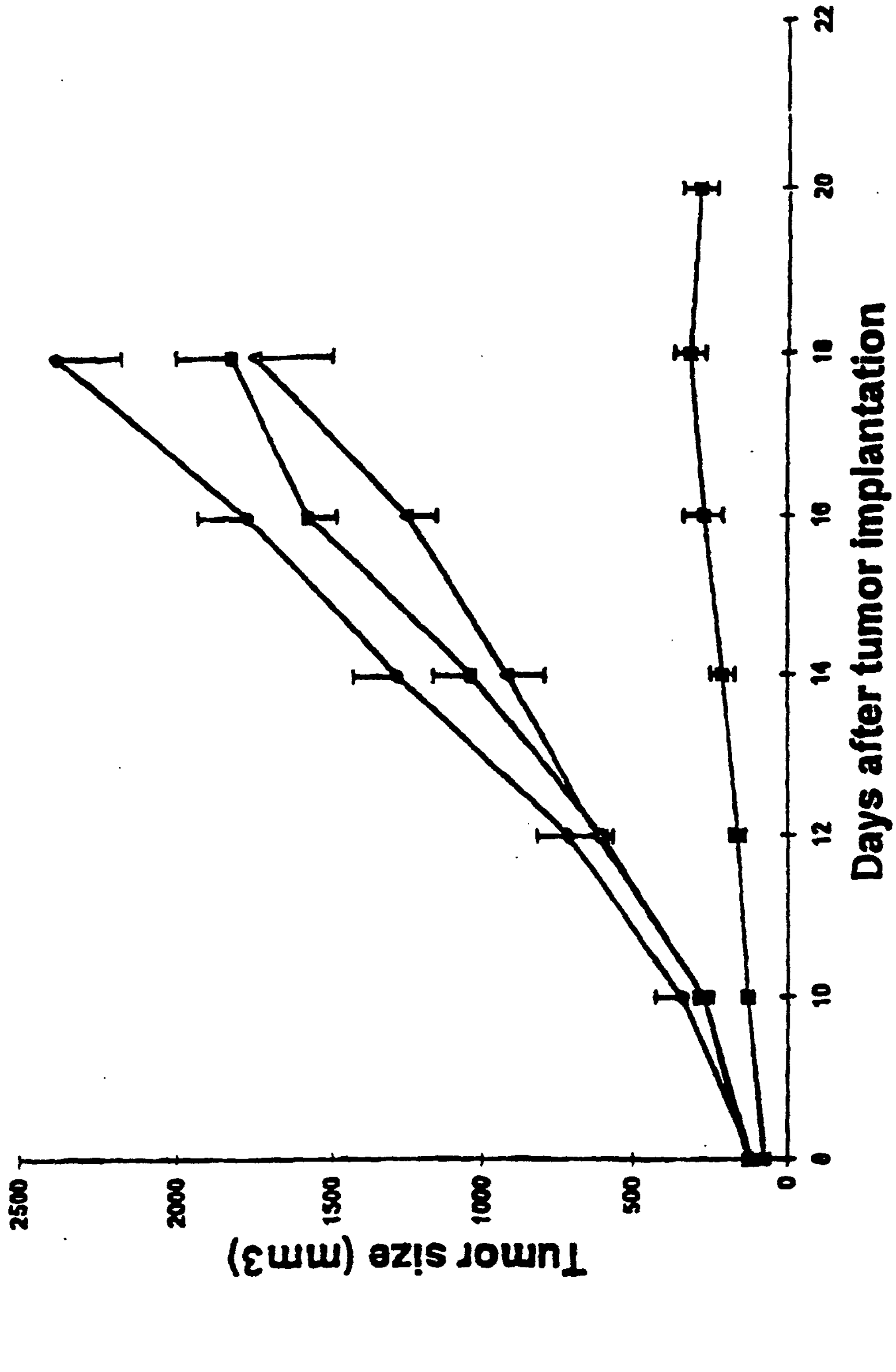
Tumor size (mm3)
0
500
1000
1500
2000
2500
Days after tumor implantation
8
10
12
14
16
18
20
22

FIG. 64

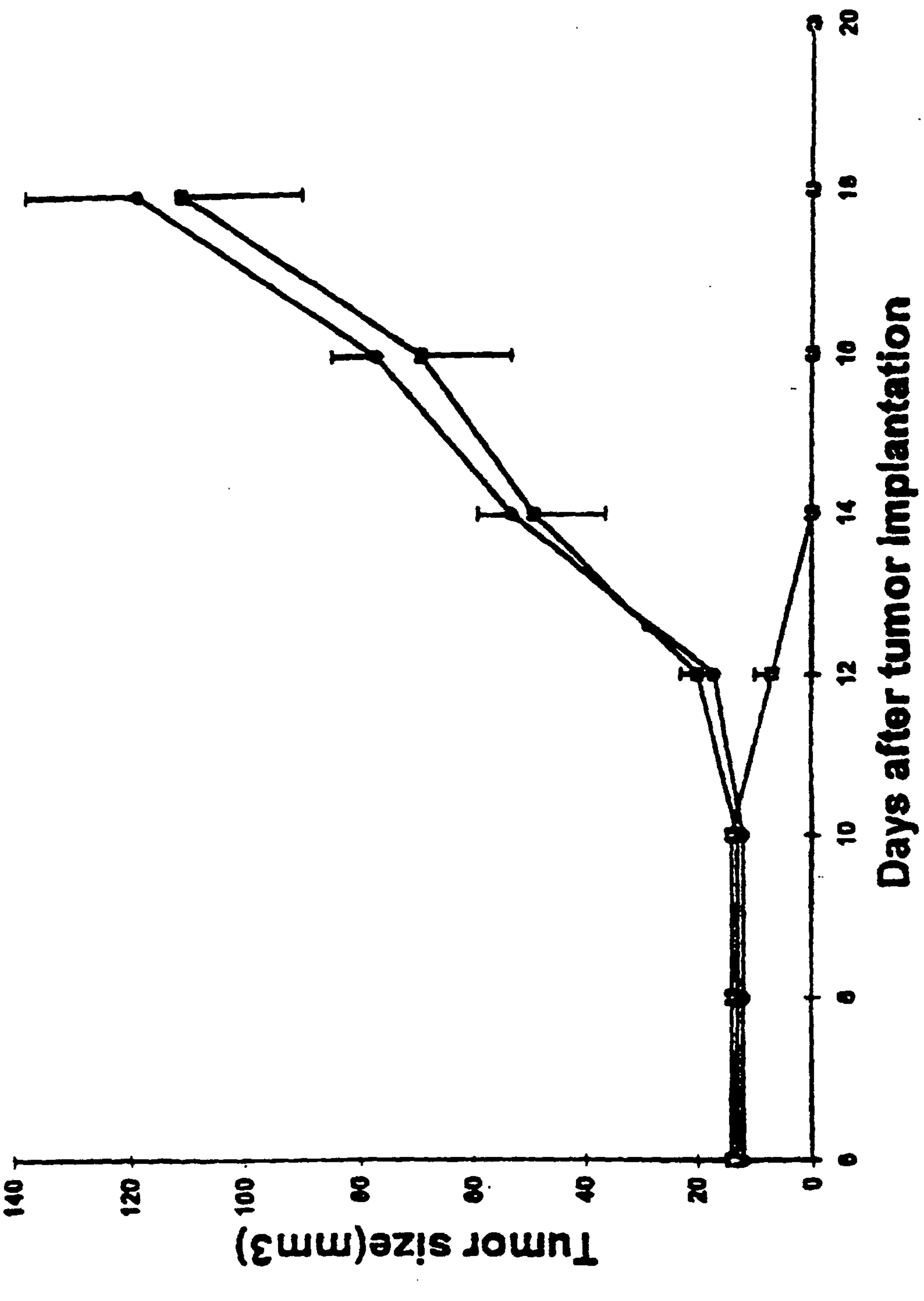

Tumor size(mm3)
0
20
40
60
80
100
120
140
Days after tumor implantation
0
12
14
20

FIG. 65A

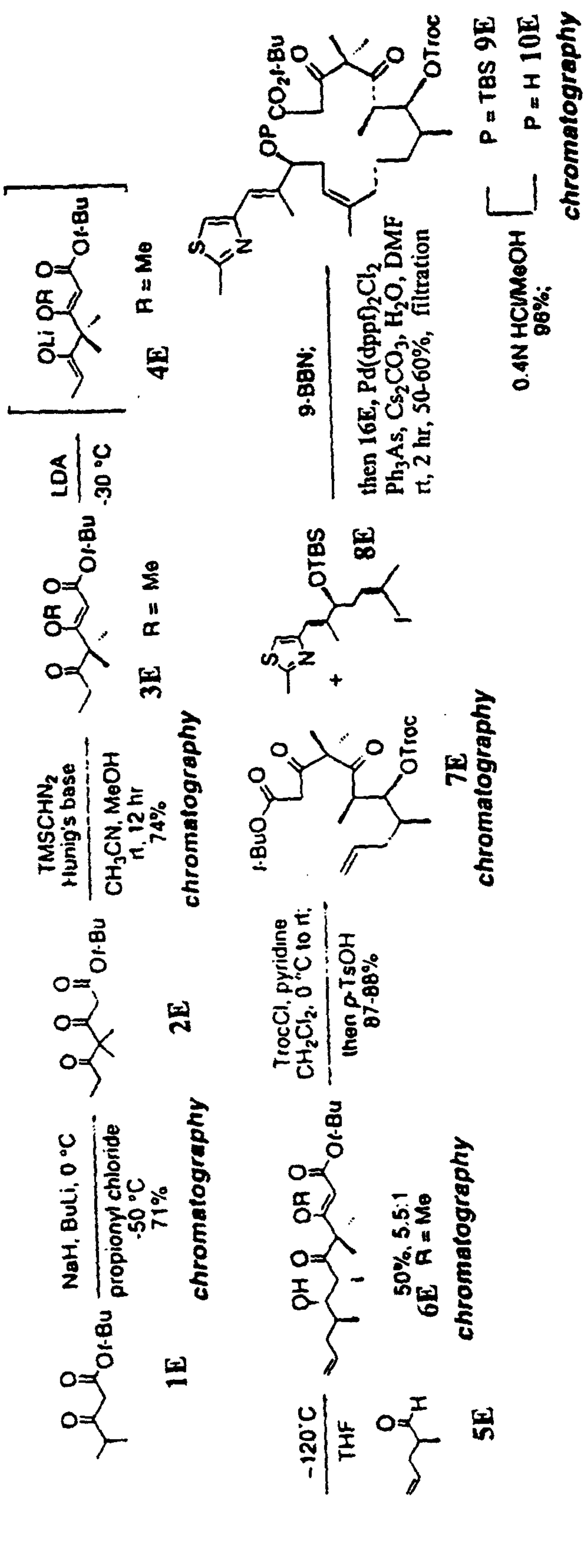
1E
NaH, BuLi, 0 °C
propionyl chloride
-50 °C
71%
chromatography
2E
TMSCHN$_2$
Hunig's base
CH$_3$CN, MeOH
rt, 12 hr
74%
chromatography
3E R = Me
LDA
-30 °C
4E R = Me
5E
-120°C
THF
50%, 5.5:1
6E R = Me
chromatography
TrocCl, pyridine
CH$_2$Cl$_2$, 0 °C to rt;
then p-TsOH
87-88%
7E
chromatography
+
8E
9-BBN;
then 16E, Pd(dppf)$_2$Cl$_2$
Ph$_3$As, Cs$_2$CO$_3$, H$_2$O, DMF
rt, 2 hr, 50-60%, filtration
P = TBS 9E
0.4N HCl/MeOH
98%;
P = H 10E
chromatography

# FIG. 65B

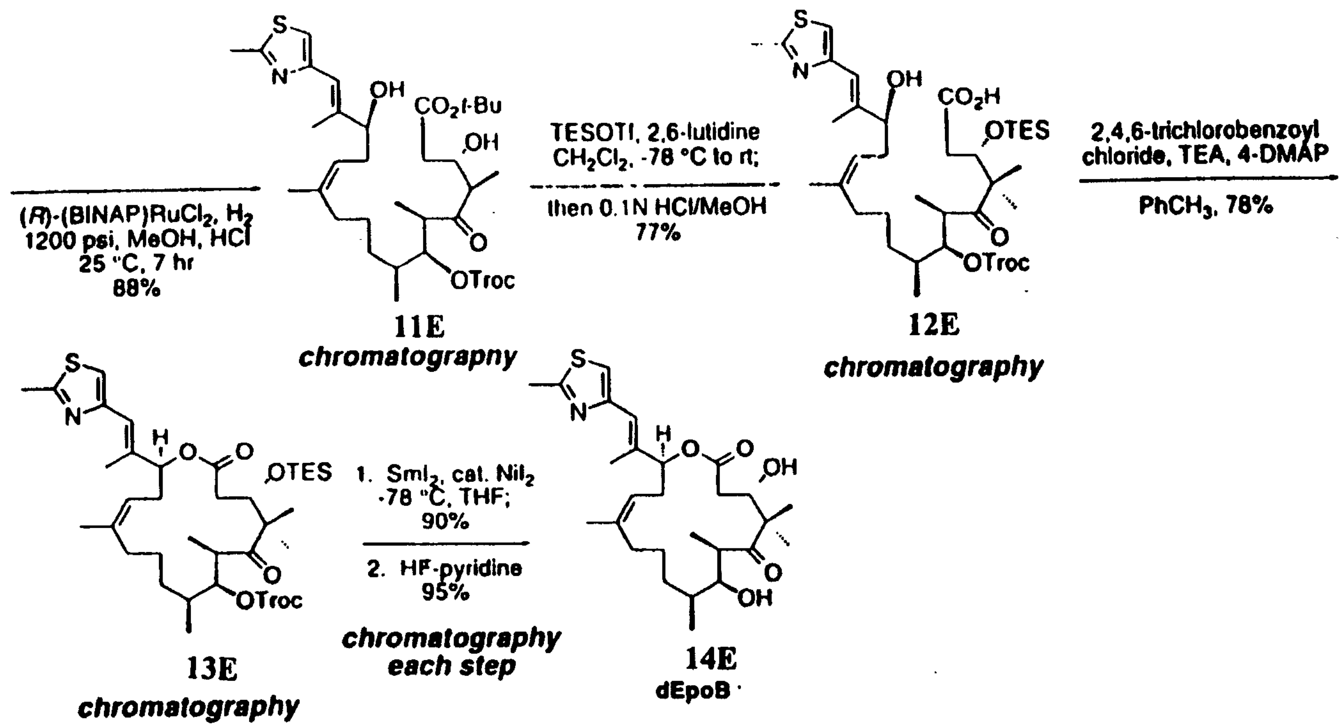


**11E**
*chromatograpny*

**12E**
*chromatography*

**13E**
*chromatography*

**14E**
**dEpoB**

# FIG. 66A

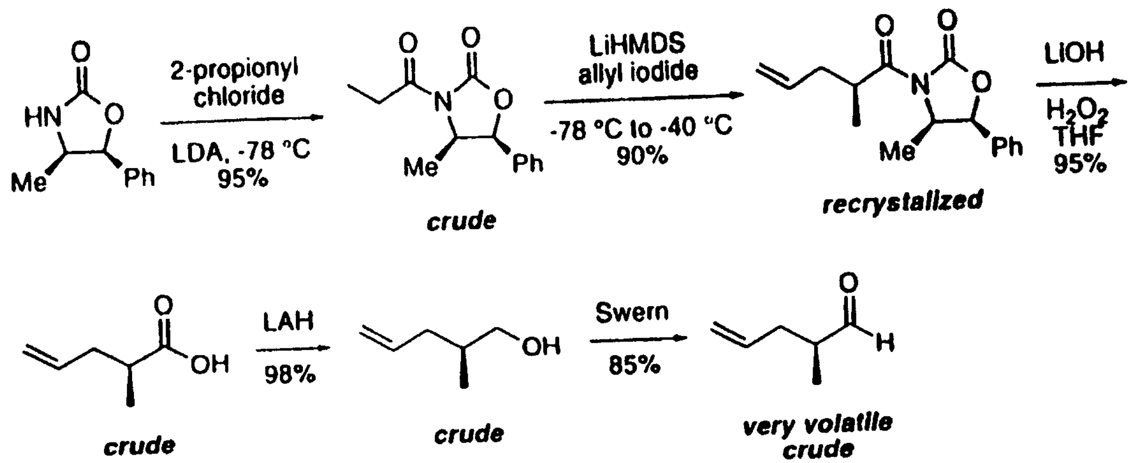
2-propionyl chloride
LDA, -78 °C
95%
crude
LiHMDS
allyl iodide
-78 °C to -40 °C
90%
recrystalized
LiOH
H2O2
THF
95%
crude
LAH
98%
crude
Swern
85%
very volatile
crude

FIG. 66B

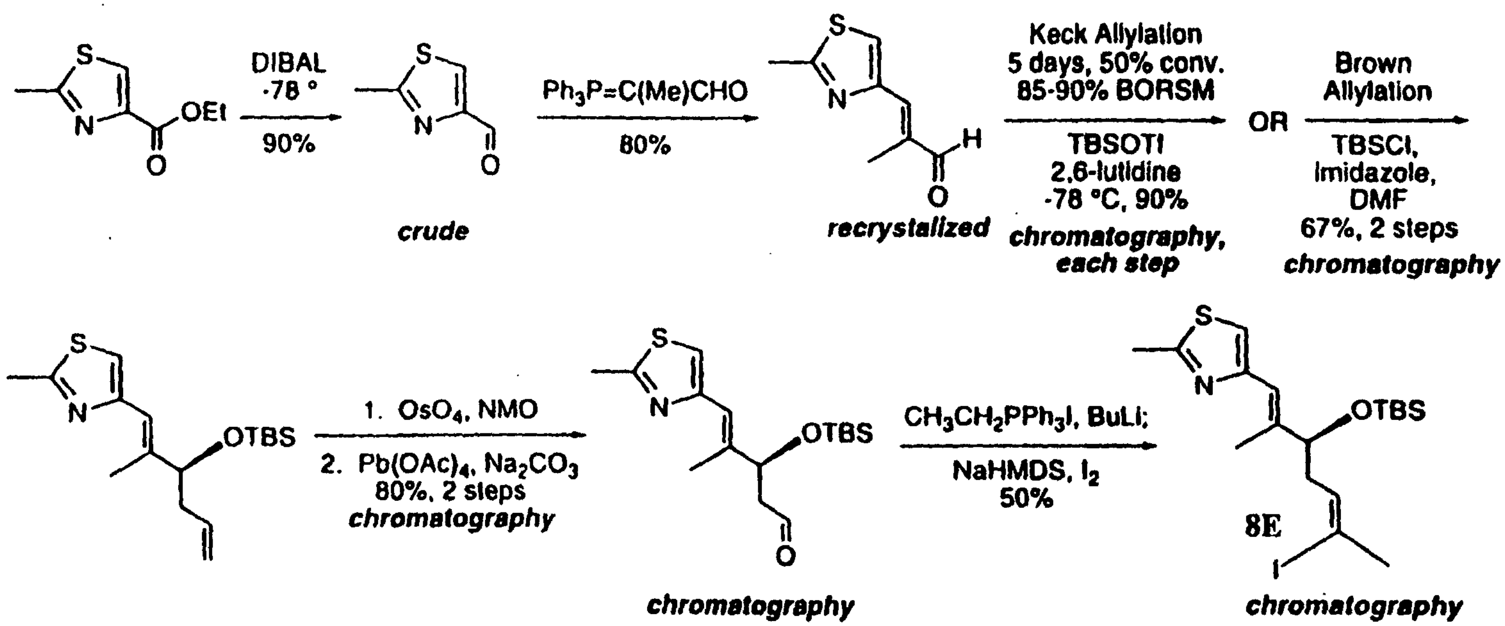
DIBAL
-78 °
90%
crude
Ph$_3$P=C(Me)CHO
80%
recrystalized
Keck Allylation
5 days, 50% conv.
85-90% BORSM
TBSOTf
2,6-lutidine
-78 °C, 90%
chromatography,
each step
OR
Brown
Allylation
TBSCl,
imidazole,
DMF
67%, 2 steps
chromatography
1. OsO$_4$, NMO
2. Pb(OAc)$_4$, Na$_2$CO$_3$
80%, 2 steps
chromatography
chromatography
CH$_3$CH$_2$PPh$_3$I, BuLi;
NaHMDS, I$_2$
50%
chromatography

8E

FIG. 67

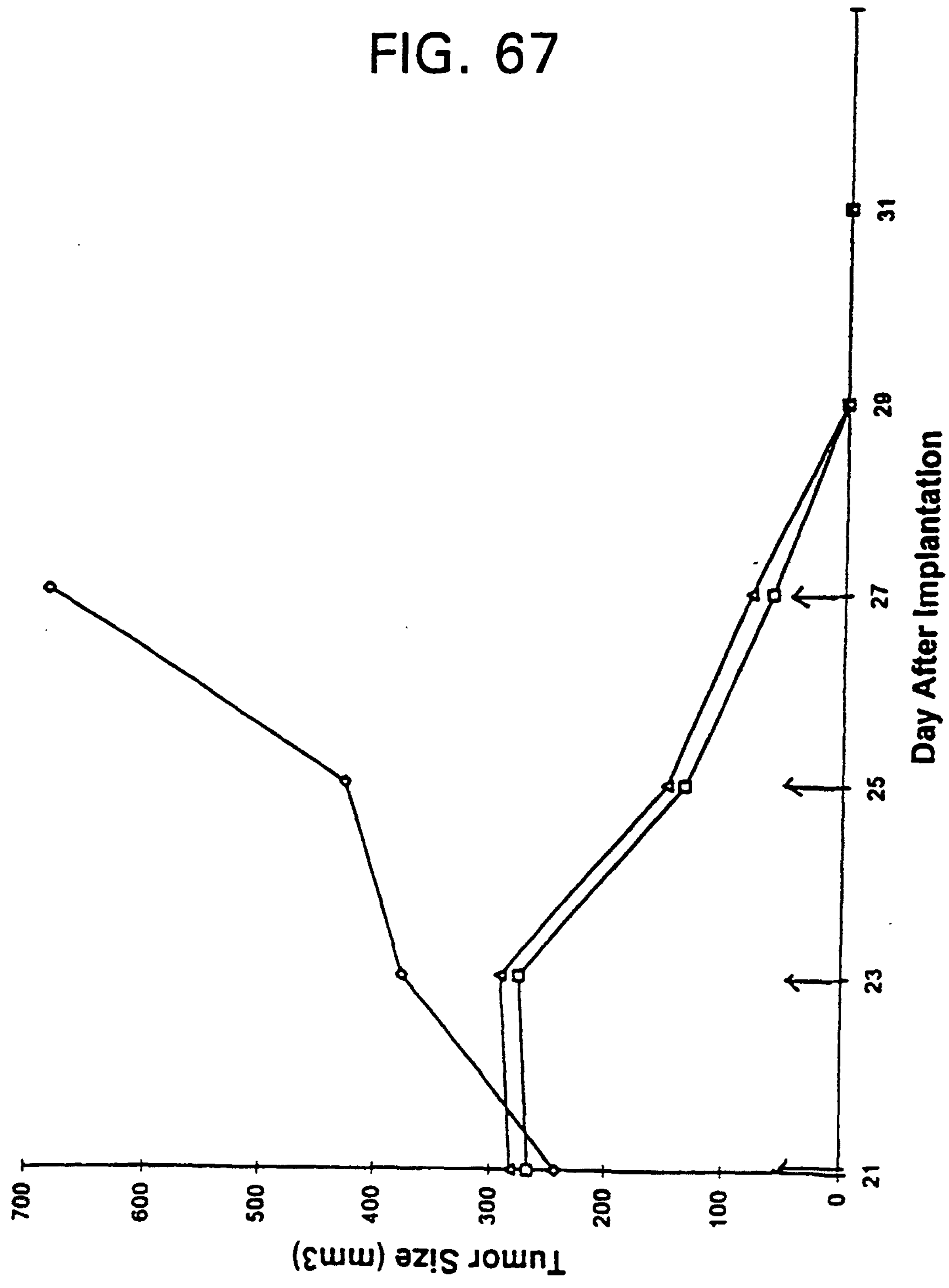
Tumor Size (mm3)
700
600
500
400
300
200
100
0
21
23
25
27
29
31
Day After Implantation

FIG. 68

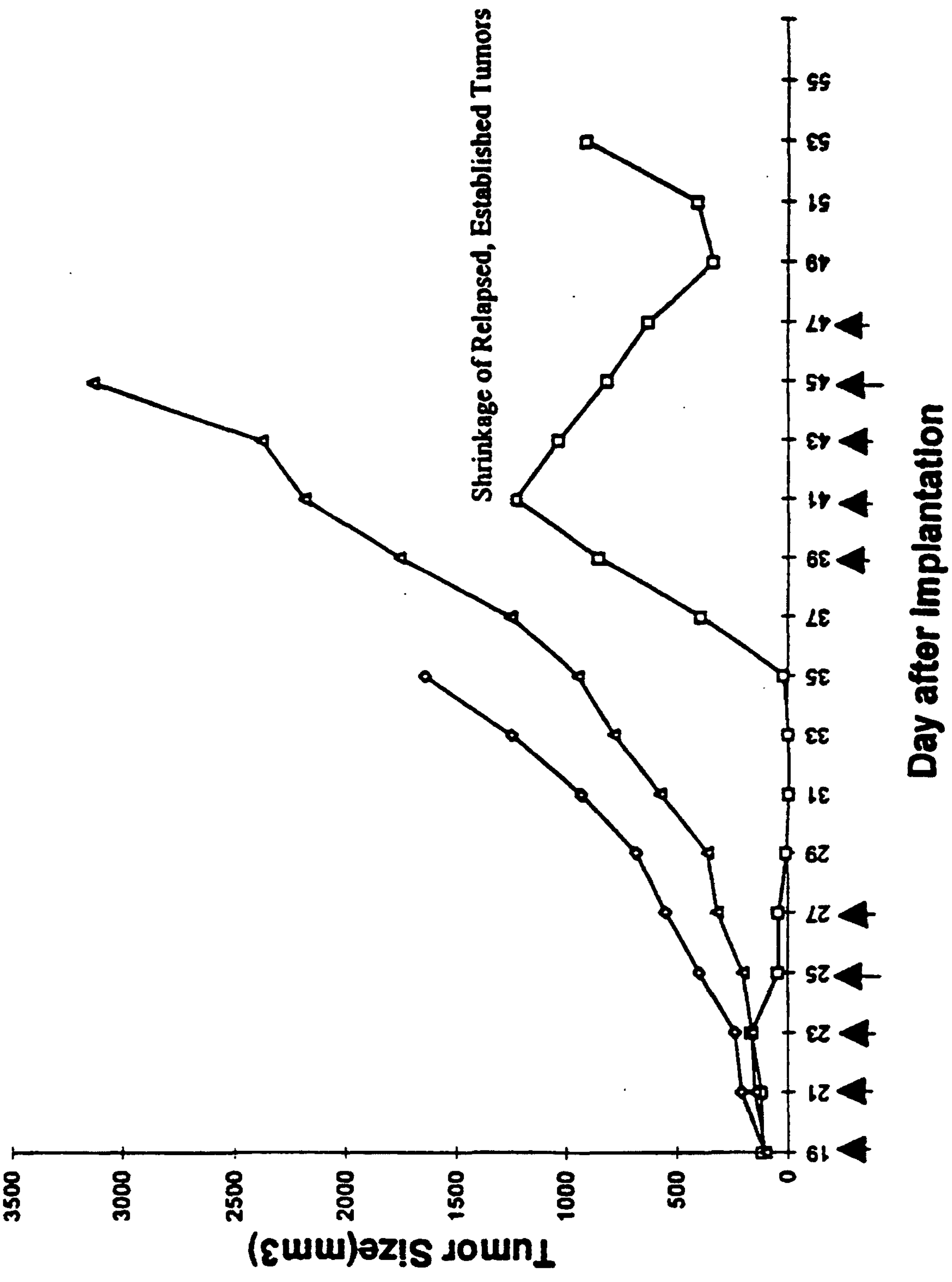
Shrinkage of Relapsed, Established Tumors
Tumor Size(mm3)
0
500
1000
1500
2000
2500
3000
3500
19
21
23
25
27
29
31
33
35
37
39
41
43
45
47
49
51
53
55
Day after Implantation

FIG. 69

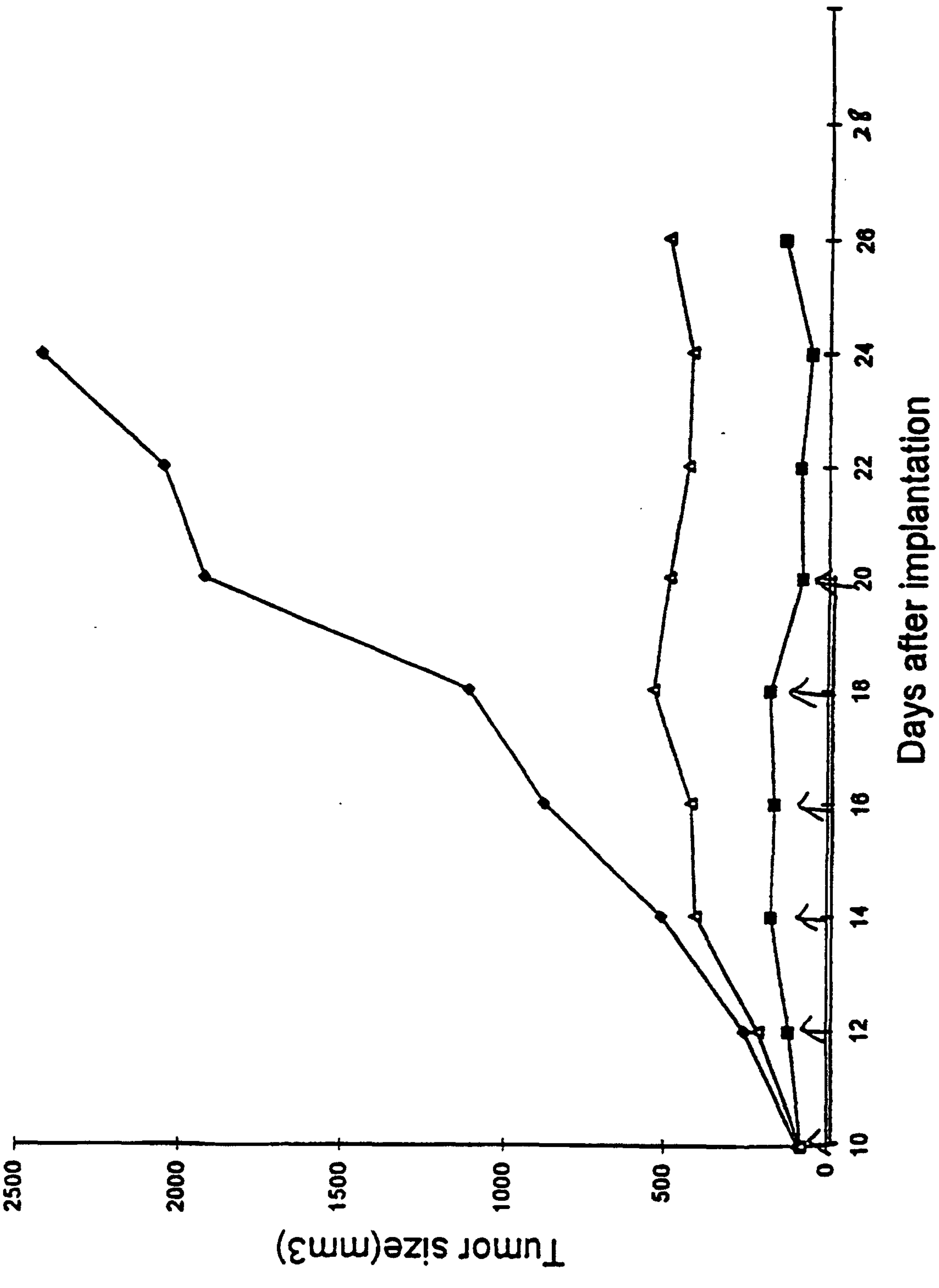
Tumor size(mm3)
0
500
1000
1500
2000
2500
Days after implantation
10
12
14
16
18
20
22
24
26
28

FIG. 70

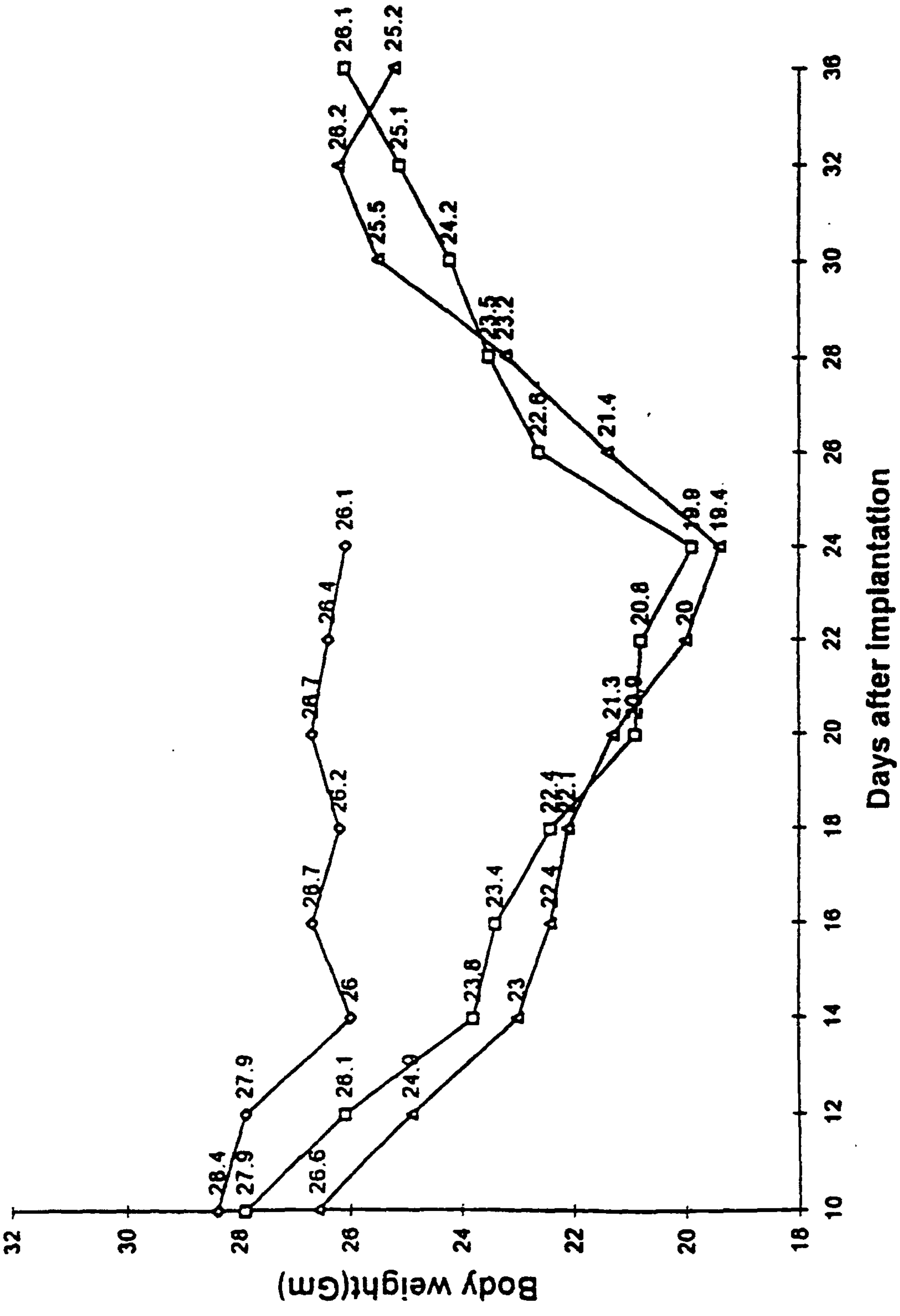
Body weight(Gm)
32
30
28
26
24
22
20
18
Days after implantation
10
12
14
16
18
20
22
24
26
28
30
32
36

FIG. 71

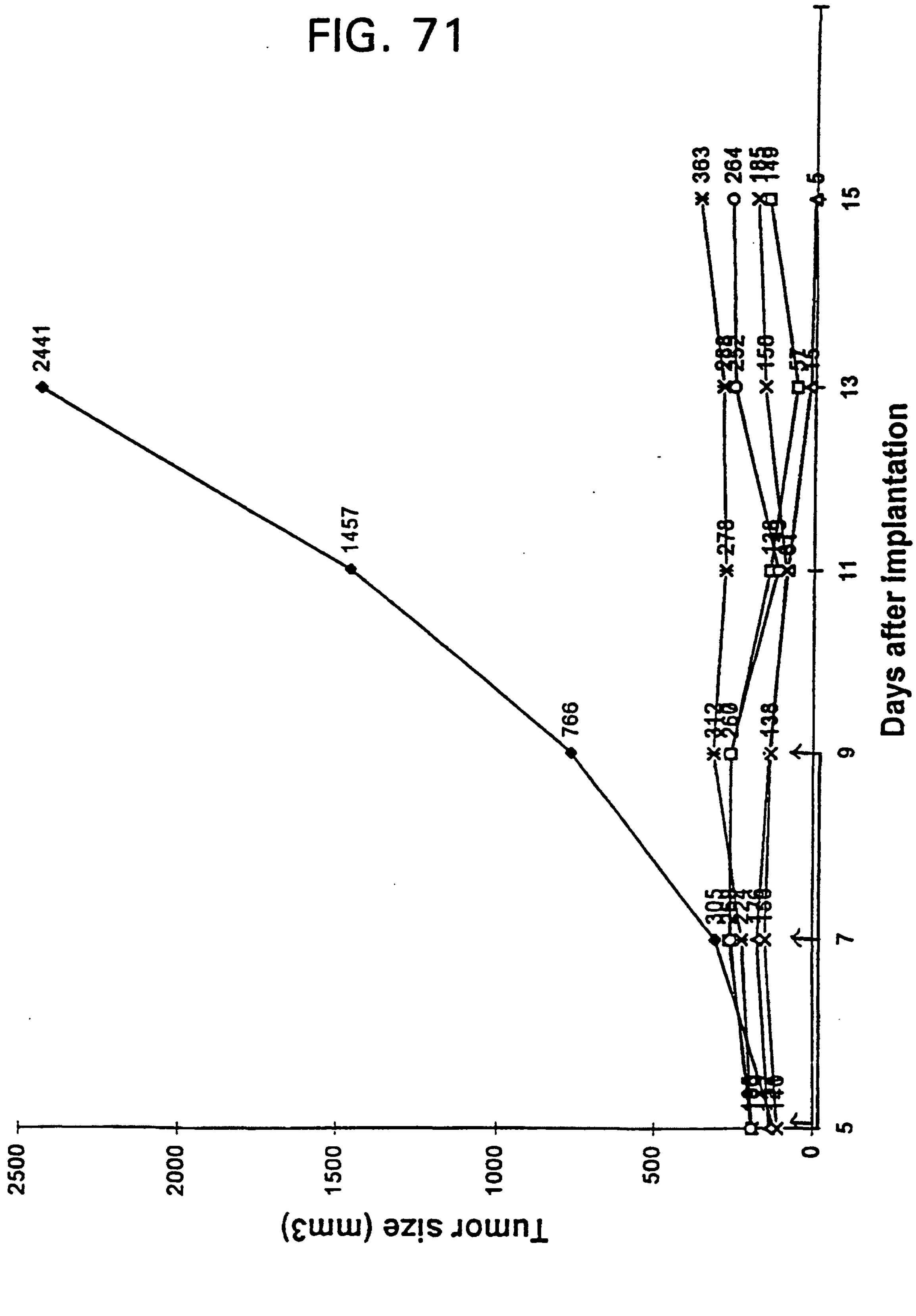
Tumor size (mm3)
0
500
1000
1500
2000
2500
Days after Implantation
5
7
9
11
13
15
305
766
1457
2441
363
264

FIG. 72

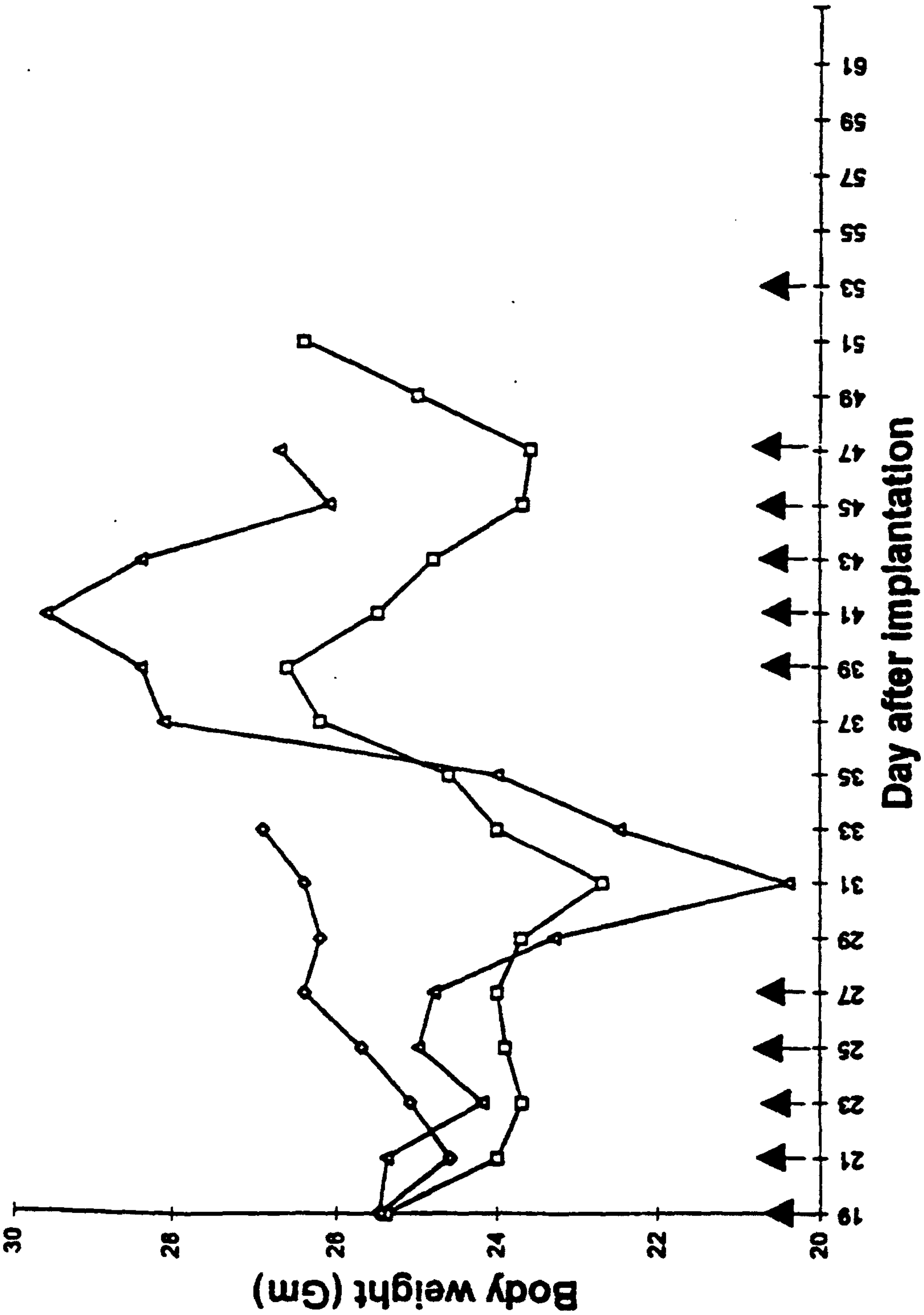
Body weight (Gm)
30
28
26
24
22
20
Day after implantation
19
21
23
25
27
29
31
33
35
37
39
41
43
45
47
49
51
53
55
57
59
61

FIG. 73

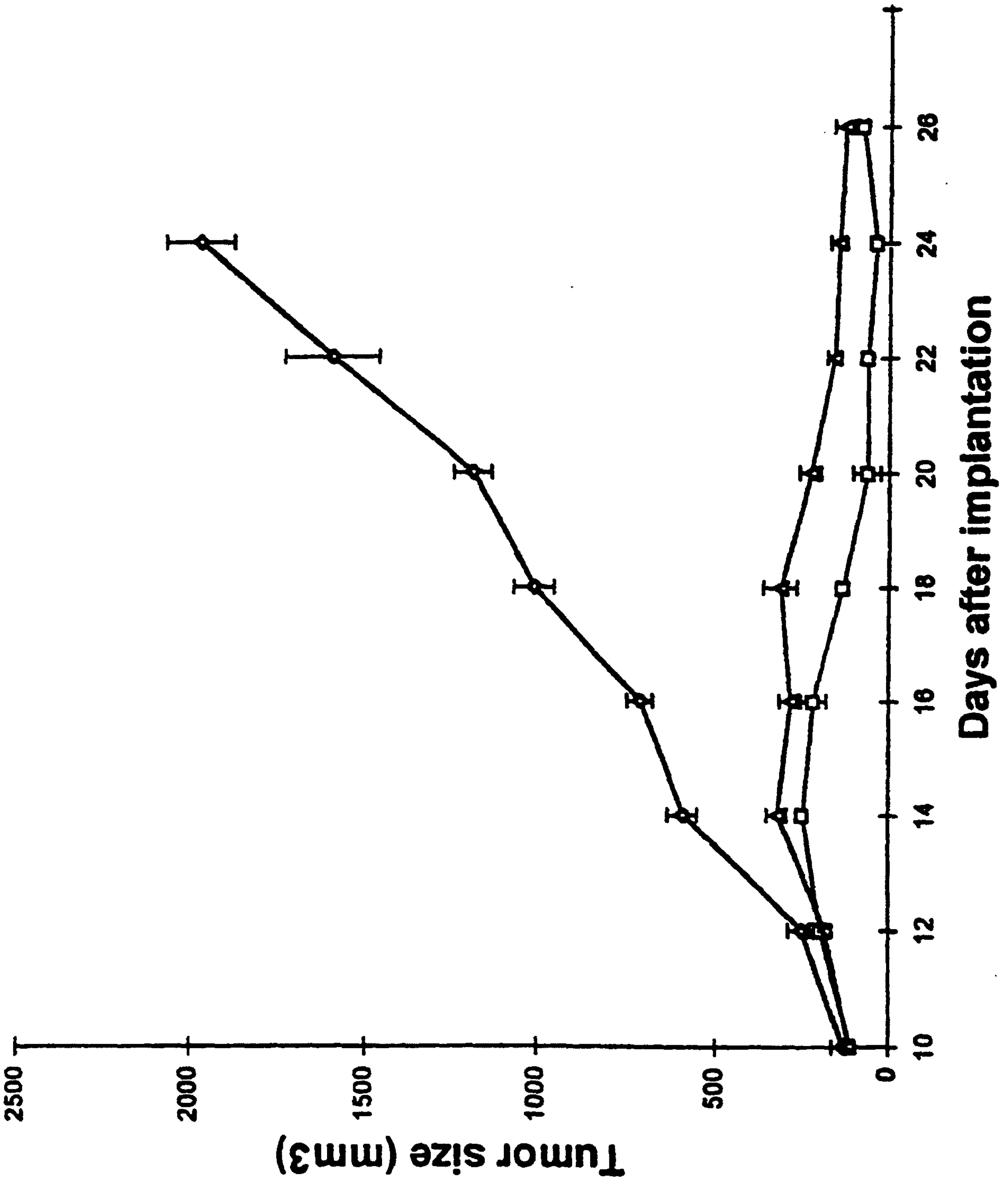

Tumor size (mm3)
2500
2000
1500
1000
500
0
10
12
14
16
18
20
22
24
28
Days after implantation

FIG. 74

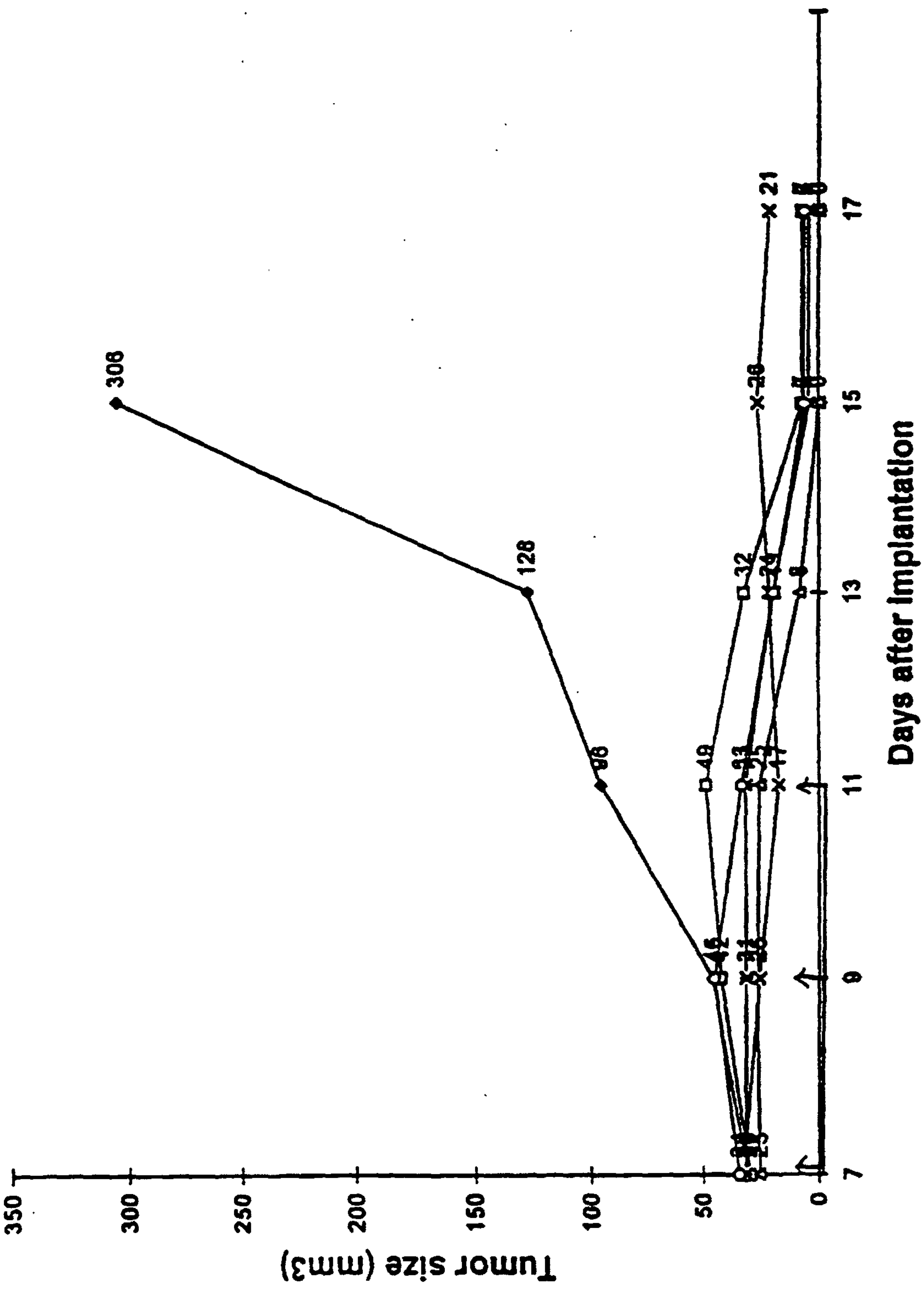
Tumor size (mm3)
Days after Implantation
0
50
100
150
200
250
300
350
7
9
11
13
15
17
306
128
98

FIG. 75